# EUROPEAN PATENT SPECIFICATION

(11) **EP 1 711 618 B1**
(45) Date of publication and mention of the grant of the patent: **04.08.2010**
(21) Application number: 04802347.7
(22) Date of filing: 21.12.2004
(51) Int. Cl.: C12Q 1/68, C12Q 1/02, G01N 33/53, G01N 33/574, A61K 45/00, A61K 49/00, A61P 35/00

(54) **METHODS FOR DETECTING MARKERS ASSOCIATED WITH ENDOMETRIAL DISEASE OR PHASE**
VERFAHREN ZUM NACHWEIS VON MIT ENDOMETRIALER KRANKHEIT ODER PHASE ASSOZIIERTEN MARKERN
PROCEDE DE DETECTION DE MARQUEURS ASSOCIES A LA MALADIE OU LA PHASE DE L'ENDOMETRE

(30) Priority: 23.12.2003 US 532601 P; 24.11.2004 US 630990 P
(43) Date of publication of application: 18.10.2006
(73) Proprietor: MOUNT SINAI HOSPITAL CORPORATION, Toronto, Ontario M5G 1X5 (CA); Siu, Michael K. W., Toronto ON M2J 2X6 (CA); University Health Network, Toronto, Ontario M5G 2M9 (CA)
(72) Inventor: COLGAN, Terence J., Toronto, Ontario M2P 1A3 (CA); SIU, K.W. Michael, Toronto, Ontario M2J 2X6 (CA); ROMASCHIN, Alexander D., Toronto, Ontario M9C 1L4 (CA); YANG, Eric C.C., Toronto, Ontario M2R 1C5 (CA); DESOUZA, Leroi, North York, Ontario M6A 1N9 (CA); DIEHL, George, D-79176 Weil am Rhein (DE); GOU, Jingzhong, Saint-Laurent, Quebec H4M 2M1 (CA)
(74) Representative: Holliday, Louise Caroline
(86) International application number: PCT/CA2004/002172
(87) International publication number: WO 2005/061725

(56) References cited:
- EP-A2- 0 316 919
- WO-A1-00/16805
- WO-A1-01/92338
- WO-A1-98/10291
- WO-A2-01/62959
- WO-A2-99/63115
- WO-A2-99/63116
- SHAARAWY M ET AL: "BIOMARKERS OF INTRINSIC AND ANTI-ANGIOGENIC ACTIVITY IN PATIENTS WITH ENDOMETRIAL HYPERPLASIA AND ENDOMETRIAL CANCER" ACTA ONCOLOGICA, INFORMA HEALTHCARE, LONDON, GB, vol. 40, no. 4, 2001, pages 513-518, XP008071359 ISSN: 0284-186X
- XIAOGUANG FAN ET AL: "A study of early pregnancy factor activity in the sera of women with trophoblastic tumor" AMERICAN JOURNAL OF REPRODUCTIVE IMMUNOLOGY, vol. 41, no. 3, March 1999 (1999-03), pages 204-208, XP008084055 ISSN: 1046-7408
- CAPPELLO FRANCESCO ET AL: "Ten kilodalton heat shock protein (HSP10) is overexpressed during carcinogenesis of large bowel and uterine exocervix." CANCER LETTERS 30 JUN 2003, vol. 196, no. 1, 30 June 2003 (2003-06-30), pages 35-41, XP002452495 ISSN: 0304-3835
- YANG ERIC C C ET AL: "Protein expression profiling of endometrial malignancies reveals a new tumor marker: chaperonin 10" JOURNAL OF PROTEOME RESEARCH, ACS, WASHINGTON, DC, US, vol. 3, no. 3, May 2004 (2004-05), pages 636-643, XP002307868 ISSN: 1535-3893
- SHAARAWY M. ET AL: 'Biomarkers of intrinsic and anti-angiogenic activity in patients with endometrial hyperplasia and endometrial cancer' ACTA ONCOLOGICA vol. 40, no. 4, 2001, pages 513 - 518, XP008071359
- KLEIN A.K. ET AL: 'Detection of circulating tumor cells by cytokeratin 20 in the blood of patients with endometrial carcinoma' GYNECOLOGIC ONCOLOGY vol. 78, no. 3 (PT.1), September 2000, pages 352 - 355, XP008071363

## Description

### FIELD OF THE INVENTION

The invention relates to methods for screening a subject for endometrial cancer.

### BACKGROUND OF THE INVENTION

The endometrium, the tissue lining the uterus, is a glandular layer of variable thickness that is very sensitive to the hormones estrogen and progesterone. During the menstrual cycle the endometrium undergoes cyclic variation with a proliferation phase where the endometrium grows under the influence of estrogen, an ovulation phase where the endometrium is exposed to estrogen and progesterone, and a secretory or progesterone dominated phase where the endometrium shows signs of increased gland growth and secretion due largely to the influence of progesterone [1]. The secretory phase is followed by the shedding of the endometrium during menstruation. The histologic changes in the endometrium have been used to detect the stage or status of the endometrium, which is important for example, in determining the receptivity of patients to fertility procedures, and in determining responses to estrogen and/or progesterone therapies.

Endometrial carcinoma is a common malignancy in women, being exceeded in incidence only by that of breast, lung, and colorectal cancers [10, 11, 48, 125-131]. The lifetime probability of a Canadian woman developing endometrial carcinoma is 2.2% [10]. Although the case-fatality rate for cancer of the endometrium is lower than that of many other cancer sites, this rate does not fully reflect the health care burden posed by endometrial carcinoma. Investigation of women with perimenopausal and postmenopausal bleeding for the presence of endometrial carcinoma is one of the most common gynecologic investigations and requires invasive endometrial sampling. Yet only a small proportion of these investigations will result in a diagnosis of endometrial carcinoma [12].

At present, no methods for screening or early detection of endometrial carcinoma are available nor are there any serum tumor markers available for the monitoring of endometrial carcinoma patients [13, 14]. Consequently, patients are diagnosed following the development of symptoms, and patients with recurrent disease are detected only following the development of recurrent symptoms, or abnormalities in imaging assessments. Sensitive and specific tumor marker(s) for endometrial carcinoma are urgently needed for screening and diagnosis. There is also a need for reliable endometrial markers for determining the stage/phase, and status of the endometrium.

Shaarawy and Sharkawy (2001; Acta Oncologica 40:513-518) report that serum vascular endothelial growth factor (VEGF) and endostatin are biomarkers for endometrial hyperplasia and endometrial cancer.

### SUMMARY OF THE INVENTION

Applicants have developed a method for identifying markers associated with the endometrium, and in particular with proliferative-endometirum, secretory endometrium, and diseased endometrial tissue. Using the method they analyzed normal endometrial tissue homogenates and endometrial tumor tissue homogenates, and identified novel endometrial markers, in particular markers of secretory and proliferative endometrium and endometrial cancer markers.

The applicants have developed a method of characterizing or classifying a sample of endometrium by detecting or quantitating in the sample one or more polypeptides extracted from the sample that are characteristic of endometrium, a phase thereof or an endometrial disease, the method comprising assaying for differential expression of polypeptides in the sample. Differential expression of polypeptides can be assayed using separation techniques known in the art, an antibody microarray, or mass spectroscopy of polypeptides extracted from the sample.

The present invention provides a method for screening a subject for endometrial cancer comprising (a) detecting in a sample from the subject an amount of an endometrial cancer marker or polynucleotide encoding the endometrial cancer marker; and (b) comparing the amount of endometrial cancer marker or polynucleotide detected to a standard, where detection of a level of endometrial cancer marker or polynucleotide different than that of a standard is indicative of endometrial cancer, and wherein the endometrial cancer marker is chaperonin 10 of SEQ ID NO.1, a sequence with 80% sequence identity to SEQ ID NO. 1 having the immunogenic activity of SEQ ID NO. I or a fragment of SEQ ID NO. 1 having the ability to form antibodies specific for SEQ ID NO. 1.

In an embodiment, a significant difference between the levels of endometrial marker levels in a patient and normal levels is indicative of endometrial cancer.

In a particular embodiment the amount of endometrial marker(s) detected is greater than that of a standard and is indicative of endometrial cancer. In another particular embodiment the amount of endometrial marker(s) detected is lower than that of a standard and is indicative of endometrial cancer.

The invention provides a non-invasive non-surgical method. The sample may be blood, plasma, serum, urine or saliva or a tissue sample from the subject.

The endometrial cancer marker may be detected by a method in which the endometrial cancer marker is detected by contacting the sample with an antibody that specifically binds to the endometrial cancer marker.

Embodiments of the method of the invention involve (a) reacting a biological sample from a subject with antibodies specific for the endometrial marker which is directly or indirectly labelled with an enzyme; (b) adding a substrate for the enzyme wherein the substrate is selected so that the substrate, or a reaction product of the enzyme and substrate forms fluorescent complexes; (c) quantitating one or more endometrial markers in the sample by measuring fluorescence of the fluorescent complexes; and (d) comparing the quantitated levels to levels obtained for other samples from the subject patient, or control subjects.

In another embodiment the quantitated levels are compared to levels quantitated for control subjects (e.g. normal or benign) without an endometrial cancer wherein an increase in endometrial marker levels compared with the control subjects is indicative of endometrial disease.

In a further embodiment the quantitated levels are compared to levels quantitated for control subjects (e.g. normal or benign) without an endometrial cancer wherein a decrease in endometrial marker levels compared with the control subjects is indicative of endometrial disease.

A particular embodiment of the invention comprises the following steps
(a) incubating a biological sample with first antibodies specific for the endometrial cancer marker which is directly or indirectly labeled with a detectable substance, and second antibodies specific for one or more endometrial cancer markers which are immobilized;
(b) detecting the detectable substance thereby quantitating endometrial cancer markers in the biological sample; and
(c) comparing the quantitated endometrial cancer markers with levels for a predetermined standard.

The standard may correspond to levels quantitated for samples from control subjects without endometrial cancer (normal or benign), with a different disease stage, or from other samples of the subject, In an embodiment, increased levels of endometrial cancer markers as compared to the standard may be indicative of endometrial cancer. In another embodiment, lower levels of endometrial cancer markers as compared to a standard may be indicative of endometrial cancer.

Endometrial marker levels can be determined by constructing an antibody microarray in which binding sites comprise immobilized, preferably monoclonal, antibodies specific to a substantial fraction of marker-derived endometrial marker proteins of interest.

Other methods of the invention employ one or more polynucleotides capable of hybridizing to one or more polynucleotides encoding the endometrial marker. Thus, methods for detecting endometrial markers can be used to monitor an endometrial cancer by detecting endometrial polynucleotide markers associated with the disease.

The endometrial marker polynucleotide may be mRNA. The presence of different levels of endometrial marker polynucleotides in the sample compared to a standard or control may be indicative of endometrium phase, disease, disease stage, and/or a positive prognosis i.e. longer progression-free and overall survival.

In embodiments of the invention, endometrial cancer marker polynucleotide positive tumors (e.g. higher levels of the polynucleotides compared to a control normal or benign tissue) are a negative diagnostic indicator. Positive tumors can be indicative of endometrial cancer, advanced stage disease, lower progression-free survival, and/or overall survival.

In other embodiments of the invention, endometrial cancer marker polynucleotide negative tumors (e.g. lower levels of the polynucleotides compared to a control normal or benign tissue) are a negative diagnostic indicator. Negative tumors can be indicative of endometrial cancer, advanced stage disease, lower progression-free survival, and/or overall survival.

Within certain embodiments, the amount of polynucleotides that are mRNA are detected via polymerase chain reaction using, for example, oligonucleotide primers that hybridize to one or more polynucleotides encoding endometrial markers, or complements of such polynucleotides. Within other embodiments, the amount of mRNA is detected using a hybridization technique, employing oligonucleotide probes that hybridize to one or more polynucleotides encoding endometrial markers, or complements thereof.

When using mRNA detection, the method may be carried out by combining isolated mRNA with reagents to convert to cDNA according to standard methods; treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence ofone or more endometrial polynucleotide markers in the sample. For mRNA the analyzing step may be accomplished using Northern Blot analysis to detect the presence of endometrial polynucleotide markers. The analysis step may be further accomplished by quantitatively detecting the presence of endometrial polynucleotide markers in the amplification product, and comparing the quantity of marker detected against a panel of expected values for the known presence or absence of the markers in normal and malignant tissue derived using similar primers.

Therefore, the invention provides a method wherein mRNA is detected by (a) isolating mRNA from a sample and combining the mRNA with reagents to convert it to cDNA; (b) treating the converted cDNA with amplification reaction reagents and nucleic acid primers that hybridize to one or more endometrial polynucleotide markers to produce amplification products; (d) analyzing the amplification products to detect an amount of mRNA encoding the endometrial markers; and (e) comparing the amount of mRNA to an amount detected against a panel of expected values for normal and diseased tissue (e.g. malignant tissue) derived using similar nucleic acid primers.

In particular embodiments of the invention, the methods described herein utilize the endometrial polynucleotide markers placed on a microarray so that the expression status of each of the markers is assessed simultaneously.

The endometrial microarray may comprise a defined set of genes whose expression is significantly altered by endometrium phase or endometrial disease. The microarray may be used as a prognostic tool to predict endometrium phase or endometrial disease. The endometrial microarray may discriminate between endometrial disease resulting from different etiologies.

Other objects, features and advantages of the present invention will become apparent from the following detailed description. It should be understood, however, that the detailed description and the specific examples while indicating preferred embodiments of the invention are given by way of illustration only, since various changes and modifications within the spirit and scope of the invention will become apparent to those skilled in the art from this detailed description.

### DESCRIPTION OF THE DRAWINGS

The invention will now be described in relation to the drawings in which:
Figure 1 shows a schematic representation of the ICAT procedure including labeling, digestion, SCX fractionation, affinity isolation, cleavage of the biotin moiety and LC-MS/MS analysis.
Figure 2 shows histologic sections of human endometrium, stained with hematoxylin and eosin. A) proliferative endometrium, sample PRO 2, and B) secretory endometrium, sample SEC2.
Figure 3 shows a NanoLC-MS TIC of SCX fraction 16, ICAT sample A:
Figure 4 shows the distribution of proteins identified zoom the middle ten SCX fractions of all three samples.
Figure 5 shows A) a mass spectral window of Sample A, SCX fraction 19, showing enhanced expression of glutamate receptor subunit zeta 1 precursor in the secretory endometrium. The line to the left of the isotope envelope of the heavy-labeled version of the protein denotes where the light-labeled version is expected. B) Resulting MS/MS spectra of the heavy labelled series showing sequence coverage.
Figure 6 shows A) a mass spectral window of Sample A, SCX fraction 13, showing enhanced expression of MIF in the secretory endometrium. B) Resulting MS/MS spectra of the heavy labelled series sequence coverage.

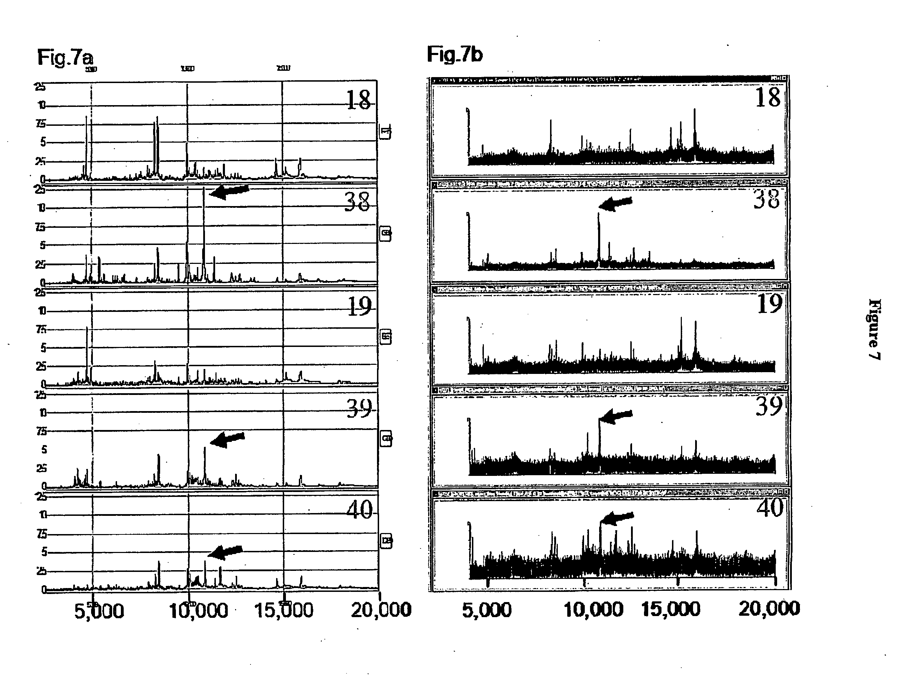

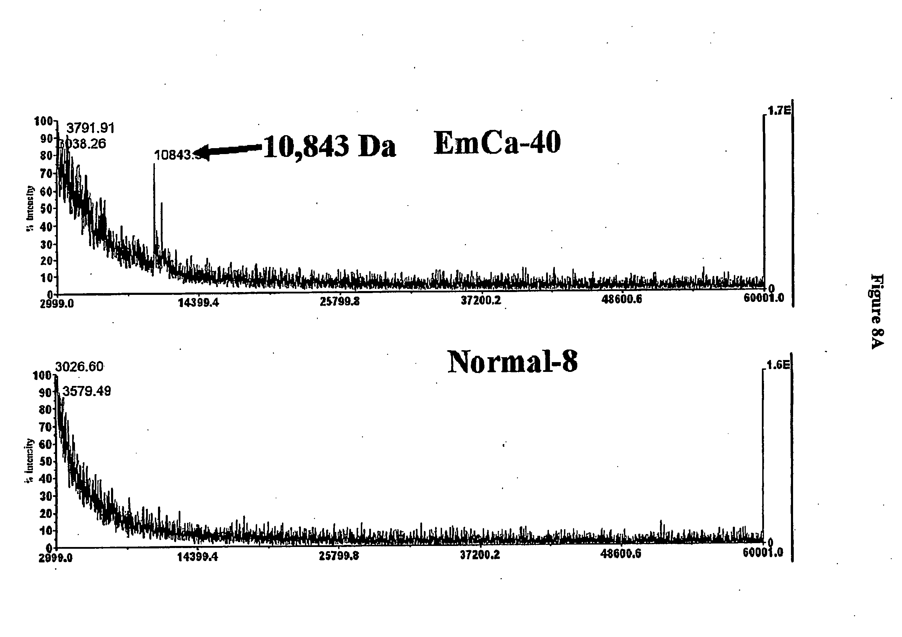

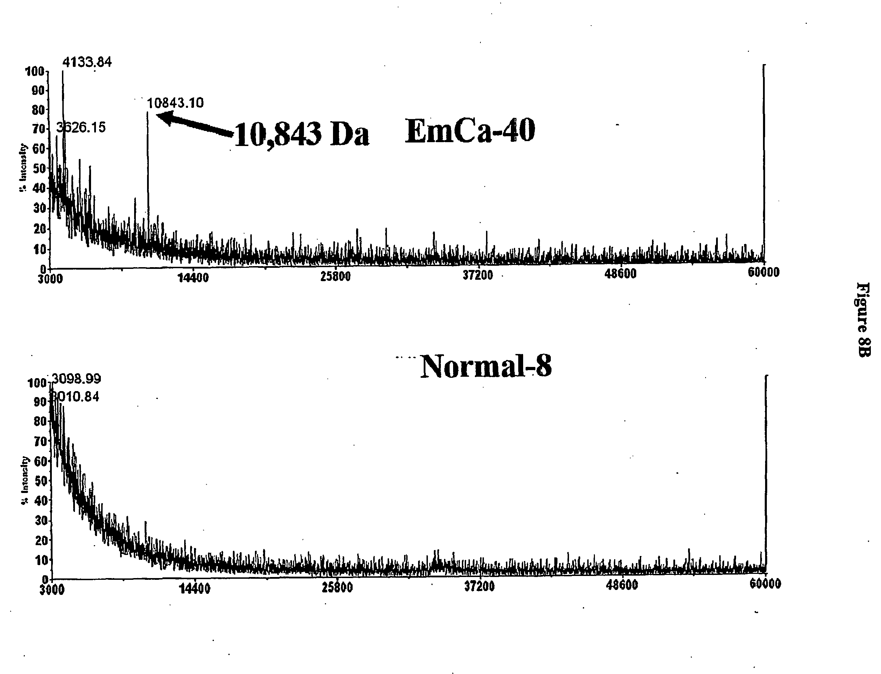

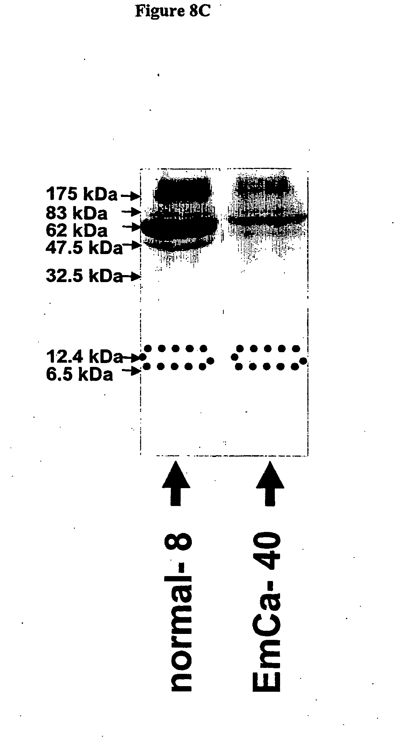

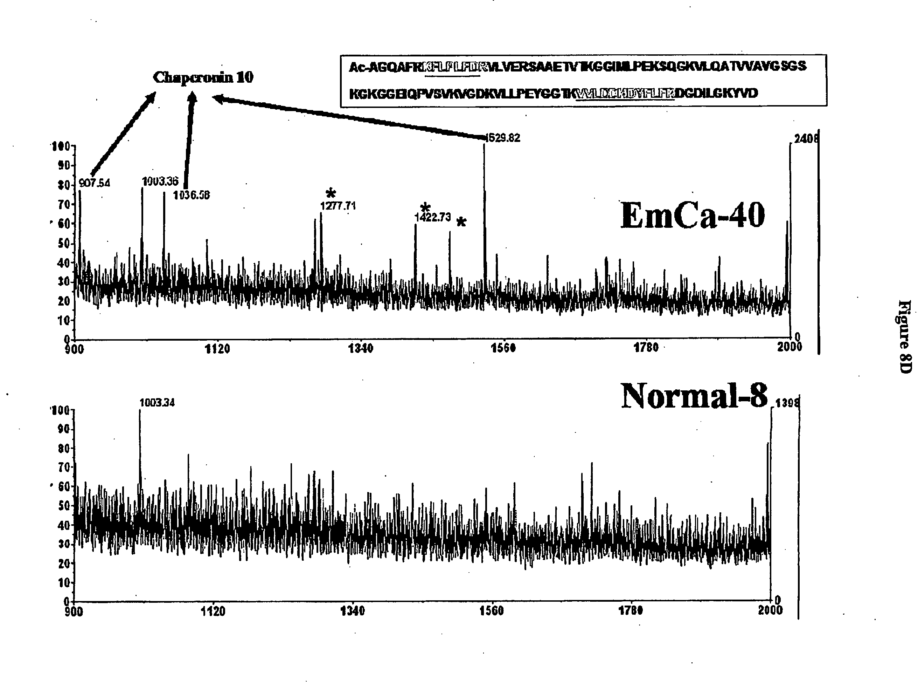

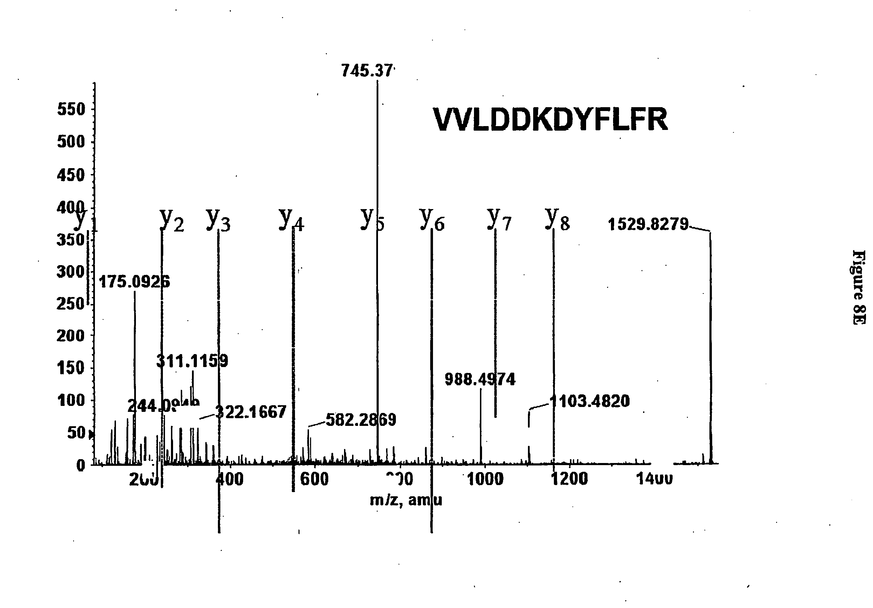

Figure 7 (a+b). STEP ONE Mass spectra obtained from five whole endometrial tissue homogeneates on Proteinchip WCX2 using (1a) SELDI-TOF-MS, PBSIIC, and (1b) QqTOF-MS, QSTAR XL. Note, using either MS method, the three malignant cases (38, 39, 40) show a distinct protein peak, which is low in the two non-malignant cases [18, 19]. The QSTARXL MS determined the weight of this peak to be 10,843 Da. Figure 7 STEP TWO Outline of protein purification. The target protein eluted in one of the early fractions of the size exclusion HPLC, was concentrated by Proteospin and then subjected to MALDI and SDS-PAGE (see Figure 8).
Figure 8 (A to E). STEP THREE (8A). Molecular weight verification of target protein after SEC. MALDI-TOF MS of samples from Proteospin was used to locate the fraction with the 10,843 Da weight of the target protein in a malignant case (40).The corresponding fraction from normal sample was used in parallel for all the following experiments. (8B) Molecular weight verification of the target protein fraction following DTT treatment. MALDI-TOF analysis of two samples indicates that the 10,843 Da peak remained in the malignant sample even after DTT treatment. (8C) SDS-PAGE analysis of the DTT treated fractions. The gel was stained by colloidal coomassie blue (Gel-Code, Pierce). The region containing the target protein was excised according to the molecular weight marker, and the intact proteins were extracted. (8D) Tryptic peptide profiles of the SDS-PAGE protein extract. MALDI-TOF MS analysis identified six "unique" tryptic peptides in the malignant samples. Three of them with mass of 907.54, 1036.58, and 1529.82 Da matched with the tryptic peptides from chaperonin 10. The remaining three peaks, marked with *, were from Keratin. (insert) Amino acid sequence of chaperonin 10. The locations of the three peptides were underlined. (8E) Fragmentation of peptide by collision induced dissociation. The 1529.82 Da peptide was fragmented. A series of y ions confirmed that the 1529.82 Da peptide comes from chaperonin 10.
Figure 9. Western blot analysis against chaperonin 10. The malignant endometrial cases (EmCa) demonstrate a higher expression of chaperonin 10 than non-malignant cases (Normal). The level of ERK1 was used to demonstrate similar loading among all lanes.
Figure 10. Immunohistochemical staining for chaperonin 10. Note granular cytoplasmic positivity in several malignant endometrioid glands, while adjacent endometrial stroma and a portion of one benign endometrial gland (right) shows much reduced staining.
Figure 11 shows mass spectral windows from isotope-coded affinity tag (ICAT) experiments for three pairs of endometrial cancer / normal samples, demonstrating the over expression of calgizzarin in the cancer samples.
Figure 12: Number of peptides/ protein identified. The first (front) series corresponds to results obtained from cICAT, the second series is from the first iTRAQ run, and the third (rearmost) series is from the second iTRAQ run.
Figure 13: Cytoplasmic actin sequencing and quantification using iTRAQ. *A*: MS/MS spectrum of the doubly protonated peptide, EITALAPSTMK [SEQ ID NO. 49], at 725.4 Th. Residues in black are sequenced from the b-ion series, while those in purple from the y-ion series. Residue 'J' in the y-ion series is the iTRAQ-modified lysine residue. *B*: Expanded view of the low-m/z end ofthe MS/MS spectrum in '*A*', showing relative abundances of the signature iTRAQ ions at 114.1, 115.1, 116.1 and 117.1 Th.

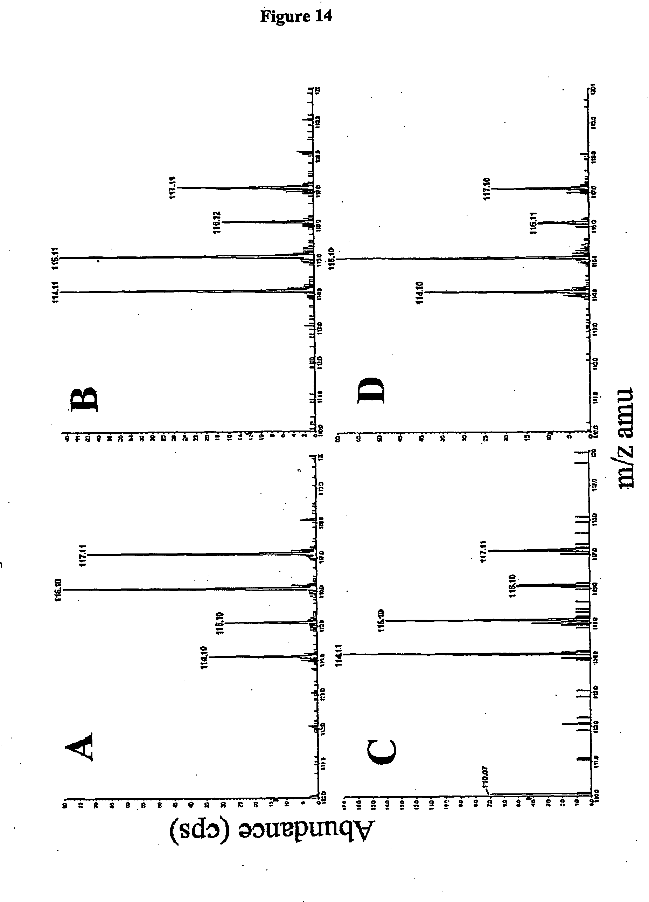

Figure 14: Relative abundances of differentially expressed proteins. The ion assignments are as follows: 114 Th, proliferative endometrium; 115 Th, secretory endometrium; 116 Th, first endometrial carcinoma; and 117 Th, second endometrial carcinoma. *A*: Tryptic peptide from chaperonin 10, showing higher abundances in the 116 and 117 Th ions relative to the 114 and 115 Th ions; *B*: tryptic peptide from alpha-1-antitrypsin precursor, showing lower abundances in the 116 and 117 Th ions relative to the 114 and 115 Th ions; *C*: tryptic peptide from creatine kinase B, showing lower abundances in the 116 and 117 Th ions relative to the 114 and 115 Th ions; *D:* tryptic peptide from transgelin, showing once more lower abundances in the 116 and 117 Th ions relative to the 114 and 115 Th ions.
Figure 15: Relative abundances of calgizzarin. clCAT analysis shows overexpression in all three cancer samples. The dotted line to the left of the heavy-label series, which originates from the cancerous sample, indicates the monoisotopic peak of the light-label series, which originates from the normal sample.
Figure 16: Distribution of proteins identified by A: iTRAQ and *B*: cICAT analyses.
Figure 17 shows histologic sections of human endometrium stained with hematoxylin and eosin: (a) proliferative endometrium, sample PRO2, and (b) secretory endometrium, sample SEC2.
Figure 18 is a nano LC/MS total ion chromatogram of strong cation exchange fraction 16 of sample A.
Figure 19 shows the distribution of 119 confirmed proteins. The legend shows the protein categories starting with "Structural" at 18% at the 2 o'clock position and continuing clockwise.
Figure 20 shows the appearance of selected proteins in the strong cation exchange fractions.
Figure 21 shows the (a) mass spectral window of sample A, fraction 19, showing enhanced expression of glutamate receptor subunit zeta I precursor in the secretory endometrium. The line to the left of the isotope envelope of the heavy-labeled version of the protein denotes where the light-labeled version is expected; (b) resulting MS/MS spectrum showing a partial sequence for the peptide LLTLALLFSCSVAR [SEQ ID NO. 28].
Figure 22 shows the mass spectral windows of the three sample pairs, traction 15, showing enhanced expression of FRAT1.
Figure 23 shows the mass spectral windows of sample A, fraction 4, showing enhanced expression of glycodelin in the secretory endometrium.
Figure 24 shows the mass spectral windows showing the isotopic clusters of cathepsin B. Sample A shows enhanced cathepsin B expression in the secretory endometrium; sample B shows the opposite trend.
Figure 25 shows SELDI MS spectral windows acquired on the QqTOF mass spectrometer (QSTAR XL): (a) typical tissue homogenate; (b) homogenate treated with 0 % hydrogen peroxide; (c) homogenate in (b) treated incubated with 3% hydrogen peroxide overnight; (d) laser fluence at 34.6 µJ; and (e) laser fluence at 76.9 µJ.
Figure 26 shows SELDI MS spectra of four malignant (designated by "C") and two endometrial tissue homogenates (designated by "N"): (a) full scan spectra collected on the linear QTOF mass spectrometer (PBS IIc); and (b) mass spectral windows collected on the PBS IIc, left panel, and on the QqTOF mass spectrometer (QSTAR XL), right panel.
Figure 27 shows SELDI MS spectral windows showing binding of both (a) chaperonin 10 and (b) the unknown protein at pH 6.0; and selective binding of the unknown protein at pH 7.0.
Figure 28 shows MS/MS spectra of three tryptic peptides from the unknown protein that identify it as calgranulin A. Peptide identities: (a) MLTELEK [SEQ ID NO. 50], (b) ALNSIIDVYHK [SEQ ID NO. 51], and (c) GADVWFK [SEQ ID NO. 52].
Figure 29 shows Calgranulin A immunohistochemical stain of endometrial TMA (US Biochem 1:150): (A) Endometrioid adenocarcinoma exhibits diffuse, immunostaining of numerous cells (3+) of the glandular component of the adenocarcinoma. Intense immunostaining of macrophages/granulocytes within the stroma and glandular lumina is also evident. No stromal staining is observed. (B) Endometrioid adenocarcinoma shows strong positive immunostaining of occasional malignant glands (1+). Intense individual cell staining is also apparent in macrophages/granulocytes within gland lumina and stroma.
Figure 30 shows Calgranulin A immunohistochemical stain of endometrial TMA (US Biochem 1: 150): Note intense (3+) cytoplasmic and nuclear immunostaining in squamous areas of this endometrioid adenocarcinoma. Glandular or columnar areas of the adenocarcinoma and adjacent stroma do not demonstrate any staining in this particular case.
Figure 31 shows Calgranulin A immunohistochemical stain of endometrial TMA (US Biochem 1: 150): The glands and stroma of this proliferative endometrium show no immunostaining. However, intense individual macrophage/granulocyte immunostaining is evident within the stroma.

### DETAILED DESCRIPTION OF THE INVENTION

Methods are provided for screening a subject for endometrial cancer.

### Glossary

"Endometrial disease" refers to any disorder, disease, condition, syndrome or combination of manifestations or symptoms recognized or diagnosed as a disorder of the endometrium, including but not limited to hyperplasia and cancer precursors, endometrial cancer or carcinoma, endometriosis, reproductive disorders, and infertility.

"Endometrial cancer" or "endometrial carcinoma" includes malignant endometrial disease including but not limited to endometrioid, mucinous, and serous adenocarcinomas, adenosquamous carcinomas, clear cell carcinomas, uterine sarcomas including stromal sarcomas, malignant mixed Mullerian tumors (carcinosarcomas), and leiomyosarcomas.

The terms "sample", "biological sample", and the like mean a material known or suspected of expressing or containing one or more endometrial polynucleotide markers or one or more endometrial markers. A test sample can be used directly as obtained from the source or following a pretreatment to modify the character of the sample. The sample can be derived from any biological source, such as tissues, extracts, or cell cultures, including cells (e.g. tumor cells), cell lysates, and physiological fluids, such as, for example, whole blood, plasma, serum, saliva, ocular lens fluid, cerebral spinal fluid, sweat, urine, milk, ascites fluid, synovial fluid, peritoneal fluid, lavage fluid, and the like. The sample can be obtained from animals, preferably mammals, most preferably humans. The sample can be treated prior to use, such as preparing plasma from blood, diluting viscous fluids, and the like. Methods of treatment can involve filtration, distillation, extraction, concentration, inactivation of interfering components, the addition of reagents, and the like.

In embodiments ofthe invention the sample is a mammalian tissue sample. In a particular embodiment, the tissue is endometrial tissue.

In another embodiment the sample is a human physiological fluid. In a particular embodiment, the sample is human serum.

The samples that may be analyzed in accordance with the invention include polynucleotides from clinically relevant sources, preferably expressed RNA or a nucleic acid derived therefrom (cDNA or amplified RNA derived from cDNA that incorporates an RNA polymerase promoter). The target polynucleotides can comprise RNA, including, without limitation total cellular RNA, poly(A)⁺ messenger RNA (mRNA) or fraction thereof, cytoplasmic mRNA, or RNA transcribed from cDNA (i.e., cRNA; see, for example., Linsley & Schelter, U.S. patent application Ser. No. 09/411,074, or U.S. Pat. Nos. 5,545,522, 5,891,636, or 5,716,785). Methods for preparing total and poly(A)⁺ RNA are well known in the art, and are described generally, for example, in Sambrook et al., (1989, Molecular Cloning - A Laboratory Manual (2nd Ed.), Vols. 1-3, Cold Spring Harbor Laboratory, Cold Spring Harbor, N.Y.) and Ausubel et al, eds. (1994, Current Protocols in Moelcular Biology, vol. 2, Current Protocols Publishing, New York). RNA may be isolated from eukaryotic cells by procedures involving lysis of the cells and denaturation of the proteins contained in the cells. Additional steps may be utilized to remove DNA. Cell lysis may be achieved with a nonionic detergent, followed by microcentrifugation to remove the nuclei and hence the bulk of the cellular DNA. (See Chirgwin et al., 1979, Biochemistry 18:5294-5299). Poly(A)+RNA can be selected using oligo-dT cellulose (see Sambrook et al., 1989, Molecular Cloning-A Laboratory Manual (2nd Ed), Vols. 1-3, Cold Spring Harbor Laboratory, Cold Spring Harbor, N.Y.). In the alternative, RNA can be separated from DNA by organic extraction, for example, with hot phenol or phenol/chloroform/isoamyl alcohol.

It may be desirable to enrich mRNA with respect to other cellular RNAs, such as transfer RNA (tRNA) and ribosomal RNA (rRNA). Most mRNAs contain a poly(A) tail at their 3' end allowing them to be enriched by affinity chromatography, for example, using oligo(dT) or poly(U) coupled to a solid support, such as cellulose or Sephadex™ (see Ausubel et al., eds., 1994, Current Protocols in Molecular Biology, vol. 2, Current Protocols Publishing, New York). Bound poly(A)+mRNA is eluted from the affinity column using 2 mM EDTA/0.1% SDS.

A sample of RNA can comprise a plurality of different mRNA molecules each with a different nucleotide sequence. In an aspect of the invention, the mRNA molecules in the RNA sample comprise at least 100 different nucleotide sequences.

Target polynucleotides can be detectably labeled at one or more nucleotides using methods known in the art. The label is preferably uniformly incorporated along the length of the RNA, and more preferably, is carried out at a high degree of efficiency. The detectable label can be a luminescent label, fluorescent label, bioluminescent label, chemi-luminescent label, radiolabel, and colorimetric label. In a particular embodiment, the label is a fluorescent label, such as a fluorescein, a phosphor, a rhodamine, or a polymethine dye derivative. Commercially available fluorescent labels include, for example, fluorescent phosphoramidites such as FluorePrime (Amersham Pharmacia, Piscataway, N.J.), Fluoredite (Millipore, Bedford, Mass.), FAM (ABI, Foster City, Calif.), and Cy3 or Cy5 (Amersham Pharmacia, Piscataway, N.J.).

Target polynucleotides from a patient sample can be labeled differentially from polynucleotides of a standard. The standard can comprise target polynucleotides from normal individuals (i.e., those not afflicted with or pre-disposed to endometrial disease, in particular pooled from samples from normal individuals. The target polynucleotides can be derived from the same individual, but taken at different time points, and thus indicate the efficacy of a treatment by a change in expression of the markers, or lack thereof, during and after the course of treatment.

The terms "subject", "individual" or "patient" refer to a warm-blooded animal such as a mammal. In particular, the terms refer to a human. A subject, individual or patient may be afflicted with or suspected of having or being pre-disposed to endometrial disease or a condition as described herein. The term also includes domestic animals bred for food or as pets, including horses, cows, sheep; poultry, fish, pigs, cats, dogs, and zoo animals.

The term "endometrial marker" includes a marker associated with normal or diseased endometrial tissue identified using a method of the invention. The term includes native-sequence polypeptides isoforms, chimeric polypeptides, complexes, all homologs, fragments, precursors, and modified forms and derivatives of the markers.

An endometrial marker may be associated with a stage or phase of endometrial tissue such as the secretory or proliferative phase. Examples of endometrial markers associated with the secretory phase are glutamate receptor subunit zeta 1 [SEQ ID NO. 26] and parts thereof (e.g. a tryptic fragment of SEQ ID NO. 28), macrophage migration inhibitory factor [SEQ ID NO. 18], FRAT1 [SEQ ID NO. 42], myosin light chain kinase 2 [SEQ ID NO. 45], and tropomyosin 1 alpha chain [SEQ ID NO. 47].

An endometrial marker may be associated with an endometrial disease, in particular it may be an endometrial cancer marker. The term "endometrial cancer marker" includes a marker associated with endometrial cancer, in particular a marker listed in Table 1.

In the invention, the endometrial cancer marker is chaperonin 10. The term "chaperonin 10", "chaperonin 10 polypeptide" or "chaperonin protein" includes human chaperonin 10, of SEQ ID NO. 1, a sequence with 80% identity to SEQ ID NO. 1 having the immunogenic activity of SEQ ID NO. 1 or a fragment of SEQ ID NO. 1 having the ability to form antibodies specific for SEQ ID NO. 1. The amino acid sequence for native human chaperonin 10 includes the sequences ofGenBank Accession No. Q04984 and AAH23518 shown in SEQ ID NO. 1.

A "native-sequence polypeptide" comprises a polypeptide having the same amino acid sequence of a polypeptide derived from nature. Such native-sequence polypeptides can be isolated from nature or can be produced by recombinant or synthetic means. The term specifically encompasses naturally occurring truncated or secreted forms of a polypeptide, polypeptide variants including naturally occurring variant forms (e.g. alternatively spliced forms or splice variants), and naturally occurring allelic variants.

The term "polypeptide variant" means a polypeptide having at least about 80%, 85%, 90%, 95%, 97%, 98%, or 99% amino acid sequence identity, more particularly at least about 95%, still more particularly at least about 90%, most particularly at least about 95% amino acid sequence identity with SEQ ID NO. 1, such variants include, for instance, polypeptides wherein one or more amino acid residues are added to, or deleted from, the Nor C-terminus of the full-length or mature sequences of the polypeptide, including variants from other species, but excludes a native-sequence polypeptide. Variants retain the immunogenic activity of the corresponding native-sequence polypeptide.

Percent identity of two amino acid sequences, or of two nucleic acid sequences is defined as the percentage of amino acid residues or nucleotides in a candidate sequence that are identical with the amino acid residues in a polypeptide or nucleic acid sequence, after aligning the sequences and introducing gaps, if necessary, to achieve the maximum percent sequence identity, and not considering any conservative substitutions as part of the sequence identity. Alignment for purposes of determining percent amino acid or nucleic acid sequence identity can be achieved in various conventional ways, for instance, using publicly available computer software including the GCG program package (Devereux J. et al., Nucleic Acids Research 12(1): 387, 1984); BLASTP, BLASTN, and FASTA (Atschul, S.F. et al. J. Molec. Biol. 215: 403-410, 1990). The BLAST X program is publicly available from NCBI and other sources (BLAST Manual, Altschul, S. et al. NCBI NLM NIH Bethesda, Md. 20894; Altschul, S. et al. J. Mol. Biol. 215: 403-410, 1990). Skilled artisans can determine appropriate parameters for measuring alignment, including any algorithms needed to achieve maximal alignment over the full length of the sequences being compared. Methods to determine identity and similarity are codified in publicly available computer programs.

An allelic variant may also be created by introducing substitutions, additions, or deletions into a polynucleotide encoding a native polypeptide sequence such that one or more amino acid substitutions, additions, or deletions are introduced into the encoded protein. Mutations may be introduced by standard methods, such as site-directed mutagenesis and PCR-mediated mutagenesis. In an embodiment, conservative substitutions are made at one or more predicted non-essential amino acid residues. A "conservative amino acid substitution" is one in which an amino acid residue is replaced with an amino acid residue with a similar side chain. Amino acids with similar side chains are known in the art and include amino acids with basic side chains (e.g. Lys, Arg, His), acidic side chains (e.g. Asp, Glu), uncharged polar side chains (e.g. Gly, Asp, Glu, Ser, Thr, Tyr and Cys), nonpolar side chains (e.g. Ala, Val, Leu, Iso, Pro, Trp), beta-branched side chains (e.g. Thr, Val, Iso), and aromatic side chains (e.g. Tyr, Phe, Trp, His). Mutations can also be introduced randomly along part or all of the native sequence, for example, by saturation mutagenesis. Following mutagenesis the variant polypeptide can be recombinantly expressed and the activity of the polypeptide may be determined.

Polypeptide variants include polypeptides comprising amino acid sequences sufficiently identical to or derived from the amino acid sequence of a native polypeptide which include fewer amino acids than the full length polypeptides. A portion of a polypeptide can be a polypeptide which is for example, 10, 15, 20, 25, 30, 35, 40, 45, 50, 60, 70, 80, 90, 100 or more amino acids in length. Portions in which regions of a polypeptide are deleted can be prepared by recombinant techniques and can be evaluated for one or more functional activities such as the ability to form antibodies specific for a polypeptide.

A naturally occurring allelic variant may contain conservative amino acid substitutions from the native polypeptide sequence or it may contain a substitution of an amino acid from a corresponding position in a polypeptide homolog, for example, a murine polypeptide.

Endometrial markers include chimeric or fusion proteins. A "chimeric protein" or "fusion protein" comprises all or part (preferably biologically active) of an endometrial marker operably linked to a heterologous polypeptide (i.e., a polypeptide other than an endometrial marker). Within the fusion protein, the term "operably linked" is intended to indicate that an endometrial marker and the heterologous polypeptide are fused in-frame to each other. The heterologous polypeptide can be fused to the N-terminus or C-terminus of an endometrial marker. A useful fusion protein is a GST fusion protein in which an endometrial marker is fused to the C-terminus of GST sequences. Another example of a fusion protein is an immunoglobulin fusion protein in which all or part of an endometrial marker is fused to sequences derived from a member of the immunoglobulin protein family. Chimeric and fusion proteins can be produced by standard recombinant DNA techniques.

A modified form of a polypeptide referenced herein includes modified forms of the polypeptides and derivatives of the polypeptides, including post-translationalty modified forms such as glycosylated, phosphorylated, acetylated, methylated or lapidated forms of the polypeptides. For example, an N-terminal methionine may be cleaved from a polypeptide, and a new N-terminal residue may or may not be acetylated. In particular, for chaperonin 10 the first residue, methionine, can be cleaved and the second first residue, alanine can be N-acetylated.

Endometrial markers identified in accordance with a method of the invention may be prepared by recombinant or synthetic methods, or isolated from a variety of sources, or by any combination of these and similar techniques.

"Endometrial polynucleotide marker(s)", polynucleotides encoding the marker(s)", and "polynucleotides encoding endometrial markers" refer to polynucleotides that encode endometrial markers including native-sequence polypeptides, polypeptide variants including a portion of a polypeptide, an isoform, precursor, complex, a chimeric polypeptide, or modified forms and derivatives of the polypeptides. In the method of the invention the polynucleotide encodes chaperonin 10, more particularly a polynucleotide sequence of GenBank Accession No. NM_002!57 and U07550 [SEQ ID NO 2], or a fragment thereof.

Endometrial polynucleotide markers include complementary nucleic acid sequences, and nucleic acids that are substantially identical to these sequences (e.g. having at least about 45%, 50%, 55%, 60%, 65%, 70%, 75%, 80%, 85%, 90%, 95%, 97%, 98%, or 99% sequence identity).

Endometrial polynucleotide markers also include sequences that differ from a native sequence [e.g. SEQ ID NO. 2], due to degeneracy in the genetic code. As one example, DNA sequence polymorphisms within the nucleotide sequence of an endometrial marker may result in silent mutations that do not affect the amino acid sequence. Variations in one or more nucleotides may exist among individuals within a population due to natural allelic variation. DNA sequence polymorphisms may also occur which lead to changes in the amino acid sequence of a polypeptide.

Endometrial polynucleotide markers also include nucleic acids that hybridize under stringent conditions, preferably high stringency conditions to the endometrial polynucleotide marker (i.e. SEQ ID NO. 2). Appropriate stringency conditions which promote DNA hybridization are known to those skilled in the art, or can be found in Current Protocols in Molecular Biology, John Wiley & Sons, N.Y. (1989), 6.3.1-6.3.6. For example, 6.0 x sodium chloride/sodium citrate (SSC) at about 45°C, followed by a wash of 2.0 x SSC at 50°C may be employed. The stringency may be selected based on the conditions used in the wash step. By way of example, the salt concentration in the wash step can be selected from a high stringency of about 0.2 x SSC at 50°C. In addition, the temperature in the wash step can be at high stringency conditions, at about 65°C.

Endometrial polynucleotide markers also include truncated nucleic acids or nucleic acid fragments and variant forms of the nucleic acids that arise by alternative splicing of an mRNA corresponding to a DNA.

The endometrial polynucleotide markers are intended to include DNA and RNA (e.g. mRNA) and can be either double stranded or single stranded. A polynucleotide may, but need not, include additional coding or non-coding sequences, or it may, but need not, be linked to other molecules and/or carrier or support materials. The polynucleotides for use in the methods of the invention may be of any length suitable for a particular method. In certain applications the term refers to antisense polynucleotides (e.g. mRNA or DNA strand in the reverse orientation to sense cancer polynucleotide markers).

"Statistically different levels", "significantly altered levels", or "significant difference" in levels of markers in a patient sample compared to a control or standard (e.g. normal levels or levels in other samples from a patient) may represent levels that are higher or lower than the standard error ofthe detection assay. In particular embodiments, the levels may be 1.5, 2, 3, 4, 5, or 6 times higher or lower than the control or standard.

"Microarray" and "array," refer to nucleic acid or nucleotide arrays or protein or peptide arrays that can be used to detect biomolecules associated with endometrium or phase threof or endometrial disease, for instance to measure gene expression. A variety of arrays are made in research and manufacturing facilities worldwide, some of which are available commercially. By way ofexample, spotted arrays and in situ synthesized arrays are two kinds of nucleic acid arrays that differ in the manner in which the nucleic acid materials are placed onto the array substrate. A widely used in situ synthesized oligonucleotide array is GeneChip™ made by Affymetrix, Inc. Oligonucleotide probes that are 20- or 25-base long can be synthesized in silico on the array substrate. These arrays can achieve high densities (e.g., more than 40,000 genes per cm²). Generally spotted arrays have lower densities, but the probes, typically partial cDNA molecules, are much longer than 20- or 25-mers. Examples of spotted cDNA arrays include LifeArray made by Incyte Genomics and DermArray made by IntegriDerm (or Invitrogen). Pre-synthesized and amplified cDNA sequences are attached to the substrate of spotted arrays. Protein and peptide arrays also are known (see for example, Zhu et al., Science 293:2101 (2001).

"Binding agent" refers to a substance such as a polypeptide or antibody that specifically binds to one or more endometrial markers. A substance "specifically binds" to one or more endometrial markers if is reacts at a detectable level with one or more endometrial markers, and does not react detectably with peptides containing an unrelated or different sequence. Binding properties may be assessed using an ELISA, which may be readily performed by those skilled in the art (see for example, Newton et al, Develop. Dynamics 197: 1-13, 1993).

A binding agent may be a ribosome, with or without a peptide component, an aptamer, an RNA molecule, or a polypeptide. A binding agent may be a polypeptide that comprises one or more endometrial marker sequence, a peptide variant thereof, or a non-peptide mimetic of such a sequence. By way of example, a chaperonin 10 sequence may be a peptide portion of a chaperonin 10 that is capable of modulating a function mediated by chaperonin 10.

An aptamer includes a DNA or RNA molecule that binds to nucleic acids and proteins. An aptamer that binds to a protein (or binding domain) of an endometrial marker or an endometrial polynucleotide marker can be produced using conventional techniques, without undue experimentation. (For example, see the following publications describing *in vitro* selection of aptamers: Klug et al Mol. Biol. Reports 20:97-107 (1994); Wallis et al., Chem. Biol. 2:543-552 (1995); Ellington, Curr. Biol. 4:427-429 (1994); Lato et al., Chem. Biol. 2:291-303 (1995); Conrad et al., Mol. Div. 1:69-78 (1995); and Uphoff et al., Curr. Opin. Struct. Biol. 6:281-287 (1996)).

Antibodies for use in the present invention include but are not limited to monoclonal or polyclonal antibodies, immunologically active fragments (e.g. a Fab or (Fab)₂ fragments), antibody heavy chains, humanized antibodies, antibody light chains, genetically engineered single chain Fᵥ molecules (Ladner et al, U.S. Pat. No. 4,946,778), chimeric antibodies, for example, antibodies which contain the binding specificity of murine antibodies, but in which the remaining portions are of human origin, or derivatives, such as enzyme conjugates or labeled derivatives.

Antibodies including monoclonal and polyclonal antibodies, fragments and chimeras, may be prepared using methods known to those skilled in the art. Isolated native or recombinant endometrial markers may be utilized to prepare antibodies. (See, for example, Kohler et al. (1975) Nature 256:495-497; Kozbor et al. (1995) J. Immunol Methods 81:31-42; Cote et al. (1983) Proc Natl Acad Sci 80:2026-2030; and Cole et al. (1984) Mol Cell Biol 62:109-120 for the preparation of monoclonal antibodies; Huse et al. (1989) Science 246:1275-1281 for the preparation of monoclonal Fab fragments; and, Pound (1998) Immunochemical Protocols, Humana Press, Totowa, NJ for the preparation of phagemid or B-lymphocyte immunoglobulin libraries to identify antibodies). Antibodies specific for an endometrial marker may also be obtained from scientific or commercial sources.

In an embodiment of the invention, antibodies are reactive against an endometrial marker if they bind with a Kₐ of greater than or equal to 10⁻⁷ M.

### Markers

The invention provides a marker correlated with endometrial disease.

The marker provided herein may be used alone or with other markers of endometrium phase or endometrial disease, or with markers for other phenotypes or conditions.

### Identification of Endometrial Markers

Markers may be identified by:
(a) obtaining a sample of endometrium from a subject;
(b) extracting proteins from the sample and producing a profile of the proteins by subjecting the proteins to mass spectrometry; and
(c) comparing the profile with a profile for normal endometrial tissue or for a known endometrium phase to identify proteins associated with the endometrium phase or an endometrial disease.

Proteins may be extracted from the samples in a manner known in the art. For example, proteins may be extracted by first digesting or disrupting cell membranes by standard methods such as detergents or homogenization in an isotonic sucrose solution, followed by ultra-centrifugation or other standard techniques.

The separated proteins may be digested into peptides, in particular using proteolytic enzymes such as trypsin, pepsin, subtilisin, and proteinase. For example, proteins may be treated with trypsin which cleaves at the sites of lysine and arginine, to provide doubly-charged peptides with a length of from about 5 to 50 amino acids. Such peptides may be particularly appropriate for mass spectrometry analysis, especially electrospray ionization mass spectrometry. Chemical reagents including cyanogens bromide may also be utilized to digest proteins.

Mass spectrometers that may be used to analyze the peptides or proteins include a Matrix-Assisted Laser Desorptioon/Ioniation Time-of-Flight Mass Spectrometer ("MALDI-TOF") (e.g. from PerSeptive Biosystems, Framingham, Mass.); an Electrospray Ionization ("ESI") ion trap spectrometer, (e.g. from Finnigan MAT, San Jose, Calif.), an ESI quadrupole mass spectrometer (e.g. from Finnigan or Perkin-Elmer Corporation, Foster City, Calif.), a quadrupole/TOF hybrid tandem mass spectrometer, QSTAR XL (Applied Biosystems/MDS Sciex), or a Surface Enhanced Laser Desorption/Ionization (SELDI-TOF) Mass Spectrometer (e.g. from Ciphergen Biosystems Inc.).

### Detection Methods

A variety of methods can be employed for the diagnostic and prognostic evaluation of endometrial disease or endometrial status involving one or more endometrial markers and polynucleotides encoding the markers, and the identification of subjects with a predisposition to endometrial diseases or that are receptive to *in vitro* fertilization and embryo transfer procedures. Such methods may, for example, utilize endometrial polynucleotide markers, and fragments thereof, and binding agents (e.g. antibodies) against one or more endometrial markers, including peptide fragments. In particular, the polynucleotides and antibodies may be used, for example, for (1) the detection of the presence of endometrial polynucleotides marker mutations, or the detection of either over- or under-expression of endometrial marker mRNA relative to a non-disorder state or different endometrium phase, or the qualitative or quantitative detection of alternatively spliced forms of endometrial polynucleotide marker transcripts which may correlate with certain conditions or susceptibility toward such conditions; and (2) the detection of either an over- or an under-abundance of one or more endometrial markers relative to a non- disorder state or a different endometrium phase or the presence of a modified (e.g., less than full length) endometrial marker which correlates with a disorder state or a progression toward a disorder state, or a particular endometrium phase.

The invention provides a method for screening a subject for endometrial cancer comprising (a) detecting in a sample from the subject an amount of an endometrial cancer marker or polynucleotide encoding the endometrial cancer marker, and (b) comparing the amount of endometrial cancer marker or polynucleotide detected to a standard, where detection of a level of endometrial cancer marker or polynucleotide different than that ofa standard is indicative of endometrial cancer, and wherein the endometrial cancer marker is chaperonin 10 of SEQ ID NO. 1, a sequence with 80% sequence identity to SEQ ID NO. 1 having the immunogenic activity of SEQ ID NO. 1 or a fragment of SEQ ID NO. 1 having the ability to form antibodies specific for SEQ ID NO. 1.

The methods described herein may be used to evaluate the probability of the presence of malignant or pre-malignant cells, for example, in a group of cells freshly removed from a host. Such methods can be used to detect tumors, quantitate their growth, and help in the diagnosis and prognosis of endometrial disease. The methods can be used to detect the presence of cancer metastasis, as well as confirm the absence or removal of all tumor tissue following surgery, cancer chemotherapy, and/or radiation therapy. They can further be used to monitor cancer chemotherapy and tumor reappearance.

The methods described herein can be adapted for diagnosing and monitoring endometrial cancer by detecting the endometrial markers or polynucleotides encoding the markers in biological samples from a subject. These applications require that the amount of markers or polynucleotides quantitated in a sample from a subject being tested be compared to a predetermined standard or cut-off value. The standard may correspond to levels quantitated for another sample or an earlier sample from the subject, or levels quantitated for a control sample. Levels for control samples from healthy subjects, different endometrial tissue phases, or subjects with an endometrial disease may be established by prospective and/or retrospective statistical studies. Healthy subjects who have no clinically evident disease or abnormalities may be selected for statistical studies. Diagnosis may be made by a finding of statistically different levels of detected endometrial markers associated with disease or polynucleotides encoding same, compared to a control sample or previous levels quantitated for the same subject.

The methods described herein may also use multiple markers for endometrial cancer. A biological sample maybe analysed for the presence of one or more endometrial markers and polynucleotides encoding the markers, and other markers that are specific indicators of an endometrial disease. The methods described herein may be modified by including reagents to detect the additional markers, or polynucleotides for the markers.

### Nucleic Acid Methods/Assays

As noted herein an endometrial disease or phase may be detected based on the level of endometrial polynucleotide markers in a sample. Techniques for detecting polynucleotides such as polymerase chain reaction (PCR) and hybridization assays are well known in the art.

Probes may be used in hybridization techniques to detect endometrial polynucleotide markers. The technique generally involves contacting and incubating nucleic acids (e.g. recombinant DNA molecules, cloned genes) obtained from a sample from a patient or other cellular source with a probe under conditions favorable for the specific annealing ofthe probes to complementary sequences in the nucleic acids. After incubation, the non-annealed nucleic acids are removed, and the presence of nucleic acids that have hybridized to the probe if any are detected.

Nucleotide probes for use in the detection of nucleic acid sequences in samples may be constructed using conventional methods known in the art. Suitable probes may be based on nucleic acid sequences encoding at least 5 sequential amino acids from regions of an endometrial polynucleotide marker, preferably they comprise 10-200, more particularly 10-30, 10-40, 20-50, 40-80, 50-150, 80-120 nucleotides in length.

The probes may comprise DNA or DNA mimics (e.g., derivatives and analogues) corresponding to a portion of an organism's genome, or complementary RNA or RNA mimics. Mimics are polymers comprising subunits capable of specific, Watson-Crick-like hybridization with DNA, or ofspecific hybridization with RNA. The nucleic acids can be modified at the base moiety, at the sugar moiety, or at the phosphate backbone.

DNA can be obtained using standard methods such as polymerase chain reaction (PCR) amplification of genomic DNA or cloned sequences. (See, for example, in Innis et al., eds., 1990, PCR Protocols: A Guide to Methods and Applications, Academic Press Inc., San Diego, Calif.). Computer programs known in the art can be used to design primers with the required specificity and optimal amplification properties, such as Oligo version 5.0 (National Biosciences). Controlled robotic systems may be useful for isolating and amplifying nucleic acids.

A nucleotide probe may be labeled with a detectable substance such as a radioactive label that provides for an adequate signal and has sufficient half life such as ³²P, ³H, ¹⁴C or the like. Other detectable substances that may be used include antigens that are recognized by a specific labeled antibody, fluorescent compounds, enzymes, antibodies specific for a labeled antigen, and luminescent compounds. An appropriate label may be selected having regard to the rate of hybridization and binding of the probe to the nucleotide to be detected and the amount of nucleotide available for hybridization. Labeled probes may be hybridized to nucleic acids on solid supports such as nitrocellulose filters or nylon membranes as generally described in Sambrook et al, 1989, Molecular Cloning, A Laboratory Manual (2nd ed.). The nucleic acid probes may be used to detect endometrial polynucleotide markers, preferably in human cells. The nucleotide probes may also be useful in the diagnosis of an endometrial disease involving one or more endometrial polynucleotide markers, in monitoring the progression of such disorder, or monitoring a therapeutic treatment.

The detection of endometrial polynucleotide markers may involve the amplification of specific gene sequences using an amplification method such as polymerase chain reaction (PCR), followed by the analysis of the amplified molecules using techniques known to those skilled in the art. Suitable primers can be routinely designed by one of skill in the art.

By way of example, at least two oligonucleotide primers may be employed in a PCR based assay to amplify a portion of a polynculeotide encoding one or more endometrial marker derived from a sample, wherein at least one of the oligonucleotide primers is specific for (i.e. hybridizes to) a polynucleotide encoding the endometrial marker. The amplified cDNA is then separated and detected using techniques well known in the art, such as gel electrophoresis.

In order to maximize hybridization under assay conditions, primers and probes employed in the methods of the invention generally have at least about 60%, preferably at least about 75%, and more preferably at least about 90% identity to a portion of a polynucleotide encoding an endometrial marker, that is, they are at least 10 nucleotides, and preferably at least 20 nucleotides in length. In an embodiment the primers and probes are at least about 10-40 nucleotides in length.

Hybridization and amplification techniques described herein may be used to assay qualitative and quantitative aspects of endometrial polynucleotide marker expression. For example, RNA may be isolated from a cell type or tissue known to express an endometrial polynucleotide marker and tested utilizing the hybridization (e.g. standard Northern analyses) or PCR techniques referred to herein.

The primers and probes may be used in the above-described methods *in situ* i.e directly on tissue sections (fixed and/or frozen) of patient tissue obtained from biopsies or resection.

In an aspect of the invention, a method is provided employing reverse transcriptase-polymerase chain reaction (RT-PCR), in which PCR is applied in combination with reverse transcription. Generally, RNA is extracted from a sample tissue using standard techinques (for example, guanidine isothiocyanate extraction as described by Chomcynski and Sacchi, Anal. Biochem.162:156-159, 1987) and is reverse transcribed to produce cDNA. The cDNA is used as a template for a polymerase chain reaction. The cDNA is hybridized to a set of primers, at least one of which is specifically designed against an endometrial marker sequence. Once the pimer and template have annealed a DNA polymerase is employed to extend from the primer, to synthesize a copy of the template. The DNA strands are denatured, and the procedure is repeated many times until sufficient DNA is generated to allow visualization by ethidium bromide staining and agarose gel electrophoresis.

Amplification may be performed on samples obtained from a subject with a suspected endometrial disease and an individual who is not afflicted with an endometrial disease. The reaction may be performed on several dilutions of cDNA spanning at least two orders of magnitude. A statistically significant difference in expression in several dilutions ofthe subject sample as compared to the same dilutions of the non-disease sample may be considered positive for the presence of an endometrial disease.

In an embodiment, the invention provides methods for determining the presence or absence of an endometrial disease in a subject comprising (a) contacting a sample obtained from the subject with oligonucleotides that hybridize to endometrial polynucleotide markers; and (b) detecting in the sample a level of nucleic acids that hybridize to the polynucleotides relative to a predetermined cut-off value, and wherefrom determining the presence or absence of an endometrial disease in the subject. In this aspect, the endometrial disease is cancer and the endometrial markers is chaperonin 10.

Endometrial marker mRNA may be is detected by (a) isolating mRNA from a sample and combining the mRNA with reagents to convert it to cDNA; (b) treating the converted cDNA with amplification reaction reagents and nucleic acid primers that hybridize to one or more endometrial marker polynucleotides, to produce amplification products; (d) analyzing the amplification products to detect amounts of mRNA encoding endometrial polynucleotide markers; and (e) comparing the amount ofmRNA to an amount detected against a panel of expected values for normal and malignant tissue derived using similar nucleic acid primers.

Endometrial cancer marker-positive samples or alternatively higher levels in patients compared to a control (e.g. non-cancerous tissue) may be indicative of late stage disease, and/or that the patient is not responsive to chemotherapy. Alternatively, negative samples or lower levels compared to a control (e.g.non-cancerous tissue or negative samples) may also be indicative of progressive disease and shorter overall survival.

The presence or absence of endometrial cancer may be determined in a subject by (a) contacting a sample obtained from the subject with oligonucleotides that hybridize to one or more endometrial cancer polynucleotide markers; and (b) detecting in the sample levels of nucleic acids that hybridize to the polynucleotides relative to a predetermined cut-off value, and therefrom determining the presence or absence of endometrial cancer in the subject. In an embodiment, the endometrial cancer polynucleotide markers encode one or more polypeptides listed in Table 1. In the invention, the endometrial marker is chaperonin 10, optionally with one or more of calgranulin A, calgranulin B, polymeric-immunoglobulin receptor (precursor), phosphatidylethanolamine-binding protein, acidic leucine-rich nuclear phosphopmtein 32 family member A, heat shock 70 kDa protein 6, macrophage migration inhibitory factor, calgizzarin (S100C protein), triosephosphate isomerase, alpha-1-andtrypsin precursor, creatine kinase, B chain, (B-CK), pyruvate, M1 or M2 isozyme, transgelin (smooth muscle protein 22-alpha), and heterologous nuclear ribonucleoprotein D0, and fragments thereof

In particular, the invention provides a method wherein chaperonin 10 mRNA is detected by (a) isolating mRNA from a sample and combining the mRNA with reagents to convert it to cDNA; (b) treating the converted cDNA with amplification reaction reagents and nucleic acid primers that hybridize to a polynucleotide encoding chaperonin 10, to produce amplification products; (d) analyzing the amplification products to detect an amount of mRNA encoding chaperonin 10; and (e) comparing the amount of mRNA to an amount detected against a panel of expected values for normal and malignant tissue derived using similar nucleic acid primers.

Endometrial cancer marker-positive samples or alternatively higher levels, in particular significantly higher levels of chaperonin 10 polynucleotides in patients compared to a control (e.g. normal or benign) are indicative of endometrial cancer. Negative samples or lower levels compared to a control (e.g. normal or benign) may also be indicative of progressive disease and poor overall survival.

Oligonucleotides or longer fragments derived from an endometrial cancer polynucleotide marker may be used as targets in a microarray. The microarray can be used to simultaneously monitor the expression levels of large numbers of genes and to identify genetic variants, mutations, and polymorphisms. The information from the microarray may be used to determine gene function, to understand the genetic basis of a disorder, to diagnose a disorder, and to develop and monitor the activities of therapeutic agents.

The preparation, use, and analysis of microarrays are well known to a person skilled in the art. (See, for example, Brennan, T. M. et al. (1995) U.S. Pat. No. 5,474,796; Schena, et al. (1996) Proc. Natl. Acad. Sci. 93:10614-10619; Baldeschweiler et al. (1995), PCT Application WO95/251116; Shalon, D. et al. (1995) PCT application WO95/35505; Heller, R. A. et al. (1997) Proc. Natl. Acad. Sci. 94:2150-2155; and Heller, M. J. et al. (1997) U.S. Pat. No. 5,605,662.)

The array comprising one or more endometrial polynucleotide markers (in particular the markers listed in Table 1, 4, 5, or 6) and optionally other markers. The array can be used to assay expression of endometrial, polynucleotide markers in the array. The invention allows the quantitation of expression of one or more endometrial polynucleotide markers.

Microarrays typically contain at separate sites nanomolar quantities of individual genes, cDNAs, or ESTs on a substrate (e.g.nitrocellulose or silicon plate), or photolithographically prepared glass substrate. The arrays are hybridized to cDNA probes using conventional techniques with gene-specific primer mixes. The target polynucleotides to be analyzed are isolated, amplified and labeled, typically with fluorescent labels, radiolabels or phosphorous label probes. After hybridization is completed, the array is inserted into the scanner, where patterns of hybridization are detected. Data are collected as light emitted from the labels incorporated into the target, which becomes bound to the probe array. Probes that completely match the target generally produce stronger signals than those that have mismatches. The sequence and position of each probe on the array are known, and thus by complementarity, the identity of the target nucleic acid applied to the probe array can be determined.

Microarrays are prepared by selecting polynucleotide probes and immobilizing them to a solid support or surface. The probes may comprise DNA sequences, RNA sequences, copolymer sequences of DNA and RNA, DNA and/or RNA analogues, or combinations thereof. The probe sequences may be full or partial fragments of genomic DNA, or they may be synthetic oligonucleotide sequences synthesized either enzymatically *in vivo,* enzymatically *in vitro* (e.g., by PCR), or non-enzymatically *in vitro.*

The probe or probes used in the methods of the invention can be immobilized to a solid support or surface which may be either porous or non-porous. For example, the probes can be attached to a nitrocellulose or nylon membrane or filter covalently at either the 3' or the 5' end of the polynucleotide probe. The solid support may be a glass or plastic surface. In an aspect ofthe invention, hybridization levels are measured to microarrays of probes consisting ofa solid support on the surface of which are immobilized a population of polynucleotides, such as a population of DNA or DNA mimics, or, alternatively, a population of RNA or RNA mimics. A solid support may be a nonporous or, optionally, a porous material such as a gel.

In accordance with embodiments of the invention, a microarray is provided comprising a support or surface with an ordered array of hybridization sites or "probes" each representing one of the markers described herein. The microarrays can be addressable arrays, and in particular positionally addressable arrays. Each probe of the array is typically located at a known, predetermined position on the solid support such that the identity of each probe can be determined from its position in the array. In preferred embodiments, each probe is covalently attached to the solid support at a single site.

Microarrays used in the present invention are preferably (a) reproducible, allowing multiple copies of a given array to be produced and easily compared with each other; (b) made from materials that are stable under hybridization conditions; (c) small, (e.g., between 1 cm² and 25 cm², between 12 cm² and 13 cm², or 3 cm²; and (d) comprise a unique set of binding sites that will specifically hybridize to the product of a single gene in a cell (e.g., to a specific mRNA, or to a specific cDNA derived therefrom). However, it will be appreciated that larger arrays may be used particularly in screening arrays, and other related or similar sequences will cross hybridize to a given binding site.

In accordance with an aspect of the invention, the microarray is an array in which each position represents one of the markers described herein. Each position ofthe array can comprise a DNA or DNA analogue based on genomic DNA to which a particular RNA or cDNA transcribed from a genetic marker can specifically hybridize. A DNA or DNA analogue can be a synthetic oligomer or a gene fragment. In an embodiment, probes representing each of the endometrial markers and endometrial polynucleotide markers is present on the array. In a preferred embodiment, the array comprises at least 5 of the endometrial polynucleotide markers.

Probes for the microarray can be synthesized using N-phosphonate or phosphoramidite chemistries (Froehler et al., 1986, Nucleic Acid Res. 14:5399-5407; McBride et al., 1983, Tetrahedron Lett. 24:246-248). Synthetic sequences are typically between about 10 and about 500 bases, 20-100 bases, or 40-70 bases in length. Synthetic nucleic acid probes can include non-natural bases, such as, without limitation, inosine. Nucleic acid analogues such as peptide nucleic acid may be used as binding sites for hybridization. (see, e.g., Egholm et al., 1993, Nature 363:566-568; U.S. Pat. No. 5,539,083).

Probes can be selected using an algorithm that takes into account binding energies, base composition, sequence complexity, cross-hybridization binding energies, and secondary structure (see Friend et al., International Patent Publication WO 01/05935, published Jan. 25, 2001).

Positive control probes, (e.g., probes known to be complementary and hybridizae to sequences in the target polynucleotides), and negative control probes, (e.g., probes known to not be complementary and hybridize to sequences in the target polynucleotides) are typically included on the array. Positive controls can be synthesized along the perimeter of the array or synthesized in diagonal stripes across the array. A reverse complement for each probe can be next to the position of the probe to serve as a negative control.

The probes can be attached to a solid support or surface, which may be made from glass, plastic (e.g., polypropylene, nylon), polyacrylamide, nitrocellulose, gel, or other porous or nonporous material. The probes can be printed on surfaces such as glass plates (see Schena et al., 1995, Science 270:467-470). This method may be particularly useful for preparing microarrays of cDNA (See also, DeRisi et al.,1996, Nature Genetics 14:457-460; Shalon et al., 1996, Genome Res. 6:639-645; and Schena et al., 1995, Proc. Natl. Acad. Sci. U.S.A. 93:10539-11286).

High-density oligonucleotide arrays containing thousands of oligonucleotides complementary to defined sequences, at defined locations on a surface can be produced using photolithographic techniques for synthesis in situ (see, Fodor et al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat. Nos. 5,578,832; 5,556,752; and 5,510,270) or other methods for rapid synthesis and deposition of defined oligonucleotides (Blanchard et al., Biosensors & Bioelectronics 11:687-690). Using these methods oligonucleotides (e.g., 60-mers) of known sequence are synthesized directly on a surface such as a derivatized glass slide. The array produced may be redundant, with several oligonucleotide molecules per RNA.

Microarrays can be made by other methods including masking (Maskos and Southern, 1992, Nuc. Acids. Res. 20:1679-1684). In an embodiment, microarrays ofthe present invention are preduced by synthesizing polynucleotide probes on a support wherein the nucleotide probes are attached to the support covalently at either the 3' or the 5' end of the polynucleotide.

The microarray may comprise a disclosed marker set For example a microarray for distinguishing endometrial disease samples may comprise a positionally-addressable array of polynucleotide probes bound to a support, the polynucleotide probes comprising a plurality of polynucleotide probes of different nucleotide sequences, each of the different nucleotide sequences comprising a sequence complementary and hybridizable to a plurality of genes, the plurality consisting of at least 2, 3, 4, 5, 6, 7, 8, 9, 10, 11, 12, 13, 14, or 15 of the genes corresponding to the markers listed in Table 1, 4, 5, or 6. The microarray may comprise at least 5, 10, or 15 of the polynucleotides encoding the markers listed in Table 1, 4, 5, or 6.

The array can be used to monitor the time course of expression of one or more endometrial polynucleotide markers in the array. This can occur in various biological contexts such as tumor progression.

The array is also useful for ascertaining differential expression patterns ofendometrial polynucleotide markers, and optionally other markers, in normal and abnormal cells. This may provide a battery of nucleic acids that could serve as molecular targets for diagnosis or therapeutic intervention.

### Protein Methods

Binding agents may be used for a variety of diagnostic and assay applications. There are a variety of assay formats known to the skilled artisan for using a binding agent to detect a target molecule in a sample. (For example, see Harlow and Lane, Antibodies: A Laboratory Manual, Cold Spring Harbor Laboratory, 1988). In general , the presence or absence of an endometrial disease (e.g. cancer) or an endometrium phase in a subject may be determined by (a) contacting a sample from the subject with a binding agent; (b) detecting in the sample a level of polypeptide that binds to the binding agent; and (c) comparing the level of polypeptide with a predetermined standard or cut-off value.

In the method of the invention, the endometrial cancer marker may be detected by contacting the sample with an antibody that specifically binds to the endometrial cancer marker. Antibodies specifically reactive with one or more endometrial marker, or derivatives, such as enzyme conjugates or labeled derivatives, may be used to detect one or more endometrial marker in various samples (e.g. biological materials). They may be used as diagnostic or prognostic reagents and they may be used to detect abnormalities in the level of expression of one or more endometrial marker, or abnormalities in the structure, and/or temporal, tissue, cellular, or subcellular location of one or more endometrial marker. Antibodies may also be used to screen potentially therapeutic compounds *in vitro* to determine their effects on disorders (e.g. endometrial cancer) involving one or more endometrial markers, and other conditions. *In vitro* immunoassays may also be used to assess or monitor the efficacy of particular therapies.

One or more endometrial markers may be quantitated in a biological sample from the subject by reacting the sample with antibodies specific for one or more endometrial markers, which are directly or indirectly labeled with detectable substances and detecting the detectable substances. The endometrial markers may be quantitated or measured.

In an aspect of the invention, a method for screening a subject for endometiral cancer comprises:
(a) contacting a sample suspected of containing one or more endometrial markers associated with an endometrial disease with antibodies that specifically bind to the endometrial markers under conditions effective to bind the antibodies and form complexes;
(b) measuring the amount of endometrial markers present in the sample by quantitating the amount of the complexes; and
(c) comparing the amount of endometrial markers present in the samples with the amount of endometrial markers in a control, wherein a change or significant difference in the amount of endometrial markers in the sample compared with the amount in the control is indicative of an endometrial disease.

The method may comprise:
(a) contacting antibodies which bind to one or more endometrial markers with a sample from the individual so as to form complexes comprising the antibodies and one or more endometrial markers in the sample;
(b) determining or detecting the presence or amount of complex formation in the sample;
(c) repeating steps (a) and (b) at a point later in time; and
(d) comparing the result of step (b) with the result of step (c), wherein a difference in the amount of complex formation is indicative of disease, disease stage, and/or progression of the disease in said individual.

The amount of complexes may also be compared to a value representative of the amount of the complexes from an individual not at risk of, or afflicted with, an endometrial disease at different stages. A significant difference in complex formation may be indicative of advanced disease e.g. advanced endometrial cancer, or an unfavourable prognosis.

In the invention, the endometrial marker is chaperonin 10.

In embodiments of the methods of the invention, chaperonin 10 is detected in samples and higher levels, in particular significantly higher levels compared to a control (normal or benign) is indicative of endometrial cancer.

Antibodies may be used to detect and quantify one or more endometrial markers in a sample in order to diagnose and treat pathological states. In particular, the antibodies may be used in immunohistochemical analyses, for example, at the cellular and sub-subcellular level, to detect one or more endometrial markers, to localize them to particular endometrial cells and tissues (e.g. tumor cells and tissues), and to specific subcellular locations, and to quantitate the level of expression.

Immunohistochemical methods for the detection of antigens in tissue samples are well known in the art. For example, immunohistochemical methods are described in Taylor, Arch. Pathol. Lab. Med. 102:112 (1978). Briefly, in the context of the present invention, a tissue sample obtained from a subject suspected of having an endometrial-related problem is contacted with antibodies, preferably monoclonal antibodies recognizing one or more endometrial markers. The site at which the antibodies are bound is determined by selective staining of the sample by standard immunohistochemical procedures. The same procedure may be repeated on the same sample using other antibodies that recognize one or more endometrial markers. Alternatively, a sample may be contacted with antibodies against one or more endometrial markers simultaneously, provided that the antibodies are labeled differently or are able to bind to a different label. The tissue sample may be normal endometrial tissue, a cancer tissue or a benign tissue.

An antibody microarray in which binding sites comprise immobilized, preferably monoclonal, antibodies specific to a substantial fraction of marker-derived endometrial markers of interest can be utilized in the present invention. Antibody arrays can be prepared using methods known in the art [(see for example, Zhu et al., Science 293:2101 (2001) and reference 20].

Antibodies specific for one or more endometrial marker may be labelled with a detectable substance and localised in biological samples based upon the presence of the detectable substance. Examples of detectable substances include, but are not limited to, the following: radioisotopes (e.g.,³H, ¹⁴C, ³⁵S, ¹²⁵I, ¹³¹I), fluorescent labels (e.g., FITC, rhodamine, lanthanide phosphors), luminescent labels such as luminol; enzymatic labels (e.g., horseradish peroxidase, beta-galactosidase, luciferase, alkaline phosphatase, acetylcholinesterase), biotinyl groups (which can be detected by marked avidin e.g., streptavidin containing a fluorescent marker or enzymatic activity that can be detected by optical or colorimetric methods), predetermined polypeptide epitopes recognized by a secondary reporter (e.g., leucine zipper pair sequences, binding sites for secondary antibodies, metal binding domains, epitope tags). In some embodiments, labels are attached via spacer arms of various lengths to reduce potential steric hindrance. Antibodies may also be coupled to electron dense substances, such as ferritin or colloidal gold, which are readily visualised by electron microscopy.

One of the ways an antibody can be detectably labeled is to link it directly to an enzyme. The enzyme when later exposed to its substrate will produce a product that can be detected. Examples of detectable substances that are enzymes are horseradish peroxidase, beta-galactosidase, luciferase, alkaline phosphatase, acetylcholinesterase, malate dehydrogenase, ribonuclease, urease, catalase, glucose-6-phosphate, staphylococcal nuclease, delta-5-steriod isomerase, yeast alcohol dehydrogenase, alpha-glycerophosphate, triose phosphate isomerase, asparaginase, glucose oxidase, and acetylcholine esterase.

For increased sensitivity in an immunoassay system a fluorescence-emitting metal atom such as Eu (europium) and other lanthanides can be used. These can be attached to the desired molecule by means of metal-chelating groups such as DTPA or EDTA.

A bioluminescent compound may also be used as a detectable substance. Bioluminescence is a type of chemiluminescence found in biological systems where a catalytic protein increases the efficiency of the chemiluminescent reaction. The presence of a bioluminescent molecule is determined by detecting the presence of luminescence. Examples of bioluminescent detectable substances are luciferin, luciferase and aequorin.

Indirect methods may also be employed in which the primary antigen-antibody reaction is amplified by the introduction of a second antibody, having specificity for the antibody reactive against one or more endometrial markers. By way of example, if the antibody having specificity against one or more endometrial markers is a rabbit IgG antibody, the second antibody may be goat anti-rabbit gamma-globulin labelled with a detectable substance as described herein.

Methods for conjugating or labelling the antibodies discussed above may be readily accomplished by one of ordinary skill in the art. (See for example Inman, Methods In Enzymology, Vol. 34, Affinity Techniques, Enzyme Purification: Part B, Jakoby and Wichek (eds.), Academic Press, New York, p. 30, 1974; and Wilchek and Bayer, "The Avidin-Biotin Complex in Bioanalytical Applications,"Anal. Biochem. 171:1-32, 1988 re methods for conjugating or labelling the antibodies with enzyme or ligand binding partner).

Cytochemical techniques known in the art for localizing antigens using light and electron microscopy may be used to detect one or more endometrial markers. Generally, antibodies may be labeled with detectable substances and one or more endometrial markers may be localised in tissues and cells based upon the presence of the detectable substances.

In the context of the methods of the invention, the sample, binding agents (e.g. antibodies specific for one or more endometrial markers), or one or more endometrial markers may be immobilized on a carrier or support. Examples of suitable carriers or supports are agarose, cellulose, nitrocellulose, dextran, Sephadex, Sepharose, liposomes, carboxymethyl cellulose, polyacrylamides, polystyrene, gabbros, filter paper, magnetite, ion-exchange resin, plastic film, plastic tube, glass, polyamine-methyl vinyl-ether-maleic acid copolymer, amino acid copolymer, ethylene-maleic acid copolymer, nylon, silk, etc. The support material may have any possible configuration including spherical (e.g. bead), cylindrical (e.g. inside surface of a test tube or well, or the external surface of a rod), or flat (e.g. sheet, test strip). Thus, the carrier may be in the shape of, for example, a tube, test plate, well, beads, disc, sphere, etc. The immobilized antibody may be prepared by reacting the material with a suitable insoluble carrier using known chemical or physical methods, for example, cyanogen bromide coupling. An antibody may be indirectly immobilized using a second antibody specific for the antibody. For example, mouse antibody specific for an endometrial marker may be immobilized using sheep anti-mouse IgG Fc fragment specific antibody coated on the carrier or support.

Where a radioactive label is used as a detectable substance, one or more endometrial marker may be localized by radioautography. The results of radioautography may be quantitated by determining the density of particles in the radioautographs by various optical methods, or by counting the grains.

Time-resolved fluorometry may be used to detect a signal. For example, the method described in Christopoulos TK and Diamandis EP Anal Chem 1992:64:342-346 may be used with a conventional time-resolved fluorometer.

The endometrial marker antibodies may be directly or indirectly labelled with enzymes, substrates for the enzymes are added wherein the substrates are selected so that the substrates, or a reaction product of an enzyme and substrate, form fluorescent complexes with a lanthanide metal (e.g. europium, terbium, samarium, and dysprosium, preferably europium and terbium). A lanthanide metal is added and one or more endometrial cancer markers are quantitated in the sample by measuring fluorescence of the fluorescent complexes. Enzymes are selected based on the ability of a substrate of the enzyme, or a reaction product of the enzyme and substrate, to complex with lanthanide metals such as europium and terbium. Suitable enzymes and substrates that provide fluorescent complexes are described in U.S. Patent No. 5,3112,922 to Diamandis. Examples of suitable enzymes include alkaline phosphatase and β-galactosidase. Preferably, the enzyme is alkaline phosphatase.

Examples of enzymes and substrates for enzymes that provide such fluorescent complexes are described in U.S. Patent No. 5,312,922 to Diamandis. By way of example, when the antibody is directly or indirectly labelled with alkaline phosphatase the substrate employed in the method may be 4-methylumbelliferyl phosphate, 5-fluorosalicyl phosphate, or diflunisal phosphate. The fluorescence intensity of the complexes is typically measured using a time-resolved fluorometer e.g. a CyberFluor 615 Imunoanalyzer (Nordion International, Kanata, Ontario).

One or more endometrial marker antibodies may also be indirectly labelled with an enzyme. For example, the antibodies may be conjugated to one partner of a ligand binding pair, and the enzyme may be coupled to the other partner of the ligand binding pair. Representative examples include avidin-biotin, and riboflavin-riboflavin binding protein. In an embodiment, the antibodies are biotinylated, and the enzyme is coupled to streptavidin. In another embodiment, an antibody specific for endometrial marker antibody is labeled with an enzyme.

Endometrial markers may be determined in a sample by measuring one or more endometrial markers by immunoassay. It will be evident to a skilled artisan that a variety of immunoassay methods can be used to measure one or more endometrial markers. In general, an immunoassay method may be competitive or noncompetitive. Competitive methods typically employ an immobilized or immobilizable antibody to one or more endometrial marker and a labeled form of one or more endometrial marker. Sample endometrial markers and labeled endometrial markers compete for binding to antibodies to endometrial markers. After separation of the resulting labeled endometrial markers that have become bound to antibodies (bound fraction) from that which has remained unbound (unbound fraction), the amount of the label in either bound or unbound fraction is measured and may be correlated with the amount of endometrial markers in the test sample in any conventional manner, e.g., by comparison to a standard curve.

In an aspect, a non-competitive method is used for the determination of one or more endometrial markers, with the most common method being the "sandwich" method. In this assay, two antibodies to endometrial markers are employed. One of the antibodies to endometrial markers is directly or indirectly labeled (sometimes referred to as the "detection antibody") and the other is immobilized or immobilizable (sometimes referred to as the "capture antibody"). The capture and detection antibodies can be contacted simultaneously or sequentially with the test sample. Sequential methods can be accomplished by incubating the capture antibody with the sample, and adding the detection antibody at a predetermined time thereafter (sometimes referred to as the "forward" method); or the detection antibody can be incubated with the sample first and then the capture antibody added (sometimes referred to as the "reverse" method). After the necessary incubation(s) have occurred, to complete the assay, the capture antibody is separated from the liquid test mixture, and the label is measured in at least a portion of the separated capture antibody phase or the remainder of the liquid test mixture. Generally it is measured in the capture antibody phase since it comprises endometrial cancer markers bound by ("sandwiched" between) the capture and detection antibodies. In an embodiment, the label may be measured without separating the capture antibodies and liquid test mixture.

In a typical two-site immunometric assay for endometrial markers, one or both of the capture and detection antibodies are polyclonal antibodies or one or both of the capture and detection antibodies are monoclonal antibodies (i.e. polyclonal/polyclonal, monoclonal/monoclonal, or monoclonal/polyclonal). The label used in the detection antibody can be selected from any ofthose known conventionally in the art. The label may be an enzyme or a chemiluminescent moiety, but it can also be a radioactive isotope, a fluorophor, a detectable ligand (e.g., detectable by a secondary binding by a labeled binding partner for the ligand), and the like. In a particular aspect, the antibody is labelled with an enzyme which is detected by adding a substrate that is selected so that a reaction product of the enzyme and substrate forms fluorescent complexes. The capture antibody may be selected so that it provides a means for being separated from the remainder of the test mixture. Accordingly, the capture antibody can be introduced to the assay in an already immobilized or insoluble form, or can be in an immobilizable form, that is, a form which enables immobilization to be accomplished subsequent to introduction of the capture antibody to the assay. An immobilized capture antibody may comprise an antibody covalently or noncovalently attached to a solid phase such as a magnetic particle, a latex particle, a microtiter plate well, a bead, a cuvette, or other reaction vessel. An example of an immobilizable capture antibody is antibody which has been chemically modified with a ligand moiety, e.g., a hapten, biotin; or the like, and which can be subsequently immobilized by contact with an immobilized form of a binding partner forthe ligand, e.g., an antibody, avidin, or the like. In an embodiment, the capture antibody may be immobilized using a species specific antibody for the capture antibody that is bound to the solid phase.

The above-described immunoassay methods and formats are intended to be exemplary and are not limiting.

### Computer Systems

Analytic methods contemplated herein can be implemented by use of computer systems and methods described below and known in the art. A computer readable media may be provided comprising one or more endometrial markers, and/or polynucleotides encoding one or more endometrial markers, and optionally other markers (e.g. markers of endometrial cancer). "Computer readable media" refers to any medium that can be read and accessed directly by a computer, including but not limited to magnetic storage media, such as floppy discs, hard disc storage medium, and magnetic tape; optical storage media such as CD-ROM; electrical storage media such as RAM and ROM; and hybrids of these categories such as magnetic/optical storage media. Thus, the invention contemplates computer readable medium having recorded thereon markers identified for patients and controls.

"Recorded" refers to a process for storing information on computer readable medium. The skilled artisan can readily adopt any of the presently known methods for recording information on computer readable medium to generate manufactures comprising information on one or more endometrial markers, and optionally other markers.

A variety of data processor programs and formats can be used to store information on one or more endometrial markers, and/or polynucleotides encoding one or more endometrial markers, and other markers on computer readable medium. For example, the information can be represented in a word processing text file, formatted in commercially-available software such as WordPerfect and MicroSoft Word, or represented in the form of an ASCII file, stored in a database application, such as DB2, Sybase, Oracle, or the like. Any number of dataprocessor structuring formats (e.g., text file or database) may be adapted in order to obtain computer readable medium having recorded thereon the marker information.

By providing the marker information in computer readable form, one can routinely access the information for a variety of purposes. For example, one skilled in the art can use the information in computer readable form to compare marker information obtained during or following therapy with the information stored within the data storage means.

A medium may be for holding instructions for performing a method for determining uterine endometrial receptivity of a patient, or whether a patient has an endometrial disease (e.g. endometrial cancer) or a predisposition to an endometrial disease (e.g. cancer), comprising determining the presence or absence of one or more endometrial markers, and/or polynucleotides encoding one or more endometrial markers, and optionally other markers, and based on the presence or absence of the one or more endometrial markers, and/or polynucleotides encoding one or more endometrial markers, and optionally other markers, determining uterine endometrial receptivity, endometrial disease (e.g. cancer) or a pre-disposition to an endometrial disease (e.g. cancer), and optionally recommending a procedure or treatment.

An electronic system and/or a network may be used in a method for determining uterine endometrial receptivity of a patient, whether a subject has an endometrial disease (e.g. cancer) or a pre-disposition to an endometrial disease (e.g. cancer), the method comprising determining the presence or absence of one or more endometrial markers, and/or polynucleotides encoding one or more endometrial markers, and optionally other markers (e.g. cancer markers), and based on the presence or absence of the one or more endometrial markers, and/or polynucleotides encoding one or more endometrial markers, and optionally other markers, determining the uterine endometrial receptivity of the patient, whether the subject has an endometrial disease (e.g. cancer) or a pre-disposition to an endometrial disease (e.g. cancer), and optionally recommending a procedure or treatment.

A network may be used in a method for determining whether a subject is receptive to *in vitro* fertilization, has an endometrial disease (e.g. cancer) or a pre-disposition to an endometrial disease (e.g. cancer) the method comprising: (a) receiving phenotypic information on the subject and information on one or more endometrial markers, and/or polynucleotides encoding one or more endometrial markers, and optionally other markers associated with samples from the subject; (b) acquiring information from the network corresponding to the one or more endometrial markers, and/or polynucleotides encoding one or more endometrial markers, and optionally other markers; and (c) based on the phenotypic information and information on the one or more endometrial markers, and/or polynucleotides encoding one or more endometrial markers, and optionally other markers, determining whether the subject is receptive to *in vitro* fertilization, has an endometrial disease (e.g. cancer) or a pre-disposition to an endometrial disease (e.g. cancer); and (d) optionally recommending a procedure or treatment.

A system may be used for identifying selected records that identify a diseased endometrial cell or tissue (e.g. cancer cell or tissue) or an endometrium phase. A system generally comprises a digital computer, a database server coupled to the computer, a database coupled to the database server having data stored therein, the data comprising records of data comprising one or more endometrial markers, and/or polynucleotides encoding one or more endometrial markers, and optionally other endometrial markers, and a code mechanism for applying queries based upon a desired selection criteria to the data file in the database to produce reports of records which match the desired selection criteria.

### Kits

The method of the invention may be carried out using a kit. Kits may typically comprise two or more components required for performing a diagnostic assay. Components include but are not limited to compounds, reagents, containers, and/or equipment.

The methods described herein may be performed by utilizing pre-packaged diagnostic kits comprising one or more specific endometrial marker polynucleotide or antibody described herein, which may be conveniently used, e.g., in clinical settings to screen and diagnose patients and to screen and identify those individuals exhibiting a predisposition to developing an endometrial disease.

In an embodiment, a container with a kit comprises a binding agent as described herein. By way of example, the kit may contain antibodies or antibody fragments which bind specifically to epitopes of one or more endometrial markers and optionally other markers, antibodies against the antibodies labelled with an enzyme; and a substrate for the enzyme. The kit may also contain microtiter plate wells, standards, assay diluent, wash buffer, adhesive plate covers, and/or instructions for carrying out a method of the invention using the kit.

The kit may include antibodies or fragments of antibodies which bind specifically to an epitope of chaperonin 10 and means for detecting binding of the antibodies to their epitope associated with tumor cells, either as concentrates (including lyophilized compositions), which may be further diluted prior to use or at the concentration of use, where the vials may include one or more dosages.

A kit may be designed to detect the level of polynucleotides encoding one or more endometrial polynucleotide markers in a sample. In an embodiment, the polynucleotides encode one or more polynucleotides encoding a chaperonin 10. Such kits generally comprise at least one oligonucleotide probe or primer, as described herein, that hybridizes to a polynucleotide encoding one or more endometrial cancer markers. Such an oligonucleotide may be used, for example, within a PCR or hybridization procedure. Additional components that may be present within the kits include a second oligonucleotide and/or a diagnostic reagent or container to facilitate detection of a polynucleotide encoding one or more endometrial cancer markers.

The kit may contain a micoarray described herein ready for hybridization to target endometrial polynucleotide markers, plus software for the data analysis of the results. The software to be included with the kit comprises data analysis methods, in particular mathematical routines for marker discovery, including the calculation of correlation coefficients between clinical categories and marker expression. The software may also include mathematical routines for calculating the correlation between sample marker expression and control marker expression, using array-generated fluorescence data, to determine the clinical classification of the sample.

The kit may be used for assessing the presence of endometrial cells, wherein the kit comprises antibodies specific for one or more endometrial markers, or primers or probes for polynucleotides encoding same, and optionally probes, primers or antibodies specific for other markers associated with an endometrial disease (e.g. cancer).

The following non-limiting examples are illustrative of the present invention:

### Example I

Isotope-coded affinity tag (ICAT) analysis with a cleavable tag was used to examine differentially expressed proteins in proliferative and secretory endometria. Sample complexity of the tissue homogenates was reduced by strong cation exchange (SCX) fractionation and subsequent affinity cleanup. Analysis of ten SCX fractions in triplicate by nanobore liquid chromatography - tandem mass spectrometry (nanoLC-MS/MS) resulted in the identification of approximately 400 labeled proteins and the discovery of potential biomarkers for the secretory phase of the endometrial cycle. This study demonstrated the feasibility of using this approach for identifying markers at a proteomics level for different stages of the endometrial cycle.

### Tissue Samples

Endometrium tissue was retrieved from an in-house dedicated, research endometrial tissue bank. All tissues were snap frozen in liquid nitrogen within 15-20 minutes of devitalization at the time of hysterectomy, and were obtained with patient consent. In each case, the endometrium was classified as proliferative or secretory by a pathologist. The histological classification was verified by examination of a histopathologic section from the frozen research tissue. Tissue was taken for proteomic analysis from the mirror-face of the residual block. After addition of 1 ml Hanks' Balanced Salt Solution containing protease inhibitors (leupeptin, aprotinin, pepstatin at 1 µg/mL), the tissue was mechanically homogenized at 30,000 rpms using a Polytron PT 1300D handheld homogenizer (Brinkmann, Westbury, USA). The samples were stored in aliquots at -80°C and/or submitted for protein profiling. These whole tissue homogenates contain endometrial epithelium, supportive stroma and vessels, as well as any secretions. Tissue samples from six different individuals were selected for the study. Three of these tissues were classified as proliferative endometria (PRO1, PRO2 and PRO3) and the other three as secretory endometria (SEC1, SEC2, SEC3).

### Chemicals

Acetonitrile, formic acid, potassium chloride, monobasic potassium phosphate, leupeptin, aprotinin, pepstatin and Hanks' Balanced Salt Solution were obtained from Sigma-Aldrich (Oakville, Canada). All reagents and buffers for the cleavable ICAT sample preparation procedure were from Applied Biosystems (Foster City, USA).

### ICAT Sample Preparation Procedure

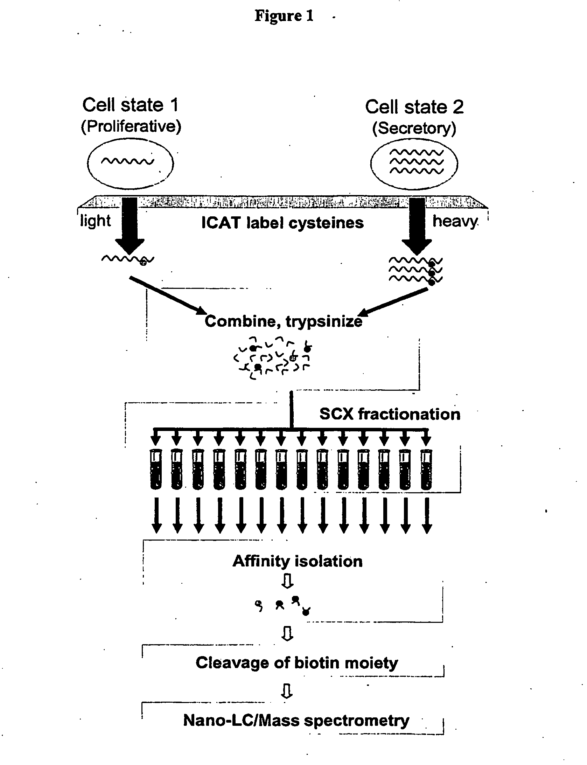

After removal of cell debris by centrifugation, the total protein content for each of the six clarified homogenates was measured using a commercially available Bradford protein assay reagent (Bio-Rad, Hercules, USA). ICAT sample preparation procedure was carried out according to the cleavable ICAT protocol (Applied Biosystems, Foster City, USA) and is illustrated in Figure 1. Following denaturing and reducing steps, 100 µg total protein of each of the samples was labeled with either the light ICAT reagent (proliferative samples) or the heavy reagent (secretory samples). The labeled PRO1 and SEC1 samples were combined to form ICAT Sample A, PRO2 and SEC2 form ICAT Sample B, and lastly PRO3 and SEC3 form ICAT Sample C. Mixing of the labeled proliferative and secretory samples in pairs in this manner ensures that any protein or peptide losses during subsequent processing steps is the same for both samples in a pair. Since the peptides are differentially labeled, they can be traced to specific samples in the pair. Any difference detected in the levels of individual peptides can then be ascribed solely to initial differences in expression level. Chromatographic separation using a strong cation exchange (SCX) column was performed after trypsin digestion to fractionate the ICAT samples. Selected SCX factions were purified by affinity chromatography according to the ICAT protocol, and subsequently analyzed by nanobore liquid chromatography-tandem mass spectrometry (nanoLC-MS/MS).

### Instrumentation for SCX fractionation

SCX fractionation was performed on an HP 1050 LC system (Agilent, Palo Alto, USA) using a 1.5 ml injection loop and a SF-2120 Super Fraction Collector (Advantec MFS, Dublin, USA).

### LC/MS/MS Instrumentation

The LC system from LC Packings (Amsterdam, The Netherlands) consisted of a Famos autosampler and Ultimate Nano LC system. It was interfaced to an API QSTAR Pulsar QqTOF mass spectrometer (Applied Biosystems/MDS Sciex, Foster City, USA) using a Protana NanoES ion source (Protana Engineering A/S, Odense, Denmark). PicoTip's SilicaTip emitters with a 10 µm tip i.d. (New Objective, Woburn, USA) were used as spray capillaries. All data were acquired using Analyst QS SP5 with Bioanalyst Extension 1.1 and analyzed with ProICAT SP2 software (Applied Biosystems/MDS Sciex, Foster City, USA).

### LC Conditions

Strong cation exchange chromatography (SCX) was performed using a PolyLC Polysulfoethyl A column (The Nest Group, Southborough, USA) equipped with a guard column of the same material with the following dimensions: 5-µm particle size, 300-A bead, 2.1-mm i.d., 10-mm length (guard column) and 100-mm length (analytical column). Eluent A of the mobile phase consisted of a 10 mM KH₂PO₄ solution in 25% acetonitrile and 75% deionized water (pH = 3.0). Eluent B consisted of a 10 mM KH₂PO₄ and 350 mM KCl solution in 25% acetonitrile and 75% deionized water (pH = 3.0). In each case, 1.5 ml of the total 2.4 ml sample (after acidifying with 2 ml Eluent A) was injected. Fractions were collected every 2 mins at a flow rate of 0.2 ml/min. using a binary gradient with the following profile:

| | | | | |
|---|---|---|---|---|
| *t*[min] | 0.01 | 2 | 58 | 60 |
| *c(Eluent B)* [%] | 0 | 0 | 100 | stop |

All reversed-phase separations were performed using PepMap C18 nano capillary columns (LC Packings, Amsterdam, The Netherlands) with the following dimensions: 3-µm particle size, 100-Å bead, 75-µm i.d. and 150-mm length. Eluent A of the mobile phase consisted of 950 ml deionized water, 50 ml acetonitrile and 1ml formic acid (pH ≈ 3). Eluent B consisted of 50 ml deionized water, 950 ml acetonitrile and 1 ml formic acid. A binary gradient at a flow rate of approximately 200 nl/min with the following profile was used:

| | | | | | | | | |
|---|---|---|---|---|---|---|---|---|
| *t* [min] | 0.01 | 5 | 125 | 135 | 157 | 160 | 163 | 190 |
| *c(Eluent B)* [%] | 5 | 5 | 30 | 60 | 80 | 80 | 5 | Stop |

Injections of 1 µl of sample were performed in full loop mode.

### MS Conditions

The source conditions were a curtain-gas setting of 20 and an ionspray voltage (in the range of 2000 - 3800 V) that was optimized daily. All data were obtained in the positive-ion detection mode. In the Q0 region, the instrument parameters were a declustering potential (DP) of 65 V and a focusing potential (FP) of 265 V. Nitrogen was used as the collision gas at a setting of CAD5 for both TOF-MS and MS/MS scans.

All LC-MS/MS data were acquired in information-dependent acquisition (IDA) mode. A TOF-MS survey scan with a mass range of m/z= 400 - 1500 and 1 s scan time was followed by two product ion scans with a mass range of m/z = 70 - 2000 and 2 s scan time. The collision energy (CE) was automatically controlled by the IDA CE Parameters script. The switching criteria were set to ions greater than m/z = 400 and smaller than m/z= 1500 with a charge state of 2 to 5 and an intensity of ≥ 10 counts/s. Former target ions were excluded for 60 s and peaks within a 4 Th window were ignored. In addition, the IDA Extensions II script was set to 2 repetitions before dynamic exclusion and to select a precursor ion nearest to a threshold of 15 count/s every 4 cycles.

### RESULTS AND DISCUSSION

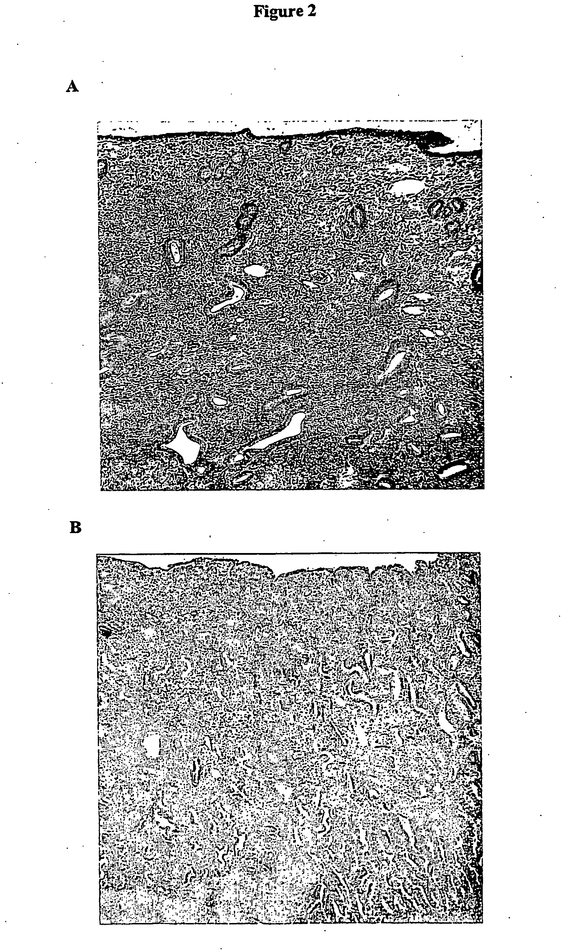

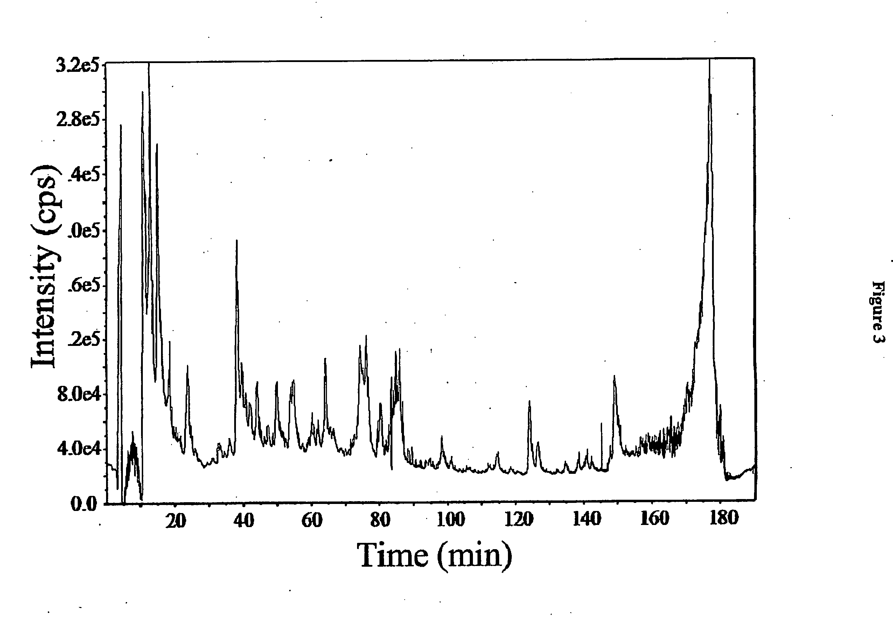

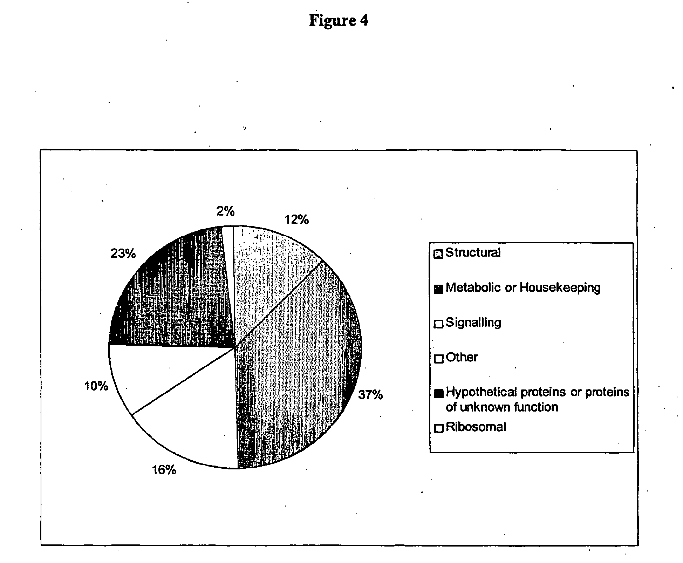

Figure 2 shows examples ofthe histologic appearance of proliferative (Figure 2A) and secretory (Figure 2B) endometrium. In both endometria the stratum basalis is characterized by a denser stroma than the physiologic responsive stratum functionalis above. Across the top of the stratum functionalis is the surface epithelium, which lines the endometrial cavity. The proliferative endometrium (PRO 2) shows small, coiled glands with lining columnar epithelium reaching to the surface. In contrast, the secretory endometrium (SEC2) is thicker, and contains more tortuous glands with intra-luminal secretions. The endometrium of both types of physiologic phases has abundant supportive stroma and vessels among the epithelial glands. In this study homogenates from the mirror faces of these tissues were used for quantitative analysis. After performing the procedure described above, SCX factions 11- 20 (of the 30 fractions) from each of the samples were chosen for further processing. This choice was based on the UV trace generated during fractionation. The 10 chosen fractions from each sample were affinity-purified, cleaved as per the ICAT protocol and analyzed using nanoLC-MS/MS. Figure 3 is an example of a nano LC-MS total ion chromatogram (TIC) from one of the ICAT Sample A SCX fraction. As the samples were run in IDA mode (See above), each such TIC resulted in hundreds of MS/MS spectra. A ProICAT confidence value of 75 was adopted after trial-and-error for reliable protein detection and identification for this initial investigation. This has resulted in identification of approximately 400 distinct proteins. A preliminary classification of these proteins based on function, is seen in Figure 4. As expected the majority of proteins fall under one of the metabolic, housekeeping or structural categories. The proteins classified under "other" are proteins like antibodies, which could not be included with the previously mentioned categories. There were also a significant number of proteins for which function could not be assigned or were identified from cDNA matches and are therefore classified as hypothetical. This last category often contains the most interesting cases for biologists. Adopting a less stringent confidence value resulted in apparent identification of many more proteins; the reliability of such identifications, however, was judged much poorer after manual inspection. In addition, manual inspection of the spectra was also found necessary to confirm automated quantifications in ProICAT.

The results show that expression levels of the majority of proteins identified were not consistently different between the two phases of the endometrial cycle. This similarity in expression levels of proteins is not surprising since most of the abundant proteins detected tend to be housekeeping or structural in nature. In addition, similar or identical, housekeeping or structural proteins such as actin and tubulin are expressed in many tissue types. This is significant since whole tissue homogenates consist not only of epithelial cells, but also supportive stromal cells and interstitium, blood, vessels and any glandular secretions. These sources provide a major contribution to the protein profile ofthe overall tissue. These high abundance proteins from these adjacent cellular types may mask differential protein expression by a cellular component of the endometrium. Thirdly the differential expression of low abundance proteins in any one cellular type within proliferative and secretory endometrium may not have been detectable by the methodology used in this study.

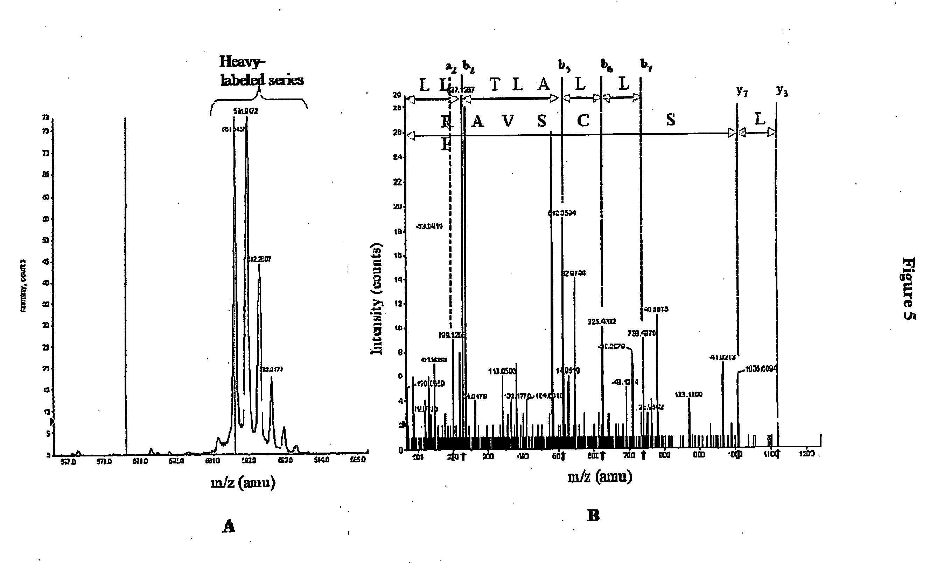

Despite these limitations, some instances of differential protein expression was noted. One such example is given in Figure 5, which shows very significant enhancement of expression of a protein, identified as glutamate receptor subunit zeta 1 precursor [SEQ ID NO. 26], in all three secretory samples. The triply charged series of peaks, starting at 581.6 Th, was MS/MS-analyzed and identified as the heavy-labeled version of the tryptic peptide LLTLALLFSCSVAR [SEQ ID NO. 28], which maps to the N-terminal region of the protein. The absence of the light-labeled analogue of this protein, which would have manifested as a series of peaks starting at 578.6 Th, suggests a significantly lower level of the same protein in the proliferative samples. This is the first evidence of this protein being a marker for the endometrial secretory phase.

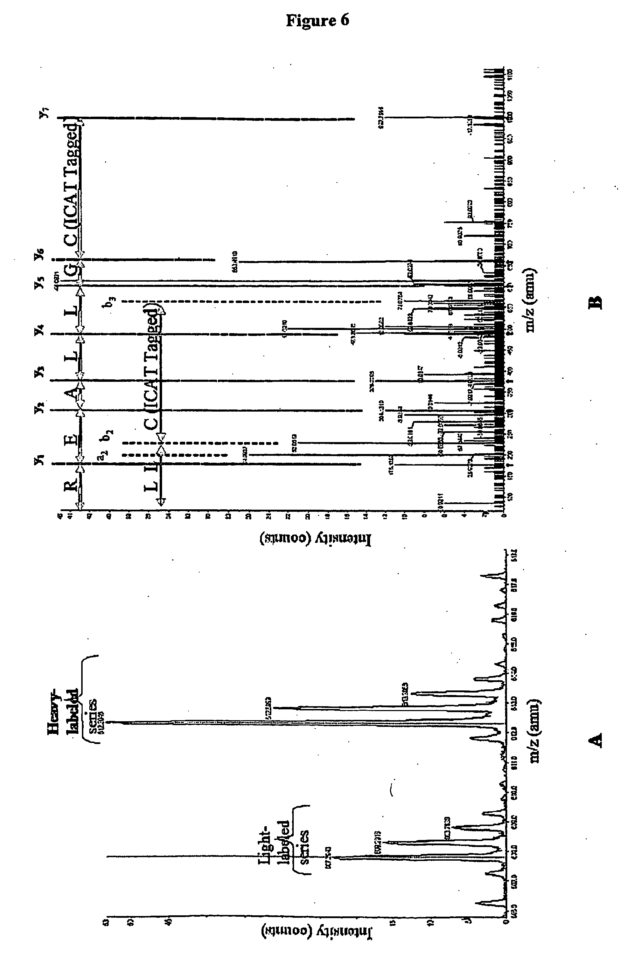

A second example is shown in Figure 6 for the protein, macrophage migration inhibitory factor (MIF). Again, the protein's expression is enhanced in the secretory endometrium (1.71 ± 0.38 times) versus the proliferative endometrium. This relative quantification is based on the three sets of samples and is calculated from the ratios of the total area of the ion peaks within the heavy-labeled series to that ofthe corresponding light-labeled series. Previous studies have demonstrated that MIF is expressed by the human endometrium throughout the menstrual cycle and that this expression is predominantly in the glandular epithelial cells [9]. It was found that MIF localized throughout the glandular epithelial cytoplasm in the proliferative phase, but that this distribution changed during the secretory phase, when it localized to the apical portion ofthe glandular epithelial cells, and was also detected in glandular secretions. Macrophages are common in female reproductive tissues. In the endometrium, they play an important role in defense. Macrophage degradation of cellular debris and foreign material may play an important role in endometrial shedding and repair. Quantification of MIF levels using ELISA assays suggested a slight increase in the mean concentration in the secretory phase (from approximately 15 to 18 ng / mg of protein based on a total of 25 samples), although the increase was not statistically significant [9]. By contrast, ICAT analysis of the six-sample set shows a significant enhancement (1.71 ± 0.38 times) of MIF expression in the secretory endometrium. The results of this study serve to illustrate the power of the ICAT method for detecting and quantifying gross as well as subtle differences in expression levels over traditional quantitative methods relied on thus far.

### CONCLUSIONS

The ICAT technology employing the new, cleavable ICAT reagent is a powerful tool that can be used for discovering differentially expressed proteins, which could potentially be significant biomarkers of different histological cell states. The results demonstrate that for the human endometrium the expression levels of the majority of proteins do not vary significantly between the proliferative and the secretory phases. Two examples of differentially expressed proteins have been discussed in this study. Further investigation of the remainder of the SCX fractions from each of the three pairs of samples can be expected to yield more markers some of which might have implications for fertility/ infertility.

### Example 2

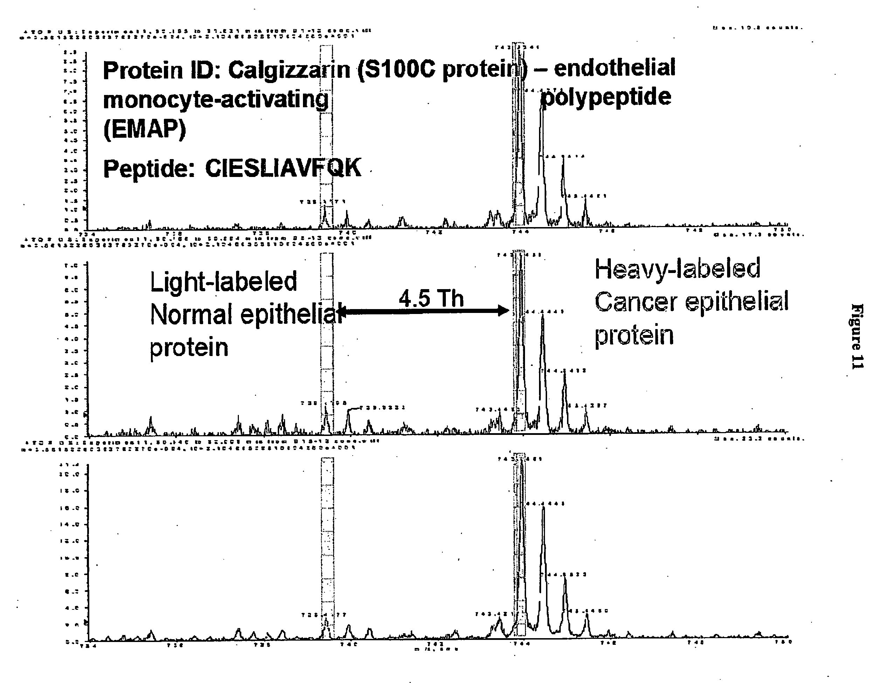

The ICAT analysis as described in Example 1 was used to examine differentially expressed proteins in cancer and normal endometrial tissue. The results of the anaylsis are illustrated in Figure 11 which shows mass spectral windows from the ICAT experiments for three pairs of endometrial cancer/ normal samples, demonstrating the over expression of calgizzarin in the cancer samples.

### Example 3

### Material and Methods:

### i) Tissue preparation and histologic classification

Endometrium and endometrial cancer tissues were retrieved from a dedicated, research in-house endometrial tissue bank. The consenting and tissue banking procedures for this tissue bank were approved by the relevant institutions. All tissues had been snap frozen in liquid nitrogen within 15-20 minutes of devitalization at the time of hysterectomy, and were obtained with patient consent. In each case, the endometrium was classified as non-malignant, or malignant by a pathologist. (26). Non-malignant endometrial cases included both normal physiologic states (atrophic, proliferative, secretory, menstrual) and pathologic states (benign endometrial polyp, disordered proliferative). Malignant endometrial cases included endometrioid, mucinous, and serous adenocarcinomas and malignant mixed Mullerian tumors (carcinosarcomas). This classification was performed using the routine surgical pathology sections. Study cases included only benign or malignant cases; cases of endometrial hyperplasia, some of which could be considered to represent an intermediate phenotype, were not included in this study. The histologic classification was verified by examination of a histopathologic section from the frozen research tissue. Tissue was taken for proteomic analysis from the mirror-face of the residual block.

Tissue was thawed in Hanks' balanced salt solution (HBSS, Sigma) containing protease inhibitors (leupeptine, aprotinin, pepstatin in I µg/mL) and followed by mechanical homogenation. The specimens were then stored in aliquots at -80°C and/or submitted for protein profiling. These whole tissue homogenates contain both endometrial epithelium or carcinoma, supportive stroma and vessels, and any secretions.

Immunohistochemical staining of selected malignant endometrial tissue was done using a polyclonal (rabbit) antibody against the putative tumor marker available from Calbiochem (San Diego, CA). Sections were cut from the paraffin embedded tissue, antibody applied in a 1:2000 dilution in Universal Strepavidin System, and immunohistochemical completed using a diaminobenzidine (DAB) chromogen.

### ii) Protein profiling

Tissue lysate was fractionated to reduce the sample complexity before protein profiling. An identical quantity of proteins was used for all samples within a method; HBSS was used to compensate the initial volume to ensure equal volumes for all samples. For C18 Zip-tip (Millipore) fractionation, 2 µg of proteins from endometrial tissue homogenate in the presence of 0.3% trifluroacetic acid (TFA) were loaded. After washing with water containing 0.3% TFA, 1 µL of 60% acetonitrile with 0.3% TFA were used to elute proteins from C18 directly onto a MALDI target containing pre-dried 1µL 10 mg/mL sinapinic acid in 60% acetonitrile. The dried protein spots were analyzed by a MALDI-TOF (Voyager DE-STR, Applied Biosystems) mass spectrometer.

For protein profiling using SELDI-TOF MS, 1 µg proteins from endometrial tissue homogenate were incubated with WCX2, SAX2, IMAC, H50 surfaces according to the manufacturer's instructions. In brief, samples were diluted to 55 µL with the corresponding binding buffer, spotted onto the appropriate ProteinChip surface, and incubated in a sealed BioProcessor for one hour at room temperature. The ProteinChip surface was washed twice with the appropriate buffer for five-minutes, briefly rinsed with water and air-dried. Two times 0.5 µL of 50% saturated sinapinic acid in 50% acetonitrile was applied on the samples to form crystals. The ProteinChips were analyzed using a linear TOF analyzer, PBSIIc (Protein Biology System IIc, Ciphergen), or a quadrupole/TOF hybrid tandem mass spectrometer, QSTAR XL (Applied Biosystems/MDS Sciex).

### iii) Protein purification and identification

A normal and an EmCa sample were subject to chromatographic separation in parallel to yield partially purified protein for identification. 500 µg proteins from the whole tissue homogenate were fractionated using size exclusion (BioSep 2000, Phenomenex) at 1mL/min flow with phosphate buffer (pH7.9) and 0.05% (w/v) sodium azide. One-millilitre fractions were collected; the eluates were then concentrated to 50 µL with a silicon carbide-based spincolumn, (ProteoSpin, MDS Sciex). Five microlitres (10%) of concentrate was desalted by C 18 zip-tip and analyzed with MALDI-TOF MS to locate the fractions containing the protein marker of interest (10,843 Da) in the EmCa sample. The fractions with the enriched 10,843 Da protein were diluted to 100 µL. with freshly prepared dithiothreitol (DTT) (5 mM final) in 150 mM Tris pH 8.5 buffer, and incubated at 60 Cfor one hour. Ten microlitres (10%) of the reaction mixture was desalted by C18 zip-tip and analyzed with MALDI-TOF MS to assess the effect of DTT on the protein of interest (see Result for detail). The remaining 90 µL was precipitated by acetone (80%(v/v) final), resuspended in SDS sample buffer, and the proteins were resolved by sodium dodecyl sulfate polyacrylamide gel electrophoresis (SDS-PAGE). Protein molecular weight markers (New England Biolabs) and cytochrome C (C-2506 Sigma) were included to guide the excision of gel portions containing the protein of interest. Intact proteins were extracted from the gel by 50 µL extraction solution (formic acid/acetonitrile/isopropanol/water in a ratio of 50/25/15/10) at room temperature for four hours. The extracts were completely dried by SpeedVac and resuspended in 40 µL 100 mM ammonium bicarbonate. Halfof the resuspended proteins was desalted by C18 zip-tip and analyzed with MALDI-TOF, the other half was digested in solution with 100 ng trypsin (Promega). The resulted tryptic peptides were analyzed by MALDI-QqTOF MS. The identity of the 10,843Da protein, chaperonin 10, was determined using amino acid sequence-tag analysis (Mascot, Matrix Science).

Identification of chaperonin 10 was verified by western blot, 12 µg of proteins was resolved by 8-16% gradient SDS-PAGE and electrophoretically transferred to nitrocellulose membrane (MSI). Membranes were incubated for two hours with 1:1000 dilution of anti-chaperonin 10 antiserum (Stressgen) or ERK I antibodies (Santa Cruz) diluted in 5% milk with 0.2% NP-40 and one hour with secondary antibodies. Chaperonin 10 and ERK1 were detected by chemiluminescence reagent (NEN) and X-ray film according to the manufacturer's instructions.

### Results:

A total of 44 malignant and non-malignant endometrial tissue samples were submitted for proteomic analysis. Twenty-three of these cases were non-malignant, and the remaining 21 cases were malignant. The exact histopathologic diagnoses are shown in Tables 2 and 3.

### Protein profiling

Both C18 zip-tip purification and retentive separation on ProteinChip WCX2 were effective in generating protein profiles that permit differentiation between normal and tumorous epithelium samples. Distinguishing features include both appearances and disappearances of proteins. One protein at approximately 10,840 ± 10 Da was present in all EmCa samples and absent or greatly diminished in all normal samples tested. Figure 7 shows the mass spectra obtained on the ProteinChip WCX2 using (a) the linear TOF mass spectrometer, PBSIIc, and (b) the QqTOF mass spectrometer, QSTAR XL. The superior mass accuracy and resolution of the latter afforded determination of the molecular weight of the marker protein as 10,843 Da.

### Marker protein purification and identification

As detailed above and outlined in Figure **8** size-exclusion LC was employed to fractionate the tissue homogeneates. The target 10,843 Da protein was found to elute in one of the early fractions from size exclusion column which suggests that this protein is a part of a large protein complex (Figure 8A). After concentration on the spin column, the proteins were treated with DTT to break the tertiary structure and reduce any disulfide bonds. The molecular weight of the target protein was verified to remain as 10,843 Da after DTT treatment and before SDS-PAGE (Figure 8B). This result suggests that the 10,843 Da target protein contains no intra- or inter- polypeptide disulfide bonds. The remaining protein concentrates were further separated by SDS-PAGE, and the gel was stained with colloidal Coomassie Blue (Figure 8C). There were, however, no visible bands at the region around 10-11 kDa, probably a consequence of low protein concentration and relatively low sensitivity of the Coomassie stain. The gel portions covering approximate 7,000-16,000 Da were excised as guided by the molecular weight markers (Figure 8C). After protein extraction, digestion with trypsin and MALDI analysis, six "unique" tryptic peptides were detected in the EmCa sample vs. the control (Figure 8D). All six peptides were sequenced by MALDI-QqTOF MS; three were traced to keratin and three to chaperonin 10 (Figure 8D and 8E). The average molecular weight of chaperonin 10 was calculated to be 10842.5 Da after considering two putative posttranslational modifications, the removal ofthe N-terminal methionine and acetylation ofthe alanine residue. This is consistent with the measurement of the target protein and the reported molecular weight of chaperonin 10 purified from human platelet (18).

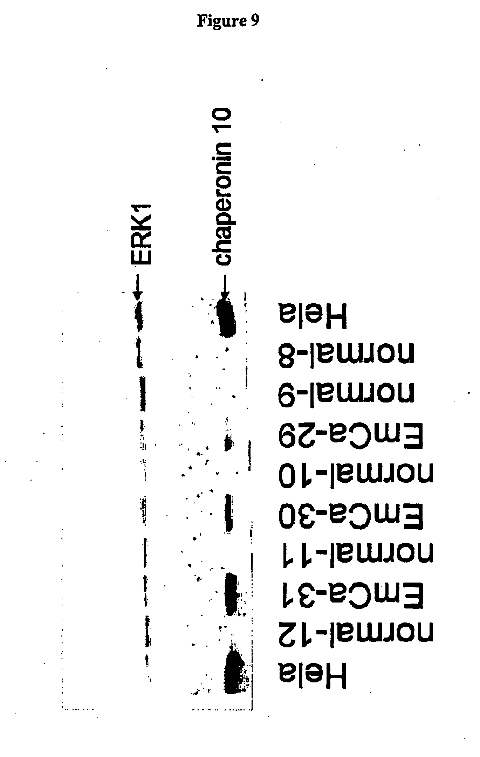

The differential expression of chaperonin 10 among cancerous and normal tissue was re-tested by western blot analysis. The signal of chaperonin 10 is higher in all EmCa specimens that also display a relative high 10,843 Da peak in their corresponding protein profiles (Figure 9).

Tables 2 and 3 summarizes the results in identifying chaperonin 10 by MS and western blot analyses in non-malignant and malignant endometrial tissue respectively. The results for both MS and western blotting have been reported using a semi-quantitative system ranging from absent (0) to 5+ (for high intensity). These two independent methodologies demonstrate consistency in the detection of chaperonin 10.

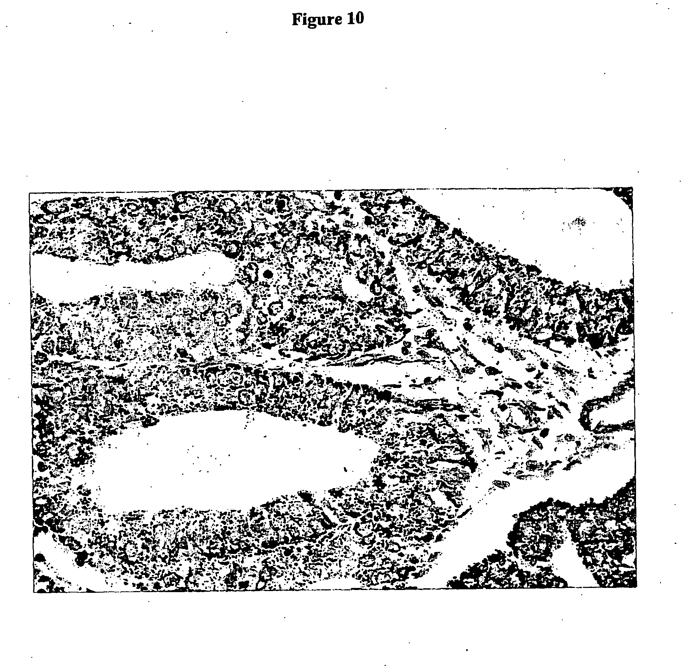

Moderately strong immunohistochemical staining for chaperonin 10 was noted in the cytoplasm of an endometrioid adenocarcinoma, as compared to adjacent stroma and benign endometrial gland. (Figure 10). This result demonstrates the association of chaperonin 10 with malignant endometrial tissues, but much less with normal endometrial epithelium or supportive stroma and vessels, which were also present in the whole tissue homogeneates.

### Discussion:

Genomic studies of endometrial carcinoma have revealed that there are two, and possibly three, types of endometrial carcinoma that are each characterized by multistep pathogenetic pathways characterized by different molecular profiles [28]. A wide variety of genetic and enzymatic markers characterize the initiation, promotion and progression toward each type of endometrial carcinoma. Large-scale messenger RNA expression analysis of the endometrioid type of endometrial carcinoma has identified 50 genes that are capable of discriminating normal from malignant endometrial tissues [29]. Many genes that are constitutively expressed in normal endometrium show either diminished or increased expression in endometrial carcinomas. In addition, there is aberrant expression of the one hundred hormonally regulated genes that are variably expressed in normal endometrial tissues, although finally endometrial carcinoma resembles proliferative endometrium more than secretory endometrium [29].

In contrast to the genomic studies of the endometrium, no studies of the endometrial proteome are available, even though proteome analysis may offer information about protein expression, functions, and modifications which might not be fully reflected by gene expression analysis. Thus, protein expression profiling of the endometrium offers a new opportunity to identify and classify endometrial phenotypes, including carcinoma. Among the available methodologies for protein profiling, SELDI-TOF based method has the advantage ofrequiring only miniscule samples and high throughput capability, although the determination of any protein identity requires much more work [30].

This proteomic study of endometrium used lysates of whole tissue homogenates from both control endometrial tissues and endometrial carcinomas. Such tissues include not only the epithelial cells of interest, but also supportive stroma (including both endometrial stromal cells or fibroblasts and extracellular matrix), blood vessels (including smooth muscle cells and endothelium), any secretions, and possibly small amounts of adjacent myometrium. While the use of such whole tissue homogenates is technically straight forward, it does have inherent limitations. The heterogeneous nature of the constituent tissue will lead to a similarly heterogeneous protein profiling [17]. The resultant proteomic analysis reflects not only the cells of interest, but also the presence of contaminating cells [15, 17,31]. Successful proteomic analysis of some types of tumors (e.g. hepatoma) may still be productive even with the limitation of whole tissue homogenization, since there is an abundance oftumor cells and minimal associated contaminating stroma [32].

In some tumor types, the inherent limitation of whole tissue homogenates must be surmounted by utilizing cell purification techniques. In the endometrium LCM may be optimal in achieving cellular purification for proteomic analysis since there is a relative abundance of stroma in endometrial carcinoma tissues [32]. Studies using laser capture microdissection (LCM) of melanoma have clearly revealed a different protein profile for melanoma than that of the surrounding epithelium, and thus supports the use of LCM in proteomic profiling studies [21, 33-35].

Despite the limitations of using whole tissue homogenates for protein expression profiling, this preliminary study of endometrial carcinoma has shown that proteomic analysis of endometrial carcinoma can detect differences from that of normal endometrium. Furthermore, a specific protein (Chaperonin 10) was strongly associated with endometrial carcinoma cases. This potential marker might be clinically useful on tissue aspirates since its differential expression pattern can be detected without the LCM procedure.

Chaperonin 10 was not identified exclusively in malignant endometrial tissues; low levels were detected in non-malignant endometrial tissues by both mass spectrometry and western blotting techniques (Table 2). Furthermore, there is no apparent association between any particular histopathologic classification and the detection of these low levels of chaperonin 10. In contrast, high levels of chaperonin 10 were detected in 17 of 22 malignant endometrial tissues by either mass spectrometry and/or western blotting techniques (Table 3). The apparent absence of chaperon in 10 in the remaining five of the 22 malignant cases may be due to either true absence or technical factors in pre-analytic processing or proteomic analysis. In two of these five cases, reexamination of the corresponding mirror image histologic section revealed minimal tumor in one case (case 28), or abundant necrosis oftumor (case 44). Furthermore, specific protein peaks of interest may be obscured in less-than-optimal mass spectromectric analysis or by adjacent protein peaks.

Chaperonin 10 (Cpn 10) is a heat shock protein (HSP) that functions intra-cellularly as a molecular chaperone for nascent proteins [36, 37]. HSP's are ubiquitous intra-cellular proteins that ensure homeostasis of metabolism [37]. Aberrations of HSP function, including chaperon in 10, have been described in a variety of pathologic conditions, including neoplasia [37, 38]. Furthermore, HSP's are differentially expressed in a variety of neoplasms. For example, immunohistochemical studies of both primary and secondary brain tumours have shown production of other HSP's [39], and that the expression of some HSP's may depend upon proliferating potential. Furthermore, the modulation of HSP expression profile has been shown to reflect the stage of prostatic carcinoma [37].

Immunohistochemical studies have identified HSP's within the nuclei and cytoplasm of epithelium, stroma, endothelium, and lymphocytes of the endometrium, with certain types of HSP's showing preferential localization to certain cell types [40]. Whereas the expression of some HSP's occurs independent of the stage of the endometrial cycle (e.g. HSP90), the expression of other HSP's is cycle dependent. The expression of chaperonin 10 can be determined among proliferative, secretory, and menstrual endometria using proteomic, western blotting, and immunohistochemical methods. It is known that the amount of HSP27 and 60 is increased during late proliferative and early secretory phases, and subsequently reduced during mid- and late secretory and menstrual phases [40]. Studies of both decidualized endometrium (decidua) and placenta have shown that there are striking differences in the cellular localization of HSP's during normal human gestation.

There have been no studies regarding the role ofHSP's or Cpn10 in endometrial carcinoma. The eutopic endometrial glands from women with endometriosis and adenomyosis shows significantly increased expression of HSP's as compared to endometria from control women - regardless of the menstrual phase [41]. The abnormal expression of HSP's may play a role in the pathophysiology of both endometriosis and adenomyosis.

Early pregnancy factor (EPF) is an extra-cellular homologue of Cpn 10 that appears within 24 hours of fertilization and persists throughout the first half of gestation [36]. It is necessary for embryonic development [42-44] and is immunosuppressive [45]. EPF is also detectable in animal models of liver regeneration and in the development ofcancer [38, 46, 47]. An association between cellular growth and the appearance of extracellular EPF has been shown [38, 46, 47]. These findings suggest a role for EPF in neoplastic growth and that the detection of Cpn 10 in the serum of endometrial carcinoma has diagnostic potential.

In conclusion, this protein profiling study of non-malignant and malignant endometrial tissues has identified chaperonin 10 as a tumor marker for endometrial malignancies.

### Example 4

The work described in the previous example using solid-phase extraction followed by matrix-assisted laser desorption/ionization mass spectrometry (MALDI MS) as well as selective surface binding and surface-enhanced laser desorption/ionization (SELDI MS, Ciphergen Biosystems Inc, CA, USA) has succeeded in identifying individual markers that show significant enhanced expression in EmCa tissues. The MALDI/SELDI MS strategy relies on side-by-side comparison of spectra from proteins that have been selected via fast separation [120-124]. Using this methodology the identities of the proteins are unknown and identifying a given protein marker typically involves offline multidimensional chromatography, concentration, trypsin digestion and MS/MS. A second available strategy that highlights differentially expressed proteins involves differential tagging of proteins from samples that are being compared using isotope-coded affinity tag (ICAT) in an isotope-dilution mass spectrometry experiment [6]. This strategy has recently been applied to discover differentially expressed proteins between the proliferative and secretory phases of the human endometrium [49] using a cleavable, second-generation ICAT reagent (cICAT, Applied Biosystems Inc, CA, USA) (see above). Recently, a variation of the ICAT technology, iTRAQ (also from the Applied Biosystems Inc, CA, USA), has been introduced. Both cICAT and iTRAQ tagging permit online identification of multiple markers and relative quantification of these proteins. Although similar in their basic concepts, the two tagging reagents and methodologies differ in significant areas. The cICAT method relies on tagging cysteine residues and isolating peptides containing these tagged residues by affinity chromatography. The net result is a reduction in the complexity of peptide pools generated by digestion with proteases including trypsin [6]. In the case of the new iTRAQ method, tagging is on primary amines. This difference in labeling strategy eliminates the dependence on relatively nonabundant cysteine containing peptides intrinsic to ICAT-based methods, thus potentially allowing the tagging of most tryptic peptides. Other noteworthy features of the iTRAQ technology are that relative quantification is performed via MS/MS and that there are four possible tags, which permit multiplexing of up to four samples (tissue states) in a single experiment. Quantification is performed via the differences in abundances of four product ions, 114, 115, 116 and 117 Th that are each cleaved from one of the four possible tags. The tags have an identical mass, a result of differences in other parts of the iTRAQ tag structure, with the consequence that an identical peptide in the four samples will have an identical mass and LC retention time after tagging. This strategy simplifies analysis and will potentially increase analytical accuracy and precision. The multi-sample capability of the iTRAQ technology is ideally suited for this study, as it now provides a means to perform a proteomic analysis of both the major phases of the normal endometrium, while simultaneously comparing them against cancer samples.

This example describes a feasibility study that compares protein expression profiles between normal and cancerous endometria using iTRAQ as well as contrasts it against a similar feasibility study using the cICAT technology. Both iTRAQ and cICAT labeling afforded discovery of a number of differentially expressed proteins that are potential cancer markers (PCMs). There is little overlap in the PCMs discovered and identified, thus pointing to the complementary nature of the two technologies. It is noteworthy that application of the iTRAQ methodology permits confirmation of the overexpression of chaperonin 10 in EmCa tissues [50]; chaperonin 10 is an approximately 10 kDa heat shock protein that does not contain the cysteine residue, which is the tagging site for cICAT. Thus, in addition to an interest in the discovery of markers for endometrial carcinoma, these experiments have also helped to illustrate the relative strengths and differences of the two tagging techniques, when applied to studies of clinical samples.

### Materials and Methods

***Sample Preparation.*** Endometrial tissue was retrieved from an in-house dedicated, research endometrial tissue bank. All tissues were snap frozen in liquid nitrogen within 15-20 minutes of devitalization at the time of hysterectomy, and were obtained with patient consent. The patient consent forms and tissue-banking procedures were approved by the Research Ethics Boards of York University, Mount Sinai Hospital, University Health Network, and North York General Hospital. In each case, the endometrium was classified by a pathologist (TJC). Histological classification was verified by examination of a section from the frozen research tissue. Tissue for proteomic analysis was taken from the mirror-face of the residual block. In the case of samples used for iTRAQ analysis, 0.5 ml phosphate buffered saline (PBS) containing protease inhibitors (1mM AEBSF, 10 µM leupeptin, 1 µg/ml aprotinin and 1 µM pepstatin) was added. The tissue was then mechanically homogenized at 30,000 rpm using a Polytron PT 1300D handheld homogenizer (Brinkmann, Westbury, USA). For samples used for cICAT analysis, tissues were similarly homogenized in 1 ml Hanks' Balanced Salt Solution with the same concentration of protease inhibitors as listed above. The samples were stored in aliquots at -70°C until used for further processing. These whole tissue homogenates contained not only endometrial epithelium, but supportive stroma and vessels, as well as secretions. The iTRAQ analysis involved one normal proliferative, one normal secretory, and two cancer homogenates, while the cICAT analysis combined one normal proliferative homogenate with three different cancer homogenates in pair-wise comparisons.

***Chemicals.*** Reagent grade chemicals were purchased from Sigma Aldrich (Oakville, ON, Canada), or Fisher Scientific (Nepean, ON, Canada). All iTRAQ and cICAT reagents and buffers were obtained from Applied Biosystems (Foster City, CA, USA).

***iTRAQ Sample Preparation Procedure.*** Cell debris from each of the homogenates was removed by centrifugation in a microfuge at 4°C for 30 min at 14,000 rpm. The clarified supernatant was then transferred to fresh microfuge tubes and the total protein content determined using a commercial Bradford assay reagent (Bio-Rad, Mississauga, ON, Canada). A standard curve for the Bradford assay was made using γ-globulin as a control. 100 µg of each sample was then denatured and the cysteines blocked as described in the iTRAQ protocol (Applied Biosystems, Foster City, CA, USA). Each sample was then digested with 0.2 mL of a 50 µg/mL trypsin (Promega) solution at 37 °C overnight and labeled with the iTRAQ tags as follows: normal proliferative endometrium, iTRAQ114; normal secretory endometrium, iTRAQ 115; and the two EmCa samples, iTRAQI 16 and iTRAQ117. The labeled samples were then pooled and acidified by mixing with Eluent A (see later) to a total volume of 2.0 mL for strong cation exchange (SCX) chromatography. 1.5 mL of this acidified labeled sample was injected into an HP 1050 LC system (Agilent, Palo Alto, CA, USA) with a 1.5 mL injection loop and a 2.1 mm internal diameter (ID) x 100 mm length PolyLC Polysulfoethyl A column packed with 5 µm beads with 300 Å pores (The Nest Group, Southborough, MA, USA). A 2.1 mm ID x 10 mm length guard column of the same material was plumbed upstream from the analytical column. Fractionation was effected by a binary mobile-phase gradient at a total flow rate of 0.2 mL/min. Eluent A consisted of a 10 mM KH₂PO₄ solution in 25% acetonitrile and 75% deionized water acidified to a pH of 3.0 with phosphoric acid. Eluent B consisted of 10 mM KH₂PO₄ and 350 mM KCl solution in 25% acetonitrile and 75% deionized water acidified to a pH of 3.0 with phosphoric acid. Initially, the gradient comprised 100% Eluent A. At the 2^{nd} minute, the % Eluent B was changed linearly from 0 to 100% at the 58^{th} min. The run was terminated at the 60^{th} min. A total of 30 fractions were collected for the sample, one every two minutes, using an SF-2120 Super Fraction Collector (Advantec MFS, Dublin, CA, USA). Following fractionation, the samples were dried by speed vacuuming and stored at -20 °C. Prior to reverse phase nanobore liquid chromatography-tandem mass spectrometric (nanoLC MS/MS) analysis, these fractions were redissolved in an aqueous buffer containing 5% acetonitrile and 0.1% formic acid for nanoLC MS/MS.

***cICAT Sample Preparation Procedure**.* Samples were clarified and their total protein content determined as described above. cICAT sample preparation procedure was carried out according to the cleavable ICAT protocol (Applied Biosystems, Foster City, CA, USA). Three 100 µg aliquots of the clarified normal proliferative homogenate were paired with 100 µg each of the three clarified cancer homogenates separately. After denaturing and reduction, the normal homogenates were labeled with the light reagent while the EmCa homogenates were labeled with the heavy reagent. A light labeled sample was then mixed with one ofthe heavy labeled samples to form in total three ICAT sample pairs: A, B and C. The final volume of each sample was 0.2 mL. The sample pairs were then digested by incubating each pair with 0.2 mL of a 50 µg/mL trypsin solution at 37°C oversight Afterwards each sample pair was mixed with 2.0 mL of Eluent A and fractionated into 30 fractions using SCX chromatography as described above. The fractions were screened by MALDI MS analysis. Those showing the signature ICAT peak pairs separated by 9 Da were further processed by affinity purification using the avidin cartridge provided with the ICAT kit. The affinity-purified sample was then dried, treated with the cleavage reagent to eliminate biotin, and dried again by speed vacuuming. The resulting solids were re-dissolved in an aqueous buffer containing 5% acetonitrile and 0.1 % formic acid for nanoLC MS/MS.

***Nanobore LC*/*MS*/*****MS**.* The nanobore LC system was from LC Packings (Amsterdam, The Netherlands) and consisted of a Famos autosampler and an Ultimate Nano LC system. It was interfaced to an API QSTAR Pulsar QqTOF mass spectrometer (Applied Biosystems/MDS Sciex, Foster City, CA, USA) using a Protana NanoES ion source (Protana Engineering A/S, Odense, Denmark). The spray capillary was a PicoTip SilicaTip emitter with a 10 µm ID tip (New Objective, Woburn, MA, USA). The nanobore LC column was a 75 µm ID x 150 mm length reverse-phase PepMap C 18 nano capillary column (LC Packings, Amsterdam, The Netherlands) packed with 3 µm beads with 100 Å pores. One µL of sample was injected via the full-loop mode. Separation was performed using a binary mobile-phase gradient at a total flow rate of 200 nL/min. Eluent A consisted of 94.9% deionized water, 5.0% acetonitrile and 0.1% formic acid (pH ≈ 3). Eluent B consisted of 5.0% deionized water, 94.9% acetonitrile and 0.1% formic acid.

The following binary gradient was used for the iTRAQ experiments:

| | | | | | | | | | |
|---|---|---|---|---|---|---|---|---|---|
| *Time (min)* | 0 | 5 | 10 | 125 | 135 | 155 | 160 | 162 | 188 |
| *% Eluent B* | 5 | 5 | 15 | 35 | 60 | 80 | 80 | 5 | Stop |

While the binary gradient used for cICAT experiments was:

| | | | | | | | | |
|---|---|---|---|---|---|---|---|---|
| *Time (min)* | 0 | 5 | 125 | 135 | 157 | 160 | 163 | 190 |
| *% Eluent B* | 5 | 5 | 30 | 60 | 80 | 80 | 5 | Stop |

For nanospray, the source conditions were a curtain-gas setting of 20 and an ionspray voltage in the range of 1800 - 3000 V that was optimized daily. In the Q0 region, the instrument parameters were a declustering potential (DP) of 65 V and a focusing potential (FP) of 265 V. Nitrogen was used as the collision gas at a setting of CAD = 5 for both TOF-MS and MS/MS scans. All nanoLC MS/MS data were acquired in information-dependent acquisition (IDA) mode in Analyst QS SP8 with Bioanalyst Extension 1.1 (Applied Biosystems / MDS Sciex). Two sets of runs for the iTRAQ fractions were performed. For the first set, MS cycles comprised a TOF MS survey scan with an m/z range of 400 -1500 Th for 2 s, followed by three product ion scans with an m/z range of 70 - 2000 Th for 10 s each. For the second, a 1 s TOF MS survey scan, followed by three product ion scans of 3 s each were used. For cICAT experiments, the MS cycles consisted of a TOF MS survey scan for 1 s, followed by two product ion scans of 2 s each. The ranges for the TOF MS and product ion scans were the same as those of the iTRAQ experiments. Collision energy (CE) was automatically controlled by the IDA CE Parameters script. Switching criteria were set to ions greater than m/z = 400. Th and smaller than m/z = 1500 Th with a charge state of 2 to 5 and an abundance of ≥10 counts/s. Former target ions were excluded for 60 s and ions within a 4 Th window were ignored. In addition, for iTRAQ runs, the IDA Extensions **II** script was set to no repetitions before dynamic exclusion for the first run, and one repetition for the second. While for the ICAT experiments, the IDA Extensions II script was set to two repetitions before dynamic exclusion. In all three experiments, the script was set to select a precursor ion nearest to a threshold of 15 count/s every 4 cycles. These settings ensured examination of not only high abundance ions, but low abundance ones as well.

***Data Analysis.*** Data analysis for the iTRAQ experiments was performed with ProQUANT 1.0, while that for cICAT experiments was with ProICAT 1.0 SP3. The cut off for the confidence settings for both analyses was at 75; that for the score was at 20. The tolerance set for peptide identification in ProQUANT searches were 0.15 Da for MS and 0.1 Da for MS/MS, while those for ProICAT searches were, 0.2 Da and 0.1 Da, respectively. All identifications were manually inspected for correctness. Relative quantification of proteins in the case of iTRAQ is performed on the MS/MS scans and is the ratio of the areas under the peaks at 114 Da, 115 Da, 116 Da and 117 Da which are the masses of the tags that correspond to the iTRAQ reagents. Relative quantification of proteins in the case of cICAT was performed on the TOF-MS scans by calculating the relative areas between the pairs of light and heavy label series of peaks. In both cases, the results of the quantification were normalized using the overall ratio obtained for all tagged peptide pairs in the sample. On the basis of previous studies utilizing cICAT reagents to compare secretory to proliferative endometrium, which involved paired tissue analysis of samples labeled with heavy and light isotopes, it was found that many proteins were expressed within a standard deviation (SD) of < 0.3 and an expression ratio close to 1.0, when protein expression was normalized against a housekeeping protein (n=6) [49]. Using this SD as an approximate benchmark for variation of protein expression among proteins whose expression is not altered when utilizing this technique, it was considered that a difference of 3 SDs i.e. 1.0 plus 0.9, or about a 2 fold difference, in normalized expression levels would be outside of the variation expected by chance, with a confidence limit of > 95% using a simple Gaussian approximation. This approximation would, therefore, apply to normalized expression levels > 2.0 or in reciprocal form < 0.5, as no assumption was made with regard to which tissue showed greater abundance. Using this approach, therefore, a two-fold expression change was chosen as an initial benchmark for potentially significant changes. In small sample sizes used for these initial studies, i.e. one sample in each group, at an alpha value of 0.05 (two-sided) and a beta value of 0.2 (80% power), the ratio of expression increase to SD must be at least 4 in order to achieve significance. This means that for minimal statistical significance to be achieved using the above alpha and beta parameters for a single paired-tissue comparison, a 2-fold mean increase in expression must be associated with an SD less than 0.5 for replicate measurements in order to satisfy the sample size equation [n = 2 SD²(Z_{α}+Z_{β})²/(expression increase)²] =1, when the power index (Z_{α} +Z_{β})² = 8 (a = 0.05, β = 0.2). For the sample size of three paired comparisons (one normal tissue and three tumor tissues were used in the cICAT experiment), the sample-size approximation of n = 2 in each group was used to compensate for the fact that one normal tissue was used (i.e. n = I in the control group, and n = 3 in the tumor group). The ratio of expression increase to SD must be greater than 2.8 in order to satisfy the above equation for an n = 2 (using the same α and β error limits of 0.05 and 0.2, respectively).

### Results

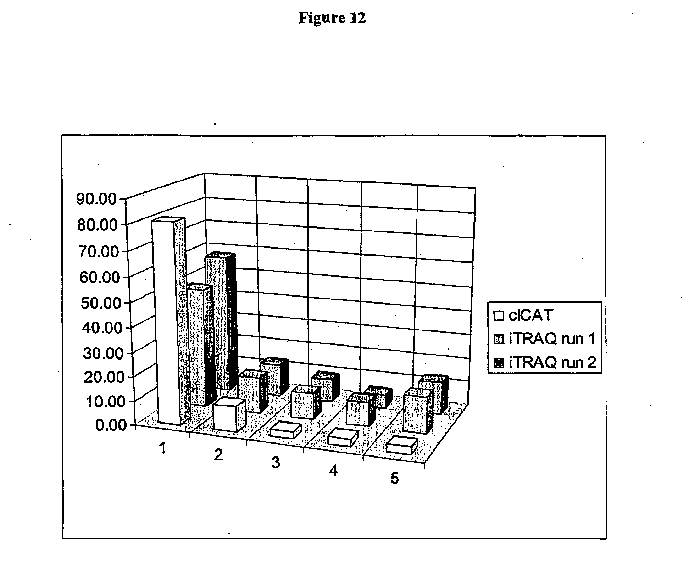

iTRAQ runs 1 and 2 led to sequencing and identification of 645 and 1,026 peptides, respectively. The previous studies (see above) showed that a confidence setting of 75 and a score setting of 20 were optimal. Manual inspection of hundreds MS/MS spectra concluded that while these conditions were sufficiently stringent in that most identifications were correct, they also did not exclude too many peptides that could have been identified based on spectral quality. The MS/MS spectra of all peptides that scored above the cut offs were manually inspected to verify proper identifications. The shorter MS and MS/MS cycles used for run 2 resulted in a higher number of peptides identified. A significant number of these peptides were identified more than once, thus the number of unique peptides dropped to 292 and 312 for runs 1 and 2, respectively. Many of the more abundant proteins were identified by several peptides (Figure 12); the numbers of proteins identified were 101 and 126, respectively. 63 proteins were identified in both runs. The numbers are modest, which were likely due to the small amount of starting materials.. Five proteins have abundance ratios that show more than a two-fold change (≥ 2.0 or ≤ 0.5) in both cancer samples relative to the proliferative as well as the secretory endometrium, and meet the criteria of differential expression. These are shown in Table 4.

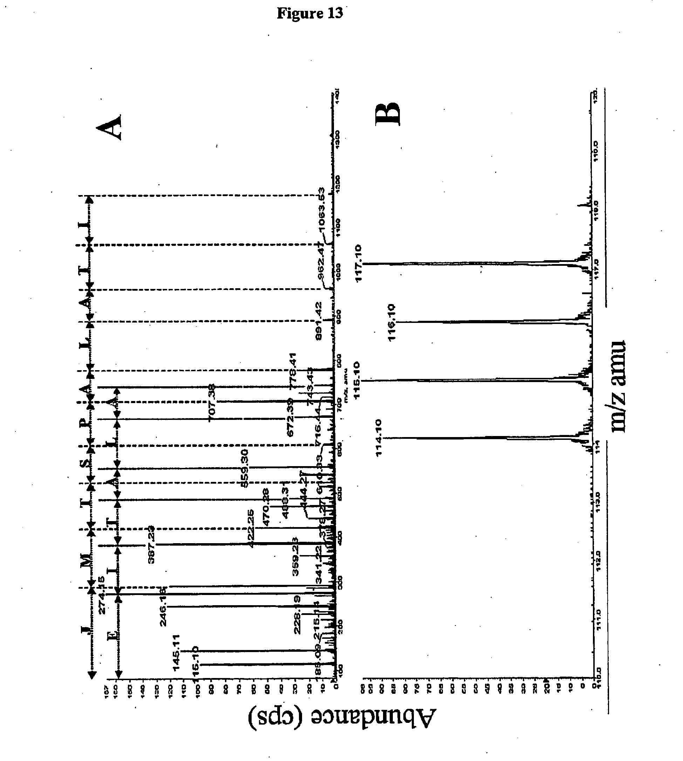

Relative quantification is expressed as three pair-wise ratios against the proliferative endometrium (iTRAQ114) for the secretory endometrium (iTRAQ 115) and the two EmCa samples (iTRAQ116 and 117). To account for small differences in protein loadings across the samples, these ratios have been normalized using the overall ratios for all proteins in the samples, as recommended by Applied Biosystems. The rationale for this choice is based on the assumption that the relative abundances for the majority of proteins are close to one. This assumption is exemplified in this study in terms of the abundance ratios in secretory versus proliferative endometrium for the following abundant proteins: cytoplasmic actin, 0.98; alpha enolase, 0.91; alpha filamin, 1.04; serum albumin precursor, 1.56; and tropomyosin alpha 4 chain, 0.99. Figure 13a shows the MS/MS spectrum for the doubly charged cytoplasmic actin peptide at 725.4 Th in one of the runs. The cluster of peaks around 115 Th is better shown in the mass spectral window 110 - 120 Th in Figure 13b, demonstrating near identical abundances in the four samples: proliferative (iTRAQ114), secretory (iTRAG115), EmCa 1 (iTRAQ116), and EmCa 2 (iTRAQ117).

Typical MS/MS windows (110-120 Th) for tryptic peptides in four differentially expressed proteins, (A) chaperonin 10, (B) alpha-1-antitrypsin precursor, (C) creatine kinase B, and (D) transgelin, are shown in Figure 14. Chaperonin 10 is overexpressed in both EmCa samples, whereas alpha-1-antitrypsin precursor, creatine kinase B, and transgelin are underexpressed. The remaining differentially expressed protein, pyruvate kinase M1 or M2 isozyme, is also overexpressed in the two EmCa samples.

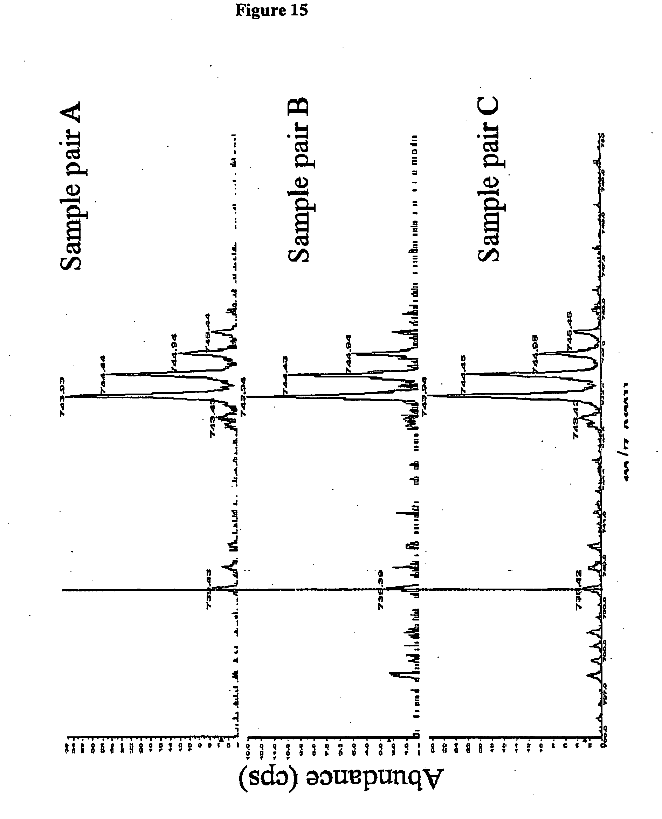

The cICAT experiments led to identification and quantification of 68 proteins, all were manually verified. Again, the modest number stemmed from the small sample size. Fewer proteins were identified by multiple peptides relative to iTRAQ (Figure 12), in accordance with the more selective nature of cICAT labeling on only cysteine residues. Five proteins that meet the two-fold differential expression criterion in all three EmCa versus proliferative endometrium pairs are: calgizzarin, heterogeneous nuclear ribonucleoprotein (hnRNP) D0, macrophage migration inhibitory factor (MIF), polymeric immunoglobulin receptor (PIGR) precursor, and pyruvate kinase M1 or M2 isozyme. These results are summarized in Table 5. All five proteins are overexpressed in EmCa tissues; pyruvate kinase is also shown to be overexpressed with iTRAQ. Figure 15 shows an example of overexpressed protein, calgizzarin A. Again, the relative abundance ratios are normalized to the overall ratio of all proteins in a given sample pair to account for small differences in protein loadings in the two samples. Cytoplasmic actin exhibits a heavy/light label ratio of 0.95 ± 0.24 (standard deviation of three samples).

### Discussion

The combination of iTRAQ and cICAT labeling results in the discovery and identification of nine differentially expressed proteins that are potential cancer markers (PCMs) for EmCa. Six of the nine PCMs are overexpressed, whereas three are underexpressed in EmCa.

***PCMs Overexpressed in EmCa**.* Chaperonin 10 is a heat shock protein that was identified as a PCM using MALDI/SELDI MS and identified by offline separation, preconcentration, trypsinization and MS/MS (see above). Overexpression of chaperonin 10 in EmCa tissues was verified independently by Western analysis; chaperonin 10 was localized to the cancerous epithelium by means of immunohistochemistry (IHC). Elevation of levels of chaperonin 10 has been associated with large bowel and cervical carcinomas [53]. The level of chaperonin 10 in serum was demonstrated to be an indicator of trophoblastic tumor [54]. The observation of chaperonin 10 overexpression in EmCa samples in this study with iTRAQ confirms the previous finding using MALDI/SELDI MS, Western analysis and IHC. Thus, chaperonin 10 may be a serum marker protein for EmCa.

Overexpression of pyruvate kinase M1 or M2 isozyme was demonstrated by both the iTRAQ and cICAT applications. Pyruvate kinase was found to be expressed at elevated levels in both plasma and fecal samples in patients ofgastrointestinal cancer [55,56]. The ability to detect pyruvate kinase in plasma samples is of particular interest as this might indicate that screening plasma for elevated pyruvate kinase levels in endometrial cancer cases may also be possible. An investigation into the activity of 12 enzymes related to the glycolytic pathway in cervical and endometrial cancers found that only two, pyruvate kinase and phosphoglucose isomerase, were significantly higher in both cancers [57].

Calgizzarin was found to be overexpressed in EmCa tissues using cICAT labeling. Calgizzarrin was one of two proteins that exhibited significant upregulation in colorectal and lung carcinoma cell lines over normal colorectal mucosal cells [58]. Calgizzarin was one of three proteins that have been identified as tumor markers in mouse colon cancer [59]. The other two tumor markers were calgranulins A and B, the former of which was also identified as a protein marker in human endometrial carcinoma using SELDI MS. Identification was made possible by size-exclusion chromatography, trypsinization and online nanoLC MS/MS; confirmation of calgranulin A overexpression in EmCa was rendered by IHC in a tissue microarray format.

HnRNP D0, also known as AU-rich element RNA-binding protein 1, binds to the AU rich 3' UTR of many proto-oncogenes. One study has shown that this protein is more abundant in murine neoplastic lung epithelial cell lines and that this abundance decreases when non-tumorigenic cells reach confluence or growth arrest [60]. Conversely, it was also found that the abundance was unaffected in spontaneous transformants from this cell line. Another more recent study using transgenic mice showed that overexpression in an isoform of this protein altered mRNA levels of several oncogenes, including c-myc, c-jun, c-fos and TNF-alpha [61]. The mouse line with the highest level of this isoform developed sarcomas [61].

Macrophage Migratory Inhibitory Factor is another protein that has been well documented as being involved with cancer [62]. This ranges from hepatocellular carcinomas [63], to non small cell lung cancer [64], to brain tumors [65] and gliblastomas [66].

PIGR has previously been detected in serum from patients with lung cancer and was shown to be significantly upregulated in the secretory component by an ELISA study involving 45 lung cancer patients compared with 45 control subjects [68]. Another study has also demonstrated a possible linkage between PIGR and bladder carcinoma with protein levels in serum being significantly increased in patients with transitional cell carcinoma [69].

***PCMs Underexpressed in EmCa**.* Alpha-1-antitrypsin precursor is one of the three proteins that are underexpressed in EmCa tissues. Recent studies have shown downregulation of alpha-1-antitrypsin to be associated with malignant lymphoma as well as liver, lung, stomach, bladder and gall bladder cancer [70,71 ]. There is also evidence for upregulation contributing to enhanced cell migration and metastases of human colon cells in a rat model [72]. Such a contradiction might be explained by the effect of alpha-1-antitrypsin being tissue specific which would prove useful for distinguishing between forms of cancer.

Creatine kinase B shown here as being underexpressed in EmCa has likewise been demonstrated as being downregulated in cervical, colon, and lung cancers but not in hepatocarcinoma [73,74].

Transgelin is another protein observed to be underexpressed in this study. It has also been implicated as being downregulated in breast and colon carcinomas as well as in a lung epithelial cell line [75,76]. Transgelin was isolated and identified as an antigen from renal cell carcinoma; subsequent *in-situ* hybridization experiments, however, found that the malignant cells were negative with respect to transgelin and that the transgelin source was from the mesenchymal cells of the stroma [77]. Thus, downregulation of transgelin appears to be true for renal cell carcinoma as well.

***Proteins Showing Possible Differential Expression**.* There are four proteins that showed differential expression by cICAT, but a smaller than critical (two-fold) change by iTRAQ labeling. One such protein is cyclophilin A, which was observed as being overexpressed by approximately four-fold in the ICAT analysis; however, the iTRAQ experiments showed a smaller overexpression of 1.47 ± 0.29 across both cancer samples in both runs compared with the normal proliferative sample, which is not considered as significant according to our criterion . of a two-fold change. Cyclophilin A has recently been reported as overexpressed in lung cancer [76].

Triosephosphate isomerase is another protein showing overexpression by cICAT tagging. However, the relative standard deviation for this protein at ± 72% was also large, suggesting a variable expression level. There is a recent study that suggests triosephosphate isomerase to be upregulated by two-fold in lung cancer [78]. Triosephosphate isomerase level was found to be highly variable in renal cell carcinoma [79]. iTRAQ labeling shows an overexpression of 1.55 ± 0.28.

Superoxide dismutase [Cu-Zn] is another protein that showed a large variation, ± 50%, in the cICAT results. This protein has been implicated in pancreatic adenocarcinoma [80]. iTRAQ results at 1.46 ± 0.08 is below the criterion of overexpression. Phosphatidylethanolamine binding protein showed a cICAT overexpression level of 3.7 ± 1.0 and an iTRAQ overexpression level of 1.39 ±10.13. There is evidence for upregulation in rat hepatoma cell lines [81].

The use of isotope-coded affinity tags and nanoLC/MS/MS afforded examination of a large number of proteins for differential expression. This method is a lot more efficient and powerful in terms of the number of proteins that can be examined in a given experiment than SELDI MS. However, complex data analysis and the need for manual examination of MS/MS data limit the number of samples that can be examined within a given period of time. As a consequence, iTRAQ tagging involved two normal endometrial tissues versus two EmCa tissues, whereas cICAT pairing involved only one normal endometrial versus three EmCa tissues. By contrast,

PCM discovery using SELDI MS involved in excess of 40 samples [see above]. A contribution to the uncertainties of the aforementioned four proteins may simply be individual variations in protein expression, both within the normal group as well as the EmCa group. The experience with the results of chaperonin 10 and calgranulin A shows that, while there is overexpression in EmCa tissues, there are considerable variations in protein abundances, possibly reflecting variability in the cellular subpopulations of the whole tissue homogenates or in the type or nature of the EmCa. In addition, there is variation in the abundances of these proteins across individual normal endometrial samples.

***Selectivity of PCMs.*** Literature data show that the nine differentially expressed proteins discovered and identified here are associated with cancers and are, indeed, potential cancer markers. Individually, these nine PCMs are nonspecific for endometrial carcinoma, as each has been linked with cancers other than EmCa. As the principal concern in screening, diagnosis, and monitoring of EmCa is the exclusion or omission ofany malignant endometrial disease, the fact that these PCMs have been noted in other cancers would rarely be a potential clinical drawback. In other words, for envisaged clinical use, the sensitivity is of far more concern than specificity for EmCa. It is probable that different cancers will share similar pathways in tumorigenesis, which will induce over- and underexpression of similar proteins [57]. Nevertheless, it is unlikely that the differential expression pattern involving many proteins will be identical for all or some primary tumor sites. Their collective use as a *panel* may be more effective in indicating the site of origin of a cancer [82,84], despite the association of individual markers with a variety of primary sites.

The absolute expression level of PCMs will be an important issue. Knowing protein abundances in normal and diseased states will allow establishment of threshold levels beyond which EmCa is signaled. iTRAQ permits simultaneous investigation of up to four samples, thus facilitating the inclusion of synthetic tryptic peptides of known amounts in absolute isotope-dilution experiments

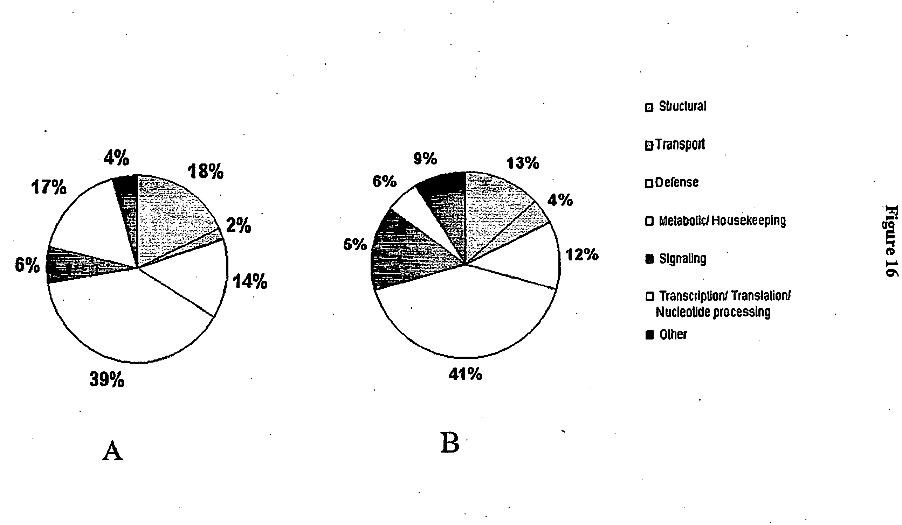

***iTRAQ versus cICAT**.* The results obtained by iTRAQ and ICAT analyses suggest that the information generated by the two methods is complementary. There are, however, a few aspects on which each of these methods has advantages over the other. Quantification by cICAT can be compromised by overlapping peaks in the MS spectrum; this complication is resolved in iTRAQ as quantification is performed on the MS/MS spectrum. iTRAQ on the other hand requires processing samples separately until after the tryptic digestion. This increases the potential for errors introduced, as a result of sample handling or different extents of tryptic digestion. Another aspect on which iTRAQ differs is complexity because of the relatively nonspecific nature of labeling. Many more proteins are identified by multiple peptides. This is not necessarily a disadvantage, as it permits quantification of multiple peptides, thereby increasing the confidence in the ratios report. After classifying identified proteins into broad categories (Figure 16), it is apparent that there is a higher proportion of signaling proteins identified by the cICAT method. Conversely, iTRAQ analysis identified a larger percentage of the more abundant ribosomal proteins and transcription factors.

### Example 5

### Summary

Proteomic analyses of the proliferative and secretory phases of the human endometrium were carried out to identify proteins and discover differentially expressed proteins using isotope-coded affinity tags and three stages of chromatographic separation and online MS/MS. From an initial list of 346 proteins identified by ProICAT, manual inspection of MS/MS spectra and confirmatory searches pared the list down to 119 positively identified proteins. Only five of the proteins showed consistent differential expression. The two proteins with unquestionable differential expressions in the secretory endometrium are: glutamate NMDA receptor subunit zeta 1 precursor and FRAT1. Some of the proteins that show no differential expression have previously been examined in gene-expression studies with similar conclusions.

Below are reported results of a study to identify proteins in the human endometrium, and especially proteins that are differentially expressed in the proliferative and secretory phases. Differences in protein expression levels are highlighted and determined by the use of the cleavable ICAT reagent [8]. Proteins that are identified are compared with genes that have been examined for regulatory changes.

The following materials and methods were used in the study.

### Materials and Methods

### Tissue samples

Endometrial tissues were retrieved from an in-house dedicated endometrial tissue bank. All tissues had been snap-frozen in liquid nitrogen within 15-20 minutes of devitalization at the time of hysterectomy. All tissue procurements were carried out after informed patient consents. The consent, procurement and banking procedures were approved by the Research Ethics Boards of York University, Mount Sinai Hospital, University Health Network, and North York General Hospital. In every case, the endometrium was classified as proliferative or secretory by a pathologist. The histological classification was verified by examination of a histopathologic section from the frozen tissue. The mirror-face of the residual block was used for proteomic analysis. After addition of 1 ml of Hanks' Balanced Salt Solution containing protease inhibitors (1 mM 4-(2-aminoethyl)benzenesulfonyl fluoride, 10 µM leupeptin, 1 µg/ml aprotinin and 1 µM pepstatin), the tissue sample was mechanically homogenized at 30,000 rpm using a Polytron PT 1300D handheld homogenizer (Brinkmann, Westbury, USA). The whole tissue homogenate contained not only endometrial epithelium, but supportive stroma, vessels as well as secretions. The homogenate was stored in aliquot at -80°C and/or submitted for proteomic analysis. Samples from six different individuals were selected for this study. Three of these tissues had been classified as proliferative endometria (randomly designated as PRO1, PRO2 and PRO3), and the other three as secretory endometria (randomly designated as SEC1, SEC2, SEC3).

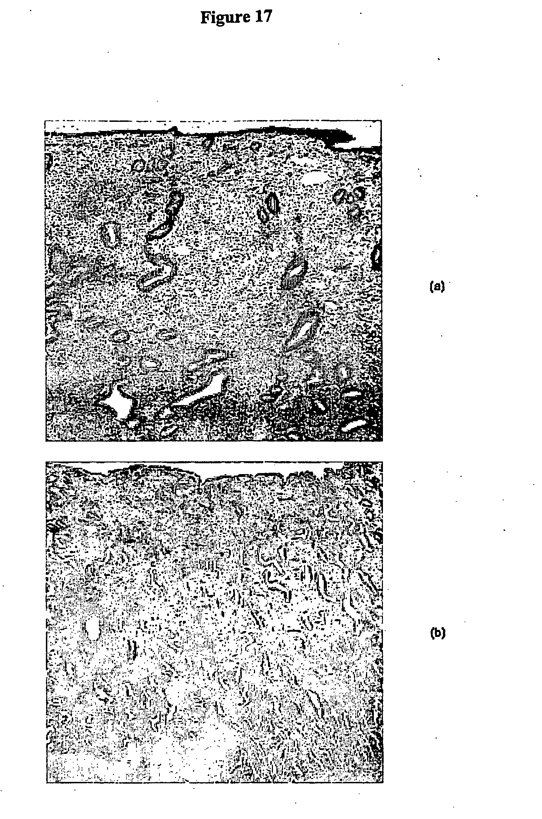

Figure 17 shows an example of the histologic appearances of (a) a proliferative (PR02) and (b) a secretory (SEC2) endometrium. In both endometria, the stratum basalis is characterized by a denser stroma than the physiologic responsive stratum functionalis above. Across the top of the stratum functionalis is the surface epithelium, which lines the endometrial cavity. The proliferative endometrium shows small, coiled glands with lining columnar epithelium reaching to the surface. By contrast, the secretory endometrium is thicker, and contains more tortuous glands with intra-luminal secretions. The endometria of both types of physiologic phases have abundant supportive stroma and vessels among the epithelial glands.

### Chemicals

Solvents, chemicals (except otherwise noted), and Hanks' Balanced Salt Solution were obtained from Sigma-Aldrich (Oakville, ON, Canada). All reagents and buffers for the cleavable ICAT sample preparation procedure were from Applied Biosystems (Foster City, CA, USA).

### Sample Preparation

After removal of cell debris by centrifugation, the total protein content for each of the six clarified homogenates was measured using a commercially available Bradford protein assay (Bio-Rad, Mississauga, ON, Canada). ICAT sample preparation procedure was carried out according to the cleavable ICAT protocol (Applied Biosystems, Foster City, CA, USA). One hundred micrograms of proteins was used per sample. The proteins were denatured with the Denaturing Buffer supplied with the ICAT reagent kit. Afterwards, disulfide bonds were cleaved by adding the reducing reagent supplied, which contained 50 mM tris-(carboxyethyl)phosphine, and boiling for 10 min. The proliferative samples were then labeled with the light ICAT reagent and the secretory samples with the heavy reagent by incubating with the respective ICAT label for 2 h at 37°C. The labeled PRO1 and SEC1 samples were combined to form ICAT sample A, PRO2 and SEC2 to form ICAT sample B, and PRO3 and SEC3 to form ICAT sample C. The final volume of each sample was 0.2 ml. Mixing of the labeled proliferative and secretory samples in pairs in this manner ensures that any protein or peptide losses during subsequent processing steps is the same for both samples in a pair. The sample pairs were then digested by incubating each pair with 0.2 ml of a 50 µg/ml trypsin solution at 37°C overnight. The resulting peptides were fractionated by means of strong cation exchange chromatography using an HP 1050 LC system (Agilent, Palo Alto, CA, USA) with a 1.5 ml injection loop and a 2.1 mm internal diameter (ID) x 100 mm length PolyLC Polysulfoethyl A column packed with 5 µm beads with 300 A pores (The Nest Group, Southborough, MA, USA). A 2.1 mm ID x 10 mm length guard column of the same material was plumbed upstream from the analytical column. Fractionation was effected by a binary mobile-phase gradient at a total flow rate of 0.2 ml/min. Eluent A consisted of a 10 mM KH₂PO₄ solution in 25% acetonitrile and 75% deionized water acidified to a pH of 3.0 with phosphorus acid. Eluent B consisted of a 10 mM KH₂PO₄ and 350 mM KCI solution in 25% acetonitrile and 75% deionized water acidified to a pH of 3.0 with phosphorus acid. The trypsinized ICAT sample pair (0.4 ml) was mixed with 2.0 ml of Eluent A. 1.5 ml of the 2.4 ml sample was injected. Initially, the gradient comprised 100% Eluent A. At the 2^{nd} minute, the % Eluent B was changed linearly from 0 to 100% at the 58^{th} min. The run was terminated at the 60^{th} min. A total of 30 fractions were collected, one every two minutes, using an SF-2120 Super Fraction Collector (Advantec MFS, Dublin, CA, USA). UV monitoring of the chromatographic eluent revealed abundant peptides in fractions 11-20. These 10 fractions were further purified by affinity chromatography according to the ICAT protocol recommended by Applied Biosystems. Eluted labeled peptides were treated with the Cleaving Reagent, which contains TFA in order to remove the biotin label, and resolved in a third stage of chromatographic separation using reverse-phase nanobore LC with online MS/MS.

### Nanobore LGMS/MS

The nanobore LC system was from LC Packings (Amsterdam, The Netherlands) and consisted of a Famos autosampler and an Ultimate Nano LC system. It was interfaced to an API QSTAR Pulsar QqTOF mass spectrometer (Applied Biosystems/MDS Sciex, Foster City, CA, USA) using a Protana NanoES ion source (Protana Engineering A/S, Odense, Denmark). The spray capillary was a PicoTip SilicaTip emitter with a 10 Jim ID tip (New Objective, Woburn, MA, USA). The nanobore LC column was a 75 µm ID x 150 mm length reverse-phase PepMap C18 nano capillary column (LC Packings, Amsterdam, The Netherlands) packed with 3 µm beads with 100 Å pores. One µl of sample was injected via the full-loop mode. Separation was performed using a binary mobile-phase gradient at a total flow rate of 200 nl/min. Eluent A consisted of 94.9% deionized water, 5.0% acetonitrile and 0.1% formic acid (H = 3). Eluent B consisted of 5.0% deionized water, 94.9% acetonitrile and 0.1% formic acid. The following binary gradient was used:

| | | | | | | | | |
|---|---|---|---|---|---|---|---|---|
| *Time (min)* | 0 | 5 | 125 | 135 | 157 | 160 | 163 | 190 |
| *% Eluent B* | 5 | 5 | 30 | 60 | 80 | 80 | 5 | Stop |

For nanospray, the source conditions were a curtain-gas setting of 20 and an ionspray voltage in the range of 1800 - 3800 V that was optimized daily. In the Q0 region, the instrument parameters were a declustering potential (DP) of 65 V and a focusing potential (FP) of 265 V. Nitrogen was used as the collision gas at a setting of CAD = 5 for both TOF-MS and MS/MS scans.

All LC-MS/MS data were acquired in information-dependent acquisition (IDA) mode in Analyst QS SP5 with Bioanalyst Extension 1.1 (Applied Biosystems / MDS Sciex). A TOF-MS survey scan with an m/z range of 400 1500 and 1 s scan time was followed by two product ion scans with an m/z range of 70 - 2000 and 2 s scan time. The collision energy (CE) was automatically controlled by the IDA CE Parameters script The switching criteria were set to ions greater than m/z = 400 and smaller than m/z = 1500 with a charge state of 2 to 5 and an abundance of ≥ 10 counts/s. Former target ions were excluded for 60 s and ions within a 4 Th window were ignored. In addition, the IDA Extensions II script was set to 2 repetitions before dynamic exclusion and to select a precursor ion nearest to a threshold of 15 count/s every 4 cycles. These settings ensured examination of not only high abundance ions, but low abundance ones as well. Each of the three sample pairs, A, B and C, was injected twice, thus yielding a total of six sets of runs.

Data analysis was performed using ProICAT 1.0 SP3 software (Applied Biosystems / MDS Sciex). An initial setting of a confidence value of 75 and a score of 15 was used. Relative quantification of proteins between the light and heavy labels was performed on the TOF-MS scans by calculating the relative areas under the series of peaks. Differential levels of expression were based upon measured protein expression ratios in proliferative versus secretory endometrium normalized to glyceraldehyde-3-phosphate dehydrogenase (GAPDH) expression and were considered significant when they exceeded three SDs from the mean expression of housekeeping proteins (actin, tubulin α-chain and triose phosphate isomerase). All expression ratios were based on a mean of two essays. The sample size calculation for determination of minimum numbers of tissue samples for ICAT analysis (N) was based upon the difference between two means, N = 8 (SD / precision) [85]. Based on preliminary experiments, an SD value of 0.3 was observed with a target precision of 0.5, yielding a minimum sample size of 3 for each tissue group. The significance of differential protein expression between proliferative and secretory endometrium was evaluated using the Wilcoxon rank sum test.

### Results

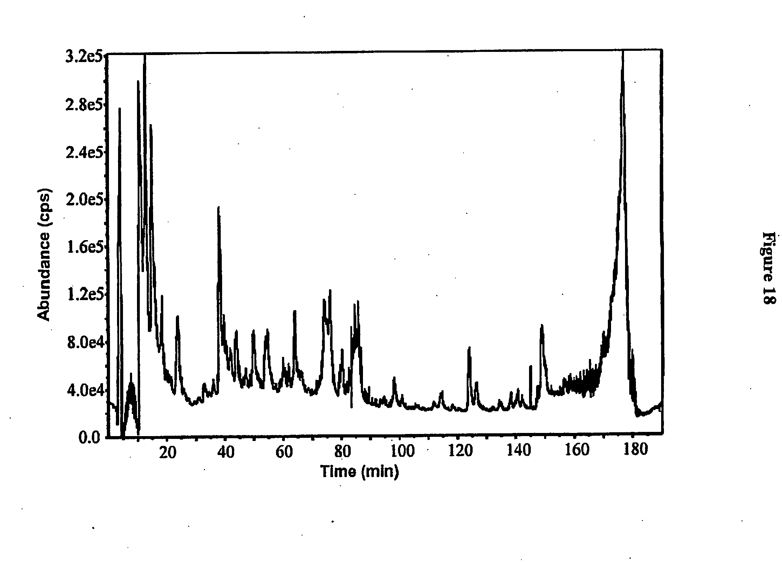

Figure 18 shows a typical nano LC/MS total ion chromatogram (TIC) from fraction 16 of sample A. As a total of 10 fractions were analyzed per sample and each sample was injected twice, there were a total of 60 TICs. The use of the IDA mode generated hundreds of MS/MS spectra per TIC.

Using the initial setting of a confidence value of 75 (ProICAT recommends a lower confidence value of 50) and a score of 15 in ProICAT 1.0 SP3 results in the identification of 346 non-redundant proteins. Manual inspection of the MS/MS spectra reveals that identification of some proteins that score between 15 and 20 may be problematic. A random selection of 50 spectra from proteins scoring between 15 and 20 shows that 15 were analyzed and sequenced correctly, 13 were probably correct, and 22 were incorrect. Manual inspection of the MS/MS spectra of the 145 proteins that score 20 or higher also reveals occasional errors in assignment, but at a rate considerably lower than that between scores 15 and 20. Unfortunately, the use of ICAT-labeling results in a reduced opportunity for corroborating identification based on a second tryptic peptide from the same protein. Although the majority of human proteins do contain more than one cysteine residue per protein, some of these residues may be located within tryptic peptides that are too small or large, others may fall into peptides that have poor ionization efficiencies. Of the 346 proteins, only 23 were identified with more than one peptide; 15 of these 23 proteins were identified with two peptides.

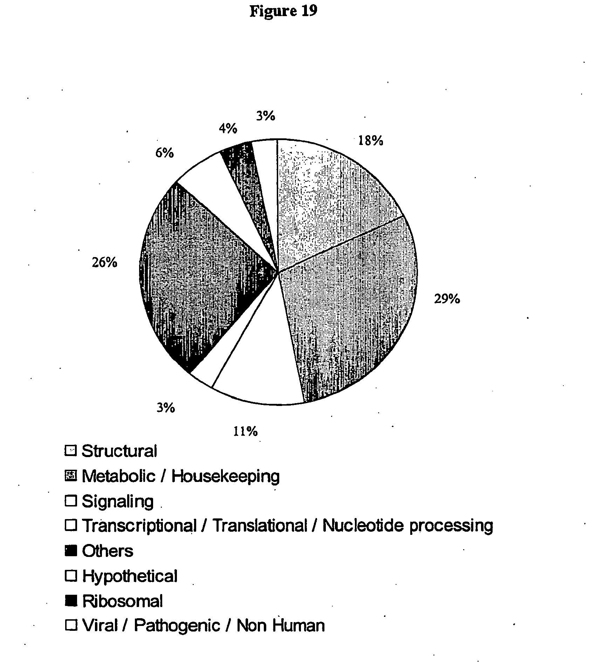

Table 6 lists the 119 proteins that have been identified by ProICAT and verified by manual inspection of MS/MS data. Figure 19 shows the distributions of the proteins in the form of a pie chart. Of the 119 proteins, 15 were added after manual inspection of 50 randomly selected proteins' MS/MS spectra. If the MS/MS spectra of the remaining 151 proteins was inspected, it would have been expected to confirm the identifications of an additional - 45 proteins. This, however, was considered impractical, especially after it was known that none of the 15 additional proteins were observed in all three runs and would contribute towards knowledge of differential expression.

The results show that expression levels of the majority of proteins identified are not consistently different between the proliferative and secretory phase of the endometrial cycle. As expected, the majority of identified proteins fall under the metabolic/housekeeping and structural categories. The proteins classified under "Others" include antibodies. A small percentage of the proteins are viral or pathogenic in origin, reflecting the possibility of infection in the biopsied endometria. A few proteins have no known functions or were identified from cDNA matches; these are, therefore, classified as hypothetical.

To normalize small variations of protein amounts, relative quantification of the protein levels is normalized to the ratio ofGAPDH. Gene expression studies involving different stages in the endometrial cycle have used GAPDH expression to normalize differential mRNA expressions [88-90]. The assumption is that the constant mRNA level of GAPDH through the endometrial cycle translate to constant protein level (no variation caused by translational controls). The GAPDH ratio of 1.5 ± 0.5 (one SD) before self-normalization in the six runs is in accordance with this assumption.

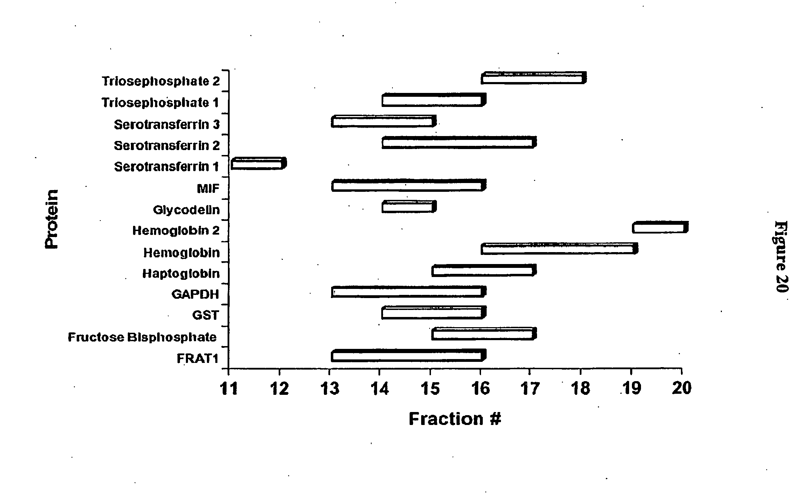

The ratios of the proteins in the secretory / proliferative phase (heavy / light label) listed in Table 6 are the averages ± SDs of the peptides in all the identified runs. In the case of more abundant proteins, e.g. serum albumin and serotransferrin, individual peptides were detected in as many as 3-4 fraction. Less abundant proteins, however, were frequently detected only in 1-2 fractions (see Figure 20). In cases of multiple fractions, multiple peptides, and/or multiple runs, the ratios listed in Table 6 are the averages and the SDs of all contributing elements. Twenty four proteins show "extreme" differential expression in that only one labeled form was observable (these are noted as "Singleton H or L". However, only five of these 24 proteins were observed in all three samples (and an additional one in two samples). All five proteins were identified by single tryptic peptides. The expression levels of three housekeeping proteins (actin, tubulin α-chain and triose phosphate isomerase) normalized against GADPH were pooled to establish a mean expression level of 1.07~1.1 and an SD of 0.3. The SD of these expression levels was calculated from expression data normalized to a value of 1 for any expression ratios less than 1 to generate linear data. (This was done by taking the reciprocal of expression values less than 1 in the original data set; for example, an expression ratio of 0.7 was converted to its reciprocal 1.42.) This data was then used to calculate the SD of housekeeping protein expression. A significant differential expression value was, therefore, defined as a normalized expression ratio that was three SDs above the ratio of 1.0. On the basis of the observed data, this ratio was set as 2.0 (approximately 1.0 + 3(0.3)). Consequently, only two-fold or larger changes in expression ratios were considered to be of significance, based on the analysis of the variation in measured expression of housekeeping proteins. An additional stringency requirement of this level of expression in all three sample pairs was imposed due to the small initial sample set. No additional proteins in Table 6 meet the differential expression criteria of abundance ratios larger than 2.0 or smaller than 0.5 in all three sample pairs.

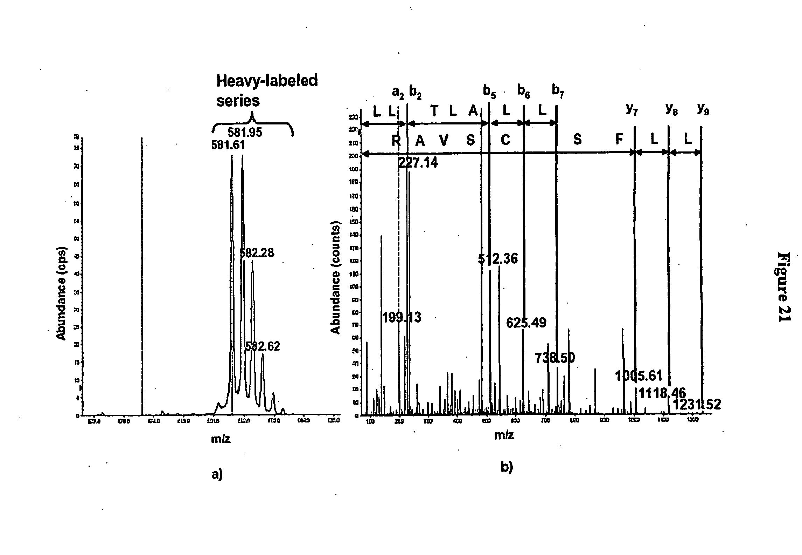

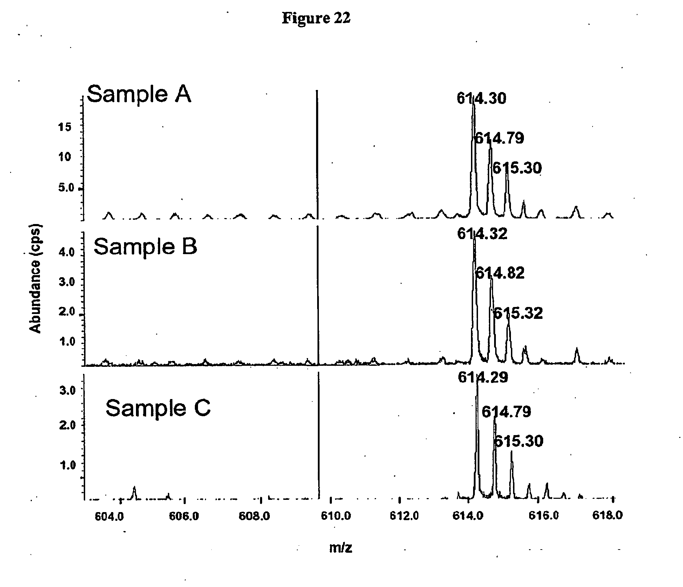

An example ofextreme differential expression observable in all three sample pairs is shown in Figure 21, which shows very significant expression enhancement of glutamate NMDA receptor subunit zeta 1 precursor in the secretory endometrium. The absence of the light-labeled analogue, which would have manifested as a cluster of peaks beginning at 578.6 Th, demonstrates the significantly lower expression of this protein in the proliferative endometrium. The triply charged tryptic peptide at 581.6 Th was identified as LLTLALLFSCSVAR [SEQ ID NO.29], which maps to the N-terminal region of the protein. A second example is shown in Figure 22, again showing extreme differential expression in the secretory phase. This protein was identified as FRAT1. For all five proteins that met the differential expression criteria stipulated above, a minimum p value of less than 0.04 was obtained on the basis of the Wilcoxon rank sum test, suggesting that the differences observed were of statistical significance.

### Discussion

The observation of similar expression levels in the majority of proteins shown in Table 6 is not surprising, as many proteins are structural and housekeeping in nature. In addition, structural and housekeeping proteins, e.g. tubulin and actin, are expressed in many tissue types. This is of significance as the biopsies that were examined comprise not only epithelial cells, but also supportive stroma cells, blood and vessels that are not expected to be affected by the endometrial cycle.

In this initial examination of three sample pairs, a conservative strategy was adopted of recognizing proteins as differentially expressed only when their expression ratios are larger than 2.0 or smaller than 0.5 in all three sample pairs. Of the 119 proteins listed in Table 6, five proteins are in this category. In fact, differential expression is in the extreme form that only single labels are apparent. There is one protein (POL polyprotein) that was seen only in the secretory endometrium, but was observed in only two sample pairs and does not meet the criteria of differential expression in this study (see later).

One of the five differentially expressed proteins is glutamate NMDA receptor subunit zeta 1 precursor, which was only observed in the secretory endometrium. This protein is a subunit of a ligand gated ion channel and is known to be involved with synaptic plasticity in neurons. A recent paper suggests that it may also play a role in glutamate-mediated toxicity to mitochondria, eventually leading to apotosis [91]. As stated earlier, the peptide maps to the N-terminal region of the precursor, which is normally cleaved to form the mature protein.

Similarly, FRAT1 is detected only in the secretory endometrial samples under the experimental conditions. There is no direct connection between FRAT1 and the endometrium reported in the literature; however, FRAT1 is expressed in a wide range of human tissues [92]. The gene expressing FRAT1 is a known proto-oncogene that activates the WNT pathway [93,94]. This pathway is important in the endometrial cycle; FRAT1 is known to inhibit c-Jun activity, thereby inhibiting subsequent apotosis [95]. c-Jun, in turn, has been shown to be expressed in the endometrium and this expression is at a higher level during the proliferative phase than the secretory phase [96].

Myosin light chain kinase 2 is another protein that was observed exclusively in the secretory endometrium in all three sample pairs. While there are no studies on the relative amounts of this enzyme during the endometrial cycle, previous studies showed that it is present in the human myometrium at a level that is four- to five-fold higher than that in endometrial stromal cells [97]. It was also demonstrated that the specific activity of myosin light chain kinase, which is believed to be essential for smooth muscle contraction, was 20-fold higher in the myometrium than that in non-muscle cells, e.g. skin fibroblasts [97].

Isopentenyl diphosphate delta-isomerase, was present only in the secretory endometrium. This protein was identified via the peptide ITMLCTGSRTLK [SEQ ID NO. 29]. In addition to this identification with ProICAT, a subsequent search with Mascot (Matrix Science) verified this assignment. This peptide, and therefore the protein, however, is unique and found only in the bacterium *Rickettsia conorii.* BLASTing the human and the *R. conorii* isopentenyl disphosphate delta-isomerase proteins showed no region of homology. In addition, MS BLASTing the peptide ITMLCTGSRTLK against non-redundant protein databases returned no other matches.

A protein that appeared in all three sample pairs was initially identified by ProICAT as a simian immunodeficient viral protein, envelope polyprotein GP160 precursor. However, searching Mascot with the same MS/MS spectra returned a different protein from clostridium, putative phosphatidylserine decarboxylase proenzyme (gi 28209853). Endometrial receptors for a clostridium toxin have been documented [98]; thus the identification of a clostridium protein is not entirely surprising.

Another protein initially identified by ProICAT as POL polyprotein from Rous sarcoma virus was observed only in the secretory endometrium in two of the three sample pairs. A subsequent Mascot search, however, returned a positive hit for an unlabeled peptide from serum albumin, and strongly suggested a wrong initial identification.

Tropomyosin 1 alpha chain was identified in two out of three sample pairs to be selectively expressed in the secretory phase at a ratio of 2.3±0.7. An enhanced expression of this protein in the secretory endometrium is in accordance with the result of a previous gene expression study that reported an up-regulation of 3.7 times [85].

A protein that showed no difference in expression levels (1.2 ± 0.7 in three samples) is macrophage migration inhibitory factor, MIF. This observation is consistent with those in previous studies that demonstrated MIF as being expressed by the human endometrium throughout the menstrual cycle and, in particular, predominantly in the glandular epithelial cells [9]. MIF was found to localize throughout the glandular epithelial cytoplasm in the proliferative phase; however, this distribution changes in the secretory phase, when IF localizes to the apical portion of the glandular epithelial cells and is also present in glandular secretions. Macrophages are common in female reproductive tissues. In the endometrium, they play an important role in defense. In addition, macrophage degradation of cellular debris and foreign material may play an important role in endometrial shedding and repair [9].

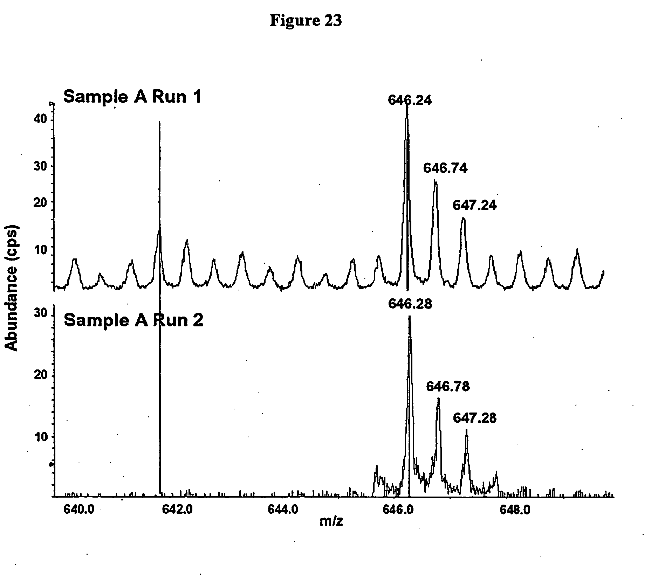

Another protein observed in this study and for which there was precedence in the literature is glycodelin. On the basis of gene expression studies [85], glycodelin was believed to be up-regulated during the secretory phase. In this study, the precursor of this protein was identified in only one sample pair, but it was identified in both runs in only the secretory endometrium (Figure 23).

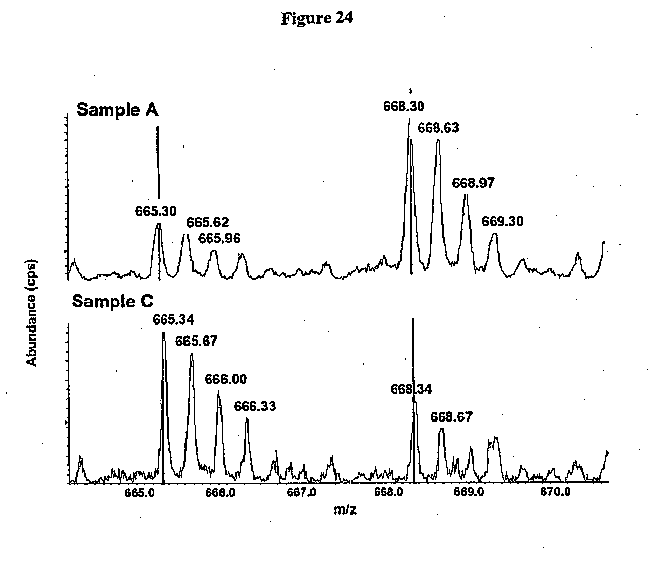

Cathepsin B which, according to a previous study, does not appear to be differentially regulated during the menstrual cycle [99], was seen in two of the sample pairs with opposite trends (Figure 24). Glutathione S transferase is known to show large variability which is believed to result from individual differences, rather than endometrial cycling [100]. The relative expression level observed in this study is 1.3 ± 0.4. Lactate dehydrogenase is another protein not believed to have any cyclic trend [101]; the relative level measured in this study, 1.4 ± 0.3, is in accordance with this expectation. Peptidyl-propyl cis-trans isomerase A or cyclophilin A has been observed in placental and decidual tissues, but there was no study on relative amounts between the phases of the endometrial cycle [102]. The relative expression results of 1.2 ± 0.2 show a constant level of expression. Haptoglobin is thought to be up-regulated during the secretory phase [103]. The average relative expression level of 2.2 ± 1.6 is based on two enhanced secretory expression level measurements in two sample pairs and one reduced secretory expression level measurement in another sample pair that account for the very large SD. Haptoglobin is a liver protein secreted into blood. As the amounts of blood in the biopsied tissues could not be controlled prior to homogenizing, the large SD might simply reflect that the samples contain different amounts of blood as opposed to truly representing increased or decreased haptoglobin expression.

It should perhaps be emphasized that protein identifications using ICAT-labeled peptides are reliable as the identification of one labeled peptide in a pair corroborates the identification of the other in the pair. This offsets somewhat the reduced opportunities in seeing multiple peptides because ICAT targets only cysteine-containing peptides. The chance of a missed identification increases when only one member of the labeled peptide pair is observable in the form of an extreme differential expression and there is uncertainty as to whether the observable peptide is labeled with the light or the heavy ICAT reagent. In addition, the presence of an unlabeled peptide may be mistakenly interpreted as a labeled peptide that is differentially expressed in the extreme. An unlabeled peptide will need to have a combination of "right" chemical properties and very high initial concentrations (e.g. a serum albumin peptide) to "survive" the affinity chromatography stage intended to select only labeled peptides. The above is intended to be a cautionary note in pointing out potential shortcomings in a powerful technique that readily highlights and quantifies differential protein expressions, and not as a negative opinion on the suitability and utility of ICAT in protein discovery or quantification.

Of the 46 studies published to date that centered on ICAT, the vast majority were on methods development that compared differential protein expressions in two different cell states. Most of these studies were on yeast cultured under different medium conditions and they compared protein levels of one sample pair. Only one recent study [104] targeted clinical samples, and even in this study only one sample pair was analyzed for differential protein expression. The present study is the only work in which multiple sample pairs have been examined. It is, of course, possible that a given observed deviation in protein ratio from 1, i.e. a heavy/light label ratio # 1, is due to individual differences, and that different pairing of the proliferative and secretory samples may produce a different ratio. It is because of this possibility that the criterion for differential expression was stringently set to a ratio of *larger than 2.0* and *smaller than 0.5* in *all three* sample pairs. It turns out that for the five proteins that met the above criterion of differential expression, the differences were in the extreme in that the proteins were observed in only one endometrial phase in a pair. Expression differences at this extreme level in all three sample pairs are highly unlikely to have originated from individual differences.

### Conclusion

From an initial list of 346 proteins identified by ProICAT 1.0 SP3, 119 proteins were identified after manual inspection of MS/MS data and additional searches using Mascot. The expression levels of the majority of proteins do not vary significantly between the proliferative and the secretory phases. Only five proteins satisfy the criteria on differential expression in having an expression ratio larger than 2.0 or smaller than 0.5 in all three sample pairs. In fact, the differential expressions observed are extreme in that the proteins are present in only one of the two endometrial phases. The two proteins with unquestionable differential expressions in the secretory endometrium are: glutamate NMDA receptor subunit zeta I precursor and FRAT1. Some of the proteins that show no differential expression have previously been examined in gene-expression studies with similar conclusions.

### Example 6

### Abstract

SELDI MS has conventionally been practiced on linear TOF which has low mass accuracy and resolution. Here in an examination of both malignant and nonmalignant endometrial tissue homogenates it is demonstrated that high mass accuracy and resolution in the MS stage are crucial. Using a commercially available QqTOF, two potential cancer markers were resolved and subsequently identified offline as chaperonin 10 and calgranulin A, that differ by 8 Da in mass. Two offline protein identification protocols were developed: the first was based on SEC, SDS PAGE, protein extraction, trypsin digestion and MALDI-MSIMS; the second on SEC and shotgun nanoLC/MS/MS. Analyses on a cohort of 44 endometrial homogenates showed 22 out of 23 nonmalignant samples had nondetectable to very low abundance of chaperonin 10 and calgranulin A; 17 of the 21 malignant samples had detectable to abundant levels of both proteins. Immunohistochemical staining of a tissue microarray of 32 samples showed that approximately half of malignant endometrial tissues exhibited positive staining for calgranulin A in the malignant epithelium, while 9 out of 10 benign tissues exhibited negative epithelial staining. In addition, macrophages/granulocytes in malignant as well as nonmalignant tissues showed positive staining. No immunostaining occurred in stroma or myometrium.

The following materials and methods were used in the study.

### Materials and methods

### Tissue samples and sample preparation

Endometrium and EmCa tissues were retrieved from a dedicated, research in-house endometrial tissue bank. The consenting and tissue banking procedures for this tissue bank were approved by the Research Ethics Boards of York University, Mount Sinai Hospital, University Health Network, and North York General Hospital. All tissues had been snap frozen in liquid nitrogen within 15-20 minutes of devitalization at the time of hysterectomy, and were obtained with patients' consent. In each case, the endometrium was classified as nonmalignant or malignant (EmCa) by one of the authors (TJC) [Kurman, R.J., Ed., Blaustein's Pathology of the Female Genital Tract, 5^{th} ed. New York, Springer-Verlag, 2002]. Nonmalignant endometrium included both benign physiologic endometrial cases (atrophic, proliferative, secretory, and menstrual) and pathologic states (benign endometrial polyp and disordered proliferative). EmCa cases included adenocarcinomas and rarer malignant mixed Mullerian tumors (carcinosarcomas). This classification was performed using routine surgical pathology sections. The histologic classification of the research tissue was verified by examination of a histopathologic section from the frozen research tissue. Tissue was taken for proteomic analysis from the mirror-face of the residual block. Cases of atypical endometrial hyperplasia, which could be considered to represent an intermediate phenotype, were not available for frozen-tissue banking and MS analysis because all tissue was required for histopathologic examination, but tissue was available for tissue microarray (TMA) preparation.

Tissue was thawed in Hanks' balanced salt solution (HHSS, Sigma) containing protease inhibitors (1 mM 4-(2-aminoethyl)benzenesulfonyl fluoride, 10 µM leupeptide, 1 µg/ml aprotinin and 1 µM pepstatin) and followed by mechanical homogenation at 30,000 rpm using a Polytron PT 1300D handheld homogenizer (Brinkmann, Westbury, USA). These whole tissue homogenates contained not only endometrial epithelium, but supportive stroma, vessels, as well as secretions. The homogenate was then stored in aliquot at -80 °C until protein profiling.

### Chemicals

Solvents, chemicals (except otherwise noted), and Hanks' Balanced Salt Solution were obtained from Sigma-Aldrich (Oakville, ON, Canada).

### Protein profiling using SELD-MS

Tissue lysate was fractionated to reduce sample complexity before protein profiling. An identical quantity of proteins was used for all samples; protein amounts were measured using Bradford Assay (Bio-Rad) on a DU-65 spectrometer (Beckman) at 595 nm. HBSS or urea/thiourea 2-D lysis buffer (7 M urea, 2 M thiourea) was used to compensate the initial volume to ensure equal volumes for all samples. Two micrograms of proteins from endometrial tissue homogenate was incubated with WCX2 or CM 10 ProteinChips (Ciphergen) according to the manufacturer's instructions. In brief, samples were diluted to 55 µl with the corresponding binding buffer, spotted onto the appropriate ProteinChip, and incubated in a sealed BioProcessor (Ciphergen) for 1h at room temperature. The ProteinChip surface was washed twice with the appropriate buffer for 5 minutes, briefly rinsed with water and air-dried. Two times 0.5 µl of 50% saturated sinapinic acid in 50% acetonitrile was applied on the samples to form crystals. The ProteinChips were analyzed using a linear TOF analyzer, PBS lie (Protein Biology System IIc, Ciphergen), or a hybrid QqTOF mass spectrometer, QSTAR XL (Applied Biosystems / MDS Sciex) fitted with the SELDI interface (Ciphergen). For accurate molecular-weight measurements, on-chip, internal mass calibration was performed using insulin (5,733 Da), cytochrome c (12,361 1 Da) and myoglobin (16,951 Da) as calibrants.

### Protein purification and identification

Selected, EmCa samples were subject to chromatographic separation to yield purified proteins for identification. Five hundred micrograms of proteins from a whole tissue homogenate was first fractionated using SEC (BioSep 2000, Phenomenex) at 1 ml/min flow with phosphate buffer (pH 7.9) containing 0.05% (w/v) sodium azide. One-millilitre fractions were collected; the eluates were then concentrated to 50 µl with a silicon carbide-based spin column (ProteoSpin, MDS Sciex). Five microlitres of a concentrate was desalted by C 18 Zip-Tip and analyzed with MALDI-MS to locate the fractions containing the protein markers of interest in the EmCa sample. Two different approaches were developed and used to identify the target proteins. Some proteins were more amenable to identification with one and others with the second approach. The first approach used SDS PAGE technique to further purify the target proteins. The fractions with the enriched proteins were diluted to 100 . µl with freshly prepared DTT (5 mM final) in 150 mM Tris pH 8.5 buffer, and incubated at 60 °C for 1h. Ten microlitres of the reaction mixture was desalted by C18 Zip-Tip and analyzed with MALDI-MS to assess the effect of DTT on the protein of interest. The remaining 90 µl was precipitated by acetone (80% (v/v) final), resuspended in SDS sample buffer, and the proteins were resolved by SDS PAGE. Protein molecular-weight markers (New England Biolabs) and cytochrome C (C-2506 Sigma) were included to guide the excision of gel portions containing the protein of interest. Intact proteins were extracted from the gel by 50 µl extraction solution (formic acid/acetonitrile/isopropanol/water in a ratio of 50l25/15/10) at room temperature for 4 h. The extracts were completely dried by SpeedVac and resuspended in 40 µl 100 mM ammonium bicarbonate. Halfof the resuspended proteins was desalted by C18 Zip-Tip and analyzed with MALDI-MS, the other half was digested in solution with 100 ng trypsin (Promega). The identity of the target protein was determined using MS/MS ion search (ProID, Applied Biosystems). The second approach used nanoLGMS/MS technology. After concentration using ProteoSpin, the fractions with the enriched target proteins were further cleaned up using C18 Zip-Tips. Four C 18 Zip-Tips were used for each 45 µl of concentrated fractions. The sorbed proteins were eluted by 100 µl of 60% acetonitrile with 0.3% TFA. The eluates were completely dried by SpeedVac and resuspended in 100 µl of 100 mM ammonium bicarbonate. The proteins were then digested in solution with 100 ng trypsin (Promega). The resulted tryptic peptides were analyzed by MALDI-MS and online nanoLC/MS1MS. The identity of the target protein was determined using MS/MS ion search (ProID, Applied Biosystems).

### NanoLC/MS/MS

The nanoLC system was from LC Packings (Amsterdam, The Netherlands) and consisted of a Famos autosampler and an Ultimate nanoLC system. It was interfaced to a second QSTAR Pulsar hybrid QqTOF mass spectrometer using a Protana NanoES ion source (Protana Engineering A/S, Odense, Denmark). The spray capillary was a PicoTip SilicaTip emitter with a 10 µm ID tip (New Objective, Woburn, MA, USA). The nanoLC column was a 75 µm ID x 150 mm length reverse-phase PepMap C18 nano capillary column (LC Packings, Amsterdam, The Netherlands) packed with 3 µm beads with 100 Å pores. One microliter of desalted sample was injected via the full-loop mode. Separation was performed using a binary mobile-phase gradient at a total flow rate of 100 nl/min. Eluent A consisted of 94.9% deionized water, 5.0% acetonitrile and 0.1 % formic acid (pH ≈ 3). Eluent B consisted of 5.0% deionized water, 94.9% acetonitrile and 0.1% formic acid. The following binary gradient was used:

| | | | | | | | | |
|---|---|---|---|---|---|---|---|---|
| *Time (min)* | 0 | 10 | 40 | 42 | 44 | 45 | 70 | 72 |
| *% Eluent B* | 5.0 | 5.0 | 30.0 | 80.0 | 80.0 | 5.0 | 5.0 | Stop |

The ion source conditions were a curtain-gas setting of 20 and an ionspray voltage in the range of 1800 - 3800 V that was optimized daily. In the Q0 region, the instrument parameters were a declustering potential (DP) of 65 V and a focusing potential (FP) of 265 V. Nitrogen was used as the collision gas at a setting of CAD = 5 for both TOF-MS and MS/MS scans.

All LC-MS/MS data were acquired in information-dependent acquisition (IDA) mode in Analyst QS SP5 with Bioanalyst Extension 1.1 (Applied Biosystems / MDS Sciex). A TOF-MS survey scan with an m/z range of 400 - 1500 and 1 s scan time was followed by two product ion scans with an m/z range of 70 -2000 and 2 s scan time. The collision energy (CE) was automatically controlled by the IDA CE Parameters script. The switching criteria were set to ions greater than m/z = 400 and smaller than m/z = 1500 with a charge state of 2 to 5 and an abundance of ≥ 10 counts/s. Former target ions were excluded for 60 s and ions within a 4 Th window were ignored. In addition, the IDA Extensions II script was set to 2 repetitions before dynamic exclusion and to select a precursor ion nearest to a threshold of 15 count/s every 4 cycles. These settings ensured examination of not only high abundance ions, but low abundance ones as well.

### Tissue microarray and immunohistochemical protein localization

A yore TMA was prepared to confirm the presence of an identified protein marker (10,835 Th, see later sections), and to determine its localization. Following fixation in 10% buffered formalin, benign, hyperplastic and malignant endometrial tissues were histologically processed and embedded in paraffin blocks. A single 2-mm core of tissue was removed zoom the paraffin-block using a Manual Tissue Arrayer (Beecher Instruments). The 26 endometrial study cases included 11 benign endometrium (five secretory, four proliferative, one atrophic and one menstrual), 13 malignant endometrium. (12 adenocarcinomas and one carcinosarcoma), and two cases of atypical endometrial hyperplasia. Six other control tissues were also embedded into the TMA, including four non-endometrial carcinomas and 2 benign tumors (salivary gland pleomorphic adenoma and ovarian thecoma). Following MS identification, monoclonal mouse antibodies against the potential cancer marker were procured from two sources: Research Diagnostics, Inc. (Flanders, NJ, labeled as RDI, and US Biological (Swampscott, MA), labeled as USB. Sections were cut from the TMA. The antibodies were applied in an appropriate dilution determined through a pilot study and immunohistochemically visualized using a diaminobenzidine chromogen.

Interpretations of the two immunohistochemically stained TMA sections were conducted using a standardized method; microscopic reviews were conducted blinded to the findings of the other TMA section. Positive staining (brown) for the potential marker was determined for three cellular components: macrophage/granulocyte, epithelium/carcinoma, and endometrial stroma and myometrium. Immunostaining of these three cell types was semi-quantitatively graded as: 0 (no immunostaining), 1+ (weak positivity and/or positivity in only occasional cells), 2+ (moderate positivity and/or positivity in more abundant cells), and 3+ (strong positivity and/or positivity in numerous cells).

### Results and discussion

### SELDI-MS details

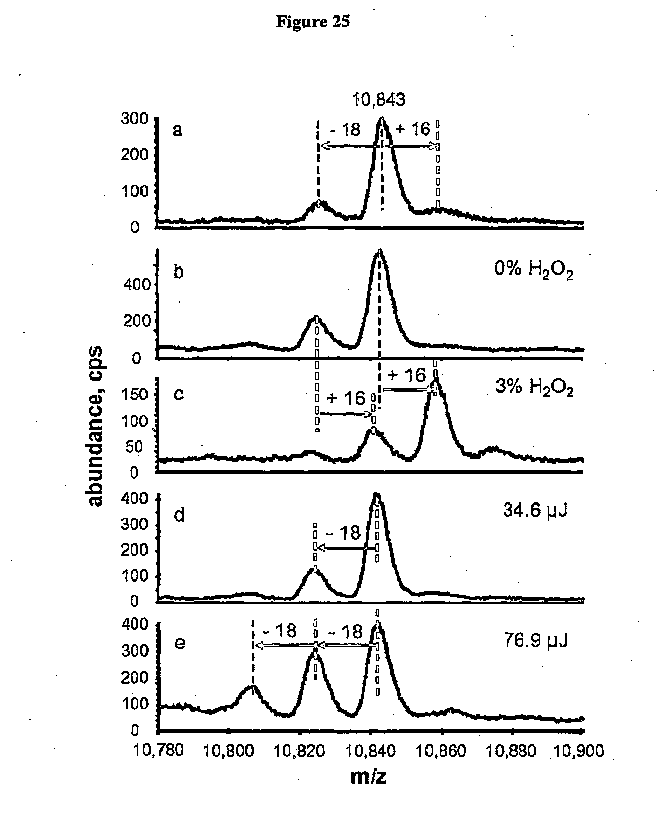

The use of SELDI-MS profiling in discovering a protein marker, chaperonin 10, for EmCa is described above. Chaperonin 10 has a molecular weight of 10,843 Da. Performing SELDI-MS on the higher resolution QqTOF mass spectrometer (QSTAR XL) was revealing: chaperonin 10 frequently appeared as three instead of a single peak (Figure 25a). On the lower resolution PBS IIc TOF mass spectrometer, which is much more widely available and used, the peaks are unresolved. Incubating the tissue homogenate with hydrogen peroxide overnight reduced the abundance ofchaperonin 10 while increased the abundance of the protein that is heavier by 16 Da (compare Figure 25b with 25c). Increasing the fluence of the MALDI laser (from 34.6 to 76.9 µJ) had the effect of increasing the abundance of the protein peak that is 18 Th lower than chaperonin 10 in m/z value. In addition, a new protein peak that is 36 Th lower in m/z value emerged (compare Figure 25d with 25e). Incubating with hydrogen peroxide enhanced oxidation of chaperonin 10, probably the lone methionine residue; as a result, the molecular weight of chaperon in 10 increased by 16 Da. Increasing the laser intensity probably promoted dehydration of chaperonin 10 within the MALDI source. The sources of the water were likely the five glutamic and eight aspartic acid residues. Laser-induced deamidation (-17 Th) could also occur, but was ruled out after careful and repeated measurements mass-calibrated with cytochrome c. Single measurements for chaperonin 10 on the QSTAR XL with external or internal calibrants carry a typical uncertainty off 2 Th.

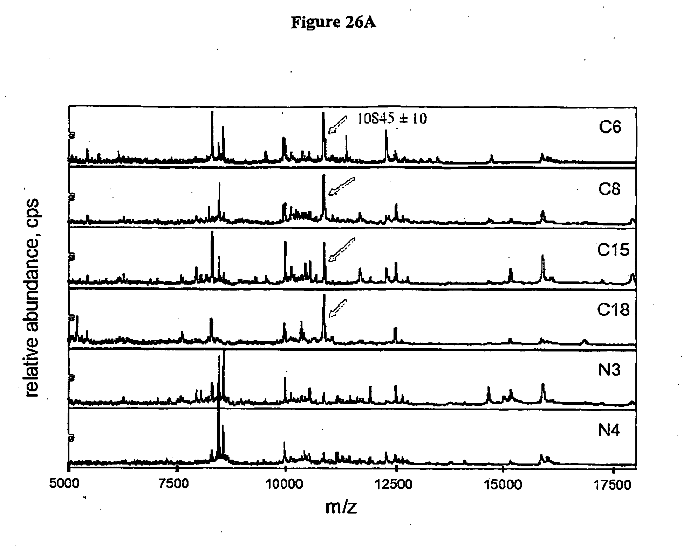

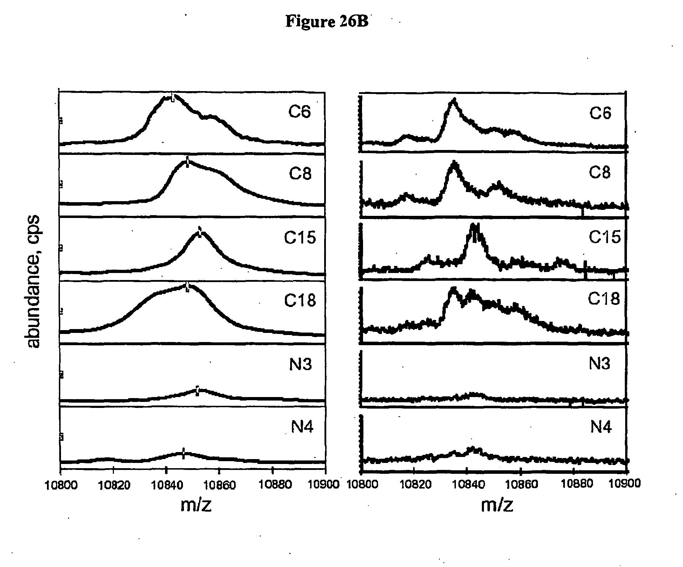

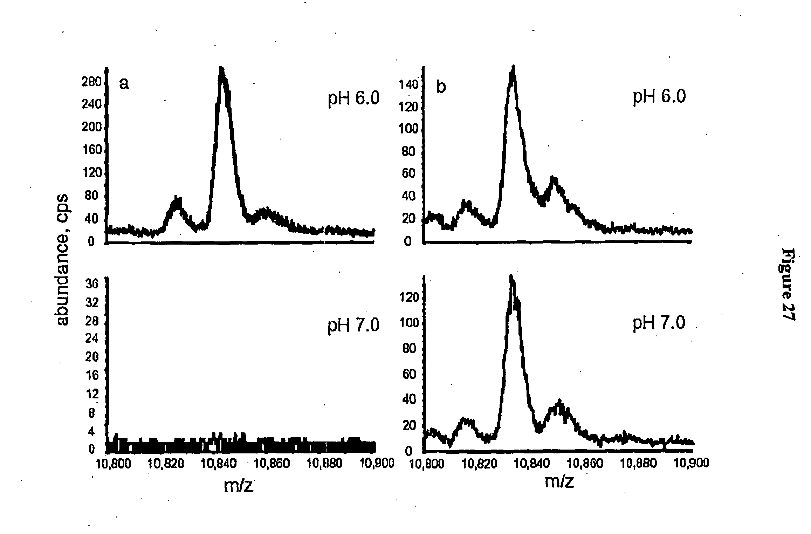

As stated earlier, the aforementioned details were unresolved on the PBS IIc TOF mass spectrometer, which displayed only one broad hump. The poor resolution in this case has no negative consequence as all peaks are different forms of chaperonin 10. However, this deficiency in resolution (and mass accuracy) may be detrimental when the sample contains a second protein of similar molecular weight. Having screened in excess of 40 endometrial homogenates (see later), it was found that a significant fraction of EmCa homogenates carry a protein that is lighter than chaperonin 10 by 8 Da. Figure 26 shows selected SELDI MS profiles, from four EmCa (designated by "C") and two normal endometria (designated by "N"), as collected on the PBS IIc TOF and the QSTAR XL QqTOF mass spectrometers. The pH of the washing buffer was 6.0. The PBS instrument exhibited better sensitivity than the QSTAR However, the profiles generated by the former contain broad humps, while those by the latter exhibit considerable details (Figure 26B). In addition, the mass accuracy of the PBS is considerably inferior to that of the QSTAR Sample C15 is the simplest of the four EmCa samples and contains only chaperonin 10, its oxidized (10,859 Th) and dehydrated (10,825 Th) forms (compare Figure 25). Sample C8 has a little chaperonin 10 and contains mostly this second protein (10,835 Th), its oxidized (10,851 Th) and dehydrated (10,817 Th) forms. Similarly, sample C6 has some chaperonin 10 admixed with the second protein. In sample C 18, the relative abundance of chaperonin 10 and the unknown protein is approximately 1 to 1. The normal samples show traces of chaperonin 10. The possibility of having two proteins that differ by 8 Da in EmCa samples was confirmed by repeating some experiments, using pH 7.0 washing buffer. In the latter condition, the ProteinChip binds only the unknown protein (Figure 27). The abundances of chaperonin 10 and this second protein in nonmalignant and malignant endometrial tissue homogenates are summarized in Tables 7 and 8, respectively. As SELDI/MALDI analyses on the same sample exhibit considerable variation from shot to shot and from spot to spot, the scales adopted, from "0" (absence) to "5+" (S/N ≥ 10 or higher) are intended to convey semiquantitative rather than quantitative information. For nonmalignant samples, chaperonin 10 and the second protein are absent or present in only very low abundance ("1+") in 22 out of 23 cases; the exception, however, is different for the two proteins. For the malignant samples, two out of 21 cases show absence of both chaperonin 10 and the second protein; in addition, two cases show absence of chaperonin 10 but high abundances of the second protein, while another two cases show high abundances of chaperonin 10 but absence of the second protein. The absence of the potential cancer markers in some malignant tissue homogenates may be due to nonoptimal MS conditions and/or absence of tumors in the tissue block homogenized and analyzed.

### Identification of the unknown protein

The vast majority of SELDI-MS applications use spectral signatures from sera to differentiate normal and diseased states without actual protein identification [22, 25, 30, 105-108]. This strategy has come under criticisms and the clinical effectiveness of serum proteomics is currently being questioned [109-119]. The identification of chaperonin 10 in EmCa tissue homogenates as a marker for endometrial cancer is described above. The identification strategy was based on SEC, SDS PAGE isolation, protein extraction, trypsin digestion and MALDI-MS/MS identification. However, the preferred strategy is shotgun nanoLC/MS/MS after initial purification using SEC.

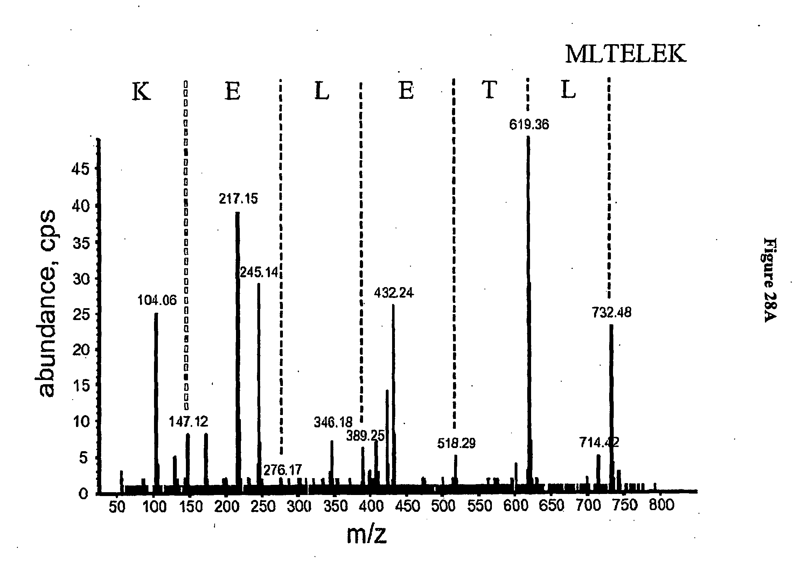

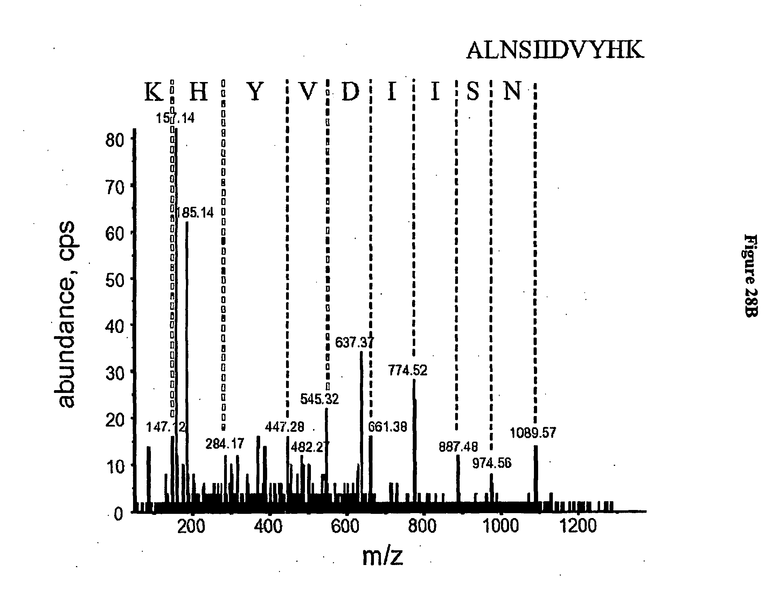

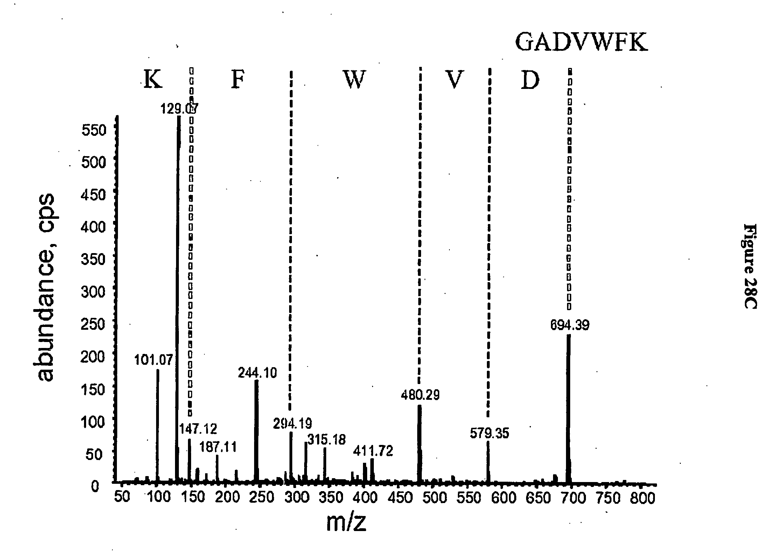

The unknown protein has a molecular weight of 10,835 Da. Using the TagIdent tool available at the ExPASy Website [133] with the following entries: molecular weight, 10,835 ± 2 Da (± 0.02%); pi, 7 ± 5; and organism classification, human to search the Swiss-Prot database that contains 151,047 entries, returned only two proteins, calgranulin A (accession number, P05109) and small nuclear ribonuclear protein D homolog (accession number, Q9Y333). A sample was used that contained the 10,835 Da protein in abundance for SEC. This protein was found to have the highest concentration in SEC fraction 7. NanoLC/MS/MS of the tryptic peptides contained in this fraction identified a number of proteins (Table 9). The only protein that has the correct molecular weight is calgranulin A, 10,834 Da. The other proteins all have higher molecular weights (see later). Figure 28 shows MS/MS spectra of three tryptic peptides, (A) MLTELEK [SEQ ID NO. 50], (B) ALNSUDVYHK [SEQ ID NO. 51] and (C) GADVWFK [SEQ ID NO. 52], that unambiguously identify calgranulin A. The first two peptides constitute residues 1-18, the third peptide residues 50-56. The three peptides constitute a sequence coverage of 25 out of a total of 93 residues or 27%.

### Calgranulin

Calgranulin A is a member of the S100 family of calcium-binding proteins [134]. It is also known as S100 calcium-binding protein A8, migration inhibitory factor-related protein 8 (MRP-8), cystic fibrosis antigen (CFAG), P8, leukocyte L1 complex light chain, and calprotectin L1L subunit. It has been reported as a specific protein for several diseases [135-156], but has not been implicated in EmCa. Calgranulin A can form homodimers as well as higher-order oligomers [139]. Calgranulin A has been found to be associated with a second protein in the S100 family, calgranulin B (13,242 Da) [137,139]. This heterodimer is thought to be involved in inflammation, including the transmigration process ofleukocytes and transport of arachidonic acid to target cells [134, 137]. In the present study, calgranulin B was also identified along with calgranulin A, albeit by only one peptide. The possibility that calgranulin A and B coexist as members of a larger unit is in accordance with the observation that calgranulin eluted early in SEC in fraction 7 along with other proteins of much higher molecular weights (Table 9). However, calgranulin B does not appear to be retained on the ProteinChips under conditions that are favorable for calgranulin A.

### Tissue microarray and immunhistochemical localization of calgranulin A

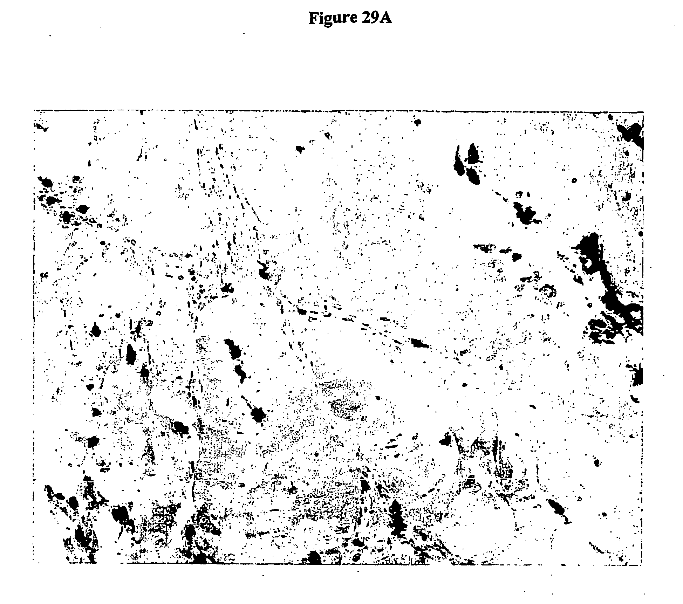

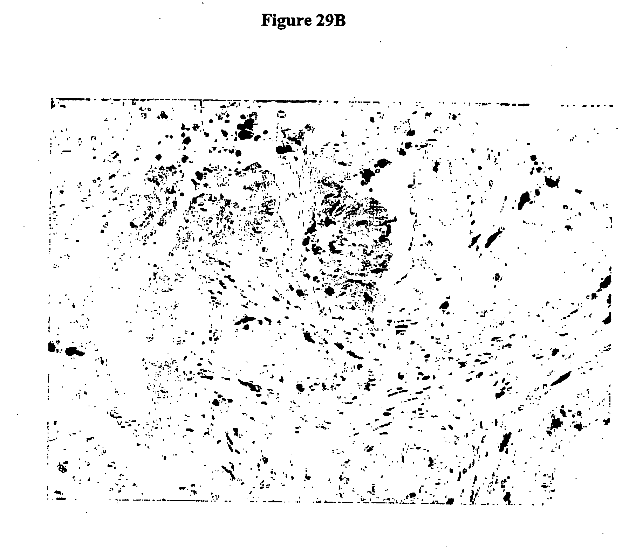

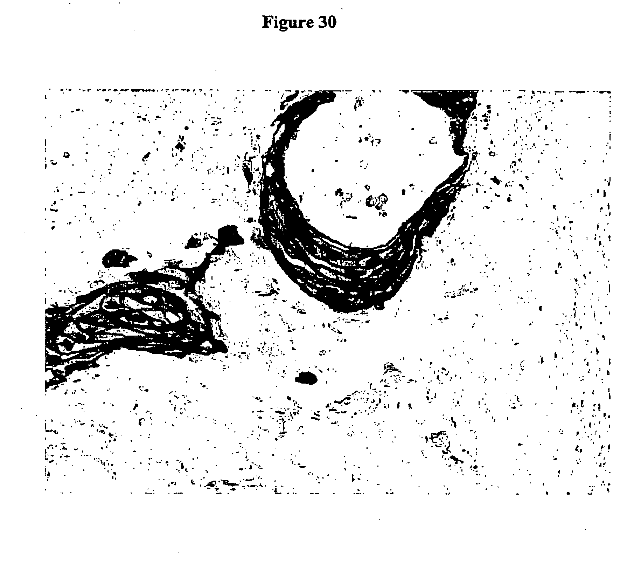

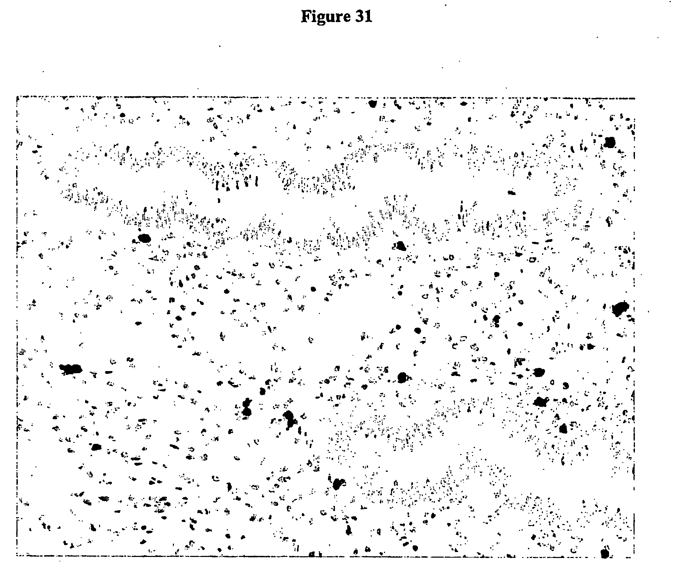

A summary of the TMA immunohistochemical studies using two different monoclonal antibodies (RDI and USB) is shown in Table 10. There is great similarity/identity between the findings from the two immunohistochemical studies. Calgranulin A was localized to the malignant epithelium of endometrial adenocarcinoma and carcinosarcoma. Approximately half of these malignant endometrial tissues showed 1+to 3+ immunostaining for calgranulin A (Figure 29A and B). The most striking positivity was noted in the squamous areas of three endometrioid carcinomas (Figure 30). Two of four non-endometrial carcinomas also exhibited immunostaining for calgranulin A in the malignant epithelium. Immunohistochemical staining for calgranulin A was absent in 9 of 10 benign endometrial cases. A single case of menstrual endometrium exhibited focal immunostaining (1+). Macrophages/granulocytes in benign endometrium (Figure 31), malignant endometrium (Figure 29), and non-endometrial carcinomas showed discrete strong immunostaining of variable numbers of cells with immunostaining ranging from 1 + to 3+. No immunostaining for calgranulin A was noted in stroma or myometrium.

The MS results presented here show that elevated levels ofcalgranulin A were detectable in about two-thirds of EmCa homogenate cases, with 14 of 21 cases demonstrating 2+ or greater abundance for this protein (Table 8). By contrast, only minor levels were observed in most nonmalignant endometrium, with 22 of23 cases demonstrating 1+ or lower abundance (Table 7).

The TMA findings not only confirm the presence of calgranulin A, at least as assessed by immunohistochemistry, but also indicate two cellular sources for the MS finding. In EmCa, the sources are both the malignant epithelium (carcinoma) and macrophage/granulocytes, whereas in nonmalignant endometrium, macrophages seem to be the predominat source, and only rarely benign (menstrual) endometrium (Table 10). The TMA analysis identified calgranulin A not only in a greater proportion of the epithelium in the EmCa cases, but also with greater intensity, than in nonmalignant endometrial cases. Both cases of atypical hyperplasia showed 1+ immunostaining of the affected endometrial glands.

Employment of calgranulin A, in combination with other protein markers (including chaperonin 10), may provide a robust endometrial cancer diagnostic and/or screening panel. The utility ofany panel will increase with the number of markers employed, and the sensitivity and specificity of individual markers. This study demonstrates the complementary nature of MS and TMA in identifying and localizing sources of potential cancer markers in this search, and a possible utility of TMA in selecting cancer markers.

### Conclusion

High mass accuracy and high resolution in the MS stage are crucial in SELDI MS. Using the QSTAR XL, two potential cancer markers were resolved and subsequently identified offline as chaperonin 10 and calgranulin A, that differ by 8 Da in mass. These two proteins were unresolved on the PBS IIc mass spectrometer. Two offline protein identification protocols were developed: the first was based on SEC, SDS PAGE, protein extraction, trypsin digestion and MALDI-MS/MS; the second on SEC and shotgun nanoLC/MS/MS. Analyses on a cohort of 44 endometrial homogenates showed 22 out of 23 nonmalignant samples had nondetectable to very low abundance of chaperonin 10 and calgranulin A; 17 of the 21 malignant samples had detectable to abundant levels of both proteins. Immunohistochemical staining of a tissue microarray of 32 samples showed that approximately half of malignant endometrial tissues exhibited positive staining for calgranulin A in the malignant epithelium, while 9 out of 10 benign tissues exhibited negative epithelial staining. In addition, macrophages/granulocytes in malignant as well as nonmalignant tissues showed positive staining. No immunostaining occurred in stroma or myometrium. Calgranulin A, in combination with chaperonin 10 and other proteins, can constitute a panel of markers to permit diagnosis and screening of endometrial cancer.

**Table 1**

| | | | |
|---|---|---|---|
| **Differentially Expressed Proteins in Endometrial Malignancies/Cancer** | | | |

| ***Protein*** | ***Gene name*** | ***Swiss-Prot Acc. No.*** | ***Expression in EmCa*** |
|---|---|---|---|
| Chaperonin 10 | HSPE1 | Q04984andAAH23518 [SEQ ID 1] NM_002157 and U07550 [SEQ ID 2] | Up |
| Calgranulin A | S100A8 | P05109 [SEQ ID 3] A12027 [SEQ ID 4] NM_002964 [SEQ ID 5] | Up |
| Calgranulin B | S100A9 | P06702 [SEQ ID 6] X06233 [SEQ ID 7] M21064 [SEQ ID 8] | Up |
| Potymenc-immunogtobuHn Receptor [precursor] | PIGS | P01833 or Q81ZV7 [SEQ ID 9] NM_002644 [SEQ ID 10] | Up |
| Phosphatidylethanolamine-binding protein (PEBP) | PBP | P30086 [SEQ ID 11] NM_002567[SEQ ID 12] | Up |
| Acidic leucine-rich nuclear phosphoprotein 32 family member | ANP32A A | P39687 [SEQ ID 13] NM_006305 [SEQ ID 14] | Up |
| Heat shock 70 kDa protein 6 | HSPA6 | P17066 [SEQ ID 15] NM 002155 [SEQ ID 16] X51757 [SEQ ID 17] | Up |
| Macrophage migration Inhibitory factor (MIF) | MIF | PI4174 [SEQ ID 18] NM_002415 [SEQ ID 19] L19686 [SEQ ID 20] | Up |
| Calgizzarin (S100C protein) | S100A11 | P31949 [SEQ ID 21] NM_005620 and D38583 [SEQ ID 22] | Up |
| Triosephosphate isomerase | TPI1 | P00938 and NP_000356 [SEQ ID 23] NM_000365 [SEQ ID 24] X69723 [SEQ ID 25] | Up |
| Alpha-1-antitrypsinprecursor | SERPINA1 | gi/1703025 ITHU and P01009 [SEQ ID 30] NM_000295[SEQ ID NO. 31] K02212 [SEQ ID 32] | Under |
| Creatine kinase, B chain (B-CK) | CKB | gi/125294 P12277[SEQ ID NO.33] NM_001823 [SEQ ID NO 34] X15334 [SEQ ID NO 35] | Under |
| Pyruvate kinase, M1 or M2 | isozyme PKM2 | gi/20178296; gi/125604; KPYI_HUMAN (P14618) [SEQ ID NO.36] X56494 [SEQ ID 37] | Up |
| Transgelin (smooth muscle protein 22-alpha) | TAGLN | gi/3123283 Q01995 [SEQ ID.38] D84342 [SEQ ID 39] | Under |
| Heterologous nuclear ribonucleoprotein D | (hnRPD | ROD_HUMAN (Q14103) [SEQ ID NO.40] AF026126 [SEQ ID 41] | Up |

**Table 2**

| **The identification of Chaperonin 10 by mass spectrometry and Western blotting techniques in non-malignant endometrial tissue homogeneates.** | | | |
|---|---|---|---|
| Case | Histopathologic Classification | MS results (relative intensity) | Western Blotting Results (relative intensity) |
| I | Proliferative | + | o |
| 2 | Proliferative | + | o |
| 3 | Secretory | + | o |
| 4 | Atrophic | + | + |
| 5 | Benign polyp | o | o |
| 6 | Atrophic | + | + |
| 7 | Secretory | o | o |
| 8 | Disordered proliferative endometrium | o | + |
| 9 | Secretory | o | + |
| 10 | Disordered proliferative | o | + |
| 11 | Secretory | o | + |
| 12 | Secretory | + | + |
| 13 | Secretory | o | + |
| 14 | Secretory | + | o |
| 15 | Atrophic | o | o |
| 16 | Secretory | + | + |
| 17 | Menstrual | + | + |
| 18 | Secretory | + | + |
| 19 | Proliferative | + | o |
| 20 | Proliferative | + | + |
| 21 | Menstrual | o | o |
| 22 | Atrophic | o | o |
| 23 | Atrophic | ++ | + |

**Table 3**

| **The identification of Chaperonin 10 by mass spectrometry and Western blotting techniques in malignant endometrial tissue homogeneates.** | | | |
|---|---|---|---|
| Case | Histopathologic Classification | MS results (relative intensity) | Western Blotting Results (relative intensity) |
| 24 | Em AdCa 1/3 | ++ | ++ |
| 25 | Em AdCa 1/3 | O | + |
| 26 | MMMT | +++++ | +++++ |
| 27 | Em AdCa | ++ | ++ |
| 28 | MMMT* | + | o |
| 29 | Em AdCa 2/3 | + | +++ |
| 30 | Em AdCa 2/3 | + | ++++ |
| 31 | Ser AdCa | + | +++++ |
| 32 | Em AdCa 1/3 | +++++ | +++++ |
| 33 | Em AdCa Grade n.k. | O | ++++ |
| 34 | Em AdCa Grade n.k. | + | +++ |
| 35 | Em AdCa Grade n.k | O | + |
| 36 | Em AdCa 1/3 | +++++ | ++++ |
| 37 | Em AdCa 1/3 | ++++ | +++ |
| 38 | Em AdCa 1/3 | +++ | +++ |
| 39 | Muc AdCa 1/3 | ++ | o |
| 40 | Em AdCa 1/3 | +++ | +++ |
| 41 | Em AdCa 1/3 | +++ | ++ |
| 42 | Em AdCa - Ser AdCa | +++++ | +++++ |
| 43 | Em** AdCa 1/3 | o | o |
| 44 | Em AdCa 2/3 | ++++ | ++++ |

| | | | |
|---|---|---|---|
| *Mirror section showed minimal tumor. **Mirror image showed necrotic tumor only. Em AdCa = Endometrioid adenocarcinoma MMMT = Malignant Mixed Mullerian Tumor Muc AdCa = Mucinous adenocarcinoma n.k. = not known Ser AdCa = Serous adenocarcinoma | | | |

**Table 4**

| **Differentially expressed proteins quantified by iTRAQ. S: P is the ratio of secretory phase relative to the proliferative phase C1: P and C2: P are the ratios of cancer samples 1 and 2 relative to the proliferative phase, respectively.** | | | | |
|---|---|---|---|---|
| **accession_id** | **name** | **S: P (± SD)** | **C1: P (± SD)** | **C2: P (± SD)** |
| gi\|461730 | 10 kDa heat shock protein, mitochondrial (Hsp 10) (10 kDa chaperonin) (CPN10) | 1.06 ± 0.12 | 2.71 ± 0.52 | 2.23 ± 0.29 |
| gi\|1703025 | Alpha-1-antitrypsin precursor | 1.25 ± 0.39 | 0.34 ± 0.07 | 0.44 ± 0.16 |
| gi\|125294 | Creatine kinase, B chain (B-CK) | 0.96 ± 0.04 | 0.38 ± 0.16 | 0.52 ± 0.06 |
| gi\|20178296 or gi\|125604 | Pyruvate kinase, M1 or M2 isozyme | 1.03 ± 0.11 | 2.75 ± 0.03 | 2.02 ± 0.08 |
| gi\|3123283 | Transgelin (Smooth muscle protein 22-alpha) | 1.46 ± 0.26 | 0.26 ± 0.04 | 0.45 ± 0.05 |

**Table 5**

| **Differentially expressed proteins quantified using cICAT. Singleton H signifies extreme Overexpression in the cancer sample relative to the normal proliferative controL Standard deviations were calculated from all three sample pairs.** | | |
|---|---|---|
| **Accession #** | **Name** | **Avg. C: N ratio (± SD)** |
| S111_HUMAN (P31949) | Calgizzarin (S100C protein) (MLN 70). | Singleton H^{a} |
| ROD_HUMAN (Q14103) | Heterogeneous nuclear ribonucleoprotein D0 | 4.62 (± 0.39) |
| MIF_HUMAN (P14174) | Macrophage migration inhibitory factor (MIF) | 2.74 (± 0.92) |
| PEBP_HUMAN (P30086) | Phosphatidylethanolamine-binding protein (PEBP) | 3.72 (± 1.03) |
| KPY1_HUMAN (P14618) | Pyruvate kinase, M1 isozyme (EC 2.7.1.40) | 4.61 (± 1.40) |
| KPY_HUMAN: PIGR_HUMAN (P01833) | Polymeric-immunoglobulin receptor precursor | 9.83 (+/- 6.61) |

| | | |
|---|---|---|
| a: Calculation of SD is not possible | | |

**Table 6**

| **Proteins Identified and Secretory / Proliferative Expression Levels** | | | | |
|---|---|---|---|---|
| **Accession #** | **Description** | **Average H: L ratio** | **SD** | **Frequency** |
| 143T_HUMAN (P27348) | 14-3-3 protein tau (14-3-3 protein theta) | **0.81** | | 1 |
| RS12_ORENI (O13019) | 40S ribosomal protein S12. | **0.52** | | 1 |
| RS21_HUMAN (P35265) | 40S ribosomal protein S21. | **NQ** | | 1 |
| HPPD_MYCGR (042764) | 4-hydroxyphenylpyruvate dioxygenase (EC 1.13.11.27) | **Singleton L** | | 1 |
| RL36_VIBCH (P78001) | 50S ribosomal protein L36. | **0.84** | | 1 |
| ACTA_HUMAN (P03996) or ACTS_HUMAN (P02568) | Actin, aortic smooth muscle or Actin alpha skeletal muscle | **1.86** | | 1 |
| ACTB_HUMAN (P02570) | Actin, cytoplasmic | **0.93** | 0.04 | 3 |
| SAH1_XENLA (P51893) | Adenosylhomocysteinase 1 (EC 3.3.1.1) | **0.70** | | 1 |
| AFAM_HUMAN (P43652) | Afamin precursor (Alpha-albumin) (Alpha-Alb). | **NQ** | | 1 |
| ADHL_HUMAN (P11766) | Alcohol dehydrogenase class III L chain | **0.67** | | 1 |
| DHA1_HUMAN (P00352) | Aldehyde dehydrogenase 1A1 (EC 1.2.1.3) | **0.60** | | 1 |
| A1AG_HUMAN (P02763) | Alpha-1-acid glycoprotein 1 precursor (AGP 1) | **1.1** | 0.5 | 3 |
| A2HS_HUMAN (P02765) | Alpha-2-HS-glycoprotein precursor (Fetuin-A) | **0.82** | 0.54 | 2 |
| ALU5_HUMAN (P39192) | Alu subfamily | **1.1** | | 1 |
| AOP2_HUMAN (P30041) | Antioxidant protein 2 (1-Cys peroxiredoxin) | **NQ** | | 1 |
| TRAI_AGRT5 (P33907) | Autoinducer synthesis protein traI. | **0.55** | | 1 |
| APOH_HUMAN (P02749) | Beta-2-glycoprotein I precursor (Apolipoprotein H) | **0.58** | | 1 |
| ARK1_HUMAN (P25098) | Beta-adrenergic receptor kinase I (EC 2.7.1.126) | **Singleton L** | | 1 |
| BPEA_HUMAN (094833) | Bullous pemphigoid antigen 1, isoforms 6/9/10 | **0.99** | | 1 |
| CLP1_HUMAN (P51911) | Calponin H1, smooth muscle (Basic calponin) | **1.8** | | 1 |
| KAP3_HUMAN (P31323) | cAMP-dependent protein kinase type II-beta | **Singleton L** | | 1 |
| CABA_MOUSE (Q99020) | CARG-binding factor-A (CBF-A). | **0.97** | 0.33 | 3 |
| CATB_HUMAN (P07858) | Cathepsin B precursor (EC 3.4.22.1) (Cathepsin B1) | **0.88** | 0.70 | 2 |
| CB32_YEAST (P40969) | Centromere DNA-binding protein complex CBF3 subunit | **NQ** | | 1 |
| BPHB_BURCE (P47227) | Cis-2,3-dihydrobiphenyl-2,3-diol dehydrogenase (EC | **Singleton L** | | 1 |
| CLH_BOVIN (P49951) | Clathrin heavy chain. | **0.62** | | 1 |
| COF1_HUMAN (P23528) | Cofilin, non-muscle isoform (18 kDa phosphoprotein) | **0.95** | 0.15 | 3 |
| CO3_HUMAN (P01024) | Complement C3 precursor | **1.1** | 0.1 | 2 |
| CFAH_HUMAN (P08603) | Complement factor H precursor (H factor 1). | **NO** | | 1 |
| AXO1_CHICK (P28685) | Contactin 2 precursor (Axonin-1). | **0.85** | | 1 |
| KCRB_HUMAN (P12277) | Creatine kinase, B chain (EC 2.7.3.2) (B-CK). | **6.0** | | 1 |
| CRBH_HUMAN (P82279) | Crumbs protein homolog I precursor. | **NQ** | | 1 |
| PYRG_STAAM (Q99SD1) | CTP synthase (EC 6.3.42) (UTP-ammonia ligase) (C | **Singleton H** | | 1 |
| CYSR_HUMAN (P21291) | Cysteine-rich protein 1 (CRP1) (CRP). | **4.4** | | 1 |
| DDX4_HUMAN (Q9NQI0) | DEAD-box protein 4 (VASA homolog). | **Singleton H** | | 1 |
| DEST_HUMAN (P18282) | Destrin (Actin-depolymerizing factor) (ADF). | **2.4** | | I |
| EF2_HUMAN (P13639) | Elongation factor 2 | **0.60** | | I |
| XYNA_PIRSP (Q12667) | Endo-1,4-beta-xylanase A precursor (EC 3.2.1.8) (1 | **0.32** | | 1 |
| ENOA_HUMAN (P06733) or MPB1_HUMAN (P22712) | Enolase (EC 4.2.1.11) (2-phosphoglycerate dehydrat.) or c-myc promoter binding protein (MBP-1) | 2.6 | 1.9 | |
| ENV_SIVM1^{a} (P05885) | Envelope polyprotein GP160 precursor | **Singleton L** | | 3 |
| LEM2_CANFA (P33730) | E-selectin precursor (Endothelial leukocyte adhesi | **Singleton H** | | 1 |
| FIBG_HUMAN (P02679) | Fibrinogen gamma chain precursor | **1.2** | | 1 |
| FLNA_HUMAN (P21333) | Filamin A (Alpha-filamin) (Filamin 1) | **1.6** | 0.8 | 3 |
| ALFA_HUMAN (P04075) | Fructose-bisphosphate aldolase A (EC 4.1.2.13) (Mu | **1.2** | 0.2 | 2 |
| G25P_HUMAN (P25763) or RAC1_HUMAN (P15154) or RHOJ_HUMAN (Q9H4E5) | G25K GTP binding protein (CDC 42 homolog) or Ras related C3 botulinum toxin substrate 1 or 2 or 3 or Rho-related GTP-binding protein Rho G or RhoJ | **NQ** | | 1 |
| LEG 1_CRIGR (P48538) | Galectin-1 (Beta-galactoside-binding lectin L-14-I) | **0.67** | 0.52 | 3 |
| GELS_HUMAN (P06396) | Gelsolin (Actin-depolymerizing factor) (ADF) | **1.4** | | 2 |
| NMZ1_HUMAN (Q05586) | Glutamate [NMDA] receptor subunit zeta 1 precursor | **Singleton H** | | 3 |
| GTP_HUMAN (P09211) | Glutathione S-transferase P (EC 2.5.1.18) | **1.3** | 0.4 | 3 |
| G3P2_HUMAN^{b} (P04406) | Glyceraldehyde 3-phosphate dehydrogenase | **1.0** | 0.0 | 3 |
| PAEP_HUMAN (P09466) | Glycodelin precursor (GD) (Pregnancy-associated en | **Singleton H** | | 1 |
| HPT1_HUMAN (P00737) | Haptoglobin-1 precursor. | **2.2** | 1.6 | 2 |
| HS70_HUMAN (08107) | Heat shock cognate 71 kDa protein. | **1.3** | 1.1 | 3 |
| HS9A_HUMAN (P07900) | Heat Shock Protein HSP90-alpha | **NQ** | | 1 |
| HBB_HUMAN (P02023) | Hemoglobin beta chain. | **0.60** | 0.45 | 3 |
| HEMO_HUMAN (P02790) | Hemopexin precursor (Beta-1B-glycoprotein). | **1.1** | 0.3 | 3 |
| ROD_HUMAN (Q14103) | Heterogeneous nuclear ribonucleoprotein D0 (hnRNP D0) | **1.0** | 0.2 | 3 |
| ROK_HUMAN (Q07244) | Heterogeneous nuclear ribonucleoprotein K (hnRNP K) | **0.68** | | 1 |
| HNT1_HUMAN (P49773) | Histidine triad nucleotide binding protein 1 | **1.4** | | 1 |
| YF48_HUMAN (Q9HCM4) | Hypothetical protein KIAA1548 | **0.85** | | 1 |
| Y885_MYCTU (Q10546) | Hypothetical protein Rv0885. | **NQ** | | 1 |
| GC1_HUMAN (P01857) | Ig gamma-1 chain C region. | **0.76** | 0.23 | 3 |
| GC2_HUMAN (P01859) or GC4_HUMAN (P01861) | Ig gamma-2 chain C region, Or Ig gamma-4 chain C region | **1.0** | 0.5 | 3 |
| GC3_HUMAN (P01860) | Ig gamma-3 chain C region | **0.85** | 0.22 | 3 |
| KAC_HUMAN (P01834) | Ig kappa chain C region. | **0.88** | 0.37 | 3 |
| HUMAN (P01876) or (P01877) | Ig alpha - 1 chain C region or Ig alpha-2 chain C region | **1.4** | | 1 |
| IDI2_RICCN^{c} (Q92HM7) | Isopentenyl-diphosphate delta-isomerase (EC 5.3.3. | **Singleton H** | | 3 |
| TRFL_MOUSE (P08071) | Lactotransferrin precursor (Lactoferrin). | **Singleton H** | | 1 |
| LGUL_HUMAN (Q04760) | Lactoylglutathione lyase (EC 4.4.1.5) (Methylglyox | **1.0** | 0.2 | 2 |
| CSR3_HUMAN (P50461) | LIM domain protein, cardiac (Muscle LIM protein) ( | **4.2** | | 1 |
| LDH_HUMAN (P00338) or (P07195) | L-lactate dehydrogenase (EC 1.1.1.27) (LDH). | **1.4** | 0.3 | 3 |
| MIF_HUMAN (P14174) | Macrophage migration inhibitory factor (MIF) | **1.2** | 0.7 | 3 |
| MSRE_MOUSE (P30204) | Macrophage scavenger receptor types I and II | **Singleton H** | | 1 |
| MDHM_HUMAN (P40926) | Malate dehydrogenase, mitochondrial precursor | **NQ** | | 2 |
| MSP1_PLAF3 (P19598) | Merozoite surface protein 1 precursor | **Singleton L** | | 1 |
| MU5A_HUMAN (P98088) | Mucin 5AC (Mucin 5 subtype AC, tracheobronchial) | **NQ** | | 1 |
| MYH9_HUMAN (P35579) or MYHA_HUMAN (P35580) | Myosin heavy chain non muscle type A or B or | **0.29** | | 1 |
| KML2_RABIT (P07313) | Myosin light chain kinase 2 | **Singleton H** | | 3 |
| NIR_NEUCR (P38681) | Nitrite reductase [NAD(P)H] (EC 1.7.1.4). | **1.3** | | 1 |
| NOG3_BRARE (Q9YHV3) | Noggin 3 precursor. | **1.3** | | 1 |
| NDKA_HUMAN (P15531) or NDKB_HUMAN (P22392) | Nucleotide diphosphate kinase A or B | **1.2** | | 1 |
| CYPH_HUMAN (P05092) | Peptidyl-prolyl cis-trans isomerase A (EC 5.2.1.8) | **1.2** | 0.2 | 3 |
| PEBP_BOVIN (P13696) | Phosphatidylethanolamine-binding protein (PEBP) (B | **1.0** | 0.2 | 2 |
| PGK_HUMAN (P00559) | Phosphoglycerate kinase (EC 2.7.2.3). | **0.99** | | 1 |
| PMG1_HUMAN (P18669) | Phosphoglycerate mutase 1 (EC 5.4.2.1) (EC 5.4.2.4 | **0.73** | | 1 |
| POL_RSVP^{d} (P03354) | POL polyprotein | **Singleton H** | | 2 |
| PIGR_HUMAN (P01833) | Polymeric-immunoglobulin receptor precursor (Poly- | **1.1** | | 1 |
| AR34_DROME (Q9VIM5) | Probable ARP2/3 complex 34 kDa subunit (P34-ARC). | **3.5** | | 1 |
| AMPA_CAMJE (Q9PP04) | Probable cytosol aminopeptidase (EC 3.4.11.1) (Leu | **0.93** | | 1 |
| PYRO1_HUMAN (P07737) | Profilin I | **1.1** | | 1 |
| PROP_HUMAN (P27918) | Properdin precursor (Factor P). | **Singleton H** | | 1 |
| PRTH_PORGI (P46071) | Protease prtH (EC 3.4.22.-). | **Singleton L** | | 1 |
| PSA5_HUMAN (P28066) | Proteasome subunit alpha type 5 (EC 3.4.25.1) (Pro | **2.1** | | 1 |
| VN02_VACCC (P20641) | Protein N2. | **Singleton L** | | 1 |
| FRT1_HUMAN (Q92837) | Proto-oncogene FRAT1 | **Singleton H** | | 3 |
| O22C_DROME (P81911) | Putative odorant receptor 22c. | **0.90** | 0.31 | 3 |
| KPY1_HUMAN (P14618) | Pyruvate kinase, M1 isozyme (EC 2.7.1.40) (Pyruvat | **1.2** | 0.4 | 3 |
| CUT1_SCHPO (P18296) | Separin (EC 3.422.-). | **NQ** | | 1 |
| TRFE_HUMAN (P02787) | Serotransferrin precursor (Transferrin) (Siderophi | **1.3** | 0.4 | 3 |
| ALBU_HUMAN (P02768) | Serum albumin precursor. | **1.1** | 0.3 | 3 |
| STC_DROME (P40798) | Shuttle craft protein. | **1.1** | | 1 |
| SLI1_HUMAN (Q13642) | Skeletal muscle LIM protein 1 | **2.0** | | 1 |
| PCP1_SCHPO (Q92351) | Spindle pole body protein pcp1. | **Singleton H** | | 1 |
| SODC_HUMAN (P00441) | Superoxide dismutase [Cu-Zn] | **0.92** | 0.33 | 3 |
| THIO_HUMAN (P10599) | Thioredoxin. | **0.87** | | 1 |
| TPIS_HUMAN (P00938) | Triosephosphate isomerase (EC 5.3.1.1) (TIM). | **1.1** | 0.5 | 3 |
| TPM1_HUMAN (P09493) | Tropomyosin 1 alpha chain (Alpha-tropomyosin). | **2.3** | 0.7 | 2 |
| TPM4_HUMAN (P07226) or TPM2_HUMAN (P07951) | Tropomyosin alpha 4 chain or beta 2 chain | **0.75** | | 1 |
| TBA_HUMAN (P05209) | Tubulin alpha chain. | **1.2** | 0.5 | 3 |
| TBB_HUMAN (P07437 or P05217)) | Tubulin beta chain (Fragment). | **0.77** | 0.25 | 3 |
| PTK7_HUMAN (Q13308) | Tyrosine-protein kinase like 7 precursor | **0.35** | | 1 |
| SPK1_DUGT1 (P42687) | Tyrosine-protein kinase SPK-1 I (EC 2.7.1.112). | **Singleton L** | | 1 |
| VSI1_TRYBB (P26326) | Variant surface glycoprotein ILTAT 1.21 precursor | **Singleton L** | | 1 |
| PGCV_MACNE (Q28858) | Versican core protein | **4.3** | | 1 |
| VIME_HUMAN (P08670) | Vimentin (Fragment). | **1.6** | 2.2 | 3 |
| VINC_HUMAN (P18206) | Vinculin (Metavinculin). | **3.1** | | 2 |
| VTDB_HUMAN (P02774) | Vitamin D-binding protein precursor (DBP) (Group-s | **1.3** | 0.4 | 2 |
| WDR1_HUMAN (075083) | WD-repeat protein 1 (Actin interacting protein 1) | **1.6** | 1.0 | 2 |
| Z363_HUMAN (Q96PM5) | Zinc finger protein 363 | **Singleton L** | | 1 |

| | | | | |
|---|---|---|---|---|
| Note: Frequency is the number of sample pairs in which the protein is observed NQ = Not quantified Singleton L = Only observed in Proliferative phase Singleton H = Only observed in Secretory phase ^{a} : Subsequently identified by Mascot as Clostridium putative phosphatidylserine decarboxylase proenzyme (gi: 28209853) ^{b} : Internal standard to which other proteins' peak areas are normalized ^{c}: *Rickettsia conorii* protein ^{d} : Subsequently identified by Mascot as unlabeled serum albumin | | | | |

**Table 7**

| **Protein abundances in nonmalignant endometrial tissue homogenates.** | | | |
|---|---|---|---|
| Case | Histopathologic Classification | Chaperonin 10 (relative abundance)^{†} | Second protein^{‡} (relative abundance)^{†} |
| 1 | Proliferative | 1+ | 0 |
| 2 | Proliferative | 1+ | 0 |
| 3 | Secretory | 1+ | 0 |
| 4 | Atrophic | 1+ | 0 |
| 5 | Benign polyp | 0 | 0 |
| 6 | Atrophic | 1+ | 1+ |
| 7 | Secretory | 0 | 1+ |
| 8 | Disordered proliferative | 0 | 0 |
| 9 | Secretory | 0 | 1+ |
| 10 | Disordered proliferative | 0 | 1+ |
| 11 | Secretory | 0 | 1+ |
| 12 | Secretory | 1+ | 0 |
| 13 | Secretory / menstrual | 0 | 5+* |
| 14 | Secretory | 1+ | 0 |
| 15 | Atrophic | 0 | 1+ |
| 16 | Secretory | 1+ | 0 |
| 17 | Menstrual | 1+ | 1+ |
| 18 | Secretory | 1+ | 0 |
| 19 | Proliferative | 1+ | 0 |
| 20 | Proliferative | 1+ | 0 |
| 21 | Menstrual | 0 | 0 |
| 22 | Atrophic | 0 | 1+ |
| 23 | Atrophic | 2+ | 1+ |

| | | | |
|---|---|---|---|
| ^{†} 0 = absence, 1+ = S/N 2-3, 2+ = S/N 3-5, 3+ = S/N 5-7, 4+ = S/N 7-10, 5+ = S/N ≥ 10. ^{‡} calgranulin A. * cervical cancer present several centimeters away. | | | |

**Table 8**

| **Protein abundances in malignant endometrial tissue homogenates.** | | | |
|---|---|---|---|
| Case | Histopathologic Classification | Chaperonin 10 (relative abundance)^{†} | Second protein^{‡} (relative abundance)^{†} |
| 24 | Em AdCa 1/3 | 2+ | 1+ |
| 25 | Em AdCa 1/3 | 0 | 5+ |
| 26 | MMMT | 5+ | 1+ |
| 27 | Em AdCa | 2+ | 5+ |
| 28 | MMMT | 1+ | 4+ |
| 29 | Em AdCa 2/3 | 1+ | 3+ |
| 30 | Em AdCa 2/3 | 1+ | 4+ |
| 31 | Ser AdCa | 1+ | 3+ |
| 32 | Em AdCa 1/3 | 5+ | 2+ |
| 33 | Em AdCa Grade n.k. | 0 | 0 |
| 34 | Em AdCa Grade n.k. | 1+ | 3+ |
| 35 | Em AdCa Grade n.k. | 0 | 0 |
| 36 | Em AdCa 1/3 | 5+ | 0 |
| 37 | Em AdCa 1/3 | 4+ | 2+ |
| 38 | Em AdCa 1/3 | 3+ | 5+ |
| 39 | Muc AdCa 1/3 | 2+ | 3+ |
| 40 | Em AdCa 1/3 | 2+ | 2+ |
| 41 | Em AdCa 1/3 | 3+ | 1+ |
| 42 | Em AdCa - Ser AdCa | 5+ | 0 |
| 43 | Em AdCa 1/3 | 0 | 5+ |
| 44 | Em AdCa 2/3 | 4+ | 4+ |

| | | | |
|---|---|---|---|
| ^{†} 0 = absence, + = S/N 2-3, 2+ = S/N 3-5, 3+ = S/N 5-7, 4+ = S/N 7-10, 5+ = S/N ≥ 10. ^{‡} calgranulin A. Em AdCa = Endometrioid adenocarcinoma MMMT = Malignant Mixed Mullerian Tumor Muc AdCa = Mucinous adenocarcinoma n.k. = not known Ser AdCa = Serous adenocarcinoma | | | |

**Table 9**

| **Proteins in SEC fraction 7 of Endometrial Cancer Tissue Identified by nano LC/MS/MS** | | |
|---|---|---|
| Name | Confidence^{a} | Averaged mol. weight (Da) |
| Calgranulin A | 99 | 10,834 |
| Calgranulin B | 99 | 13,242 |
| Heat shock protein 10 | 99 | 10,800 |
| Hemoglobin alpha chain | 90 | 15,126 |
| Hemoglobin beta chain | 99 | 15,867 |
| Putative nucleoside diphosphate kinase | 75 | 15,529 |
| Nucleoside diphosphate kinase A | 75 | 17,149 |
| Nucleoside diphosphate kinase B | 75 | 17,298 |
| Cofilin, non-muscle isoform | 90 | 18,502 |
| Cofilin, muscle isoform | 90 | 18,736 |
| Translationally controlled tumor protein | 90 | 19,595 |
| Peroxiredoxin 1 | 99 | 22,110 |
| Peroxiredoxin 2 | 99 | 21,892 |
| Neutrophil gelatinase-associated lipocalin | 99 | 22,588 |
| Glutathione S-transferase P | 99 | 23,224 |
| Triosephosphate isomerase | 90 | 26,538 |
| Peroxiredoxin 4 | 90 | 30,540 |
| NADPH dehydrogenase | 90 | 30,867 |
| Ig gamma-3 chain C region | 75 | 32,331 |
| Ig gamma-2 chain C region | 75 | 35,884 |
| Ig gamma-4 chain C region | 75 | 35,940 |
| Ig gamma-1 chain C region | 75 | 36,106 |
| C-myc promoter-binding protein | 99 | 37,087 |
| Hetero. nuclear ribonucleoproteins A2 | 99 | 37,429 |
| Cathepsin B precursor | 99 | 37,807 |
| Beta enolase | 75 | 46,855 |
| Alpha enolase | 99 | 47,037 |
| Gamma enolase | 75 | 47,137 |
| Alpha enolase, lung specific | 99 | 49,477 |
| Serum albumin precursor | 99 | 69,366 |
| Polymeric-immunoglobulin receptor precursor | 75 | 83,313 |
| Cone cGMP-specific 3',5'-cyclic phosphodiesterase | 75 | 99,102 |

| | | |
|---|---|---|
| ^{a} "Confidence" value in ProID: the highest possible score is 99. | | |

**Table 10**

| **Immunohistochemical identification of calgranulinA in tissue microarray using two monoclonal mouse antibodies (RDI & USB)** | | | | | | |
|---|---|---|---|---|---|---|
| **Histologic Classification** | **Macrophage/ Granulocytic** | | **Epithelium/ Carcinoma** | | **Stroma/ Myometrium** | |
| | **RDI** | **USB** | **RDI** | **USB** | **RDI** | **USB** |
| Benign endometrium, n = 10‡ | *0* (1) | 0 (0) | *0* (9) | 0 (9) | *0* (10) | 0 (10) |
| | *1*+(6) | 1+ (10) | *1*+ (1) | 1+ (1) | *1*+ (0) | 1+ (0) |
| | *2*+ (3) | 2+ (0) | *2*+ (0) | 2+ (0) | *2*+ (0) | 2+ (0) |
| | *3*+ (0) | 3+ (0) | *3*+ (0) | 3+ (0) | *3*+ (0) | 3+ (0) |
| Atypical endometrial hyperplasia, n = 2 | *1*+ (2) | 1+ (2) | *1*+ (2) | *1*+ (2) | *0* (2) | 0 (2) |
| EmCa, n = 13† | *0*(1) | 0 (1) | *0* (6) | 0 (7) | *0*(12) | 0 (13) |
| | *1*+ (7) | 1+ (8) | *1*+ (4) | 1+ (2) | *1*+ (0) | 1+ (0) |
| | *2*+ (3) | 2+ (3) | *2*+ (2) | 2+ (2) | *2*+ (0) | 2+ (0) |
| | *3*+ (1) | 3+ (1) | *3*+ (0) | 3+ (2) | *3*+ (0) | 3+ (0) |
| Non-endometrial carcinomas, n = 4 | *0* (0) | 0 (1) | *0* (2) | 0 (2) | *0* (4) | 0 (4) |
| | *1*+ (1) | 1+ (1) | *1*+ (1) | 1+ (1) | *1*+ (0) | 1+ (0) |
| | *2*+ (3) | 2+ (2) | *2*+ (1) | 2+ (1) | *2*+ (0) | 2+ (0) |
| | *3*+ (0) | 3+ (1) | *3*+ (0) | 3+ (0) | *3*+ (0) | 3+ (0) |
| Benign non-endometrial neoplasms, n = 2 | *0* (0) | 0 (1) | *0* (0) | 0 (0) | *0* (0) | 0 (0) |

| | | | | | | |
|---|---|---|---|---|---|---|
| ( ) = Number of samples *0* = No immunostaining *1*+ = Positivity in occasional or few cells *2*+ = Positivity in more abundant cells *3*+ = Strong positivity in numerous cells † = In TMA- RDI, one core of EmCa was not displayed ‡ = 1 of 11 benign endometrial cores (secretory) did not display in either TMA. | | | | | | |

### Full Citations for References

1. Buckley CH: Normal endometrium and non-proliferative conditions of the endometrium. In Obstetrical and Gynaecological Pathology. Fox H, Wells M. Eds. Churchill-Livingstone. 5th Ed. London, 2003, Pgs. 391 - 442.
2. Andersen JS, Mann M (2000) FEBS Lett 480: 25-31.
3. Yao X, Freas A, Ramirez J, Demirev PA, Fenselau C (2001) Anal Chem 73: 2836-2842.
4. Geng M, Ji J, Regnier F (2000) J Chromoatogr A 870: 295-313.
5. Manchbach M, Quadroni M, Miotto G, James P (2000) Anal Chem 72: 4047-4057.
6. Gygi SP, Rist B, Gerber SA, Turecek F, Gelb MH, Aebersold R (1999) Nat Biotechnol 17: 994-999.
7. Goshe MB, Conrads TP, Panisko EA, Angell NH, Veenstra TD, Smith RD (2001) Anal Chem 73: 2578-2586.
8. Williamson B, Marchese J, Juhasz P, Hatten S, Patterson D, Graber A, Khainovski N, Romeo A, Martin S (2002) Proceedings of the 50th ASMS Conference on Mass Spectrometry and Allied Topics, Orlando.
9. Arcuri F, Ricci C, Ietta F, Cintorino M, Tripodi SA, Cetin I, Garzia E, Schatz F, Klemi P, Santopietro R, Paulesu L (2001) Biol Reprod 64: 1200 -1205.
10. Canadian Cancer Statistics 2003. Canadian Cancer Society. 2003. 7-28-0003. Ref Type: Electronic Citation
11. Cancer Facts and Figures. American Cancer Society. 2003. 7-25-0003. Ref Type: Electronic Citation
12. Rose PG: Endometrial carcinoma. N Engl J Med 1996,335:640-649.
13. Agboola OO, Grunfeld E, Coyle D, Perry GA: Costs and benefits of routine follow-up after curative treatment for endometrial cancer. CMAJ 1997, 157:879-886.
14. Duffy MJ: Clinical uses of tumor markers: a critical review. Crit Rev Clin Lab Sci 2001, 38:225-262.
15. Chambers G, Lawrie L, Cash P, Murray Gl: Proteomics: a new approach to the study of disease. J Pathol 2000, 192:280-288.
16. Peng J, Gygi SP: Proteomics: the move to mixtures. J Mass Spectrom 2001, 36:1083-1091.
17. Wu W, Hu W, Kavanagh JJ: Proteomics in cancer research. Int J Gynecol Cancer 2002, 12:409-423.
18. Bryant-Greenwood PK, Petricoin 3rd EF, Abati A, Liotta L: High-Throughput Proteomic Analysis of Microdissected Cytological Specimens Yields Distinct Expression Profiles of Follicular vs Papillary Thyroid Carcinomas. Mod Pathol 2000, 13:30A.
19. Jones MB, Krutzsch H, Shu H, Zhao Y, Liotta LA, Kohn EC, Petricoin EF, III: Proteomic analysis and identification of new biomarkers and therapeutic targets for invasive ovarian cancer. Proteomics 2002, 2:76-84.
20. Knezevic V, Leethanakul C, Bichsel VE, Worth JM, Prabhu VV, Gutkind JS, Liotta LA, Munson PJ, Petricoin EF, III, Krizman DB: Proteomic profiling of the cancer microenvironment by antibody arrays. Proteomics 2001, 1:1271-1278.
21. Paweletz CP, Gillespie JW, Ornstein DK, Simone NL, Brown MR, Cole KA, Wang Q-H, Huang J, Hu N, Yip T-T, Rich WE, Kohn EC, Linehan WM, Weber T, Taylor P, Emmert-Buck MR, Liotta LA, Petricoin EF, III: Rapid Protein Display Profiling of Cancer Progression Directly From Human Tissue Using a Protein Biochip. Drug Dev Res 2000, 49:34-02.
22. Petricoin EF, Ardekani AM, Hitt BA, Levine PJ, Fusaro VA, Steinberg SM, Mills GB, Simone C, Fishman DA, Kohn EC, Liotta LA: Use of proteomic patterns in serum to identify ovarian cancer. Lancet 2002, 359:572-577.
23. Vlahou A, Schellhammer PF, Mendrinos S, Patel K, Kondylis FI, Gong L, Nasim S, Wright JG, Jr.: Development of a novel proteomic approach for the detection of transitional cell carcinoma of the bladder in urine. Am J Pathol 2001, 158:1491-1502.
24. Wright Jr GL, Cazares LH, Leung S-M, Nasim S, Adam B-L, Yip T-T, Schellhammer PF, Gong L, Vlahou A: Proteinchip® surface enhanced laser desorption/ionization (SELDI) mass spectrometry: a novel protein biochip technology for detection of prostate cancer biomarkers in complex protein mixtures. Prostate Cancer and Prostatic Diseases 1999, 2:264-276.
25. Wulfkuhle JD, McLean KC, Paweletz CP, Sgroi DC, Trock BJ, Steeg PS, Petricoin EF, III: New approaches to proteomic analysis of breast cancer. Proteomics 2001, 1:1205-1215.
26. Blaustein's Pathology of the Female Genital Tract. New York, Springer-Verlag, 2002.
27. Cavanagh AC, Morton H: The purification of early-pregnancy factor to homogeneity from human platelets and identification as chaperonin 10. Eur J Biochem 1994, 222:551-560.
28. Inoue M: Current molecular aspects of the carcinogenesis of the uterine endometrium. Int J Gynecol Cancer 2001, 11:339-348.
29. Mutter GL, Baak JP, Fitzgerald JT, Gray R, Neuberg D, Kust GA, Gentleman R, Gullans SR, Wei LJ, Wilcox M: Global expression changes of constitutive and hormonally regulated genes during endometrial neoplastic transformation. Gynecol Oncol 2001, 83:177-185.
30. Wulfkuhle JD, Liotta LA, Petricoin EF: Proteomic applications for the early detection of cancer. Nat Rev Cancer 2003, 3:267-275.
31. Walch A, Komminoth P, Hutzler P, Aubele M, Hofler H, Werner M: Microdissection oftissue sections: application to the molecular genetic characterisation of premalignant lesions. Pathobiology 2000, 68:9-17.
32. Sugiyama Y, Sugiyama K, Hirai Y, Akiyama F, Hasumi K: Microdissection is essential for gene expression profiling of clinically resected cancer tissues. Am J Clin Pathol 2002, 117:109-116.
33. Batorfi J, Ye B, Mok SC, Cseh I, Berkowitz RS, Fulop V: Protein profiling of complete mole and normal placenta using ProteinChip analysis on laser capture microdissected cells. Gynecol Oncol2003, 88:424-428.
34. Jr GW, Cazares LH, Leung SM, Nasim S, Adam BL, Yip TT, Schellhammer PF, Gong L, Vlahou A: Proteinchip(R) surface enhanced laser desorption/ionization (SELDI) mass spectrometry: a novel protein biochip technology for detection of prostate cancer biomarkers in complex protein mixtures. Prostate Cancer Prostatic Dis 1999, 2:264-276.
35. von Eggeling F, Davies H, Lomas L, Fiedler W, Junker K, Claussen U, Ernst G: Tissue-specific microdissection coupled with ProteinChip array technologies: applications in cancer research. BioTechniques 2000, 29:1066-1070.
36. Somodevilla-Torres MJ, Hillyard NC, Morton H, Alewood D, Halliday JA, Alewood PF, Vesey DA, Walsh MD, Cavanagh AC: Preparation and characterization of polyclonal antibodies against human chaperonin 10. Cell Stress Chaperones 2000, 5:14-20.
37. Cornford PA, Dodson AR, Parsons KF, Desmond AD, Woolfenden A, Fordham M, Neoptolemos JP, Ke Y, Foster CS: Heat shock protein expression independently predicts clinical outcome in prostate cancer. Cancer Res 2000, 60:7099-7105.
38. Quinn KA, Morton H: Effect of monoclonal antibodies to early pregnancy factor (EPF) on the in vivo growth of transplantable murine tumours. Cancer Immunol Immunother 1992, 34:265-271.
39. Kato S, Kato M, Hirano A, Takikawa M, Ohama E: The immunohistochemical expression of stress-response protein (srp) 60 in human brain tumours: relationship of srp 60 to the other five srps, proliferating cell nuclear antigen and p53 protein. Histol Histopathol 2001, 16:809-820.
40. Tabibzadeh S, Kong QF, Satyaswaroop PG, Babaknia A: Heat shock proteins in human endometrium throughout the menstrual cycle. Hum Reprod 1996, 11:633-640.
41. Ota H, Igarashi S, Hatazawa J, Tanaka T: Distribution of heat shock proteins in eutopic and ectopic endometrium in endometriosis and adenomyosis. Fertil Steril 1997, 68:23-28.
42. Athanasas-Platsis S, Quinn KA, Wong TY, Rolfe BE, Cavanagh AC, Morton H: Passive immunization of pregnant mice against early pregnancy factor causes loss of embryonic viability. J Reprod Fertil 1989, 87:495-502.
43. Athanasas-Platsis S, Morton H, Dunglison GF, Kaye PL: Antibodies to early pregnancy factor retard embryonic development in mice in vivo. J Reprod Fertil 1991, 92:443-451.
44. Athanasas-Platsis S, Corcoran CM, Kaye PL, Cavanagh AC, Morton H: Early pregnancy factor is required at two important stages of embryonic development in the mouse. Am J Reprod Immunol 2000, 43:223-233.
45. Noonan FP, Halliday WJ, Morton H, Clunie GJ: Early pregnancy factor is immunosuppressive. Nature 1979,278:649-651.
46. Quinn KA, Cavanagh AC, Hillyard NC, McKay DA, Morton H: Early pregnancy factor in liver regeneration after partial hepatectomy in rats: relationship with chaperonin 10. Hepatology 1994, 20:1294-1302.
47. Quinn KA: Active immunization with EPF suppresses the formation of immune ascites in BALB/c mice. Immunol Cell Biol 1991, 69 (Pt 1):1-6.
48. National Cancer Institute of Canada: Canadian Cancer Statistics 2003, Toronto, Canada, 2003 [Serial online] April 2003; Available from: URL: http://www.bc.cancer.ca/vgn/images/portal/cit 776/61/38/56158640niw stats en.pdf
49. DeSouza, L.; Diehl, G.; Yang, E.C.C.; Guo, J.; Rodrigues, M.J.; Romaschin, A.D.; Colgan, T.J.; Siu, K.W.M. Proteomic Analysis of the Proliferative and Secretory Phases of the Human Endometrium: Protein Identification and Differential Protein Expression. Proteomics. 2004, in press.
50. Yang, E.C.C.; Guo, J.; Diehl, G.; DeSouza, L.; Rodrigues, M.J.; Romaschin, A.D.; Colgan, T.J.; Siu, K.W.M. Expression Profiling of Endometrial Malignancies Reveals a New Tumor Marker. Chaperonin 10.J. Proteome Res. 2004 3, 636-643.
51. Guo, J.; Yang, E.C.C.; DeSouza, L.; Diehl, G.; Rodrigues, M.J.; Romaschin, A.D.; Colgan, T.J.; Siu, K.W.M. A Strategy for High-Resolution and Protein Identification in Surface- Enhanced Laser Desorption / Ionization (SELDI) Mass Spectrometry: Calgranulin A and Chaperonin 10 as Protein Markers for Endometrial Carcinoma. Proteomics.
52. Li, J.; Steen, H.; Gygi, S.P. Protein profiling with cleavable isotope-coded affinity tag (cICAT) reagents. Mol. Cell. Proteomics 2003, 2, 1198 - 1204.
53. Cappello, F.; Bellafiore, M.; David, S.; Anzalone, R.; Zummo; G. Ten kilodalton heat shock protein (HSP10) is overexpressed during carcinogenesis of large bowel and uterine exocervix. Cancer Lett. 2003, 196, 35 - 81.
54. Fan, X.; Yan, L.; Jia, S.; Ma, A.; Qiao, C. A study of early pregnancy factor activity in the sera of women with trophoblastic tumour. Am. J. Reprod. Immunol. 1999, 41, 204-208.
55. Kim, C.W.; Kim, J.I.; Park, S.H.; Han, J.Y.; Kim, J.K.; Chung, K.W.; Sun, H.S. Usefulness of plasma tumor M2-pyruvate kinase in the diagnosis of gastrointestinal cancer. Korean J Gastroenterol. 2003,42, 387 -393.
56. Hardt, P.D.; Toepler, M.; Ngoumou, B.; Rupp, J.; Kloer, H.U. Fecal pyruvate kinase concentrations (ELISA based on a combination of clone 1 and clone 3 antibodies) for gastric cancer screening. Anticancer Res. 2003, 23, 855 - 857.
57. Marshall, M.J.; Goldberg, D.M.; Neal, F.E.; Millar, D.R. Enzymes of glucose metabolism in carcinoma of the cervix and endometrium of the human uterus. Br J Cancer. 1978, 37, 990 - 1001.
58. Tanaka, M.; Adzuma, K.; Iwami, M.; Yoshimoto, K.; Monden, Y.; Itakura, M. Human calgizzarin; one colorectal cancer-related gene selected by a large scale random cDNA sequencing and northern blot analysis. Cancer Lett. 1995, 89, 195 - 200.
59. Chaurand, P.; DaGue, B.B.; Pearsall, R.S.; Threadgill, D.W.; Caprioli, R.M. Profiling proteins from azoxymethane-induced colon tumors at the molecular level by matrix-assisted laser desorption/ionization mass spectrometry. Proteomics 2001, 1, 1320- 1326.
60. Blaxall, B.C.; Dwyer-Nield, L.D.; Bauer, A.K.; Bohlmeyer, T.J.; Malkinson, A.M.; Port, J.D. Differential expression and localization of the mRNA binding proteins, AU-rich element mRNA binding protein (AUFI) and Hu antigen R (HuR), in neoplastic lung tissue. Mol. Carcinog. 2000, 28, 76 - 83.
61. Gouble, A.; Grazide, S.; Meggetto, F.; Mercier, P.; Delsol, G.; Morello, D. A new player in oncogenesis: AUFI/hnRNPD overexpression leads to tumorigenesis in transgenic mice. Cancer Res. 2002, 62, 1489-1495.
62. Nishihira, J.; Ishibashi, T.; Fukushima, T.; Sun, B.; Sato, Y.; Todo, S. Macrophage migration inhibitory factor (MIF): Its potential role in tumor growth and tumor-associated angiogenesis. Ann. New York Acad. Sci. 2003, 995, 171 - 182.
63. Ren, Y.; Tsui, H.T.; Poon, R.T.; Ng, I.O.; Li, Z.; Chen, Y.; Jiang, G.; Lau, C.; Yu, W.C.; Bacher, M.; Fan, S.T. Macrophage migration inhibitory factor: roles in regulating tumor cell migration and expression of angiogenic factors in hepatocellular carcinoma. Int. J. Cancer. 2003, 107, 22 - 29.
64. Campa, M.J.; Wang, M.Z.; Howard, B.; Fitzgerald, M.C.; Patz, E.F. Jr. Protein expression profiling identifies macrophage migration inhibitory factor and cyclophilin a as potential molecular targets in non-small cell lung cancer. Cancer Res. 2003, 63, 1652 - 1656.
65. Markert, J.M.; Fuller, C.M.; Gillespie, G.Y.; Bubien, J.K.; McLean, L.A.; Hong, R.L.; Lee, K.; Gullans, S.R.; Mapstone, T.B.; Benos, D.J. Differential gene expression profiling in human brain tumors. Physiol. Genomics. 2001, 555, 21 - 33.
66. Bacher, M.; Schrader, J.; Thompson, N.; Kuschela, K.; Gemsa, D.; Waeber, G.; Schlegel, J. Up-regulation of macrophage migration inhibitory factor gene and protein expression in glial tumor cells during hypoxic and hypoglycemic stress indicates a critical role for angiogenesis in glioblastoma multiforme. Am. J. Pathol. 2003, 162, 11 - 17.
67. Mahutte, N.G.; Matalliotakis, I.M.; Goumenou, A.G.; Koumantakis, G.E.; Vassiliadis, S.; Arici, A. Elevations in peritoneal fluid macrophage migration inhibitory factor are independent of the depth of invasion or stage of endometriosis. Fertil. Steril. 2004, 82, 97 - 101.
68. Rossel, M.; Brambilla, E.; Billaud, M.; Vuitton, D.A.; Blanc-Jouvan, F.; Biichle, S.; Revillard, J.P. Nonspecific increased serum levels of secretory component in lung tumors: relationship to the gene expression of the transmembrane receptor form. Am. J. Respir. Cell. Mol. Biol. 1993, 9, 341 - 346.
69. Rossel, M.; Billerey, C.; Bittard, H.; Ksiazek, P.; Alber, D.; Revillard, J.P.; Vuitton, D.A. Alterations in polymeric immunoglobulin receptor expression and secretory component levels in bladder carcinoma. Urol. Res. 1991, 19, 361 - 366.
70. Sun, Z.; Yang, P. Role of imbalance between neutrophil elastase and alpha 1-antitrypsin in cancer development and progression. Lancet Oncol. 2004, 5, 182 - 190.
71. Ryu, J.W.; Kim, H.J.; Lee, Y.S.; Myong, N.H.; Hwang, C.H.; Lee, G.S.; Yom, H.C. The proteomics approach to find biomarkers in gastric cancer. J. Korean Med. Sci. 2003, 18, 505 - 509.
72. Nejjari, M.; Berthet, V.; Rigot, V.; Laforest, S.; Jacquier, M.F.; Seidah, N.G.; Remy, L.; Bruyneel, E.; Scoazec, J.Y.; Marvaldi, J.; Luis, J. Inhibition of proprotein convertases enhances cell migration and metastases development of human colon carcinoma cells in a rat model. Am. J. Pathol. 2004, 164, 1925 - 1933.
73. Choi, H.; Park, C.S.; Kim, B.G.; Cho, J.W.; Park, J.B.; Bae, Y.S.; Bae, D.S. Creatine kinase B is a target molecule of reactive oxygen species in cervical cancer. Mol. Cells. 2001, 12, 412 - 417.
74. Joseph, J.; Cardesa, A.; Carreras, J. Creatine kinase activity and isoenzymes in lung, colon and liver carcinomas. Br. J. Cancer. 1997, 7776, 600 - 605.
75. Shields, J.M.; Rogers-Graham, K.; Der, C.J. Loss of transgelin in breast and colon tumors and in RIE-1 cells by Ras deregulation of gene expression through Raf-independent pathways. J. Biol. Chem. 2002, 277, 9790 - 9799.
76. Chang, J.W.; Jeon, H.B.; Lee, J.H.; Yoo, J.S.; Chun, J.S.; Kim, J.H.; Yoo, YJ. Augmented expression of peroxiredoxin I in lung cancer. Biochem. Biophys. Res. Commun. 2001, 289, 507 - 512.
77. Klade, C.S.; Voss, T.; Krystek, E.; Ahom, H.; Zatloukal, K.; Pummer, K.; Adolf, G.R. Identification of tumor antigens in renal cell carcinoma by serological proteome analysis. Proteomics 2001, 1, 890 - 898.
78. Chen, G.; Gharib, T.G.; Huang, C.C.; Thomas, D.G.; Shedden, K.A.; Taylor, J.M.; Kardia, S.L.; Misek, D.E.; Giordano, T.J.; Iannettoni, M.D.; Orringer, M.B.; Hanash, S.M.; Beer, D.G. Proteomic analysis of lung adenocarcinoma: identification of a highly expressed set of proteins in tumors. Clin. Cancer Res. 2002, 8, 2298 - 2305.
79. Lichtenfels R, Kellner R, Atkins D, Bukur J, Ackermann A, Beck J, Brenner W, Melchior S, Seliger B. Identification of metabolic enzymes in renal cell carcinoma utilizing PROTEOMEX analyses. Biochim. Biophys. Acta. 2003, 1646, 21 - 31.
80. Crnogorac-Jurcevic, T.; Efthimiou, E.; Nielsen, T.; Loader, J.; Terris, B.; Stamp, G.; Baron, A.; Scarpa, A.; Lemoine, N.R. Expression profiling of microdissected pancreatic adenocarcinomas. Oncogene 2002, 21, 4587-4594.
81. Kuramitsu, Y.; Fujimoto, M.; Tanaka, T.; Ohata, J.; Nakamura, K. Differential expression of phosphatidylethanol-aroine-binding protein in rat hepatoma cell lines: analyses of tumor necrosis factor-alpha-resistant cKDH-8/11 and -sensitive KDH-8/YK cells by two-dimensional gel electrophoresis. Electrophoresis 2000, 21, 660 - 664.
82. Srivastava, S.; Verma, M.; Henson,D.E. Biomarkers for early detection of colon cancer. Clin. Cancer Res. 2001, 7, 1 I 18-1126.
83. Vlahou, A.; Schellhammer, P.F.; Mendrinos, S.; Patel, K.; Kondylis, F.I.; Gong, L.; Nasim,S.; Wright, J.G., Jr. Development of a novel proteomic approach for the detection of transitional cell carcinoma of the bladder in urine. Am. J. Pathol. 2001, 158, 1491-1502.
84. Bundred, N.J. Prognostic and predictive factors in breast cancer. Cancer Treat. Rev. 2001, 27, 137-142.
85. Kao, L.C., Tulac, S., Lobo, S., Imani, B., Yang, J.P., Germeyer, A., Osteen, K., Taylor, R.N., Lessey, B.A., Guidice, L.C., Endocrinology 2002, 143, 2119-2138.
86. Hathout, Y., Riordan, K., Gehrmann, M., Fenselau, C., J. Prot. Res. 2002, 1,435-442.
87. Rautajoki, K., Nyman, T.A., Lahesmaa, R., Proteomics 2004, 4, 84 - 92.
88. Bigonnesse, F., Labelle, Y., Akoum, A., Fertility and Sterility 2001, 75, 79 - 87.
89. Fujiwaki, R., Iida, K., Nakayama, K., Kanasaki, H., Ozaki, T., Hata, K., Sakai, E., Miyazaki, K., Virchows Arch. 2003, 443, 672 - 677.
90. von Wolff, M., Ursel, S., Hahn, U., Steldinger, R., Strowitzki, T., J. Clin. Endocrin. Met. 2003, 88, 3885-3892.
91. Duchen, M.R., Diabetes 2004, 53, Suppl. I, S96 - S 102.
92. Freemantle, S.J., Portland, H.B., Ewings, K., Dmitrovsky, F., DiPetrillo, K., Spinella, M.J., Dmitrovsky, E., Gene 2002, 291, 17 - 27.
93. Jonkers, J., Korswagen, H.C., Acton, D., Breuer, M., Berns, A., EMBO J. 1997, 16, 441 -450.
94. Saitoh, T., Mine, T., Katoh, M., Int. J. Oncol. 2002, 20, 785 - 795
95. Hongisto, V., Smeds, N., Brecht, S., Herdegen, T., Courtney, MJ., Coffey, E.T., Mol. Cell Biol. 2003, 23, 6027 - 6036.
96. Maldonado, V., Castilla, J.A., Martinez, L., Herruzo, A., Concha, A., Fontes, J., Mendoza, N., Garcia-Pena, M.L., Mendoza, J.L., Magan, R., Ortiz, A., Gonzalez, E., J. Assist. Reprod. Genet. 2003, 20, 474 - 481.
97. Richardson, M.R., Taylor, D.A., Casey, M.L., MacDonald, P.C., Stull, J.T. In Vitro Cell Dev. Biol. 1987, 23, 21 - 28.
98. Carson, D.D., Lagow, E., Thathiah, A., Al-Shami, R., Farach-Carson, M.C., Vernon, M., Yuan, L., Fritz, M.A., Lessey, B. Mol. Hum. Reprod. 2002, 8, 871 - 879.
99. Jokimaa, V., Oksjoki, S., Kujari, H., Vuorio, E., Anttila, L., Mol. Hum. Reprod. 2001, 7, 73 - 78.
100. Barnette, K.G., Sarkar, M.A., Glover, D.D., Li, P., Boyd, C., Lalka, D., Gynecol. Obstet. Invest. 1999, 47, 114-119.
101. Herrmann, U, Jr., Degiampietro, P., Peheim, E., Bachmann, C., Arch. Gynecol. 1987, 240, 233 - 240.
102. Meier, U., Beier-Hellwig, K., Klug, J., Linder, D., Beier, H.M., Hum. Reprod. 1995, 10, 1305 - 1310.
103. Berkova, N., Lemay, A., Dresser, D.W., Fontaine, J.-Y., Kerizit, J., Goupil, S., Mol. Hum. Reprod. 2001, 7, 747-754.
104. Li, C., Hong, Y., Tan, Y.-X., Zhou, H., Ai, J.-H., Li, S.-J., Zhang, L., Xia, Q.-C., Wu, J.-R., Wang, H.-Y., Zeng, R., Mol. Cell Proteomics 2004, 3, 399 - 409.
105. Issaq, H.J., Conrads, T.P., Prieto, D.A., Tirumalai, R., Veenstra, T.D., Anal. Chem. 2003, 75, 149A . -155A.
106. Petricoin III, E.F., Ornstein, D.K., Pawletz, C.P., Ardekani, A., Hackett, P.S., Hitt, B.A., Valassco, A., Trucco, C., Wiegand, L., Wood, K., Simone, C.V., Leving, P.J., Linehan, W.M., Emmert-Buck, M.R., Steinberg, S.M., Kohn, E.C., Liotta, L.A. J. Natl. Cancer Inst. 2002, 94, 1576 - 1578.
107. Kozak, K.R., Amneus, M.W., Pusey, S.M., Su, F., Luong, M.N., Luong, S.A., Reddy, S.T., Farias-Eisner, R., Proc. Natl. Acad. Sci. USA 2003, 100, 12343 - 12348.
108. Zhu, W., Wang, X., Ma, Y., Rao, M., Glimm, J., Kovach, J.S., Proc. Natl. Acad. Sci. USA 2003, 100, 14666 -14671.
109. Diamandis, E.P., Lancet 2002, 360, 170.
110. Diamandis, E.P., J. Natl. Cancer Inst. 2003, 95, 489-490.
111. Diamandis, E.P., Clin. Chem. 2003, 49, 1271 - 1278.
112. Sorace, J.M., Zhan, B. BMC Bioinformatics 2003, 4, 24.
113. Cottingham, K., Anal. Chem. 2003, 75, 472A -476A.
114. Marshall, J., Kupchak, P., Zhu, W., Yantha, J., Vrees, T., Furesz, S., Jacks, K., Smith, C., Kireeva, I., Zhang, R., Takahashi, M., Stanton, E., Jackowski, G., J. Proteome Res. 2003, 2, 361-372.
115. Diamandis, E.P., Mol. Cell Proteomics 2004, 3, 367-378.
116. Baggerly, K.A., Morris, J.S., Coombes, K.R. Bioinformatics 2004, 20, 777 - 785.
117. Check, E., Nature 2004, 429, 496 - 497.
118. Marshall, J., Jankowski, A., Furesz, S., Kireeva, I., Barker, L., Dombrovsky, M., Zhu, W., Jacks, K., Ingratta, L., Bruin, J., Kristensen, E., Zhang, R., Stanton, E., Takahashi, M., Jackowski, G. J. Proteome Res. 2004, 3, 364 - 382.
119. Wagner, L., J. Natl. Cancer Inst. 2004, 96, 500 - 501.
120. He, Q.Y., Chen, J., Kung, H.F., Yuen, A.P., Chiu, J.F., Proteomics 2004, 4, 271- 278.
121. Zhang, Z., Bast Jr., R.C., Fung, E.T., Yu, Y., Li, J., Rosenzweig, J. Proc. AACR Abstract No. 5739, 2003.
122. Ye, B., Cramer, D.W., Skates, J., Gygi, S.P., Pratomo, V., Fu, L., Hornick, N.K., Licklider, L.J., Schorge, J.O., Berkowitz, R.S., Mok, S.C., Clin. Cancer Res. 2003, 9, 2904 - 2911.
123. Chan, D. HUPO 2nd Annual & IUBIMB XIX World Congress, Montreal, Quebec, October 8-11, 2003.
124. Guo, J., Yang, E.C.C., DeSouza, L., Rodrigues, M.J., Romaschin, A.D., Colgan, T.J., Siu, K.W.M., Proc. 52nd American Society for Mass Spectrometry Conference, Nashville, Tennessee, May 23 - 27,2004.
125. Creasman, W.T., Endometrial Carcinoma Available from: URL: http://www.emedicine.com/med/topic674.htm
126. National Cancer Institute of Canada: Canadian Cancer Statistics 2003, Toronto, Canada, 2003 [Serial online] April 2003; Available from: URL: http://www.bc.cancer.ca/v images/portal/cit 776/61/38/56158640niw stats en.pdf
127. American Cancer Society, Statistics Available from: URL: http://www.cancer.org/docroot/STT/stt 0.asp
128. Bernstein, L., Deapen, D., Cerhan, J.R., Schwartz, S.M., J. Natl Cancer Inst. 1999, 91, 1654 - 1662.
129. Grady, G., Ernster V.L., Endometrial cancer In: Schottenfeld, D., Fraumeni Jr., J., Ed., Cancer epidemiology and prevention 2nd ed. New York, Saunders, 1996. p. 1058 - 1089.
130. Endometrial cancer Available from: URL: http://health.allrefer.com/health/endometrial-cancer-info.html
131. Uterine Cancer (Endometrial carcinima) Available from: URL: http://alwaysyourchoice.com/ayc/adult/womens/uterine cancer.php
132. Kurman, R.J., Ed., Blaustein's Pathology of the Female Genital Tract, 5th ed. New York, Springer-Verlag, 2002.
133. ExPASy, URL: http://ca.expasy.org/
134. Odink, K., Cerletti, N., Bruggen, J., Clerc, G.R., Tarcsay, L., Zwadlo, G., Gerhards, G., Schlegel, R., Sorg, C., Nature 1987, 330, 80 - 82.
135. Stulik, J., Osterreicher, J., Koupilova, K., Knizek, J., Macela, A., Bures, J., Jandik, P., Langr, F., Dedic, K., Jungblut, P.R., Electrophoresis 1999,20, 1047 - 1054.
136. Kerkhoff, C., Eue, I., Sorg, C., Pathobiology 1999, 67, 230 - 232.
137. Kerkhoff, C., Klempt, M., Sorg, C., Biochim. Biophys. Acta. 1998, 1448, 200 - 211.
138. Schluesene, H.J., Kremsner, P.G., Meyermann, R., Acta Neuropathol. (Berl.) 1998, 96, 575 -580.
139. Hunter, M.J., Chazin, W.J., J. Biol. Chem. 1998, 273, 12427 - 22435.
140. Bordmann, G., Burmeister, G., Saladin, S., Urassa, H., Mwankyusye, S., Weiss, N., Tanner, M., Clin. Diagn. Lab. Immunol. 1997, 4, 435 -439.
141. Lugering, N., Stoll, R., Kucharzik, T., Burmeister, G., Sorg, C., Domschke, W., Clin. Exp. Immunol. 1995, 101, 249 - 253.
142. Fanjul, M., Renaud, W., Merten, M., Guy-Crotte, O., Hollande E., Figarella, C., Am. J. Physio. 1995, 268, C1241- C1251.
143. Schmid, K.W., Lugering, N., Stoll, R., Brinkbaumer, P., Winde, G., Domschke, W., Bocker, W., Sorg, C., Hum. Pathol. 1995, 26, 334 - 337.
144.. Uchida, T., Fukawa, A., Uchida, M., Fujita, K., Saito, K., J. Proteome Res. 2002, 1, 495 - 499.
145. Lundy, F.T., Chalk, R., Lamey, P.-J., Shaw, C., Linden, G.J., J. Pathol. 2000, 1992, 540 - 544.
146. Bhardwaj, R.S., Zotz, C., Zwadlo-Kkarwasser, G., Roth, J., Geobeler, M., Mahnke, K., Falk, M., Meinardus-Hager, G., Sorg, C., Eur. J. Immunol. 1992, 22, 1891-1897.
147. Burkhardt, K., Radespiel-Troger, M., Rupprecht, H.D., Goppelt-Strube, M., Riess, R., Renders, L., Hauser, I.A., Kunzendorf, U., J. Am. Soc. Nephrol. 2001, 12, 1947 - 1957.
148. Roth, J., Teigelkamp, S., Weike, M., Grun, L., Tummler, B., Sorg, C., Immunobiology 1992, 186, 304-314.
149. Munaron, L., Antoniotti, S., Pla, A.F., Lovisolo, D. Curr. Med. Chem. 2004, 11, 1533 - 1543.
150. Lin, J., Black, M., Tang, C., Zimmer, D., Rustandi, R.R., Weber, D.J., Carrier, F., J. Biol. Chem. 2001, 276, 35037 - 35041.
151. Lin, J., Yang, Q., Yan, Z., Markowitz, J., Wilder, P.T., Carrier, F., Weber, D.J. J. Biol. Chem. 2004, 279,34071-34077.
152. Gebhardt, C., Breitenbach, U., Tuckermann, J.P., Dittrich, B.T., Richter, K.H., Angel, P. Oncogene 2002, 21, 4266 - 4276.
153. Kristinsson, J., Nygaard, K., Aadland, E., Barstad, S., Sauar, J., Hofstad, B., Stray, N., Stallemo, A., Haug, B., Ugstad, M., Ton, H., Fuglerud, P. Digestion 2001, 64, 104 - 110.
154. Tibble, J., Sigthorsson, G., Foster, R., Sherwood, R., Fagerhol, M., Bjarnason, I. Gut 2001, 49, 402 -408.
155. Nacken, W., Roth, J., Sorg, C., Kerkhoff, C. Microsc. Res. Tech. 2003, 60, 569 - 580.
156. Donato, R. Int. J. Biochem. Cell Biol. 2001, 33, 637 -668.

### Sequence Listing

**SEQ ID NO. 1**
   Q04984 and AAH23518
   Chaperonin 10 
1 magqafrkfl plfdrvlver saaetvtkgg imlpeksqgk vlqatvvavg sgskgkggei
61 qpvsvkvgdk vllpeyggtk vvlddkdyfl frdgdilgky vd
**SEQ ID NO. 2**
   NM_002157 and U07550
   Human chaperonin 10 mRNA, complete cds 
1 gctacactag agcagagtac gagtctgagg cggagggagt aatggcagga caagcgttta
61 gaaagtttct tccactcttt gaccgagtat tggttgaaag gagtgctgct gaaactgtaa
121 ccaaaggagg cattatgctt ccagaaaaat ctcaaggaaa agtattgcaa gcaacagtag
181 tcgctgttgg atcgggttct aaaggaaagg gtggagagat tcaaccagtt agcgtgaaag
241 ttggagataa agttcttctc ccagaatatg gaggcaccaa agtagttcta gatgacaagg
301 attatttcct atttagagat ggtgacattc ttggaaagta cgtagactga aataagtcac
361 tattgaaatg gcatcaacat gatgctgccc attccactga agttctgaaa tctttcgtca
421 tgtaaataat ttccatattt ctcttttata ataaactaat gataactaat gacatccagt
481 gtctccaaaa ttgtttcctt gtactgatat aaacacttcc aaataaaaat atgtaaat
**SEQ ID NO. 3**
   P05109
   Calgranulin A 
1 mltelekaln siidvyhkys likgnfhavy rddlkkllet ecpqyirkkg advwfkeldi
61 ntdgavnfqe flilvikmgv aahkkshees hke
**SEQ ID NO. 4**
   A12027
   Macrophage migration inhibition factor (MRP-14)cDNA from Human placenta (formula v) 
1 cttgggttgc ttccaccttt tggctcttgt aaataatgct gctatgaaca tgaatgtaca
61 aacatctgtt tgaatccctg cattcaattc ttttgcatat atacccagga gcagaatgat
121 ggatcatatg gtaattctgt gtttatttat ttgaggaaca aacttgccgt tttccataac
181 agctgcacta ttttacattc ccactaacag tgcattaggc ttccaattct ctatgccctc
241 accaacactt gttttctggg ttttaaaaga agtagtagtc atccttgtag gtgtcaggtg
301 gtatctcatt gtcgttttgc ttcatgtttt cctaaagatt agtaattttc atatgcttat
361 tgaccatttg tatatcttct tcggagaagt gtctatttga gtctttcccc aattttgatt
421 ggtttgtttg ttttttgttg ttgagttgta gggattcttt tatattctgg atattaatcc
481 cttatcagat atttgtttta caaatatttt ctttgtaaca acagaaacac accacagtct
541 tcaaggttgg aagccagtta atctgagtag cattttgtta gtggtgggga gaggatttgt
601 tcctcctgaa atcctgggga attggccacc tcctcttctc ctcttaggca tgaagcgcgt
661 ctggcttctc caaagaactc ttcccctcca ctacctcaga gttagcttcc tctcttcagc
721 cagtgatcct ggggtcccag acacaataat taaccaagag agggtgaaag gctccctgct
 
781 gtgtttatgc aatggctcag gcccttgtga agtgccgagg gaccccaagc agcctccatc
841 tcccagggca tggtccatcc ccagctttca cagaacagga aagctgtgga ggagtgtggg
901 cagcagggta ggaatggata tagcccttgg caacaacaca tttccccaca aagcacccac
961 ccaaaagaac aacaacgata gttttagttt ttagtaatga gaacaatagt tctcatgact
1021 aaaagccatc agccaggaca ctgttctcaa cccttttgcg gtctttggac cctttgaaac
1081 tctgacagaa gccatggagg aatgttctca ctgagtgcat gcactcaaaa tgatgcattc
1141 aacttcaatt cagtttcagg gatgtatggc ctgaccacca atgcagggga ttagcaatcg
1201 caatagtgga gagggcatgg gagtgggaat ctggctggat caagcaagtg gatgccagca
1261 gcccagaaaa agagcccccc tacctgcttt ttccttcctg ggcactattg cccagcaaat
1321 gccttcctct ttccgcttct cctacctccc cacccaaaat tttcattctg cacagtgatt
1381 gccacattca ctggttgaga aacagagact gtagcaactc tggcagggag aagctgtctc
1441 tgatggcctg aagctgtggg cagctggcca agcctaaccg ctataaaaag gagctgcctc
1501 tcagccctgc atgtctcttg tcagctgtct ttcagaagac ctggtaagtg ggactgtctg
1561 ggttggcccc gcactttggg cttctcttgg ggagggtcag ggaagtggag cagccttcct
1621 gagagaggag agagaaagct cagggaggtc tggagcaaag atactcctgg aggtggggag
1681 tgaggcaggg ataaggaagg agagtatcct ccagcacctt ccagtgggta agggcacatt
1741 gtctcctagg ctggactttt cttgagcaga gggtggggtg gtaaggaaag tctacgggcc
1801 cccgtgtgtg tgcacatgtc tctgtgtgaa tggacccttc cccttcccac acgtgtatcc
1861 ctatcatccc acccttccca ccagaggcca tagccatctg ctggtttggt tatttgagag
1921 tgcaggccag gacaaggcca tcgcttgggg catgaatcct ctgcgtactg ccctggccag
1981 atgcaaattc cctgccatgg gattccccag aaggttctgt ttttcaggtg gggcaagttc
2041 cgtgggcatc atgttgaccg agctggagaa agccttgaac tctatcatcg acgtctacca
2101 caagtactcc ctgataaagg ggaatttcca tgccgtctac agggatgacc tgaagaaatt
2161 gctagagacc gagtgtcctc agtatatcag ggtgaggagg ggctgggtgt ggcgggggct
2221 ctctgcctgg tcctggggct gccctgggcc agcggtcctc cctgccaccc ttcatagatg
2281 ctatgcctcg gctctctctg agatctttaa actctggctt cttcctcctc aatcttgaca
2341 gaaaaagggt gcagacgtct ggttcaaaga gttggatatc aacactgatg gtgcagttaa
2401 cttccaggag ttcctcattc tggtgataaa gatgggcgtg gcagcccaca aaaaaagcca
2461 tgaagaaagc cacaaagagt agctgagtta ctgggcccag aggctgggcc cctggacatg
2521 tacctgcaga ataataaagt catcaatacc tcatgcctct ctcttatgct tttgtggaat
2581 gaggttcctc ggtgtggagg gagggttgga aaacccaaag gaagaaaaag aaatctatgt
2641 tatcccaccc tacctctcac aagcctttcc tgctttaccc ctcacctggc ctctgcccca
2701 cattccttca gcccctcatt tcgagcattg gatttgaggc ttaaggattc aaaaagtcgt
2761 catgaatata gctgatgatt ttatagtggt tctgaaatgg gtcggggatt tgggaacagg
2821 gtggtagtat aagaacaact gatactgttc tctaagctaa atcttagctt ccagctacct
2881 gtcttagatg tggctcttgg gaaccttaga gtgatagcta catagaagtg tgtgggtgtg
2941 tgtgtgtgtg tctgtgtgtg tgtgtgtgag agagagacag acagaaagag agcaagagag
3001 ggaagggggg agaggctgat tgtgtgtgtg gtgtgatgta ggtggacaat gttcagagtc
3061 ctccattaac aggataatcc tcacacctgt ccacatacct gtagtttgtc cttggggatt
3121 ttgaaaattt ttcctccctc tccactccca aactcccaac tcaattaaat gataaaggaa
3181 taggcaaata ggaaaataaa ttagtaaaac ttaagtcaaa gaataggtta ttcatacgct
3241 gcctatggga ttctatgctt tgtgatcaga aaattatcta aaaaatactt cccaagggct
3301 ggtacaaggg aggccagaag acgagtggtt cttctctgag gtggacatta aaaaaagaag
3361 aaaatgaagg ggaacctttt gacaagaatg tcaccccaaa ctggatttc atgctgtggt
3421 gtggggaatt ttctgttgtc ctcacttagg tgctggggca gtggtgttag tgatgggtaa
3481 aaaggtagga agctgtcaca gaatcactaa accagggttc ttaacttgtc tgtctataca
3541 tctctgaaat tgggttgaag ttgtgtgcat cattttgagt gacgcactga gaacattcct
3601 ccacggcttc catcgagagt ctcgaaaagg cccaacacct caaaaaggtt aagaacactt
3661 gtcctgctta ctggttttta gtaacaaatg gcagagtatt tctctctgtc tctctctctt
3721 tttttttttt tttttttgag acacagggtc ttgtctgtca cgtggactag agtacaatgg
3781 gcatgatcat gggctcactg tagcctcgaa cacctgggct caagtaatcc tcccacctca
3841 gcctctttag tagctgggac tacagcatga gccactgccc ttggctaatt tttaaattat
3901 tttttgtag agatggaaac ttgctatgtt gcccaggcta gtctcaaact cctggactca
3961 agcgatcctc ctaccttggc ctcccaaagt gctgagatta cagtgtgatc cacaccacac
4021 ctggccaaag attggagtat ttttattgct attgttgtgc tgggtgggtg ggtgggtgta
 
4081 tgctttgtgg ggacgtgtgt tgttgccaag ggctaaatca gttcctaccc tgctgcccac
4141 agtcctccac agctttcctg ctctgtgaag ctaaggatac accccgatga taagctgtca
4201 acata
**SEQ ID NO. 5**
   NM_002964
   Homo sapiens S100 calcium binding protein A8 (calgranulin A) S100A8, mRNA 
1 atgtctcttg tcagctgtct ttcagaagac ctggtggggc aagtccgtgg gcatcatgtt
61 gaccgagctg gagaaagcct tgaactctat catcgacgtc taccacaagt actccctgat
121 aaaggggaat ttccatgccg tctacaggga tgacctgaag aaattgctag agaccgagtg
181 tcctcagtat atcaggaaaa agggtgcaga cgtctggttc aaagagttgg atatcaacac
241 tgatggtgca gttaacttcc aggagttcct cattctggtg ataaagatgg gcgtggcagc
301 ccacaaaaaa agccatgaag aaagccacaa agagtagctg agttactggg cccagaggct
361 gggcccctgg acatgtacct gcagaataat aaagtcatca atacctcaaa aaaaaaaaaa
421 aaaaaaa
**SEQ ID NO. 6**
   P06702
   Calgranulin B/MRP-14 
1 mtckmsqler nietiintfh qysvklghpd tlnqgefkel vrkdlqnflk kenknekvie
61 himedldtna dkqlsfeefi mlmarltwas hekmhegdeg pghhhkpglg egtp
**SEQ ID NO. 7**
   X06233
   Human mRNA for calcium-binding protein in macrophages (MRP-14) macrophage migration inhibitory factor (MIF)-related protein 
aaaacactct gtgtggctcc tcggctttga cagagtgcaa gacgatgact tgcaaaatgt
cgcagctgga acgcaacata gagaccatca tcaacacctt ccaccaatac tctgtgaagc
tggggcaccc agacaccctg aaccaggggg aattcaaaga gctggtgcga aaagatctgc
aaaatttctt caagaaggag aataagaatg aaaaggtcat agaacacatc atggaggacc
tggacacaaa tgcagacaag cagctgagct tcgaggagtt catcatgctg atggcgaggc
taacctgggc ctcccacgag aagatgcacg agggtgacga gggccctggc caccaccata
agccaggcct cggggagggc accccctaag accacagtgg ccaagatcac agtggccacg
gccacggcca cagtcatggt ggccacggcc acagccaccc at
**SEQ ID NO. 8**
   M21064
   Human migration inhibitory factor-related protein 14 (MRP14) gene, complete cds 
1 atcactgtgg agtaggggaa gggcactcct ggggtggcaa ggtgggaggt gggccctgtg
61 ttcccacagt gggcagggag gtagtgaaag ggaagctggc cggacaggaa gggccattcc
121 aagagggctt tgtgcgcagg gctaagccaa gctttctcca taggcaatgg ggagcaactg
 
181 gaggttcgta gcaggagaag gacacatcaa gcccaccagg aggctaagta aaaacagttg
241 tctcccaagt tataagttcc tggaaccctt gctgggagca ggatttagaa aaatgatgct
301 gagagatgct agaaacatat tcgccctgag gctctctcac tcagactgca agaggaaggt
361 atcatcagaa ttgcccttaa ccaggaacca gaatagctgg gtccccttcc tgccaagtca
421 gcaaccagct atgtgacctt gctcaggtcc atctccgggt gtcagtttct tcatctacaa
481 tgcaagaggg ttgcccacct ctgagaaccc ttctaacccc aaatctcacc ctatgaatct
541 aagaacacaa cccctcgcca tcctaagtat cacagagcca ggcaagcatg ggtgagagct
601 cagaccatcc ttgttggact aaaaggaagg ggcagactgc catgggggc agccgagagg
661 gtcaggcccc cataggtcct cagcctgctt caacctcaaa ggggatgggg ggctgagtgg
721 tgccagagga gcagcaggct cgctcgggga gagtagggcc ttaggataga agggaaatga
781 actaaacaac cagcttcctg caaaccagtt tcaggccagg gctgggaatt tcacaaaaaa
841 gcagaaggcg ctctgtgaac atttcctgcc ccgccccagc cccttcctg gcagcattag
901 cacactgctc acctgtgaag caatcttccg gagacagggc caaagggcaa gtgccccagt
961 caggagctgc ctataaatgc cgagcctgca cagctctggc aaacactctg tgtggctcct
1021 cggctttggt aagtgagctg ccagcttccc caggcagaag cctgcctgcc gattccttct
1081 ttccttccct gacccaactt ccttccaaat cctcctccta gaagccctcc ttggttggcc
1141 ctgcctactt taaagcttct ttcacatttt cttaggtcat gttcccctgg ggcctcctgc
1201 cctcaaatgc tttgcttttt ggcactctgt agatattcta aaaaatcatt ttgtacatgt
1261 gtgtgacagg ccatctccca gttaagttgc agcctgtgct ttctttttat tttgcacttc
1321 ccccactatt tctgtgagtg cttagtagga agtgtcaaag aagcttgaca gcattttctt
1381 ctaagtgtcc caactcttgg ttttccatta cacagacaga gtgcaagacg atgacttgca
1441 aaatgtcgca gctggaacgc aacatagaga ccatcatcaa caccttccac caatactctg
1501 tgaagctggg gcacccagac accctgaacc agggggaatt caaagagctg gtgcgaaaag
1561 atctgcaaaa ttttctcaag gtagggctgg actctggcag gtctgaccca gcctcaccgc
1621 agtttgggtt gacaagggag gatgggagta tgggctacag caatcaaggg gaagatttga
1681 gctcctggag cccagcccca agacgcagcg agtgtcctgt tatacagggc aggtgctcac
1741 agttacacag gacgacaggg tcaagaaatt gctcaattga acacctgcta tttgtcgggc
1801 cctgttctgg gcagagggat gtagtggtaa atgggagccc actattccat gaggagacac
1861 acagtaaagt tgttggccaa taaagagcac agataaagcc aaatgccaat aagtgcctgg
1921 aagaaaatga gatagagtgc gctgtgggca atggggctgg gtggggtgga ggtgaccagt
1981 tagggtacat gagaagggcc tctttgagga ggtaacattt gagctgagcc ccgaatgttg
2041 gggagggaag cccctgagga tgacacttgg cacaaagctg aggagaccct aagcctcagg
2101 gcgaacttgg ggtggaagac ttgggggctt ttctaatcct aagggtctgc ggtggaaaat
2161 gaatgcataa agagcacatg gagagcacct gcacagcact cagggaactg ggaggttttt
2221 cccccgctcc aaaaatgatt aggcagttct aagaaaaagg ctgagcactt ccaacagcct
2281 ttttgttttc ttttcaaatt tggggaaagt cgggaaacag aggcctgcat taagaagggt
2341 ggaacacatg ggtctcagtc tcagttccag tcccggagcc agacatcctg gggtaggtcc
2401 ccagccctcc cagtgcccct ccctccgcct tggtaaggtg gagaattgca gccttcagag
2461 ttaggggccc tgacagctct ccataggtgg aggcctcagg caggcaggat gctgggtggg
2521 gtaggcaaga aagggcccag cagagaggcc gcatcggaaa actatcctcc atgtgacccc
2581 ctatgcccgc ttcacccccc acctgacatc ccccaccaga agcaaagcga tgctgtggga
2641 aaggaagcag agcctcatgg atgggctgca caggagagtg ctcgcattgg ctgggtaccc
2701 cacaggttct gggaggggac ttagcgaggt gactcagtgc ctcggcctcc caaagtgctg
2761 ggattacaag catgagccac cctgtccgac catctcccct tttatacttt atcacaccct
2821 tgaggtcagc ggagcacata ctctgctctc tgaccctcca tctcccctgc ccacacctag
2881 gtttttctag tgtttccccg ttgtattggt tgaaataagt ttcactaatt ggtaacctcc
2941 agagggaagg gaagggaggg caggggaagg agtgaagtgc agaggggtag cagagtggaa
3001 ctggcctcta agtcagatct gaatttgcat gccctcaata gtcaagcctg tgaaaactaa
3061 tgaccctctc taggactggt ttcaagtctt cctccaggaa gataccattc ctagctgtta
3121 aagttgttat aaggaccaaa tgaggtgaca tttccaggct tactcatgcc atgaccaggg
3181 caagaccctg gaactcagct tcctcttcta taaatagaga atcagcaccc aagtcacagg
3241 gtcatggagg gaataaactg gagagcgttt ggtatgtgct cagtgtctgc tccattgtgc
3301 gcactcagcc tatggtcatt tttaattttt aaatccagcc ccagggtcga ggcttccttg
3361 tacatttgcc agctggtcat ttactgtgct cccagtcccc acctctggcc acacccagct
3421 ctcacagcct tctctcccca cccgcagaag gagaataaga atgaaaaggt catagaacac
 
3481 atcatggagg acctggacac aaatgcagac aagcagctga gcttcgagga gttcatcatg
3541 ctgatggcga ggctaacctg ggcctcccac gagaagatgc acgagggtga cgagggccct
3601 ggccaccacc ataagccagg cctcggggag ggcaccccct aagaccacag tggccaagat
3661 cacagtggcc acggccacgg ccacagtcat ggtggccacg gccacaggcc actaatcagg
3721 aggccaggcc accctgcctc tacccaacca gggccccggg gctgttatgt caaactgtct
3781 tggctgtggg gctaggggct ggggcaaata agtctcttcc tccaagtcag tgctctgtgt
3841 gcttcttcca cctcttctcc aaccctgcct tcccagggct ctggcattta gacagccctg
3901 tccttatctg tgactcagcc ccctcattca gtattaacaa aatgagaagc agcaaaacat
3961 gggtctgtgc tgggcccctt ggctcacctc cctgaccatg tcctcacctc tgacttcagg
4021 ccccactgtt cagatcccag gctccctgcc ccatctcaga caccctgtcc agcctgtcca
4081 gcctgacaaa tggcccttgt cactgtacac tgtagaaagc aaaaaggcat atctctaccc
4141 cttgatatgc ctgctacctc accaaccagc cccaagcctg tcttcaccca tcactgtcta
4201 cacagccctc tctctctcct aacagaattc tattcctctg aaagtcttca gaaactggac
4261 ctagatagtg ccatgtctgg ggaggaatat ggcaccaggc agtggaaaca aggacagatc
4321 ggtgtgttat ctcacatttg atcagagagc atgatctctc ttaacagacc tgccacccta
4381 atcaacggga gtgctcacac aagtgggagt ctgagagctt agccctatgc ccaccctgg
**SEQ ID NO. 9**
   P01833 and Q81ZY7
   Polymeric-immunoglobulin receptor (precursor) 
1 mllfvltcll avfpaistks pifgpeevns vegnsvsitc yypptsvnrh trkywcrqga
61 rggcitliss egyvsskyag ranltnfpen gtfvvniaql sqddsgrykc glginsrgls
121 fdvslevsqg pgllndtkvy tvdlgrtvti ncpfktenaq krkslykqig lypvlvidss
181 gyvnpnytgr irldiqgtgq llfsvvinql rlsdagqylc qagddsnsnk knadlqvlkp
241 epelvyedlr gsvtfhcalg pevanvakfl crqssgencd vvvntlgkra pafegrilln
301 pqdkdgsfsv vitglrkeda grylcgahsd gqlqegspiq awqlfvnees tiprsptvvk
361 gvagssvavl cpynrkesks ikywclwega qngrcpllvd segwvkaqye grlslleepg
421 ngtftvilnq ltsrdagfyw cltngdtlwr ttveikiieg epnlkvpgnv tavlgetlkv
481 pchfpckfss yekywckwnn tgcqalpsqd egpskafvnc densrlvslt lnlvtradeg
541 wywcgvkqgh fygetaavyv aveerkaags rdvslakada apdekvldsg freienkaiq
601 dprlfaeeka vadtrdqadg srasvdsgss eeqggssral vstlvplglv lavgavavgv
661 ararhrknvd rvsirsyrtd ismsdfensr efgandnmga ssitqetslg gkeefvatte
721 sttetkepkk akrsskeeae maykdfllqs stvaaeaqdg pqea
**SEQ ID NO. 10**
   NM_002644
   Homo sapiens polymeric immunoglobulin receptor (PIGR), mRNA 
1 agagtttcag ttttggcagc agcgtccagt gccctgccag tagctcctag agaggcaggg
61 gttaccaact ggccagcagg ctgtgtccct gaagtcagat caacggagga gaaggaagtg
121 gctaaaacat tgcacaggag aagtcggcct gagtggtgcg gcgctcggga cccaccagca
181 atgctgctct tcgtgctcac ctgcctgctg gcggtcttcc cagccatctc cacgaagagt
241 cccatatttg gtcccgagga ggtgaatagt gtggaaggta actcagtgtc catcacgtgc
301 tactacccac ccacctctgt caaccggcac acccggaagt actggtgccg gcagggagct
361 agaggtggct gcataaccct catctcctcg gagggctacg tctccagcaa atatgcaggc
421 agggctaacc tcaccaactt cccggagaac ggcacatttg tggtgaacat tgcccagctg
481 agccaggatg actccgggcg ctacaagtgt ggcctgggca tcaatagccg aggcctgtcc
541 tttgatgtca gcctggaggt cagccagggt cctgggctcc taaatgacac taaagtctac
601 acagtggacc tgggcagaac ggtgaccatc aactgccctt tcaagactga gaatgctcaa
661 aagaggaagt ccttgtacaa gcagataggc ctgtaccctg tgctggtcat cgactccagt
 
721 ggttatgtaa atcccaacta tacaggaaga atacgccttg atattcaggg tactggccag
781 ttactgttca gcgttgtcat caaccaactc aggctcagcg atgctggcca gtatctctgc
841 caggctgggg atgattccaa tagtaataag aagaatgctg acctccaagt gctaaagccc
901 gagcccgagc tggtttatga agacctgagg ggctcagtga ccttccactg tgccctgggc
961 cctgaggtgg caaacgtggc caaatttctg tgccgacaga gcagtgggga aaactgtgac
1021 gtggtcgtca acaccctggg gaagagggcc ccagcctttg agggcaggat cctgctcaac
1081 ccccaggaca aggatggctc attcagtgtg gtgatcacag gcctgaggaa ggaggatgca
1141 gggcgctacc tgtgtggagc ccattcggat ggtcagctgc aggaaggctc gcctatccag
1201 gcctggcaac tcttcgtcaa tgaggagtcc acgattcccc gcagccccac tgtggtgaag
1261 ggggtggcag gaggctctgt ggccgtgctc tgcccctaca accgtaagga aagcaaaagc
1321 atcaagtact ggtgtctctg ggaaggggcc cagaatggcc gctgccccct gctggtggac
1381 agcgaggggt gggttaaggc ccagtacgag ggccgcctct ccctgctgga ggagccaggc
1441 aacggcacct tcactgtcat cctcaaccag ctcaccagcc gggacgccgg cttctactgg
1501 tgtctgacca acggcgatac tctctggagg accaccgtgg agatcaagat tatcgaagga
1561 gaaccaaacc tcaaggtacc agggaatgtc acggctgtgc tgggagagac tctcaaggtc
1621 ccctgtcact ttccatgcaa attctcctcg tacgagaaat actggtgcaa gtggaataac
1681 acgggctgcc aggccctgcc cagccaagac gaaggcccca gcaaggcctt cgtgaactgt
1741 gacgagaaca gccggcttgt ctccctgacc ctgaacctgg tgaccagggc tgatgagggc
1801 tggtactggt gtggagtgaa gcagggccac ttctatggag agactgcagc cgtctatgtg
1861 gcagttgaag agaggaaggc agcggggtcc cgcgatgtca gcctagcgaa ggcagacgct
1921 gctcctgatg agaaggtgct agactctggt tttcgggaga ttgagaacaa agccattcag
1981 gatcccaggc tttttgcaga ggaaaaggcg gtggcagata caagagatca agccgatggg
2041 agcagagcat ctgtggattc cggcagctct gaggaacaag gtggaagctc cagagcgctg
2101 gtctccaccc tggtgcccct gggcctggtg ctggcagtgg gagccgtggc tgtgggggtg
2161 gccagagccc ggcacaggaa gaacgtcgac cgagtttcaa tcagaagcta caggacagac
2221 attagcatgt cagacttcga gaactccagg gaatttggag ccaatgacaa catgggagcc
2281 tcttcgatca ctcaggagac atccctcgga ggaaaagaag agtttgttgc caccactgag
2341 agcaccacag agaccaaaga acccaagaag gcaaaaaggt catccaagga ggaagccgag
2401 atggcctaca aagacttcct gctccagtcc agcaccgtgg ccgccgaggc ccaggacggc
2461 ccccaggaag cctagacggt gtcgccgcct gctccctgca cccatgacaa tcaccttcag
2521 aatcatgtcg atcctggggc cctcagctcc tggggacccc actccctgct ctaacacctg
2581 cctaggtttt tcctactgtc ctcagaggcg tgctggtccc ctcctcagtg acatcaaagc
2641 ctggcctaat tgttcctatt ggggatgagg gtggcatgag gaggtcccac ttgcaacttc
2701 tttctgttga gagaacctca ggtacggaga agaatagagg tcctcatggg tcccttgaag
2761 gaagagggac cagggtggga gagctgattg cagaaaggag agacgtgcag cgcccctctg
2821 cacccttatc atgggatgtc aacagaattt ttccctccac tccatccctc cctcccgtcc
2881 ttcccctctt cttctttcct tccatcaaaa gatgtatttg aattcatact agaattcagg
2941 tgctttgcta gatgctgtga caggtatgcc accaacactg ctcacagcct ttctgaggac
3001 accagtgaaa gaagccacag ctcttcttgg cgtatttata ctcactgagt cttaactttt
3061 caccaggggt gctcacctct gcccctattg ggagaggtca taaaatgtct cgagtcctaa
3121 ggccttaggg gtcatgtatg atgagcatac acacaggtaa ttataaaccc acattcttac
3181 catttcacac ataagaaaat tgaggtttgg aagagtgaag cgtttttctt tttctttttt
3241 ttttttgaga cggagtctct cactgtcgcc caggctggag tgcagtggcg caatctcggc
3301 tcactgcaac ctccgcctcc caggttgaca ccattctcct gcctcaccct cccaagtagc
3361 tgggactaca ggcgcctgcc agcacgcctg gctaattttt tgtattttta gtagagacag
3421 ggtttcaccg tgttagccag gatggtctcg atctcctgac ctcgtgatcc gcctgcctct
3481 gcctcccaaa gtgctgggat tacaggcgtg agccaccgcg tccggcctct tttttctttt
3541 tctttttttt gagacaaagt ctcactgtgt cacccagact ggaatgcagt gacacaatct
3601 cggctcactg aaacctctgc cttccaggtt caagctattc tcatgcctca gcctctcaag
3661 tagctgggac tacagatgtg ggccaccatg tctggctaat tttttttttt tttttttttt
3721 tttgtagaga cagggtttcg ccatgttgac gagactggtc tcgaactcct ggcctcaagt
3781 gatctgccgc ctcagcttct caaagtactg ggattatata ggcatgagcc actgagcctg
3841 gccctgaagc gtttttctca aaggccctca gtgagataaa ttagatttgg catctcctgt
3901 cctgggccag ggatctctct acaagagccc ctgcccctct gttggaggca cagttttaga
3961 ataaggagga ggagggagaa gagaaaatgt aaaggaggga gatctttccc aggccgcacc
 
4021 atttctgtca ctcacatgga cccaagataa aagaatggcc aaaccctcac aacccctgat
4081 gtttgaagag ttccaagttg aagggaaaca aagaagtgtt tgatggtgcc agagaggggc
4141 tgctctccag aaagctaaaa tttaatttct tttttcctct gagttctgta cttcaaccag
4201 cctacaagct ggcacttgct aacaaatcag aaatatgaca attaatgatt aaagactgtg
4261 attgcc
**SEQ ID NO. 11**
   P30086 - Homo sapiens
   Phosphatidylethanolamine binding protein (PEBP) 
1 mpvdlskwsg plslqevdeq pqhplhvtya gaavdelgkv ltptqvknrp tsiswdglds
61 gklytlvltd pdapsrkdpk yrewhhflvv nmkgndissg tvlsdyvgsg ppkgtglhry
121 vwlvyeqdrp lkcdepilsn rsgdhrgkfk vasfrkkyel rapvagtcyq aewddyvpkl
181 yeqlsgk
**SEQ ID NO. 12**
   NM_002567
   Homo sapiens prostatic binding protein (PBP), mRNA 
1 tgggcggcgg ctgaggcgcg tgctctcgcg tggtcgctgg gtctgcgtct tcccgagcca
61 gtgtgctgag ctctccgcgt cgcctctgtc gcccgcgcct ggcctaccgc ggcactcccg
121 gctgcacgct ctgcttggcc tcgccatgcc ggtggacctc agcaagtggt ccgggccctt
181 gagcctgcaa gaagtggacg agcagccgca gcacccgctg catgtcacct acgccggggc
241 ggcggtggac gagctgggca aagtgctgac gcccacccag gttaagaata gacccaccag
301 catttcgtgg gatggtcttg attcagggaa gctctacacc ttggtcctga cagacccgga
361 tgctcccagc aggaaggatc ccaaatacag agaatggcat catttcctgg tggtcaacat
421 gaagggcaat gacatcagca gtggcacagt cctctccgat tatgtgggct cggggcctcc
481 caagggcaca ggcctccacc gctatgtctg gctggtttac gagcaggaca ggccgctaaa
541 gtgtgacgag cccatcctca gcaaccgatc tggagaccac cgtggcaaat tcaaggtggc
601 gtccttccgt aaaaagtatg agctcagggc cccggtggct ggcacgtgtt accaggccga
661 gtgggatgac tatgtgccca aactgtacga gcagctgtct gggaagtagg gggttagctt
721 ggggacctga actgtcctgg aggccccaag ccatgttccc cagttcagtg ttgcatgtat
781 aatagatttc tcctcttcct gccccccttg gcatgggtga gacctgacca gtcagatggt
841 agttgagggt gacttttcct gctgcctggc ctttataatt ttactcactc actctgattt
901 atgttttgat caaatttgaa cttcattttg gggggtattt tggtactgtg atggggtcat
961 caaattatta atctgaaaat agcaacccag aatgtaaaaa agaaaaaact gggggaaaa
1021 agaccaggtc tacagtgata gagcaaagca tcaaagaatc tttaagggag gtttaaaaaa
1081 aaaaaaaaaa aaaaagattg gttgcctctg cctttgtgat cctgagtcca gaatggtaca
1141 caatgtgatt ttatggtgat gtcactcacc tagacaacca gaggctggca ttgaggctaa
1201 cctccaacac agtgcatctc agatgcctca gtaggcatca gtatgtcact ctggtccctt
1261 taaagagcaa tcctggaaga agcaggaggg agggtggctt tgctgttgtt gggacatggc
1321 aatctagacc ggtagcagcg ctcgctgaca gcttgggagg aaacctgaga tctgtgtttt
1381 ttaaattgat cgttcttcat gggggtaaga aaagctggtc tggagttgct gaatgttgca
1441 ttaattgtgc tgtttgcttg tagttgaata aaaatagaaa cctgaatgaa gaaaaaaaaa
1501 aaaaaaa
**SEQ ID NO. 13**
   P39687 - Homo sapiens
   Acidic leucine-rich nuclear phosphoprotein 32 family member A 
1 memgrrihle lrnrtpsdvk elvldnsrsn egklegltde feeleflsti nvgltsianl
61 pklnklkkle lsdnrvsggl evlaekcpnl thlnlsgnki kdlstieplk klenlksldl
121 fncevtnlnd yrenvfkllp qltyldgydr ddkeapdsda egyvegldde eededeeeyd
181 edaqvvedee dedeeeegee edvsgeeeed eegyndgevd deedeeelge eergqkrkre
241 pedegeddd
**SEQ ID NO. 14**
   NM_006305
   Homo sapiens acidic (leucine-rich) nuclear phosphoprotein 32 family, member A (ANP32A), mRNA 
1 cgggtgctgg gggctcgaga accgagcgga gctggttgag ccttcaaagt cctaaaacgc
61 gcggccgtgg gttcggggtt tattgattga attccgccgg cgcgggagcc tctgcagaga
121 gagagcgcga gagatggaga tgggcagacg gattcattta gagctgcgga acaggacgcc
181 ctctgatgtg aaagaacttg tcctggacaa cagtcggtcg aatgaaggca aactcgaagg
241 cctcacagat gaatttgaag aactggaatt cttaagtaca atcaacgtag gcctcacctc
301 aatcgcaaac ttaccaaagt taaacaaact taagaagctt gaactaagcg ataacagagt
361 ctcagggggc ctggaagtat tggcagaaaa gtgtccgaac ctcacgcatc taaatttaag
421 tggcaacaaa attaaagacc tcagcacaat agagccactg aaaaagttag aaaacctcaa
481 gagcttagac cttttcaatt gcgaggtaac caacctgaac gactaccgag aaaatgtgtt
541 caagctcctc ccgcaactca catatctcga cggctatgac cgggacgaca aggaggcccc
601 tgactcggat gctgagggct acgtggaggg cctggatgat gaggaggagg atgaggatga
661 ggaggagtat gatgaagatg ctcaggtagt ggaagacgag gaggacgagg atgaggagga
721 ggaaggtgaa gaggaggacg tgagtggaga ggaggaggag gatgaagaag gttataacga
781 tggagaggta gatgacgagg aagatgaaga agagcttggt gaagaagaaa ggggtcagaa
841 gcgaaaacga gaacctgaag atgagggaga agatgatgac taagtggaat aacctatttt
901 gaaaaattcc tattgtgatt tgactgtttt tacccatatc ccctctcccc cccccctcca
961 atcctgcccc ctgaaactta tttttttctg attgtaacgt tgctgtggga acgagagggg
1021 aagagtgtac tgggggttgc ggggggaggg atggcgggtg ggggtggaat aaaatactat
1081 ttttactgcc actctttaaa aaaaaaaaaa aaaaaaaaaa aaaaaaaaaa aaaaaa
**SEQ ID NO. 15**
   P17066 - Homo sapiens
   Heat shock 70kDa protein 
1 mqaprelavg idlgttyscv gvfqqgrvei landqgnrtt psyvaftdte rlvgdaaksq
61 aalnphntvf dakrligrkf adttvqsdmk hwpfrvvseg gkpkvrvcyr gedktfypee
121 issmvlskmk etaeaylgqp vkhavitvpa yfndsqrqat kdagaiagln vlriinepta
181 aaiaygldrr gagernvlif dlgggtfdvs vlsidagvfe vkatagdthl ggedfdnrlv
241 nhfmeefrrk hgkdlsgnkr alrrlrtace rakrtlssst qatleidslf egvdfytsit
301 rarfeelcsd lfrstlepve kalrdakldk aqihdvvlvg gstripkvqk llqdffngke
361 lnksinpdea vaygaavqaa vlmgdkcekv qdllllldvap lslgletagg vmttliqrna
421 tiptkqtqtf ttysdnqpgv fiqvyegera mtkdnnllgr felsgippap rgvpqievtf
481 didangilsv tatdrstgka nkititndkg rlskeeverm vheaeqykae deaqrdrvaa
541 knsleahvfh vkgslqeesl rdkipeedrr kmqdkcrevl awlehnqlae keeyehqkre
601 leqicrpifs rlyggpgvpg gsscgtqarq gdpstgpiie evd
**SEQ ID NO. 16**
   NM_002155
   Homo sapiens heat shock 70kDa protein 6 (HSP70B') (HSPA6), mRNA. 
1 agagccagcc cggaggagct agaaccttcc ccgcatttct ttcagcagcc tgagtcagag
61 gcgggctggc ctggcgtagc cgcccagcct cgcggctcat gccccgatct gcccgaacct
121 tctcccgggg tcagcgccgc gccgcgccac ccggctgagt cagcccgggc gggcgagagg
181 ctctcaactg ggcgggaagg tgcgggaagg tgcggaaagg ttcgcgaaag ttcgcggcgg
241 cgggggtcgg gtgaggcgca aaaggataaa aagcccgtgg aagcggagct gagcagatcc
301 gagccgggct ggctgcagag aaaccgcagg gagagcctca ctgctgagcg cccctcgacg
361 gcggagcggc agcagcctcc gtggcctcca gcatccgaca agaagcttca gccatgcagg
421 ccccacggga gctcgcggtg ggcatcgacc tgggcaccac ctactcgtgc gtgggcgtgt
481 ttcagcaggg ccgcgtggag atcctggcca acgaccaggg caaccgcacc acgcccagct
541 acgtggcctt caccgacacc gagcggctgg tcggggacgc ggccaagagc caggcggccc
601 tgaaccccca caacaccgtg ttcgatgcca agcggctgat cgggcgcaag ttcgcggaca
661 ccacggtgca gtcggacatg aagcactggc ccttccgggt ggtgagcgag ggcggcaagc
721 ccaaggtgcg cgtatgctac cgcggggagg acaagacgtt ctaccccgag gagatctcgt
781 ccatggtgct gagcaagatg aaggagacgg ccgaggcgta cctgggccag cccgtgaagc
841 acgcagtgat caccgtgccc gcctatttca atgactcgca gcgccaggcc accaaggacg
901 cgggggccat cgcggggctc aacgtgttgc ggatcatcaa tgagcccacg gcagctgcca
961 tcgcctatgg gctggaccgg cggggcgcgg gagagcgcaa cgtgctcatt tttgacctgg
1021 gtgggggcac cttcgatgtg tcggttctct ccattgacgc tggtgtcttt gaggtgaaag
1081 ccactgctgg agatacccac ctgggaggag aggacttcga caaccggctc gtgaaccact
1141 tcatggaaga attccggcgg aagcatggga aggacctgag cgggaacaag cgtgccctgc
1201 gcaggctgcg cacagcctgt gagcgcgcca agcgcaccct gtcctccagc acccaggcca
1261 ccctggagat agactccctg ttcgagggcg tggacttcta cacgtccatc actcgtgccc
1321 gctttgagga actgtgctca gacctcttcc gcagcaccct ggagccggtg gagaaggccc
1381 tgcgggatgc caagctggac aaggcccaga ttcatgacgt cgtcctggtg gggggctcca
1441 ctcgcatccc caaggtgcag aagttgctgc aggacttctt caacggcaag gagctgaaca
1501 agagcatcaa ccctgatgag gctgtggcct atggggctgc tgtgcaggcg gccgtgttga
1561 tgggggacaa atgtgagaaa gtgcaggatc tcctgctgct ggatgtggct cccctgtctc
1621 tggggctgga gacagcaggt ggggtgatga ccacgctgat ccagaggaac gccactatcc
1681 ccaccaagca gacccagact ttcaccacct actcggacaa ccagcctggg gtcttcatcc
1741 aggtgtatga gggtgagagg gccatgacca aggacaacaa cctgctgggg cgttttgaac
1801 tcagtggcat ccctcctgcc cccacgtgga gtcccccagat agaggtgacc tttgacattg
1861 atgctaatgg catcctgagc gtgacagcca ctgacaggag cacaggtaag gctaacaaga
1921 tcaccatcac caatgacaag ggccggctga gcaaggagga ggtggagagg atggttcatg
1981 aagccgagca gtacaaggct gaggatgagg cccagaggga cagagtggct gccaaaaact
2041 cgctggaggc ccatgtcttc catgtgaaag gttctttgca agaggaaagc cttagggaca
2101 agattcccga agaggacagg cgcaaaatgc aagacaagtg tcgggaagtc cttgcctggc
2161 tggagcacaa ccagctggca gagaaggagg agtatgagca tcagaagagg gagctggagc
2221 aaatctgtcg ccccatcttc tccaggctct atggggggcc tggtgtccct gggggcagca
2281 gttgtggcac tcaagcccgc cagggggacc ccagcaccgg ccccatcatt gaggaggttg
2341 attgaatggc ccttcgtgat aagtcagctg tgactgtcag ggctatgcta tgggccttct
2401 agactgtctt ctatgatcct gcccttcaga gatgaacttt ccctccaaag ctagaacttt
2461 cttcccagga taactgaagt cttttgactt tttgggggga gggcggttca tcctcttctg
2521 cttcaaataa aaagtcatta atttattaaa acttgtgtgg cactttaaca ttgctttcac
2581 ctatattttg tgtactttgt tacttgcatg tatgaatttt gttatgtaaa atatagttat
2641 agacctaaat aaaaaaaaaa aaaa
**SEQ ID NO. 17**
   X51757
   Human heat-shock protein HSP70B gene 
1 cccgggcggg cgagaggctc tcaactgggc gggaaggtgc gggaaggtgc ggaaaggttc
61 gcgaaagttc gcggcggcgg gggtcgggtg aggcgcaaaa ggataaaaag cccgtggaag
121 cggagctgag cagatccgag ccgggctggc tgcagagaca ccgcagggag agcctcactg
181 ctgagcgccc ctcgacggcg gacgggcagc agcctccgtg gcctccagca tccgacaaga
241 agcttcagcc atgcaggccc cacgggagct cgcggtgggc atcgacctgg gcaccaccta
301 ctcgtgcgtg ggcgtgtttc agcagggccg cgtggagatc ctggccaacg accagggcaa
361 ccgcaccacg cccagctacg tggccttcac cgacaccgag cggctggtcg gggacgcggc
421 caagagccag gcggccctga acccccacaa caccgtgttc gatgccaagc ggctgatcgg
481 gcgcaagttc gcggacacca cggtgcagtc ggacatgaag cactggccct tccgggtggt
541 gagcgagggc ggcaagccca aggtgccggt atcgtaccgc ggggaggaca agacgttcta
601 ccccgaggag atctcgtcca tggtgctgag caagatgaag gagacggccg aggcgtacct
661 gggccagccc gtgaagcacg cagtgatcac cgtgcccgcc tatttcaatg actcgcagcg
721 ccaggccacc aaggacgcgg gggccatcgc ggggctcaac gtgttgcgga tcatcaatga
781 gcccacggca gctgccatcg cctatgggct ggaccggcgg ggcgcgggag agcgcaacgt
841 gctcattttt gacctgggtg gggcaccttt cgatgtgtcg gttctctcca ttgacgctgg
901 tgtctttgag gtgaaagcca ctgctggaga tacccacctg ggaggagagg acttcgacaa
961 ccggctcgtg aaccacttca tggaagaatt ccggcggaag catgggaagg acctgagcgg
1021 gaacaagcgt gccctcggca ggctgcgcac agcctgtgag cgcgccaagc gcaccctgtc
1081 ctccagcacc caggccaccc tggagataga ctccctgttc gagggcgtgg acttctacac
1141 gtccatcact cgtgcccgct ttgaggaact gtgctcagac ctcttccgca gcaccctgga
1201 gccggtggag aaggccctgc gggatgccaa gctggacaag gcccagattc atgacgtcgt
1261 cctggtgggg ggctccactc gcatccccaa ggtgcagaag ttgctgcagg acttcttcaa
1321 cggcaaggag ctgaacaaga gcatcaaccc tgatgaggct gtggcctatg ggctgctgt
1381 gcaggcggcc gtgttgatgg gggacaaatg tgagaaagtg caggatctcc tgctgctgga
1441 tgtggctccc ctgtctctgg ggctggagac agcaggtggg gtgatgacca cgctgatcca
1501 gaggaacgcc actatcccca ccaagcagac ccagactttc accacctact cggacaacca
1561 gcctggggtc ttcatccagg tgtatgaggg tgagagggcc atgaccaagg acaacaacct
1621 gctggggcgt tttgaactca gtggcatccc tcctgcccca cgtggagtcc cccagataga
1681 ggtgaccttt gacattgatg ctaatggcat cctgagcgtg acagccactg acaggagcac
1741 aggtaaggct aacaagatca ccatcaccaa tgacaagggc cggctgagca aggaggaggt
1801 ggagaggatg gttcatgaag ccgagcagta caaggctgag gatgaggccc agagggacag
1861 agtggctgcc aaaaactcgc tggaggccca tgtcttccat gtgaaaggtt ctttgcaaga
1921 ggaaagcctt agggacaaga ttcccgaaga ggacaggcgc aaaatgcaag acaagtgtcg
1981 ggaagtcctt gcctggctgg agcacaacca gctggcagag aaggaggagt atgagcatca
2041 gaagagggag ctggagcaaa tctgtcgccc catcttctcc aggctctatg gggggcctgg
2101 tgtccctggg ggcagcagtt gtggcactca agcccgccag ggggacccca gcaccggccc
2161 catcattgag gaggttgatt gaatggccct tcgtgataag tcagctgtga ctgtcagggc
2221 tatgctatgg gccttctaga ctgtcttcta tgatcctgcc cttcagagat gaactttccc
2281 tccaaagcta gaactttctt cccaggataa ctgaagtctt ttgacttttt gcggggaggg
2341 cggttcatcc tcttctgctt caaataaaaa gtcattaatt tattaaaact tgtgtggcac
2401 tttaacattg ctttcaccta tattttgtgt actttgttac ttgcatgtat gaattttgtt
2461 atgtaaaata tagttataga cctaaataag ct
**SEQ ID NO. 18**
   P14174
   macrophage migration inhibitory factor - Homo Sapiens 
1 mpmfivntnv prasvpdgfl seltqqlaqa tgkppqyiav hvvpdqlmaf ggssepcalc
61 slhsigkigg aqnrsyskll cgllaerlri spdrvyinyy dmnaanvgwn nstfa
**SEQ ID NO. 19**
   NM_002415 - Homo Sapiens
   Homo sapiens macrophage migration inhibitory factor (glycosylation-inhibiting factor) (MIF), mRNA 
1 accacagtgg tgtccgagaa gtcaggcacg tagctcagcg gcggccgcgg cgcgtgcgtc
61 tgtgcctctg cgcgggtctc ctggtccttc tgccatcatg ccgatgttca tcgtaaacac
121 caacgtgccc cgcgcctccg tgccggacgg gttcctctcc gagctcaccc agcagctggc
181 gcaggccacc ggcaagcccc cccagtacat cgcggtgcac gtggtcccgg accagctcat
241 ggccttcggc ggctccagcg agccgtgcgc gctctgcagc ctgcacagca tcggcaagat
301 cggcggcgcg cagaaccgct cctacagcaa gctgctgtgc ggcctgctgg ccgagcgcct
361 gcgcatcagc ccggacaggg tctacatcaa ctattacgac atgaacgcgg ccaatgtggg
421 ctggaacaac tccaccttcg cctaagagcc gcagggaccc acgctgtctg cgctggctcc
481 acccgggaac ccgccgcacg ctgtgttcta ggcccgccca ccccaacctt ctggtgggga
541 gaaataaacg gtttagagac t
**SEQ ID NO. 20**
   L19686
   Homo sapiens macrophage migration inhibitory factor (MIF) gene, complete cds 
1 ctgcaggaac caatacccat aggctatttg tataaatggg ccatggggcc tcccagctgg
61 aggctggctg gtgccacgag ggtcccacag gcatgggtgt ccttcctata tcacatggcc
121 ttcactgaga ctggtatatg gattgcacct atcagagacc aaggacagga cctccctgga
181 aatctctgag gacctggcct gtgatccagt tgctgccttg tcctcttcct gctatgtcat
241 ggcttatctt ctttcaccca ttcattcatt cattcattca ttcagcagta ttagtcaatg
301 tctcttgata tgcctggcac ctgctagatg gtccccgagt ttaccattag tggaaaagac
361 atttaagaaa ttcaccaagg gctctatgag aggccataca cggtggacct gactagggtg
421 tggcttccct gaggagctga agttgcccag aggcccagag aaggggagct gagcacgttt
481 gaaccactga acctgctctg gacctcgcct ccttccttcg gtgcctccca gcatcctatc
541 ctctttaaag agcaggggtt cagggaagtt ccctggatgg tgattcgcag gggcagctcc
601 cctctcacct gccgcatgac taccccgccc catctcaaac acacaagctc acgcatgcgg
661 gactggagcc cttgaggaca tgtggcccaa agacaggagg tacaggggct cagtgcgtgc
721 agtggaatga actgggcttc atctctggaa gggtaagggg ccatcttccg ggttcaccgc
781 cgcatcccca cccccggcac agcgcctcct ggcgactaac atcggtgact tagtgaaagg
841 actaagaaag acccgaggcg aggccggaac aggccgattt ctagccgcca agtggagaac
901 aggttggagc ggtgcgccgg gcttagcggc ggttgctgga ggaacgggcg gagtcgccca
961 gggtcctgcc ctgcgggggt cgagccgagg caggcggtga cttccccact cggggcggag
1021 ccgcagcctc gcgggggcgg ggcctggcgc cggcggtggc gtcacaaaag gcgggaccac
1081 agtggtgtcc gagaagtcag gcacgtagct cagcggcggc cgcggcgcgt gcgtctgtgc
1141 ctctgcgcgg gtctcctggt ccttctgcca tcatgccgat gttcatcgta aacaccaacg
1201 tgccccgcgc ctccgtgccg gacgggttcc tctccgagct cacccagcag ctggcgcagg
1261 ccaccggcaa gcccccccag gtttgccggg aggggacagg aagagggggg tgcccaccgg
1321 acgaggggtt ccgcgctggg agctggggag gcgactcctg aacggagctg gggggcgggg
1381 cggggggagg acggtggctc gggcccgaag tggacgttcg gggcccgacg aggtcgctgg
1441 ggcgggctga ccgcgccctt tcctcgcagt acatcgcggt gcacgtggtc ccggaccagc
1501 tcatggcctt cggcggctcc agcgagccgt gcgcgctctg cagcctgcac agcatcggca
1561 agatcggcgg cgcgcagaac cgctcctaca gcaagctgct gtgcggcctg ctggccgagc
1621 gcctgcgcat cagcccggac aggtacgcgg agtcgcggag ggcgggggga ggggcggcgg
1681 cgcgcggcca ggcccgggac tgagccaccc gctgagtccg gcctcctccc cccgcagggt
1741 ctacatcaac tattacgaca tgaacgcggc caatgtgggc tggaacaact ccaccttcgc
1801 ctaagagccg cagggaccca cgctgtctgc gctggctcca cccgggaacc cgccgcacgc
1861 tgtgttctag gcccgcccac cccaaccttc tggtggggag aaataaacgg tttagagact
1921 aggagtgcct cggggttcct tggcttgcgg gaggaattgg tgcagagccg ggacattggg
 
1981 gagcgaggtc gggaaacggt gttgggggcg ggggtcaggg ccgggttgct ctcctcgaac
2041 ctgctgttcg ggagcccttt tgtccagcct gtccctccta cgctcctaac agaggagccc
2101 cagtgtcttt ccattctatg gcgtacgaag ggatgaggag aagttggcac tctgccctgg
2161 gctgcag
**SEQ ID NO. 21**
   P31949
   Calgizzarin - Homo sapiens 
 1 makissptet erciesliav fqkyagkdgy nytlsktefl sfmntelaaf tknqkdpgvl
61 drmmkkldtn sdgqldfsef lnligglama chdsflkavp sqkrt
**SEQ ID NO. 22**
   NM_005620 and D38583 - Homo sapiens
   Homo sapiens S100 calcium binding protein All (calgizzarin)(S100A11), mRNA 
1 gggcaaggct gggccgggaa gggcgtgggt tgaggagagg ctccagaccc gcacgccgcg
61 cgcacagagc tctcagcgcc gctcccagcc acagcctccc gcgcctcgct cagctccaac
121 atggcaaaaa tctccagccc tacagagact gagcggtgca tcgagtccct gattgctgtc
181 ttccagaagt atgctggaaa ggatggttat aactacactc tctccaagac agagttccta
241 agcttcatga atacagaact agctgccttc acaaagaacc agaaggaccc tggtgtcctt
301 gaccgcatga tgaagaaact ggacaccaac agtgatggtc agctagattt ctcagaattt
361 cttaatctga ttggtggcct agctatggct tgccatgact ccttcctcaa ggctgtccct
421 tcccagaagc ggacctgagg accccttggc cctggccttc aaacccaccc cctttccttc
481 cagcctttct gtcatcatct ccacagccca cccatcccct gagcacacta accacctcat
541 gcaggcccca cctgccaata gtaataaagc aatgtcactt ttttaaaaca tgaaa
**SEQ ID NO. 23**
   P00938 and NP_000356 - Homo sapiens
   Triosephosphate isomerase 
1 mapsrkffvg gnwkmngrkq slgeligtln aakvpadtev vcapptayid farqkldpki
61 avaaqncykv tngaftgeis pgmikdcgat wvvlghserr hvfgesdeli gqkvahalae
121 glgviacige kldereagit ekvvfeqtkv iadnvkdwsk vvlayepvwa igtgktatpq
181 qaqevheklr gwlksnvsda vaqstriiyg gsvtgatcke lasqpdvdgf lvggaslkpe
241 fvdiinakq
**SEQ ID NO. 24**
   NM_000365
   Homo sapiens triosephosphate isomerase 1 (TPI1), mRNA 
1 ccttcagcgc ctcggctcca gcgccatggc gccctccagg aagttcttcg ttgggggaaa
61 ctggaagatg aacgggcgga agcagagtct gggggagctc atcggcactc tgaacgcggc
121 caaggtgccg gccgacaccg aggtggtttg tgctccccct actgcctata tcgacttcgc
181 ccggcagaag ctagatccca agattgctgt ggctgcgcag aactgctaca aagtgactaa
241 tggggctttt actggggaga tcagccctgg catgatcaaa gactgcggag ccacgtgggt
 
301 ggtcctgggg cactcagaga gaaggcatgt ctttggggag tcagatgagc tgattgggca
361 gaaagtggcc catgctctgg cagagggact cggagtaatc gcctgcattg gggagaagct
421 agatgaaagg gaagctggca tcactgagaa ggttgttttc gagcagacaa aggtcatcgc
481 agataacgtg aaggactgga gcaaggtcgt cctggcctat gagcctgtgt gggccattgg
541 tactggcaag actgcaacac cccaacaggc ccaggaagta cacgagaagc tccgaggatg
601 gctgaagtcc aacgtctctg atgcggtggc tcagagcacc cgtatcattt atggaggctc
661 tgtgactggg gcaacctgca aggagctggc cagccagcct gatgtggatg gcttccttgt
721 gggtggtgct tccctcaagc ccgaattcgt ggacatcatc aatgccaaac aatgagcccc
781 atccatcttc cctacccttc ctgccaagcc aggactaagc agcccagaag cccagtaac
841 tgccctttcc ctgcatatgc ttctgatggt gtcatctgct ccttcctgtg gcctcatcca
901 aactgtatct tcctttactg tttatatctt caccctgtaa tggttgggac caggccaatc
961 ccttctccac ttactataat ggttggaact aaacgtcacc aaggtggctt ctccttggct
1021 gagagatgga aggcgtggtg ggatttgctc ctgggttccc taggccctag tgagggcaga
1081 agagaaacca tcctctccct tcttacaccg tgaggccaag atcccctcag aaggcaggag
1141 tgctgccctc tcccatggtg cccgtgcctc tgtgctgtgt atgtgaacca cccatgtgag
1201 ggaataaacc tggcactagg aaaaaaaaaa aaaaaaaaaa aaaaaaaaaa aaaa
**SEQ ID NO. 25**
   X69723
   H.sapiens TPI1 gene for triosephosphate isomerase. 
1 ctgcagttcc tgccaggcct tgccagccgg ggcgagggtt gggatgatcc tggcggccta
61 tgcctgtgtg ggctgcccct cccgctgtga accctgcatt tgtcccgcaa gttttcactc
121 aggtagactc cctgggtaca agggtgcctg ctcagcagtc gggcatgagc tgctccgatg
181 ggcgaaggag gttgtctatt ccacagttgg agaggggccc tctctgcccc agtgggcgat
241 ctgggctacg gccaagttgc caccagctag ttccgcttga aaaccacttc tggccccgtg
301 ggggactcaa gtcgccaagc gagggttccc ctgagcgccg gagctcacag gtctcgcctt
361 gtcccgaaag ccccgcaatc gaggcggagg cgaccgagcc cccgactctc ctagaacgtt
421 gccacaagaa gggggaacgt cggaacagtg catcatcggg cggcggccgg ggcggcggca
481 ggagggcggg cggggggcag ggctccgggg gactgggcgg gccatggcgg aggacggcga
541 ggaggcggag ttccacttcg cggcgctcta tataagtggg cagtggccgc gactgcgcgc
601 agacactgac cttcagcgcc tcggctccag cgccatggcg ccctccagga agttcttcgt
661 tgggggaaac tggaagatga acgggcggaa gcagagtctg ggggagctca tcggcactct
721 gaacgcggcc aaggtgccgg ccgacaccgg taagccctcg ccgaggaggg gtctggccgg
781 gccggggccg ggccggggca ggagtggcag cgcctctccc gaggcccgag gtccgggccg
841 gtatccgcgc ggacctgatg cagggctgtg ggacgagggc cgctggggtc cgggcagggg
901 cctcgcagcc gcagccccgt cggtgcgtcg agggggcagg gcggagcaca tgatgcccct
961 tggactacgg ggcaggtaag gacgttttgg gtctcctgga ggaaggcggc cccggggcgc
1021 gcactggctg tgcccgccag gcgacggggt taggagccga gcccgaggct ctgcgggaga
1081 ccgggggagg ctgggccgcg tgggcttccg ctccctgccc tggcctccgc gtgcgcgccg
1141 ccgcacgtag ccccagactc ctccccctcc tcgccggcgt cgtcccgcgc cgagctgctg
1201 ctgccctgag cccccagatc tgaacccctt cccttcggca acctgagcga ctcccgcctt
1261 ccacggaagg gaccgagccc gtgccaaaca ggctgagcga tttgggagtg aggagccatc
1321 ctaccgcttt ccccaacctg gaaacagcaa agcgcaaggc ctctgagtca gttaggtctc
1381 tgccacccac gggcaaagga tgctctcctc catcctcctt cctccctcca ccgaaatcgg
1441 agagccgcgg gcctgatcca aagaggcatc cccttctcgt tcattcccca gaggcctcaa
1501 tacaaacccc aggagttggc ccctctcctt ttgctacaaa tccttgcctt gcaaagggga
1561 ggtgaggatg ggctatttta gaagggaagc agggttgctc cctggagaat gctgagtctg
1621 tgaggtgcct atgccgagaa tagctcgagg aaattggagc cccagctgtt aaaagagcag
1681 agggcagggt gagggccgtg gctctcaggg gtatctggaa ggctcttcga gttgagtgca
1741 gacccagcct tgggctggaa aatggacaaa ggtcatcttg ctggggtgaa aagggggaga
1801 gcagaaccaa gaagaagagg gtgagggctg gggggctcca gggcactggt taggaattgt
1861 ggggaatgaa ggctttcttt agtctcatcc ccctgtggta ccatcttgtc ctcagaggtg
 
1921 gtttgtgctc cccctactgc ctatatcgac ttcgcccggc agaagctaga tcccaagatt
1981 gctgtggctg cgcagaactg ctacaaagtg actaatgggg cttttactgg ggagatcagg
2041 tgagatcgag gtggagaggg gtgtgtggga cccttccctc actttcctcg ttgaggggaa
2101 agccacaggg tgggctccct gctgaacctt ggcttcatct cttcctttag ccctggcatg
2161 atcaaagact gcggagccac gtgggtggtc ctggggcact cagagagaag gcatgtcttt
2221 ggggagtcag atgaggttag tagccaagag agaagataag ggatgtcttt ttccaagaag
2281 gatgtctcac caagtctgtt tctcaacagc tgattgggca gaaagtggcc catgctctgg
2341 cagagggact cggagtaatc gcctgcattg gggagaagct agatgaaagg gaagctggca
2401 tcactgagaa ggttgttttc gagcagacaa aggtcatcgc aggtatctct ggagaaaggg
2461 acctttgagc ctatccaggg ccacagagac tcagagggta gggtcaggcc ctggagcctg
2521 tcttggtccc catgctgatc cagaaaagga aaaaggggag ggggagtgac aatctttgct
2581 tggggcctat gacttctcca gccccaaggt agatgccacc tggaaatccc ccaatgtcca
2641 ctagggggca gtaggccacc gttcttcgta ctccggagaa cctggctgga gagctctttc
2701 ttgttcaccc ttccctccat ctgtatctct gccctgcaga taacgtgaag gactggagca
2761 aggtcgtcct ggcctatgag cctgtgtggg ccattggtac tggcaagact gcaacacccc
2821 aacaggtaac cgggcccagg agccctgccc tcatcccagc ctgcctcaat aggtttggac
2881 agacacagcc cacatggagc aaccccttat ttcaaagaca cagagacctt gaacccagag
2941 acagtgactt gtccaagggc atccagtcca gggcctggct tggatcagag ccctggtact
3001 ctgactcagt cagaaaccac actaagtgtc cactggtgcc agtgattttt cctcttagag
3061 aggcagaaaa ggtcttactt aggccagctt cttgttctag gcccaggaag tacacgagaa
3121 gctccgagga tggctgaagt ccaacgtctc tgatgcggtg gctcagagca cccgtatcat
3181 ttatggaggt gagtggcttt ggttcccggc tgaggtggag tgggctgagg actagactga
3241 gccctcggac atggaggtgg ggatggggca gactcatccc attcttgacc aagcccttgt
3301 tctgctccct tcccaggctc tgtgactggg gcaacctgca aggagctggc cagccagcct
3361 gatgtggatg gcttccttgt gggtggtgct tccctcaagc ccgaattcgt ggacatcatc
3421 aatgccaaac aatgagcccc atccatcttc cctacccttc ctgccaagcc agggactaag
3481 cagcccagaa gcccagtaac tgccctttcc ctgcatatgc ttctgatggt gtcatctgct
3541 ccttcctgtg gcctcatcca aactgtatct tcctttactg tttatatctt caccctgtaa
3601 tggttgggac caggccaatc ccttctccac ttactataat ggttggaact aaacgtcacc
3661 aaggtggctt ctccttggct gagagatgga aggcgtggtg ggatttgctc ctgggttccc
3721 taggccctag tgagggcaga agagaaacca tcctctccct tcttacaccg tgaggccaag
3781 atcccctcag aaggcaggag tgctgccctc tcccatggtg cccgtgcctc tgtgctgtgt
3841 atgtgaacca cccatgtgag ggaataaacc tggcactagg tcttgtggtt tgtctgcctt
3901 cactggactt gcccagataa tcttcctttt tgaggcagct atataaatga tcatttgtgc
3961 aagaaaaaaa aaaaaacaag aacaggtttc tataacaaca tctcttacta tttttacttg
4021 aaaaaatgtt ttgcgtagca gactgtcata gccttgaacg ccggctccct ttcttcctcc
4081 ctccaagtgg ctctggggct gttgatttcc gcagagcttg ggttggggta gggctcagcc
4141 tcaccagctt tcagcagctg gtctaggcca gcagtgcctc cccacctccc caagggaggg
4201 tggtggcaag acctcagcac agtctgtggt atcacaggct cactggtaga gcagtagcgc
4261 ttcatgcagg gggcaagggc agggcagaca cctggccgag cggtatcccc aggttgtggc
4321 gcacacacag gcggctcagg tgcagaaggg agtgtggctc cgctgggaga gagaaggagg
4381 ggaatgtaag tatgggtgca gccaccagcc agatgtcctc aaactacggg gtcctcatca
4441 gatgcctttc tgctttcctg cttcgagtgt gcccacctgg ctgaaagggg aatttgagat
4501 acccggaagt tctgcctccc agataagatt tcacacatcc ctagtcagag ctgggggtga
4561 agagctggct aaggccctct aaacaacagg ccaaggtggc tctgacagtg gtggagctgg
4621 cccaggcttt gactccagag gcttgggagc tggggctgag gtgaggaggg atggccctcc
4681 actctacagc ccaacacaac tgcagagagc agctccaagc cctggaccca gtcagttcct
4741 ggggaggctc ctcccctgct gccccaccct aaggcctgcc tcctccactg ctctcctcct
4801 ccctggtgcc cagggcccca gtgtctccat cctgaggtgt ggctgaggaa ggaagtaggt
4861 atgtggcaca gagacaggtt agagcccagg gaatccggta tacagcctgg gtacctcgtc
4921 tgcccatcct tcttttggac ctgtacatca aacccagtac ctaaccgttt gcacctcttg
4981 cctaggggtg attactcctg aattc
**SEQ ID NO. 26**
   Q05586 - Homo sapiens
   Glutamate [NMDA] receptor subunit zeta 1 precursor
**SEQ ID NO. 27**
   D13515
   Homo sapiens mRNA for key subunit of N-methyl-D-aspartate receptor, complete cds 
1 gcttcagcgc cccttccctc ggccgacgtc ccgggaccgc cgctccgggg gagacgtggc
61 gtccgcagcc cgcggggccg ggcgagcgca ggacggcccg gaagccccgc gggggatgcg
121 ccgagggccc cgcgttcgcg ccgcgcagag ccaggcccgc ggcccgagcc catgagcacc
181 atgcgcctgc tgacgctcgc cctgctgttc tcctgctccg tcgcccgtgc cgcgtgcgac
241 cccaagatcg tcaacattgg cgcggtgctg agcacgcgga agcacgagca gatgttccgc
301 gaggccgtga accaggccaa caagcggcac ggctcctgga agattcagct caatgccacc
361 tccgtcacgc acaagcccaa cgccatccag atggctctgt cggtgtgcga ggacctcatc
421 tccagccagg tctacgccat cctagttagc catccaccta cccccaacga ccacttcact
481 cccacccctg tctcctacac agccggcttc taccgcatac ccgtgctggg gctgaccacc
541 cgcatgtcca tctactcgga caagagcatc cacctgagct tcctgcgcac cgtgccgccc
601 tactcccacc agtccagcgt gtggtttgag atgatgcgtg tctacagctg gaaccacatc
661 atcctgctgg tcagcgacga ccacgagggc cgggcggctc agaaacgcct ggagacgctg
721 ctggaggagc gtgagtccaa ggcagagaag gtgctgcagt ttgacccagg gaccaagaac
781 gtgacggccc tgctgatgga ggcgaaagag ctggaggccc gggtcatcat cctttctgcc
841 agcgaggacg atgctgccac tgtataccgc gcagccgcga tgctgaacat gacgggctcc
901 gggtacgtgt ggctggtcgg cgagcgcgag atctcgggga acgccctgcg ctacgcccca
961 gacggcatcc tcgggctgca gctcatcaac ggcaagaacg agtcggccca catcagcgac
1021 gccgtgggcg tggtggccca ggccgtgcac gagctcctcg agaaggagaa catcaccgac
1081 ccgccgcggg gctgcgtggg caacaccaac atctggaaga ccgggccgct cttcaagaga
1141 gtgctgatgt cttccaagta tgcggatggg gtgactggtc gcgtggagtt caatgaggat
1201 ggggaccgga agttcgccaa ctacagcatc atgaacctgc agaaccgcaa gctggtgcaa
1261 gtgggcatct acaatggcac ccacgtcatc cctaatgaca ggaagatcat ctggccaggc
1321 ggagagacag agaagcctcg agggtaccag atgtccacca gactgaagat tgtgacgatc
1381 caccaggagc ccttcgtgta cgtcaagccc acgctgagtg atgggacatg caaggaggag
1441 ttcacagtca acggcgaccc agtcaagaag gtgatctgca ccgggcccaa cgacacgtcg
1501 ccgggcagcc cccgccacac ggtgcctcag tgttgctacg gcttttgcat cgacctgctc
1561 atcaagctgg cacggaccat gaacttcacc tacgaggtgc acctggtggc agatggcaag
 
1621 ttcggcacac aggagcgggt gaacaacagc aacaagaagg agtggaatgg gatgatggc
1681 gagctgctca gcgggcaggc agacatgatc gtggcgccgc taaccataaa caacgagcgc
1741 gcgcagtaca tcgagttttc caagcccttc aagtaccagg gcctgactat tctggtcaag
1801 aaggagattc cccggagcac gctggactcg ttcatgcagc cgttccagag cacactgtgg
1861 ctgctggtgg ggctgtcggt gcacgtggtg gccgtgatgc tgtacctgct ggaccgcttc
1921 agccccttcg gccggttcaa ggtgaacagc gaggaggagg aggaggacgc actgaccctg
1981 tcctcggcca tgtggttctc ctggggcgtc ctgctcaact ccggcatcgg ggaaggcgcc
2041 cccagaagct tctcagcgcg catcctgggc atggtgtggg ccggctttgc catgatcatc
2101 gtggcctcct acaccgccaa cctggcggcc ttcctggtgc tggaccggcc ggaggagcgc
2161 atcacgggca tcaacgaccc tcggctgagg aacccctcgg acaagtttat ctacgccacg
2221 gtgaagcaga gctccgtgga tatctacttc cggcgccagg tggagctgag caccatgtac
2281 cggcatatgg agaagcacaa ctacgagagt gcggcggagg ccatccaggc cgtgagagac
2341 aacaagctgc atgccttcat ctgggactcg gcggtgctgg agttcgaggc ctcgcagaag
2401 tgcgacctgg tgacgactgg agagctgttt ttccgctcgg gcttcggcat aggcatgcgc
2461 aaagacagcc cctggaagca gaacgtctcc ctgtccatcc tcaagtccca cgagaatggc
2521 ttcatggaag acctggacaa gacgtgggtt cggtatcagg aatgtgactc gcgcagcaac
2581 gcccctgcga cccttacttt tgagaacatg gccggggtct tcatgctggt agctgggggc
2641 atcgtggccg ggatcttcct gattttcatc gagattgcct acaagcggca caaggatgct
2701 cgccggaagc agatgcagct ggcctttgcc gccgttaacg tgtggcggaa gaacctgcag
2761 gatagaaaga gtggtagagc agagcctgac cctaaaaaga aagccacatt tagggctatc
2821 acctccaccc tggcttccag cttcaagagg cgtaggtcct ccaaagacac gagcaccggg
2881 ggtggacgcg gcgctttgca aaaccaaaaa gacacagtgc tgccgcgacg cgctattgag
2941 agggaggagg gccagctgca gctgtgttcc cgtcataggg agagctgaga ctccccgccc
3001 gccctcctct gccccctccc ccgcagacag acagacagac ggacgggaca gcggcccggc
3061 ccacgcagag ccccggagca ccacggggtc gggggaggag cacccccag
**SEQ ID NO. 28**
   LLTLLALLFSCSVAR
**SEQ ID NO. 29**
   ITMLCTGSRTLK
**SEQ ID NO. 30**
   ITHU and P01009 - Homo sapiens
   α-1-antitrypain precursor
**SEQ ID NO. 31**
   NM_000295
   Homo sapiens serine (or cysteine) proteinase inhibitor, clade A(alpha-1 antiproteinase, antitrypsin), member 1 (SERPINA1),transcript variant 1, mRNA 
1 aatgactcct ttcggtaagt gcagtggaag ctgtacactg cccaggcaaa gcgtccgggc
61 agcgtaggcg ggcgactcag atcccagcca gtggacttag cccctgtttg ctcctccgat
121 aactggggtg accttggtta atattcacca gcagcctccc ccgttgcccc tctggatcca
181 ctgcttaaat acggacgagg acagggccct gtctcctcag cttcaggcac caccactgac
241 ctgggacagt gaatcgacaa tgccgtcttc tgtctcgtgg ggcatcctcc tgctggcagg
301 cctgtgctgc ctggtccctg tctccctggc tgaggatccc cagggagatg ctgcccagaa
361 gacagataca tcccaccatg atcaggatca cccaaccttc aacaagatca cccccaacct
421 ggctgagttc gccttcagcc tataccgcca gctggcacac cagtccaaca gcaccaatat
481 cttcttctcc ccagtgagca tcgctacagc ctttgcaatg ctctccctgg ggaccaaggc
541 tgacactcac gatgaaatcc tggagggcct gaatttcaac ctcacggaga ttccggaggc
601 tcagatccat gaaggcttcc aggaactcct ccgtaccctc aaccagccag acagccagct
661 ccagctgacc accggcaatg gcctgttcct cagcgagggc ctgaagctag tggataagtt
721 tttggaggat gttaaaaagt tgtaccactc agaagccttc actgtcaact tcggggacac
781 cgaagaggcc aagaaacaga tcaacgatta cgtggagaag ggtactcaag ggaaaattgt
841 ggatttggtc aaggagcttg acagagacac agtttttgct ctggtgaatt acatcttctt
901 taaaggcaaa tgggagagac cctttgaagt caaggacacc gaggaagagg acttccacgt
961 ggaccaggtg accaccgtga aggtgcctat gatgaagcgt ttaggcatgt ttaacatcca
1021 gcactgtaag aagctgtcca gctgggtgct gctgatgaaa tacctgggca atgccaccgc
1081 catcttcttc ctgcctgatg aggggaaact acagcacctg gaaaatgaac tcacccacga
1141 tatcatcacc aagttcctgg aaaatgaaga cagaaggtct gccagcttac atttacccaa
1201 actgtccatt actggaacct atgatctgaa gagcgtcctg ggtcaactgg gcatcactaa
1261 ggtcttcagc aatggggctg acctctccgg ggtcacagag gaggcacccc tgaagctctc
1321 caaggccgtg cataaggctg tgctgaccat cgacgagaaa gggactgaag ctgctggggc
1381 catgttttta gaggccatac ccatgtctat cccccccgag gtcaagttca acaaaccctt
1441 tgtcttctta atgattgaac aaaataccaa gtctcccctc ttcatgggaa aagtggtgaa
1501 tcccacccaa aaataactgc ctctcgctcc tcaacccctc ccctccatcc ctggccccct
1561 ccctggatga cattaaagaa gggttgagct ggtccctgcc tgcaaaa
**SEQ ID NO. 32**
   K02212
   Human alpha-1-antitrypsin gene (S variant), complete cds 
1 gaattccagg ttggaggggc ggcaacctcc tgccagcctt caggccactc tcctgtgcct
61 gccagaagag acagagcttg aggagagctt gaggagagca ggaaaggtgg aacattgctg
121 ctgctgctca ctcagttcca caggtgggag gaacagcagg gcttagagtg ggggtcattg
181 tgcagatggg aaaacaaagg cccagagagg ggaagaaatg cctaggagct accgagggca
241 ggcgacctca accacagccc agtgctggag ctgtgagtgg atgtagagca gcggaatatc
301 cattcagcca gctcagggga aggacagggg ccctgaagcc aggggatgga gctgcaggga
361 agggagctca gagagaaggg gaggggagtc tgagctcagt ttcccgctgc ctgaaaggag
421 ggtggtacct actcccttca cagggtaact gaatgagaga ctgcctggag gaaagctctt
481 caagtgtggc ccaccccacc ccagtgacac cagcccctga cacgggggag ggagggcagc
541 atcaggaggg gctttctggg cacacccagt acccgtctct gagctttcct tgaactgttg
601 cattttaatc ctcacagcag ctcaacaagg tacataccgt caccatcccc attttacaga
661 tagggaaatt gaggctcgga gcggttaaac aactcacctg aggcctcaca gccagtaagt
721 gggttccctg gtctgaatgt gtgtgctgga ggatcctgtg ggtcactcgc ctggtagagc
781 cccaaggtgg aggcataaat gggactggtg aatgacagaa ggggcaaaaa tgcactcatc
841 cattcactct gcaagtatct acggcacgta cgccagctcc caagcaggtt tgcgggttgc
901 acagcggagc gatgcaatct gatttaggct tttaaaggat tgcaatcaag tgggacccac
961 tagcctcaac cctgtacctc ccctcccctc cacccccagc agtctccaaa ggcctccaac
 
1021 aaccccagag tgggggccat gtatccaaag aaactccaag ctgtatacgg atcacactgg
1081 ttttccagga gcaaaaacag aaacagcctg aggctggtca aaattgaacc tcctcctgct
1141 ctgagcagcc tagggggcag actaagcaga gggctgtgca gacccacata aagagcctac
1201 tgtgtgccag gcacttcacc cgaggcactt cacaagcatg cttgggaatg aaacttccaa
1261 ctctttggga tgcaggtgaa acagttcctg gttcagagag gtgaagcggc ctgcctgagg
1321 cagcacagct cttctttaca gatgtgcttc cccacctcta ccctgtctca cggcccccca
1381 tgccagcctg acggttgtgt ctgcctcagt catgctccat ttttccatcg ggaccatcaa
1441 gagggtgttt gtgtctaagg ctgactgggt aactttggat gagcggtctc tccgctccga
1501 gcctgtttcc tcatctgtca aacgggctct aacccactct gatctcccag ggcggcagta
1561 agtcttcagc atcaggcatt ttggggtgac tcagtaaatg gtagatcttg ctaccagtgg
1621 aacagccact aaggattctg cagtgagagc agagggccag ctaagtggta ctctcccaga
1681 gactgtctga ctcacgccac cccctccacc ttggacacag gacgctgtgg tttctgagcc
1741 aggtacaatg actcctttcg gtaagtgcag tggaagctgt acactgccca ggcaaagcgt
1801 ccgggcagcg taggcgggcg actcagatcc cagccagtgg acttagcccc tgtttgctcc
1861 tccgataact ggggtgacct tggttaatat tcaccagcag cctcccccgt tgcccctctg
1921 gatccactgc ttaaatacgg acgaggacag ggccctgtct cctcagcttc aggcaccacc
1981 actgacctgg gacagtgaat cgtaagtatg cctttcactg cgaggggttc tggagaggct
2041 tccgagctcc ccatggccca ggcaggcagc aggtctgggg caggaggggg gttgtggagt
2101 gggtatccgc ctgctgaggt gcagggcaga tggagaggct gcagctgagc tcctatttc
2161 ataataacag cagccatgag ggttgtgtcc tgtttcccag tcctgcccgg tccccctcg
2221 gtacctcctg gtggatacac tggttcctgt aagcagaagt ggatgagggt gtctaggtct
2281 gcagtcctgg caccccagga tggggacacc cagccaagat acagcaacag caacaaagcg
2341 cagccatttc tttctgtttg cacagctcct ctgtctgtcg ggggctcctg tctgttgtct
2401 cctataagcc tcaccacctc tcctactgct tgggcatgca tctttctccc cttctataga
2461 tgaggaggtt aaggttcaga gaggggtggg gaggaacgcc ggctcacatt ctccatcccc
2521 tccagatatg accaggaaca gacctgtgcc agcctcagcc ttacatcaaa atgggcctcc
2581 ccatgcaccg tggacctctg ggccctcctg tcccagtgga ggacaggaag ctgtgagggg
2641 cactgtcacc cagggctcaa gctggcattc ctgaataatc gctctgcacc aggccacggc
2701 taagctcagt gcgtgattaa gcctcataac cctccaaggc agttactagt gtgattccca
2761 ttttacagat gaggaagatg gggacagaga ggtgaataac tggccccaaa tcacacacca
2821 tccataattc gggctcaggc acctggctcc agtccccaaa ctcttgaacc tggccctagt
2881 gtcactgttt ctcttgggtc tcaggcgctg gatggggaac aggaaacctg ggctgaactt
2941 gaggcctctc tgatgctcgg tgacttcaga cagttgctca acctctctgt tctcttgggc
3001 aaaacatgat aacctttgac ttctgtcccc tcccctcacc ccacccgacc ttgatctctg
3061 aagtgttgga aggatttaat ttttcctgca ctgagttttg gagacaggtc aaaaagatga
3121 ccaaggccaa ggtggccagt ttcctataga acgcctctaa aagacctgca gcaatagcag
3181 caagaactgg tattctcgag aacttgctgc gcagcaggca cttcttggca ttttatgtgt
3241 atttaatttc acaatagctc tatgacaaag tccacctttc tcatctccag gaaactgagg
3301 ttcagagagg ttaagtaact tgtccaaggt cacacagcta atagcaagtt gacgtggagc
3361 aatctggcct cagagccttt aattttagcc acagactgat gctcccctct tcatttagcc
3421 aggctgcctc tgaagttttc tgattcaaga cttctggctt cagctttgta cacagagatg
3481 attcaatgtc aggttttgga gcgaaatctg tttaatccca gacaaaacat ttaggattac
3541 atctcagttt tgtaagcaag tagctctgtg atttttagtg agttatttaa tgctctttgg
3601 ggctcaattt ttctatctat aaaatagggc taataatttg caccttatag ggtaagcttt
3661 gaggacagat tagatgatac ggtgcctgta aaacaccagg tgttagtaag tgtggcaatg
3721 atggtgacgc tgaggctgtg tttgcttagc atagggttag gcagctggca ggcagtaaac
3781 agttggataa tttaatggaa aatttgccaa actcagatgc tgttcactgc tgagcaggag
3841 ccccttcctg ctgaaatggt cctggggagt gcagcaggct ctccgggaag aaatctacca
3901 tctctcgggc aggagctcaa cctgtgtgca ggtacaggga gggcttcctc acctggtgcc
3961 cactcatgca ttacgtcagt tattcctcat ccctgtccaa aggattcttt tctccattgt
4021 acagctatga agctagtgct caaagaagtg aagtcattta ccccaggccc cctgccagta
4081 agtgacaggg cctggtcaca cttgggttta tttattgccc agttcaacag gttgtttgac
4141 cataggcgag attctcttcc ctgcaccctg ccgggttgct cttggtccct tattttatgc
4201 tcctgggtag aaatggtgcg agattaggca gggagtggac gcttccctgt ccctggcccc
4261 gcaaagagtg ctcccacctg ccccgatccc agaaatgtca ccatgaagcc ttcattcttt
 
4321 tggtttaaag cttggcctca gtgtccgtac accatggggt ccttggccag atggcgactt
4381 tctcctctcc agtcgccctc ccaggcacta gcttttagga gtgcagggtg ctgcctctga
4441 tagaagggcc aggagagagc aggttttgga gacctgatgt tataaggaac agcttgggag
4501 gcataatgaa cccaacatga tgcttgagac caatgtcaca gcccaattct gacattcatc
4561 atctgagatc tgaggacaca gctgtctcag ttcatgatct gagtgctggg aaagccaaga
4621 cttgttccag ctttgtcact gacttgctgt atagcctcaa caaggccctg accctctctg
4681 ggcttcaaac tcttcactgt gaaaggagga aaccagagta ggtgatgtga caccaggaaa
4741 gatggatggg tgtgggggaa tgtgctcctc ccagctgtca ccccctcgcc accctccctg
4801 caccagcctc tccacctcct ttgagcccag aattcccctg tctaggaggg cacctgtctc
4861 gtgcctagcc atgggaattc tccatctgtt ttgctacatt gaacccagat gccattctaa
4921 ccaagaatcc tggctgggtg caggggctct cgcctgtaac cccagcactt tgggaggcca
4981 aggcaggcgg atcaagaggt caggagttca agacctgcct ggccaacacg gtgaaacctc
5041 agctctacta aaaatacaaa aattagccag gcgtggtggc acacgcctgt aatcccagct
5101 atttgggaag ctgagacaga agaatttctt gaacccggga ggtggaggtt tcagtgagcc
5161 gagatcacgc cactgcactc caccctggcg gataaagcga gactctgtct caaaaaaaac
5221 ccaaaaacct atgttagtgt acagagggcc ccagtgaagt cttctcccag ccccactttg
5281 cacaactggg gagagtgagg ccccaggacc agaggattct tgctaaaggc caagtggata
5341 gtgatggccc tgccaggcta gaagccacaa cctctggccc tgaggccact cagcatattt
5401 agtgtcccca ccctgcagag gcccaactcc ctcctgacca ctgagccctg taatgatggg
5461 ggaatttcca taagccatga aggactgcac aaagttcagt tgggagtgaa agagaaatta
5521 aagggagatg gaaatataca gcactaattt tagcaccgtc ttcagttcta acaacactag
5581 ctagctgaag aaaatacaaa catgtattat gtaatgtgtg gtctgttcca tttggattac
5641 ttagaggcac gagggccaag gagaaaggtg gtggagagaa accagctttg cacttcattt
5701 gttgctttat tggaaggaaa cttttaaaag tccaaggggg ttgaagaatc tcaatatttg
5761 ttatttccag ctttttttct ccagtttttc atttcccaaa ttcaaggaca cctttttctt
5821 tgtattttgt taagatgatg gttttggttt tgtgactagt agttaacaat gtggctgccg
5881 ggcatattct cctcagctag gacctcagtt ttcccatctg tgaagacggc aggttctacc
5941 tagggggctg caggcaggtg gtccgaagcc tgggcatatc tggagtagaa ggatcactgt
6001 ggggcagggc aggttctgtg ttgctgtgga tgacgttgac tttgaccatt gctcggcaga
6061 gcctgctctc gctggttcag ccacaggccc caccactccc tattgtctca gccccgggta
6121 tgaaacatgt attcctcact ggcctatcac ctgaagcctt tgaatttgca acacctgcca
6181 acccctccct caaaagagtt gccctctcta gatccttttg atgtaaggtt tggtgttgag
6241 acttatttca ctaaattctc atacataaac atcactttat gtatgaggca aaatgaggac
6301 cagggagatg aatgacttgt cctggctcat acacctggaa agtgacagag tcagattaga
6361 tcctaggtct atctgaagtt aaaagaggtg tcttttcact tcccacctcc tccatctact
6421 ttaaagcagc acaaacccct gctttcaagg agagatgagc gtctctaaag cccctgacag
6481 caagagccca gaactgggac accattagtg acccagacgg caggtaagct gactgcagga
6541 gcatcagcct attcttgtgt ctgggaccac agagcattgt ggggacagcc ccgtctcttg
6601 ggaaaaaaac cctaagggct gaggatcctt gtgagtgttg ggtgggaaca gctcccagga
6661 ggtttaatca cagcccctcc atgctctcta gctgttgcca ttgtgcaaga tgcatttccc
6721 ttctgtgcag cagtttccct ggccactaaa tagtgggatt agatagaagc cctccaaggg
6781 ctccagcttg acatgattct tgattctgat ctgacccgat tctgataatc gtgggcaggc
6841 ccattcctct tcttgtgcct cattttcttc ttttgtaaaa caatggctgt accatttgca
6901 tcttagggtc attgcagatg aaagtgttgc tgtccagagc ctgggtgcag gacctagatg
6961 taggattctg gttctgctac ttcctcagtg acattgaata gctgacctaa tctctctggc
7021 tttggtttct tcatctgtaa aagaaggata ttagcattag cacctcacgg gattgttaca
7081 agaaagcaat gaattaacac atgtgagcac ggagaacagt gcttggcata tggtaagcac
7141 tacgtacatt ttgctattct tctgattctt tcagtgttac tgatgtcggc aagtacttgg
7201 cacaggctgg tttaataatc cctaggcact ttcacgtggt gtcaatccct gatcactggg
7261 agtcatcatg tgccttgact cgggcctggc ccccccatct ctgtcttgca ggacaatgcc
7321 gtcttctgtc tcgtggggca tcctcctgct ggcaggcctg tgctgcctgg tccctgtctc
7381 cctggctgag gatccccagg gagatgctgc ccagaagaca gatacatccc accatgatca
7441 ggatcaccca accttcaaca agatcacccc caacctggct gagttcgcct tcagcctata
7501 ccgccagctg gcacaccagt ccaacagcac caatatcttc ttctccccag tgagcatcgc
7561 tacagccttt gcaatgctct ccctggggac caaggctgac actcacgatg aaatcctgga
 
7621 gggcctgaat ttcaacctca cggagattcc ggaggctcag atccatgaag gcttccagga
7681 actcctccgt accctcaacc agccagacag ccagctccag ctgaccaccg gcaatggcct
7741 gttcctcagc gagggcctga agctagtgga taagtttttg gaggatgtta aaaagttgta
7801 ccactcagaa gccttcactg tcaacttcgg ggacaccgaa gaggccaaga aacagatcaa
7861 cgattacgtg gagaagggta ctcaagggaa aattgtggat ttggtcaagg agcttgacag
7921 agacacagtt tttgctctgg tgaattacat cttctttaaa ggtaaggttg ctcaaccagc
7981 ctgagctgtt tcccatagaa acaagcaaaa atatttctca aaccatcagt tcttgaactc
8041 tccttggcaa tgcattatgg gccatagcaa tgcttttcag cgtggattct tcagttttct
8101 acacacaaac actaaaatgt tttccatcat tgagtaattt gaggaaataa tagattaaac
8161 tgtcaaaact actgacgctc tgcagaactt ttcagagcct ttaatgtcct tgtgtatact
8221 gtatatgtag aatatataat gcttagaact atagaacaaa ttgtaataca ctgcataaag
8281 ggatagtttc atggaacata ctttacacga ctctagtgtc ccagaatcag tatcagtttt
8341 gcaatctgaa agacctgggt tcaaatcctg cctctaacac aattagcttt tgacaaaaac
8401 aatgcattct acctctttga ggtgctaatt tctcatctta gcatggacaa aataccattc
8461 ttgctgtcag gtttttttag gattaaacaa atgacaaaga ctgtggggat ggtgtgtggc
8521 atacagcagg tgatggactc ttctgtatct caggctgcct tcctgcccct gaggggttaa
8581 aatgccaggg tcctgggggc cccagggcat tctaagccag ctcccactgt cccaggaaaa
8641 cagcataggg gaggggaggt gggaggcaag gccaggggct gcttcctcca ctctgaggct
8701 cccttgctct tgaggcaaag gagggcagtg gaggcaagcc aggctgcagt cagcacagct
8761 aaagtcctgg ctctgctgtg gccttagtgg gggcccaggt ccctctccag ccccagtctc
8821 ctccttctgt ccaatgagaa agctgggatc aggggtccct gaggcccctg tccactctgc
8881 atgcctcgat ggtgaagctc tgttggtatg gcagagggga ggctgctcag gcatctgcat
8941 ttcccctgcc aatctagagg atgaggaaag ctctcaggaa tagtaagcag aatgtttgcc
9001 ctggatgaat aactgagctg ccaattaaca aggggcaggg agccttagac agaaggtacc
9061 aaatatgcct gatgctccaa cattttattt gtaatatcca agacaccctc aaataaacat
9121 atgattccaa taaaaatgca cagccacgat ggcatctctt agcctgacat cgccacgatg
9181 tagaaattct gcatcttcct ctagttttga attatcccca cacaatcttt ttcggcagct
9241 tggatggtca gtttcagcac cttttacaga tgatgaagct gagcctcgag ggatgtgtgt
9301 cgtcaagggg gctcagggct tctcagggag gggactcatg gtttcttatt ctgctacact
9361 cttccaaacc ttcactcacc cctggtgatg cccaccttcc cctctctcca ggcaaatggg
9421 agagaccctt tgaagtcaag gacaccgagg aagaggactt ccacgtggac caggtgacca
9481 ccgtgaaggt gcctatgatg aagcgtttag gcatgtttaa catccagcac tgtaagaagc
9541 tgtccagctg ggtgctgctg atgaaatacc tgggcaatgc caccgccatc ttcttcctgc
9601 ctgatgaggg gaaactacag cacctggtaa atgaactcac ccacgatatc atcaccaagt
9661 tcctggaaaa tgaagacaga aggtgattcc ccaacctgag ggtgaccaag aagctgccca
9721 cacctcttag ccatgttggg actgaggccc atcaggactg gccagagggc tgaggagggt
9781 gaaccccaca tccctgggtc actgctactc tgtataaact tggcttccag aatgaggcca
9841 ccactgagtt caggcagcgc cgtccatgct ccatgaggag aacagtaccc agggtgagga
9901 ggtaaaggtc tcgtccctgg gaacttccca ctccagtgtg gacactgtcc cttcccaata
9961 tccagtgccc aaggcaggga cagcagcacc accacacgtt ctggcagaac caaaaaggaa
10021 cagatgggct tcctggcaaa ggcagcagtg gagtgtggag ttcaaggtta gaatgtccct
10081 gggggacggg ggaagagcc tgtgtggcaa ggcccagaaa agcaaggttc ggaattggaa
10141 cagccaggcc atgttcgcag aaggcttgcg tttctctgtc actttatcgg tgctgttaga
10201 ttgggtgtcc tgtagtaagt gatacttaaa catgagccac acattagtgt atgtgtgtgc
10261 attcgtgatt atgcccatgc cctgctgatc tagttcgttt tgtacactgt aaaaccaaga
10321 tgaaaataca aaaggtgtcg ggttcataat aggaatcgag gctggaattt ctctgttcca
10381 tgccagcacc tcctgaggtc tctgctccag gggttgagaa agaacaaaga ggctgagagg
10441 gtaacggatc agagagccca gagccagctg ccgctcacac cagaccctgc tcagggtggc
10501 attgtctccc catggaaaac cagagaggag cactcagcct ggtgtggtca ctcttctctt
10561 atccactaaa cggttgtcac tgggcactgc caccagcccc gtgtttctct gggtgtaggg
10621 ccctggggat gttacaggct gggggccagg tgacccaaca ctacagggca agatgagaca
10681 ggcttccagg acacctagaa tatcagagga ggtggcattt caagcttttg tgattcattc
10741 gatgttaaca ttctttgact caatgtagaa gagctaaaag tagaacaaac caaagccgag
10801 ttcccatctt agtgtgggtg gaggacacag gagtaagtgg cagaaataat cagaaaagaa
10861 aacacttgca ctgtggtggg tcccagaaga acaagaggaa tgctgtgcca tgccttgaat
 
10921 ttcttttctg cacgacaggt ctgccagctt acatttaccc aaactgtcca ttactggaac
10981 ctatgatctg aagagcgtcc tgggtcaact gggcatcact aaggtcttca gcaatggggc
11041 tgacctctcc ggggtcacag aggaggcacc cctgaagctc tccaaggtga gatcaccctg
11101 acgaccttgt tgcaccatgg tatctgtagg gaagaatgtg tgggggctgc agcactgtcc
11161 tgaggctgag gaaggggccg agggaaacaa atgaagaccc aggctgagct cctgaagatg
11221 cccgtgattc actgacacgg gacggtgggc aaacagcaaa gccaggcagg ggctgctgtg
11281 cagctggcac tttcggggcc tcccttgagg ttgtgtcact gaccctgaat ttcaactttg
11341 cccaagacct tctagacatt gggccttgat ttatccatac tgacacagaa aggtttgggc
11401 taagttgttt caaaggaatt tctgactcct tcgatctgtg agatttggtg tctgaattaa
11461 tgaatgattt cagctaaagt gacacttatt ttggaaaact aaaggcgacc aatgaacaac
11521 ctgcagttcc atgaatggct gcattatctt ggggtctggg cactgtgaag gtcactgcca
11581 gggtccgtgt cctcaaggag cttcaagccg tgtactagaa aggagagagc cctggaggca
11641 gacgtggagt gacgatgctc ttccctgttc tgagttgtgg gtgcacctga gcagggggag
11701 aggcgcttgt caggaagatg gacagagggg agccagcccc atcagccaaa gccttgagga
11761 ggagcaaggc ctatgtgaca gggagggaga ggatgtgcag ggccagggcc gtccaggggg
11821 agtgagcgct tcctgggagg tgtccacgtg agccttgctc gaggcctggg atcagcctta
11881 caacgtgtct ctgcttctct cccctccagg ccgtgcataa ggctgtgctg accatcgacg
11941 agaaagggac tgaagctgct ggggccatgt ttttagaggc catacccatg tctatccccc
12001 ccgaggtcaa gttcaacaaa ccctttgtct tcttaatgat tgaacaaaat accaagtctc
12061 ccctcttcat gggaaaagtg gtgaatccca cccaaaaata actgcctctc gctcctcaac
12121 ccctcccctc catccctggc cccctccctg gatgacatta aagaagggtt gagctggtcc
12181 ctgcctgcat gtgatctgta aatccctggg atgttttctc tg
**SEQ ID NO. 33**
   gi/125294, P12277 - Homo sapiens
   Creatine kinase, B chain (B-CK) 
mpfsnshnal klrfpaedef pdlsahnnhm akvltpelya elrakstpsg ftlddviqtg
vdnpghpyim tvgcvagdee syevfkdlfd piiedrhggy kpsdehktdl npdnlqggdd
ldpnyvlssr vrtgrsirgf clpphcsrge rraieklave alssldgdla gryyalksmt
eaeqqqlidd hflfdkpvsp lllasgmard wpdargiwhn dnktflvwvn eedhlrvism
qkggnmkevf trfctgltqi etlfkskdye fmwnphlgyi ltcpsnlgtg lragvhiklp
nlgkhekfse vlkrlrlqkr gtggvdtaav ggvfdvsnad rlgfsevelv qmvvdgvkll
iemeqrleqg qaiddlmpaq k
**SEQ ID NO. 34**
   NM_001823 Homo sapiens creatine kinase, brain (CKB), mRNA
   Creatine kinase, B chain (B-CK) 
1 gctgttcgcc tgcgtcgctc cgggagctgc cgacggacgg agcgcccccg cccccgcccg
61 gccgcccgcc cgccgccgcc atgcccttct ccaacagcca caacgcactg aagctgcgct
121 tcccggccga ggacgagttc cccgacctga gcgcccacaa caaccacatg gccaaggtgc
181 tgacccccga gctgtacgcg gagctgcgcg ccaagagcac gccgagcggc ttcacgctgg
241 acgacgtcat ccagacaggc gtggacaacc cgggccaccc gtacatcatg accgtgggct
301 gcgtggcggg cgacgaggag tcctacgaag tgttcaagga tctcttcgac cccatcatcg
361 aggaccggca cggcggctac aagcccagcg atgagcacaa gaccgacctc aaccccgaca
421 acctgcaggg cggcgacgac ctggacccca actacgtgct gagctcgcgg gtgcgcacgg
481 gccgcagcat ccgtggcttc tgcctccccc cgcactgcag ccgcggggag cgccgcgcca
541 tcgagaagct cgcggtggaa gccctgtcca gcctggacgg cgacctggcg ggccgatact
601 acgcgctcaa gagcatgacg gaggcggagc agcagcagct catcgacgac cacttcctct
661 tcgacaagcc cgtgtcgccc ctgctgctgg cctcgggcat ggcccgcgac tggcccgacg
 
721 cccgcggtat ctggcacaat gacaataaga ccttcctggt gtgggtcaac gaggaggacc
781 acctgcgggt catctccatg cagaaggggg gcaacatgaa ggaggtgttc acccgcttct
841 gcaccggcct cacccagatt gaaactctct tcaagtctaa ggactatgag ttcatgtgga
901 accctcacct gggctacatc ctcacctgcc catccaacct gggcaccggg ctgcgggcag
961 gtgtgcatat caagctgccc aacctgggca agcatgagaa gttctcggag gtgcttaagc
1021 ggctgcgact tcagaagcga ggcacaggcg gtgtggacac ggctgcggtg ggcggggtct
1081 tcgacgtctc caacgctgac cgcctgggct tctcagaggt ggagctggtg cagatggtgg
1141 tggacggagt gaagctgctc atcgagatgg agcagcggct ggagcagggc caggccatcg
1201 acgacctcat gcctgcccag aaatgaagcc cggcccacac ccgacaccag ccctgctgct
1261 tcctaactta ttgcctgggc agtgcccacc atgcacccct gatgttcgcc gtctggcgag
1321 cccttagcct tgctgtagag acttccgtca cccttggtag agtttatttt tttgatggct
1381 aagatactgc tgatgctgaa ataaactagg gttttggcct gcctgcgtct g
**SEQ ID NO. 35**
   X15334
   Human gene for creatine kinase B (EC 2.7.3.2). 
1 gatcagtttt tttttttaat cgcacttatg cttattgttt attagcgttt cctcccatct
61 ttgcctgaag tctccgggga ctgcctttgg gggtcgggta aacttgtccc ctgcgaagag
121 ggcccagggt tggggtctgg aaactccgag gctgcacttg ccagcggcct cttaaggcca
181 cagcgtcccc gtggtttctg gctcgcagcc ccccgagacc caggacttgt ccaaggtcag
241 ggcaccgcgg gtgcccccgg gctgggccgc agcagactgc gcttcccgcg cgccttcgct
301 ttgcaccagg atcgcccagg aaatgcctgc gggcaccttg aggaaggtcg gcggctccgg
361 gccagctcgc actggccggg gtggggcggg ggccgtacct gctgcggaag ccccgaaagc
421 tttcgcccgg cccctcgccg ccgccgcggg ggctggctgg actaggcggg caggctcgag
481 gatgcggatg aacccaagcg tcctcgagtg cccggaggct ctccgcctca gtttcccgcc
541 cagaggcaag ggcgtgcgag gggatccaga tatccaagga cctgaggttt cggcctcgag
601 gtcttgggcg ggggactggg caggctgcgc ggggtcccag cgaggggaca gctcgggtgg
661 gcggccaggg tgttgggggc tgcgggcggc ggacaaagcg gcggcaccac cccgcggcgc
721 gggccaatgg aatgaatggg ctataaatag ccgccaatgg gcggcccgcg ttgtgcccct
781 taagagccgc gggagcgcgg agcggccgct gttcgcctgc gtcgctccgg gagctgccga
841 cggacggagc gcccccgccc ccgcccggcc gcccggtgag tgggcccggg ggccgggggc
901 gtccgcgccc gggctagggg cgctgcgagc aaagggggcg cgtcgcctgg agcgcgcgcc
961 ggaccggccg ggggtccccg gcgatgatgg cgctccccgc gcgcgctgcg gaccccgctg
1021 accttggccg cgtcccgggg ggcgccgggg ggcccggcgg cgggggcctg agtggtacgc
1081 gggagcccgg gaaccccggc gtgccggtcc cctctgaccc cgcgtctccc cgcagcccgc
1141 cgccgccatg cccttctcca acagccacaa cgcactgaag ctgcgcttcc cggccgagga
1201 cgagttcccc gacctgagcg cccacaacaa ccacatggcc aaggtgctga cccccgagct
1261 gtacgcggag ctgcgcgcca agagcacgcc gagcggcttc acgctggacg acgtcatcca
1321 gacaggcgtg gacaacccgg gtacgcgacc cctcggggcc ggggtcccgg ccccccctcc
1381 ccccgcgcag ccgcagggtc ctcagcagcg cgctcgggcc cggcagtgac gtcactgtcc
1441 ccgtcccgcg ccccctcccc caggccaccc gtacatcatg accgtgggct gcgtggcggg
1501 cgacgaggag tcctacgaag tgttcaagga tctcttcgac cccatcatcg aggaccggca
1561 cggcggctac aagcccagcg atgagcacaa gaccgacctc aaccccgaca acctgcaggt
1621 gcggggctgc gggcgggccg ggcgggcggg gccggggtct tcgggcgctc actcccgtct
1681 cgcctcccag ggcggcgacg acctggaccc caactacgtg ctgagctcgc gggtgcgcac
1741 gggccgcagc atccgtggct tctgcctccc cccgcactgc agccgcgggg agcgccgagc
1801 catcgagaag ctcgcggtgg aaggtagggg ccgggcgggc cgaggggcgg cggcggccgc
1861 gtccccctcc cggcgcggtc cccgcccgct tttgtttacg tcgcccggga gcggcagccg
1921 ccgtcgcgct cttatctgcg cgcgcccggg ttcagtttcc cggacccacc gagggacgga
1981 ggcccagccc ccgcgcccac agcggcctgg ggcccaggga gggcgggtcc tggcgcgggg
2041 tcaccgcctg ggaccgtcgc ccgggccgtg aggactggac gcccgcagat ccgggcgggt
2101 ggggccctct gacgtccccc gaggtggggc acgggggcgg gcgggtccgc gctgcgggct
 
2161 ggaggggcgg gcgcgggagc ccagcgtcct gagcgcaccc ctcgcagccc tgtccagcct
2221 ggacggcgac ctggcgggcc gatactacgc gctcaagagc atgacggagg cggagcagca
2281 gcagctcatc gacgaccact tcctcttcga caagcccgtg tcgcccctgc tgctggcctc
2341 gggcatggcc cgcgactggc ccgacgcccg cggtatctgg tgcgtgtccc tctgcgccct
2401 ctcgcggcgt cctccctccc cgctacctcc gctttccctc tcgcccccct cgcgggggtg
2461 gggcccctcg cggcgaggag gaggaggagg aggagggagg ggccggccgc gctccgggtc
2521 tgggttccgt gccgcgcctc ctcctgcgcc ggtgaccttg gccgagcagg tgcgttaagg
2581 gactgggccc cggcccgtgg gggctcagga ctcagcaaca cctccccacc ccgagacgtg
2641 aggtgggggc ggggctctct ggcgcctctc cccgacggcc ctgggagctg gagctctttg
2701 ttttcttttc tcactcctcc gccgctggga ttctaccagg ggctggtgac gccaaagctt
2761 ctccaggggc agggctccta cccccactgt gggggcgggg tcggctgtcc ctggcggtcc
2821 ctggccccgc cccacctcgg gccacagcgc atgatggcag ctggggttct cctgctgtga
2881 ggcgtcccgg ttcccccgcc cgccccgtgt tggcgggtgg agtcttggca gcagcctcca
2941 ctcctgggca tggcagggag cagcacctca gggacttggg aagttccttt ggtctggggg
3001 cggcctgggg cttttttctg ggtatgccct gagaccagcc ctcccgcagg cacaatgaca
3061 ataagacctt cctggtgtgg gtcaacgagg aggaccacct gcgggtcatc tccatgcaga
3121 aggggggcaa catgaaggag gtgttcaccc gcttctgcac cggcctcacc caggtgccag
3181 ggacggggca ggcccagacc ccagggcccc agcagggatg tgggtgcccc agcatcagtc
3241 cccccggggg atttccggca ctggggagtc tcagggcctg taggggtttc aggcaggcct
3301 tctccctcat accctcttct ccgtctgcag attgaaactc tcttcaagtc taaggactat
3361 gagttcatgt ggaaccctca cctgggctac atcctcacct gcccatccaa cctgggcacc
3421 gggctgcggg caggtgtgca tatcaagctg cccaacctgg gcaagcatga gaagttctcg
3481 gaggtgctta agcggctgcg acttcagaag cgaggcacag gtgagcaggg caggtgctgc
3541 ggcttcccgt ggcctttggg cagccctgtt tcctccgccc tgacttgctg tctccccagg
3601 cggtgtggac acggctgcgg tgggcggggt cttcgacgtc tccaacgctg accgcctggg
3661 cttctcagag gtggagctgg tgcagatggt ggtggacgga gtgaagctgc tcatcgagat
3721 ggaacagcgg ctggagcagg gccaggccat cgacgacctc atgcctgccc agaaatgaag
3781 cccggcccac acccgacacc agccctgctg cttcctaact tattgcctgg gcagtgccca
3841 ccatgcaccc ctgatgttcg ccgtctggcg agcccttagc cttgctgtag agacttccgt
3901 cacccttggt agagtttatt tttttgatgg ctaagatact gctgatgctg aaataaacta
3961 gggttttggc ctgcctgcgt ctgagtggtg cctctccttt cccagggggg aggggaagg
4021 gcagcagcca ggccccagga gtcttgagtc ctgggcctgc tgtgggcctc gccttctgtg
4081 agatgggaca agagccagga ggtggccact ctgttctgcc tgccctacct agtccatggg
4141 ccccttccct cgtgtctatc gggctgtgca ggcaggaaca tgggagagag cgagggagga
**SEQ ID NO. 36**
   P14618 - Homo sapiens
   Pyruvate kinase M1 or M2 isozyme 
mskphseagt afiqtqqlha amadtflehm crldidsppi tarntgiict igpasrsvet
lkemiksgmn varlnfshgt heyhaetikn vrtatesfas dpilyrpvav aldtkgpeir
tglikgsgta evelkkgatl kitldnayme kcdenilwld yknickvvev gskiyvddgl
islqvkqkga dflvteveng gslgskkgvn lpgaavdlpa vsekdiqdlk fgveqdvdmv
fasfirkasd vhevrkvlge kgknikiisk ienhegvrrf deileasdgi mvargdlgie
ipaekvflaq kmmigrcnra gkpvicatqm lesmikkprp traegsdvan avldgadcim
lsgetakgdy pleavrmqhl iareaeaaiy hlqlfeelrr lapitsdpte atavgaveas
fkccsgaiiv ltksgrsahq varyrprapi iavtrnpqta rqahlyrgif pvlckdpvqe
awaedvdlrv nfamnvgkar gffkkgdvvi vltgwrpgsg ftntmrvvpv p
**SEQ ID NO. 37**
   X56494
   H.sapiens M gene for M1-type and M2-type pyruvate kinase 
1 ggtcttcaca ttttgaatgc gcaacattgt atctgtgaat gaaggcaaga gttaacagct
61 gtttaattga taactgctcg catcattagt tgctggctaa caactgggaa atcagaaaat
121 gtcttgtaga aaaatgtaag aaaagttcca acaatactga cttaaacacg agcaaaggtg
181 aaaacagaaa tgctgactcc tgcataggtt atcggcccta atgttctgac ttgatatttc
241 cagatgccca gctctgcgct aatatcaaca ccgtctattt actttctact ctgaggcatt
301 cgctctgcag gattccagac cctactaaat tattcacatg gccccaaccg gtccttcctt
361 gttccgcggt cctaacacaa tgaatggtcc taagaggaaa acggcctcgg ctcccgctcc
421 aggcccactt cgcagtccct agttctccct actgccgctc cagtgccaga gcccctccga
481 aggcggccag gacctccaac cacgcacaag tctgcagctc tccccaactt tccgttcagc
541 tcagtctccg agggtgcgcc agagcagaca cccggaggag tggggagtgg cagggcgggg
601 ccgggagaat gctgccccgg aacccataaa ttcggccctg cccaggtagg ccgggacagc
661 tggggtggcc tgggccgaga gccaagaaaa gagaccccat ctggacgccc aacttggcgg
721 caacaggtgg ccggcgcccg ggggtctggg aggaaagtcg ctccgggcgg gccccgttgc
781 cccgccgcgt ccccattggt catcaggttt cttaaaatgt gactctgaat ctgtgtcctt
841 ccgccgcaga atttagtccc accgaaaggg caacctgccc gcgcgttccg ccaccgccgc
901 cgcgcttcct cctgaaggtg actcgagccc gcggggacgc agggggcggg gcccgggtcg
961 cccggagccg ggattgggca gagggcgggg cggcggaggg attgcggcgg cccgcagcgg
1021 gataaccttg aggctgaggc agtggctcct tgcacagcag ctgcacgcgc cgtggctccg
1081 gatctcttcg tctttgcagc gtagcccgag tcggtcagca gccggaggtg agcggtgcag
1141 gcagtacgcc atcagtcccc accaagggcc agtcgcccgg ctagtgcgga atcccggcgc
1201 gccggccggc cccgggcacg caggcagggc ggcgcaggat ccctgtgcta aatggtatat
1261 taaccacttc tcagtcttac cactctcttt caatttgtct cgacccagga cctcagcagc
1321 catgtcgaag ccccatagtg aagccgggac tgccttcatt cagacccagc agctgcacgc
1381 agccatggct gacacattcc tggagcacat gtgccgcctg gacattgatt caccacccat
1441 cacagcccgg aacactggca tcatctgtac cattggtgag tgggtgtccc ccttccccca
1501 aaaagggctt catgggcagt gacctttctc tcctgaaaag agctccatgc actttttaaa
1561 gacttttgag ctatttggga gaggaaaaat tttcagggaa aaaaattctt taaacttaaa
1621 gcaaacttaa atgtttttcc ttggttgaat aattaatact tgtggcttta aaacttttcc
1681 taataggccc agcttcccga tcagtggaga cgttgaagga gatgattaag tctggaatga
1741 atgtggctcg tctgaacttc tctcatggaa ctcatgaggt gagctgtggc tggaccctat
1801 cctggcaggg gaattggagc tggattctag tgtgggagca cgcttgtcat cttccttctt
1861 ttcccccagt accatgcgga gaccatcaag aatgtgcgca cagccacgga aagctttgct
1921 tctgacccca tcctctaccg gcccgttgct gtggctctag acactaaagg acctgagatc
1981 cgaactgggc tcatcaaggg cgtgagtatt ctgcggagag cgaggggaag gctcagtagg
2041 caatatgccc cagagacatg attccttccg aggtgatgct gctactggtg tctccagttt
2101 ggactcttcc ttactctctt gtccctagag cggcactgca gaggtggagc tgaagaaggg
2161 agccactctc aaaatcacgc tggataacgc ctacatggaa aagtgtgacg agaacatcct
2221 gtggctggac tacaagaaca tctgcaaggt ggtggaagtg ggcagcaaga tctacgtgga
2281 tgatgggctt atttctctcc aggtgaagca gaaaggtacg tatgggagct ggagtccagt
2341 tgtctaaaac agtcttttgt ctctaaactt ctcgtctctg cctccccaac ttaccctttt
2401 ttatacaggt gccgacttcc tggtgacgga ggtggaaaat ggtggctcct tgggcagcaa
2461 gaagggtgtg aaccttcctg gggctgctgt ggacttgcct gctgtgtcgg agaaggacat
2521 ccaggatctg aagtttgggg tcgagcagga tgttgatatg gtgtttgcgt cattcatccg
2581 caaggcatct gatgtccatg aagttaggaa ggtcctggga gagaagggaa agaacatcaa
2641 gattatcagc aaaatcgaga atcatgaggg ggttcggagg caagtccccg ttgtccctgg
2701 tctactgcca tacttgtggc ctctgttcta tataacctct ctccccccca ctttgtccat
2761 caggtttgat gaaatcctgg aggccagtga tgggatcatg gtggctcgtg gtgatctagg
2821 cattgagatt cctgcagaga aggtcttcct tgctcagaag atgatgattg gacggtgcaa
2881 ccgagctggg aagcctgtca tctgtgctac tcaggcatgt gcccaccctt ccccacattc
2941 tcatgtgcac actcgcatgt ttgtatggga aagctctgga ggctgtctga tctcttccca
3001 tggaattgtc gcaacgtaac acacagataa tccccttccc ccatgtacct acacaaagcc
3061 atactctgtg tacctactca ctatccagag gatcagcttg ctgtcatttg tctctgaaga
3121 cagctcaagc tacatctcac taatgctctg tcccctccca gatgctggag agcatgatca
 
3181 agaagccccc gcccactcgg gctgaaggca gtgatgtggc caatgcagtc ctggatggag
3241 ccgactgcat catgctgtct ggagaaacag ccaaagggga ctatcctctg gaggctgtgc
3301 gcatgcagca cctggtgagt tctgggcctg ccccatcccc cagggcttcg gactgggcct
3361 gggatggatg caagctctgg tgcagagctt tttaggtttc tccatcctct tatgcacagc
3421 ctttcattat cctccaagtt acagcagcaa gagggtgggg gtggaagtgg aggtggcttt
3481 ttttttttct cctgttcctg cattcctgcc cacaccccca cccctctcat ttccttctgc
3541 tctggaggca cctccttcat tggacaccac acagtttatt tcacttctga cttcaaggtt
3601 gtgaattctt cccatggctt aagtcctggg atacttctgc agtgaaagga ggtcttgtac
3661 ctcttcctca gagtcagaag ttctgagtac ctttgcccta ttctgaaaag ggctaggggc
3721 tcctgctccc agctgccctc ttcctttggc ttccaattca gttccctctg ccccgcatcc
3781 tgcagacagg cgctcccgca gggggccctt gtggacctgc actggagtct gttgccttca
3841 ctgagctgcc tgtgctggcc ttgcatggtg cctgtagggg gatttgcttt gctgtgccat
3901 tggggtacag ctgctgctct tactctagac caaaaagtcg ggttgagtga ctggtggcag
3961 ggccaagata gagacagcgg ggagggtggc tgaccctggc ggccctggac tgagcgtctg
4021 gaggagtcgt ggaggctctt tcccttcttt ctcctctgag agctcgttct tcaggctctt
4081 ccagcttgtc atgtcgagtg cctggccact gctcagggtt ggaggctcag tccctttgcc
4141 ctgtctgttc cagctctgga gctaactcag ggatccctga tcagggttac gtaggtttgg
4201 taaaatgagt gctggaaatt aactttctcc cagtagtctt aggtctagct cagtgaactt
4261 aaactttatc cagatatggt ttttccttca gcctttctat tccctttcta gccagtgaaa
4321 gacccgctgc cctttgacct cagccccctc caagccccca agtttaaaac gccaccccct
4381 gccaccagaa aaaacagaaa aaaaaaaaaa aaaaaaaact aaaacaccca tctggtctgg
4441 gcatcttcct tcctttttca ctatgtatcc tgttactggg cttaaacagc tttcagagaa
4501 gagatgtcat ttctattaaa tgctctttca gtagcgaact gagttcacac ttgactaagg
4561 atattttccg gactgtctgt catcagcatc cttagtgggt ttccccatat ttaaattggt
4621 agaggccagg gatggtggct cacacctgta atctcagtac tttgggaggc caaggtaggt
4681 ggattgcttg agctcagaag accagcctgg gcaacctggt gaaaccctgt ctctactaaa
4741 aattcaagtt agctagctgg gcatggtgat gcacttctgt agtcccagct acttggagag
4801 ggggtggtgc tggggcagca ggatcgctta aacccaggag gttaaggttg cagtcagcca
4861 agatggtacc agcctaggtg acaaagtgac accctgtctc aaaaaagaaa ccaaacaaac
4921 ataaaaaaaa aaacaaaaaa atcggtagag agtgatttct ctcccaggcc cacttaatgt
4981 agactgggcc tggctgacac ctcaccattc gtgtgatgtg attgctgttc tgatgcttag
5041 atactcttgg cgcagtctca caattgccac catggtagga aggtgtccag gagacggtgc
5101 accttgaacc agtcaccact aaagtggctg cctttctggg tctctccaca catcccctct
5161 ctctaatttc cctacttaat cgtgtgactt catggtctca aaggaggaac agaggctgat
5221 cttgacttag atatactgaa ccatgaaatc actgcataga atgtggggac ttgaatgtgt
5281 ctttgggcaa gtcatttaac ctcttaagac ctcatctgta aaatggatta gatatgttta
5341 attatagcct tagcattaaa tattcattgc tgttattatt aagtgtctga taagtctctg
5401 tgtacatgga tgtaatcttc ctaactccca ttacctccat ttatagatga gggttatatg
5461 gccaataaag cctgggtttg aatctaggtc tactgcctcc aaagccagtc ttctctcctg
5521 caacatcatg ctctgtctag caggagatga gaacaggtct ccatttggag cctgtcagtg
5581 gggtcagaga ctaagattca ggctcagggt ctaaattccg tatcctttct tccataccct
5641 ggtgtttcct atgaacagat agatacttta gggctgcaag gtttggattg catggcactg
5701 ctcagaagat aagttacagg tctgggctag gctgtagctg cccctccagg tggctagacc
5761 tttcctttct gtgtcaccag ttaacactgg ccaacagttc cttccattaa ctgttcactg
5821 ctttctcctg tgtctaactg atgcagttta tgacccataa ctaagagcag taccaggtat
5881 ggctctgttt cctgttcatg tcccctgtcc tctgggctgc atgcattccg ttcttacaga
5941 aagaatacct ttaacctagt acatcctgcc acacatctgc ttctactgtg aaattgatga
6001 gggggtatta ccgattcttc cctctcccat catttactga gatgctggtg attgcattat
6061 aatcctctta agcttacatt gtctttctga ttcttggtct tatctgagca agtgatctat
6121 aaataactca gtggctttct catgactgtt ttaattatta gattttaatc aagtgtctta
6181 ttaaatatat ctgcatgctt ccacaggcat ctgtctcttc acatggctgt tcagtgtgcc
6241 tctcacaact tagcccaaac tcagttgagc tgccttgctt tggctttgac ccagctttcc
6301 agcgctgctc aatctgttgc catggcaggc cattggaaag gctcagttca tccccgtgcc
6361 tgaagccaag tgagcgctca ctccatgcat gcatggaggc tgggcaggag cctgcctaat
6421 caaccagcca tgtgaggagg gagggcctgt tccttcctgt aagctatgtc atgaggcagc
 
6481 gtggtcaagt cctctgccag ggagtggcct ggcccagcct gggcatgttt tcatgccagg
6541 gtgctagagc ctactgccag attgtctccc tccaccccca atgaaaaaat ccttccagaa
6601 gggaagagcc aatttcccct gtattggagg ggaagtggca gcacctcctg aagcagttgg
6661 actttcatca ccctacctct gcatctgcct gaaggacaga tttagccaat taacctaagg
6721 ttaccttcct ctctgataaa ttccccattc tgtcttccca tgtgttgtgt ctcgtttttt
6781 tcctcctcct tccctcttcc ttgccccctc ttcccctaaa ccttacagat agctcgtgag
6841 gctgaggcag ccatgttcca ccgcaagctg tttgaagaac ttgtgcgagc ctcaagtcac
6901 tccacagacc tcatggaagc catggccatg ggcagcgtgg aggcttctta taagtgttta
6961 gcagcagctt tgatagttct gacggagtct ggcaggtagg gccctaaggg caggtaacac
7021 tgttaggata accagcctct tgctgcacct gccccaggag aagagagaag gcccaacctg
7081 gcatctggga acagagcctc ttctcgtctg taggaacacc gccagggagg tcatggcagg
7141 gcagaccaaa gggtcctgtg gctcagtagg cacagtagat gtcacaggca cttggtgaag
7201 gactggtttc tgtggagtct tgatcttggc tcagctcaga atctccagtg attgggctcc
7261 tcttggcctt tgttcccagg aacatgttcc tcaccagctg tccggtgact cttcccctcc
7321 ctctcctttt gtgacaaagc tctgacaaag ctctgtcccc ctctcgtccc tctggacgga
7381 tgttgctccc ctagattgcc cgtgaggcag aggctgccat ctaccacttg caattatttg
7441 aggaactccg ccgcctggcg cccattacca gcgaccccac agaagccacc gccgtgggtg
7501 ccgtggaggc ctccttcaag tgctgcagtg gggccataat cgtcctcacc aagtctggca
7561 ggtaggaggc ggcagcggct ccctggaatg ccctgctcag tggtacctca ccttgggggt
7621 cctgggagca gtccattgaa caatgctcag gtggcactga gccaaggtaa gacccctctg
7681 cctgccacct tgggcctgca gggaaggatt gagcagagcc ccttcccagg gcccaaagga
7741 ctctaggtag cactcataag gaatgtcaga acatttggat caaaagcaaa tttatgctgg
7801 agatttatta cataacagtg cacaggctga ctacaaatgg ttatttgata ttgaaaattt
7861 agtcctctaa aattgtaaaa gataccactt ttgcttattc cagttactat gtgctcttta
7921 aaaatttcag ttgggaaatg aatttattta aatgctgttt actgtgcctc catttggcac
7981 actagtccct gctgtttttg agccctaaag acaaattggg ttccagctca ggagaggttg
8041 ctgtgctatc ttggctgaca ttctgtgggc ctggcagcca ggctgaggac tgtgtggcct
8101 atgctgggcc tccaacttgg gatcccttcc ttggcccagg acattgagtt aatgtccttc
8161 actctcctag ttagggagta tgctccttgt ccctgtccac aggggagcaa gggtttcctg
8221 gaagagggga gcaaacaggc agtgcccatg cactgaggag cagcagatgg gcgtgggcag
8281 cccagagaac caggacacaa gctctgtgca gatccctcag cagagggctc cagcctccca
8341 ctcttggctg aacagctcca acccgtaggg ttgacctttc ttaaaaggtc cagttcttgc
8401 tgtttggcta ttttaagctc tagtcttctg gggtttcact cagctggtcc tggcttcagc
8461 aattgcttcc ctctgaaggc cttgcataga ggccaagcgt gaagtgcagg gacttctctg
8521 ctgtgatgtg gcttaagttt ccctgacacc tgttgagtgt cctcataact tcccttctgg
8581 tgcccctccc cagctcctga gaccagctgc agctacaagt gtgcagtgtc agtgttcaag
8641 aaagtgcctg gcagaggggc tttagaaggg tcccctgcct tccaaaggag ctttggcagg
8701 cagacgtgct cctgcagcaa cactcccatt tcctgttctt gcctgctgag tagcacctag
8761 atttctaagc ctcatctaga tactcagatt tgattctggg cctttatagc ccagttgctg
8821 ggactgtttc aggagctagg ggccatgtgg ggcagggaga gggcacaaaa gtagagaagc
8881 ctgatgttga ttcccagggg gctggtcagc tctgctactg ctccttgcag atgtcaagag
8941 tcaggtgcta gtcacgtgct gcttggcttg tcactgtcat tggcagcgag aggaatgggt
9001 gctggtgaca ttgggccagg gctgcctctc tgtgtcagag ttcagggtgt aggaggggtt
9061 ctgccaacca tgggctgtgt ggggtaagtg ggttgaggct gatctttctg ggtcaaggtg
9121 atcctgagcc cttgcctgtg gaatgggggt agagggcaat ggtaacctag ctagcatgct
9181 gtgggggata taggatgagg ggctgcccga ccctcgggag gggtcctagg gagcagatgt
9241 tgaagaggcc agagccctca gtgagctgga tgagggggtg agccgtttga actccctgag
9301 ggtacttcct ggggcctcgt gtaatggtct cttctgtatg tcccccatcc catctcaggt
9361 ctgctcacca ggtggccaga taccgcccac gtgcccccat cattgctgtg acccggaatc
9421 cccagacagc tcgtcaggcc cacctgtacc gtggcatctt ccctgtgctg tgcaaggacc
9481 cagtccagga ggcctgggct gaggacgtgg acctccgggt gaactttgcc atgaatgttg
9541 gtacgtggct ggagcagggg ctagagccta gaggagcttg gggatgcttg agcattggcc
9601 accaacctcc cttctcttcc tccaggcaag gcccgaggct tcttcaagaa gggagatgtg
9661 gtcattgtgc tgaccggatg gcgccctggc tccggcttca ccaacaccat gcgtgttgtt
9721 cctgtgccgt gatggacccc agagcccctc ctccagcccc tgtcccaccc ccttccccca
 
9781 gcccatccat taggccagca acgcttgtag aactcactct gggctgtaac gtggcactgg
9841 taggttggga caccagggaa gaagatcaac gcctcactga aacatggctg tgtttgcagc
9901 ctgctctagt gggacagccc agagcctggc tgccccatca tgtggcccca cccaatcaag
9961 ggaagaagga ggaatgctgg actggaggcc cctggagcca gatggcaaga gggtgacagc
10021 ttcctttcct gtgtgtactc tgtccagttc ctttagaaaa aatggatgcc cagaggactc
10081 ccaaccctgg cttggggtca agaaacagcc agcaagagtt aggggtcctt agggcactgg
10141 gctgttgttc cattgaagcc gactctggcc ctggccctta cttgcttctc tagctctcta
10201 ggcctctcca gtttgcacct gtccccaccc tccactcagc tgtcctgcag caaacactcc
10261 accctccacc ttccatttcc cccactactg cagcacctcc aggcctgttg ctatagagcc
10321 tacctgtatg taataaacaa cagctgaagc acctgtttcc tctctttt
SEQ ID NO. 38
   Q01995 - Homo sapiens
   Transgelin 
mankgpsygm srevqskiek kydeeleerl vewiivqcgp dvgrpdrgrl gfqvwlkngv
ilsklvnsly pdgskpvkvp enppsmvfkq meqvaqflka aedygviktd mfqtvdlfeg
kdmaavqrtl malgslavtk ndghyrgdpn wfmkkaqehk reftesqlqe gkhviglqmg
snrgasqagm tgygrprqii s
SEQ ID NO. 39
   D84342
   Homo sapiens DNA for SM22 alpha, complete cds 
1 ccgggtgaaa gcagagtgct ccctgaccct ctgcccctcc ctcctccacc ctggcctgct
61 ttagctttcc ccagacatgg ccaacaaggg tccttcctat ggcatgagcc gcgaagtgca
121 gtccaaaatc gagaagaagt atgacgagga gctggaggag cggctggtgg agtggatcat
181 agtgcagtgt ggccctgatg tgggccgccc agaccgtggg cgcttgggct tccaggtctg
241 gctgaagaat ggcgtggtga gtggcaccct gggctagggc gctggggggc tggggtgtga
301 ccccctgtga gtcctgggcc aatccctgag gactgctaag ctgcgtccta tgccctatgc
361 ctggtagatt ctgagcaagc tggtgaacag cctgtaccct gatggctcca agccggtgaa
421 ggtgcccgag aacccaccct ccatggtctt caagcagatg gagcaggtgg ctcagttcct
481 gaaggcggct gaggactatg gggtcatcaa gactgacatg ttccagactg ttgacctctt
541 tgaaggtaga gaggagaatg ctgggggagg aggtgggcag gaggacaggg tgctgggaca
601 gggagagggt atgaccaaat atgccacaac taggggtgtg ctcgcccgca cacagcaggg
661 atgggatatg ccgagaataa cacgccacgc tcacagggcc cactgagagg cctcccttga
721 attggggaca actcttggcc ctggtttggc catttttttg tgagagacgg gggcaggccc
781 tggcttggag tcttgtttat acgttcttga tgttcatctc ctctctcctg tcttctcaca
841 ggcaaagaca tggcagcagt gcagaggacc ctgatggctt tgggcagctt ggcagtgacc
901 aagaatgatg ggcactaccg tggagatccc aactggttta tgaagtatgt ggcccccagg
961 gagcttgagt ctccgcatgg ggtgggaggt ggcttgttct aaggagcttg cgggaaggat
1021 taggggaagc agatagccaa gaaaggataa agtgagggtc tgggatgggg aataatgggt
1081 ccttaatact ccttgacccc tccctttcca ccctcctgcg ctcagtctcc ctagcctatg
1141 aggcaagcta gattagggaa aaaaagtgca acaggaaggc aatgggattg ggctaggacg
1201 taacagaggg atcagaaaac gggtggaaaa cacacagttc taccaagtct ttatcctgct
1261 tcctcctctt ctaggaaagc gcaggagcat aagagggaat tcacagagag ccagctgcag
1321 gagggaaagc atgtcattgg ccttcagatg ggcagcaaca gaggggcctc ccaggccggc
1381 atgacaggct acggacgacc tcggcagatc atcagttaga gcggagaggg ctagccctga
1441 gcccggccct cccccagctc cttggctgca gccatcccgc ttagcctgcc tcacccacac
1501 ccgtgtggta ccttcagccc tggccaagct ttgaggctct gtcactgagc aatggtaact
1561 gcacctgggc agctcctccc tgtgccccca gcctcagccc aacttcttac ccgaaagcat
 
1621 cactgccttg gcccctccct cccggctgcc cccatcacct ctactgtctc ctccctgggc
1681 taagcagggg agaagcgggc tgggggtagc ctggatgtgg gccaagtcca ctgtcctcct
1741 tggcggcaaa agcccattga agaagaacca gcccagcctg ccccctatct tgtcctggaa
1801 tatttttggg gttggaactc tc
SEQ ID NO. 40
   Q14103 - human
   Heterogeneous nuclear ribonucleoprotein 
mseeqfggdg aaaaataavg gsageqegam vaatqgaaaa agsgagtggg tasggteggs
aesegakida skneedeghs nssprhseaa taqreewkmf igglswdttk kdlkdyfskf
gevvdctlkl dpitgrsrgf gfvlfkeses vdkvmdqkeh klngkvidpk rakamktkep
vkkifvggls pdtpeekire yfggfgeves ielpmdnktn krrgfcfitf keeepvkkim
ekkyhnvgls kceikvamsk eqyqqqqqwg srggfagrar grgggpsqnw nqgysnywnq
gygnygynsq gyggyggydy tgynnyygyg dysnqqsgyg kvsrrgghqn sykpy
SEQ ID NO. 41
   AF026126
   Homo sapiens heterogeneous nuclear ribonucleoprotein D (HNRPD) gene, complete cds 
1 tcgcagaggt gcagccacac cccggcctaa cgtgttgttc cccccgatac tggagtggtg
61 gggagggtga gtggactcca ggaatcctcg gaagggcggg ggcggaggca gggggcccct
121 ctagccgcta cttcgaaaca gcattccttg ttctcgatgg tccccgcgcg actgtcttag
181 ctcacgacac ttccggttcc ttttaaaggc ccccaaggct gtgcaacgcg gagcgtgaga
241 ggaaggtata aagggtagcg agagggcggg accgaggagg aaagggaaaa aaaaaaaact
301 agggggatag gggtgggggg acgcgcgaag ggcgcgctct cgcgtcacgt gaccgggacg
361 cgccgttctt ccgtcggcca ttttaggtgg tccgcggcgg cgccattaaa gcgaggagga
421 ggcgagagcg gccgccgctg gtgcttattc ttttttagtg cagcgggaga gagcgggagt
481 gtgcgccgcg cgagagtggg aggcgaaggg ggcaggccag ggagaggcgc aggagccttt
541 gcagccacgc gcgcgccttc cctgtcttgt gtgcttcgcg aggtagagcg ggcgcgcggc
601 agcgcgggga ttactttgct gctagtttcg gttcgcggca ggcgggtgta gtctcggcgg
661 cagcggcgga gacactagca ctatgtcgga ggagcagttc ggcggggacg gggcggcggc
721 agcggcaacg gcggcggtag gcggctcggc gggcgagcag gagggagcca tggtggcggc
781 gacacagggg gcagcggcgg cggcgggaag cggagccggg accgggggcg gaaccgcgtc
841 tggaggcacc gaagggggca gcgccgagtc ggagggggcg aagattgacg ccagtaagaa
901 cgaggaggat gaagggtgag taaggggcat cccaggatag tcaggcccaa ctagtccccc
961 tccccctctt tattcccccg ccattagcgc tggttcccgc tctcagcccg ttctccgggc
1021 cccatgcagc ctccttgcac tggtctccct ttttctatgt tgggttcccc ggaccttcat
1081 ttccttcttc ctttgcctgc ttatcgcccc cctcccccat cacacacgtt tccaccttta
1141 gccgtcacca tgctgctcct cggcccgccc tcctttcctc cctcgccatg ggtctcctcc
1201 caccgactta gccgccagat tttttcccgc ctgtcgtggg gtaccttttt tctttccatg
1261 ctgtcccctc tttttccctt tttctacagt cttgggcata aaaacacaga cacaaacagc
1321 ttctctgttt gtattactaa ggtttatttg gtgcttctcc accatcctga aacgatgcga
1381 ttgtttataa gcacatgttt tggggaacgc gtaggctgtc cacctctgcc tctcccttgt
1441 cggccttacg ccgactgttt tcttgggatt ttgaataagt ttcgcctaag gatttactca
1501 ttttctccac catacgctat cgcacaatgg catactcata caggccctac attttgacat
1561 gcagaccaaa ttgggtctgg tgaaatgctc cgagtttctt gttggtacat tggttttgct
1621 ccgcgggctc tggttaagtt cttagtcgat cgggcctgca cttgactgga gctgttccta
1681 tctccggcct agcatctacc cttcccccac cccactgagt tattctaacc gcgcaccctt
1741 ttcgcgccct cagctattgg gttccccaac tgttagtaca gattgtacct tactttttat
 
1801 gtgcaaccta atttttaca accccagcc ccctttttt cccggtgccc aggatcctat
1861 tttggtgtct tgatatctgt ttcccgccca ctaagggagg cctgtagtcc tcttaagaga
1921 aacaaatcac tgtattgtgc tatgggatac tttttttttc tttttggtgc aaatttctta
1981 gtattaactt gcttcctcca attgaaataa cagttgtata tactacctaa atctgcattt
2041 agtgtattaa aatgcagcat ttgggattgg gaacataata cgggagttag caggagggac
2101 taaatcaggt ttcttggttg agttttctta gctccgatta ctgaccgatg atcgtgggtt
2161 cacgcttcca ttcctttttg taatggggag gggtgtgtgt gtgtctgaat tttttttaa
2221 ctgtaaagag aaaggtgttg gtggatataa caagatgtcg ctttaatcta gtattagaaa
2281 acacgatttc ttttactgag aaagagccca ggatttggag ggaaagttgg gaggggagaa
2341 gcactttgga atatagggtt tattaagccc agcttaggca gattcttgtc aggctgtttg
2401 atgatagtgc tggttttggg ggagtggtgg tgggaaggga agcaaacttt taaaaattaa
2461 acagaaccaa ctcgaattgt atatagctta tttgagaaag gaagatgtta gatatttgga
2521 aacttaaaac ctttaacatt ttggttttcg tttgctgaat tctgttcttt ggacagaggc
2581 accaaaatga ttaattgtat aacttcctgt ttggggcagg ctttctgtta gccctgataa
2641 ttcaaatcgc aaagcagctg actttatagt tacctactgt tgaagtgtaa atgaattaaa
2701 gtttttaacc ctaacagtgt caaaaactaa aactagaatt ttgattggtt gctgtaccgg
2761 ttgttaattc cctagccatt caaactcctc cccacgacac tctgaagcag cgacggcaca
2821 gcgggaagaa tggtaaatct tttaaatttt atttctgtat tataaaggtt tcagtagtca
2881 taatatgtag aggctgtgta ttggttagtt gcccattgat ccccaggaaa ttctagacca
2941 tagtacatac agtaggtttg tacctgggat atataccttt tataggcatc atgtgtaatc
3001 tttgggcaaa attgatgccg ttacatggta tttggtctgt ggtaatgcat gtagttgcag
3061 tttggcaatt taagctaaac aattagtaac gatattttta tatcgagcaa gaaaaaatat
3121 taccgtaatc ttcacatttg atttatttta agatgaaaaa tatttttgtt ggaatttaag
3181 tttaatggct aaatatgaag actccttgat ataaaatatt gtgtttaaaa tgtcatgcat
3241 gttatactga aatattctgg agatgtctga agcagttctt taagcagcac tgtgtattat
3301 ggaaaaatgt ttgtctttta cctagcatat ttgtaaatag acctttaata aaatgtgtgt
3361 gtgtgtgtgt gtgtgtgtgt gtgtatatat atataccatt aaaaggtggg ctaatgtggc
3421 tttccactac agctcttacc agatttttta ttttcagtgt gatttggctg gaatattaat
3481 aacagttggt tgacatgtat tcacaattgc atgacttctc tttggaagct ggtaggttga
3541 gtattgttct ttttatccat cctttcccct cccctaaatg tcagtctttc tgcttttagg
3601 attatatttg aattttcagg ggtttataca ccaccactgg agagtagaag gccactaact
3661 tgcaggcagt taacttatat ctttacaagc tacattctat tttaagagcc tatttagggt
3721 aacatttaaa gatttgctta tcactgtatt ctcatccact gaaatcagtc cagctttacc
3781 ttggagaaaa agcagatggg aaacaggcaa ggggaaaagc aaaaaagagg aaaaaaaaag
3841 actggggtca cagtttcgat gaatgttatt ccctgtattt ggtgagtggt gggcactagc
3901 agctcaattt cactgctagc taatagtggc aaattaagat taaactataa actatttgac
3961 attactccat tatgtatttt gagtctcatg taattgtttc cagtgatttg aaatgaccat
4021 aaacttaaat ttgaaggtca gtgttttgac tcttcgaagt aattacactt gacctgctac
4081 ctaaaacgat gtaatattac aacttttttg aataatctca ggaaaaatgg agaaatgttt
4141 atattcatag ttaataaaca ttctagatag taaccaactg tcatttactg aaaataaaat
4201 tttacccagg tttattttat ttttgagacg gagtttagct tttgttgccc aggctggagt
4261 gcagtggtgt gatcttggct cagcgcaacc tccacctccc aggttcaagc aattctcctg
4321 cctcagcctc caaagtagtt gggattacag gcatattcca ccatgcctgg ctaattttgt
4381 atttttagta gagacagggt ttcttcatgt tggtcaggct ggtctcgaac tcccgacctc
4441 aggtgatccg cctgcctcgg cctccccaag tgctgtaatc acaggcgtga gccactgcgc
4501 ccggcctagc caggttgatt ttaaaattac acttaaaaat aatgttctca tttttaaggg
4561 ctctaatatt tgacttttct taactttcca attatgcagc cctatcctgt tccagacgtt
4621 aaggctttct ttttgaatga aaaactttca gacttttttc tctctctttt tttttttttt
4681 tggtgtctgc actccccacc gtatctgtcc cttcctcttt ctcccatttt gagggatatt
4741 ataagggcaa cagttttata gtctcaatat tgagggtaag actggttaat agatagagta
4801 tccaaattga atttattaaa cacagttctg tgtgcatttc cttctgcaaa tctgagaata
4861 aagtgattac agtttcatcc taaaatactt taaaaatcag ttggttttag aaataggttt
4921 ttttcctttt gcatgtaaga atggacagct atttttagga aggcatgcac ctgttatccc
4981 agctactcag gaggctgagg caggagaatt gcttgaaccc gggaggtgga ggttgcagtg
5041 agcctagatc atgccattgc actccagccc gggcgacaga gtgagactct gtttaaaaag
 
5101 aaaaaaataa gtatttgtaa atgtatatat atatatatat acacacacac acacacatat
5161 atatataaaa tcattatgcc atcagtttga gggagagcat gtatgccaac attgtaaaga
5221 ctaagtgccc atttatccat aattaagata agcagacttt tccccattgt actataaagt
5281 tagagtttgg ggctgggtat ggtacttccg cctgtaatcc cagcagtttg ggaggctgga
5341 gcgggcggat cacttgaggc caggagttca agaccagcct ggccaacgag gggaaacccc
5401 gtctctacta aaaatgcaaa aatcaagtca ggcgtggtgg tgcagcctgt aatccctccc
5461 acggaggctg aggaatgaga atcactttga acctgggagg cagaggttgc agtgagctga
5521 gattgtgcca ctgcgctcca gcttgggcga cagagtgaga ctgtctcaaa aactaagcaa
5581 ccagaaacag aaaaagatgg aatggaatta atatgttcca tttagtaaag atggaataga
5641 attggtccag tttgtgttca ttgtagcata ttcacctatt tggtctgcaa ccattttttt
5701 ttttttaaag cctaagcata ccctgagaac aacaaccttt tttgattaga acagaagaaa
5761 aagccagaat gttcttgggc tgccaaaata ttaatacgtt tgtctcaaag acgaaccact
5821 taggtgcttg cttagagact tctactttac acagaatttg aaaagtttga cagctggcta
5881 cttactctgc aaaacggaag ggagactttt tagaacagca actggtgatc tggcaaaaat
5941 gaaaagtaga acttgtgaag aaatgagatg gacttggtgt attaatagct aattttagaa
6001 gaccctgtgg atattctaaa tcagcataat agtgatgtct gagccatagt gttaaattat
6061 gattactgta tttgaagtat aagaaggcag aaaagtgtga tgctgttaga aaaaaatctc
6121 atttcaaatg attgaaggtt aaaaacaatt ggggaaagat tagagagggc aggcggtgct
6181 ttaagggagg tggaataata cttggctcct tattctccaa aagtgctgga tagctgaagt
6241 ttttaatatt ctggataatt gatatttgat tattagttta ggagggatag catcttcagt
6301 gaacccttgt agctctaagg taggcatatt aaacaactac catactaaag gtggggagta
6361 cgtttggaga gcactttgct gggccagctg atgatatgct taggtgatat ttaagaaaat
6421 ctgcttcttg tctgaaaaac atttggggct tcagaataat accacatacc tcattcttgg
6481 tgttttaatt tatttctttg taattgtttt ctgtttctta ataattttaa attaagggta
6541 ttttcccaat ataaacattg ctttttggtt aaataaaatc aaaactagtt ctgtctccat
6601 atctgaatag accagacaga gaattttctt gcctttcaac agcttaaaaa atgtttttgt
6661 tagaactgtt gtgttcaggc tgaaatactt tcaaagtttg ttagttatta ttgagaaatt
6721 tctgtaataa tcatggaaag ggtaatttaa tagttaaatc tcaacttaat tgaagttatt
6781 tctgttgtgg aacttatggt catcttagca agaggtcatt gctttatagt cagttctctt
6841 tctcattaaa aatatagtgt actcaccttt acagggtgaa tgttggtaaa taacttctgt
6901 ctgtgaagga agtattctgg acctgtaagt taaaaataag gtgtttatag caaattatgt
6961 aaataaagat tgtatattag aaggtacaca ctattcaaat ttaaagaaaa tgtatattga
7021 gaaaataact caaattcttc catgaaattg gcaagaagta aacatttcaa atacacaaaa
7081 cattatgggt atttttgttc atttgattat aaaatgccaa agttgtttta taaatgctac
7141 ttcaagcctg gaaactttga ggaagtcctg aacattaagc atataaatgg cccagctcta
7201 gaatacatgt aagttgaaaa gctaacctga agtgggaagc gcagtatata cctaagactt
7261 actctgcact gaaagtttgc tttgtcacta gaagtaaaac aagactgtgg taggatagta
7321 agatcagtaa cacctcagtt aatcaggtat cttggaggaa gtgaagaaag accctaattc
7381 aagggacagt taattggcct tttattccaa gaatgggcct tagtggcagt atcttaaaag
7441 cccacaagat ggagatgttt cctaatgaaa ggcctttaat ttctttatag agctgagtta
7501 gtgtcacatc ccagtcccca cccatgaccc ttccccagtt aaaaagaaga gaaaatgttg
7561 agcaagtctg atttgattcc catggtgaca tttttagcca ttatgtaaca aattctgaca
7621 gtttaccctt aaaattaaaa acctccagtc ctgtcttttt aaagggtaga aagaaggtta
7681 ggtataggat agctttttat ttatttattt atttattttt gtacaaaggg agcctatgta
7741 aagctgccag atctgaactt tctggtgttt tgctgtaatg ttagtaagat ttcgccttaa
7801 aatattttat tttgagtata tacgtttggt cttagagtgt cttggtggat ttccgcttac
7861 cacccatcac tttctgcatt ttaaaggctt aaatacttta ttgctggtta actgaggttc
7921 tactgtaaac gaatcatcta agttaattag tggattgtac ttcaatggat aattttcact
7981 aaattgttta tattgcacat tacttttgtc ttaaggactc ttagcacatc aaaaaaattg
8041 gctcacaact taattgtgag atgtagaatt ttccatttta tgtgctaaga gttttgttaa
8101 tgagagaact gttaaaatag aaaaggagct tcagcataga caccaatgct ggttgctgag
8161 atcagtgggg aactgcatag cattttaaca agtttaatga acatttggaa gagaaatttg
8221 aagctaagat tctggttttt ttgctgttaa ctttttaatt ttttaatcta aggaaaactt
8281 ttatgtacag tatttttact ttgggtatat gtttatcttt tagcaagttg aaagacttaa
8341 tttgctgctt gctcactatt ttgtaattat ttgggagcag cagtaataag ccagcttttt
 
8401 ggaataggat gttcctgatg tgtggttatg taggaagaat gatgttttaa tatactgccc
8461 agtaaactgg tgcagtttgg aaaaggtgtg ttattgatgt ggataatatt taaggcaatt
8521 ttttttaagc attttaatac tgctttttgt ctttacagga aaatgtttat aggaggcctt
8581 agctgggaca ctacaaagaa agatcctaag gaaaactttt atgtacagta tttttacttt
8641 gggtatatgt ttatctttta gcaagttgaa agacttaatt tgctgcttgc tcactatttt
8701 gtaattattt gggagcagca gtaataagcc agctttttgg aataggatgt tcctgatgtg
8761 tggttatgta ggaagaatga tgttttaata tactgcccag taaactggtg cagtttggaa
8821 aaggtgtgtt attgatgtgg ataatattta aggcaatttt ttttaagcat tttaatactg
8881 ctttttgtct ttacaggaaa atgtttatag gaggccttag ctgggacact acaaagaaag
8941 atctgaagga ctacttttcc aaatttggtg aagttgtaga ctgcactctg aagttagatc
9001 ctatcacagg gcgatcaagg ggttttggct ttgtgctatt taaagaatcg gagagtgtag
9061 ataaggtagt gtgttacgtg ttctgatcag ttaataatat aaaatattaa catatggata
9121 gtttgataca atgagtttgc ctatttgtgg ttccccattt tgatagtata ggaaggaaga
9181 atagttcttg ccccaatacg ttttatgaag atagaggtag gttcaggaat tatttcctga
9241 ataatttgtg ttccaggctc tgctaaattt tgaaattaac tttaaagata ctatagactt
9301 aaagatgcct agttaaaagg atgtgtttta gcaattcaca gaagtccata ttttgaaatt
9361 ttgttaggca agcaactttt aactgaatca ttattttgat cctgggctaa agggaagtag
9421 cagttatgtt tgtatatagt gctaaaggga agtagcagtc atgtttgtat atattgaagg
9481 taatggttat ctagtaattg gttaaatttg tgtatgtcct accattctta cctttagatt
9541 taaacagtat atgttagttg atgttacatc accaccatga cttgacagtt taatcttgag
9601 caagtcaaca tatgcttggc attatctgta tttatagtta tttttaagta agttaatagt
9661 ctctgaactt cagttaagat atatattttt taaatgaaaa cttcaatttc cttaggtcat
9721 ggatcaaaaa gaacataaat tgaatgggaa ggtgattgat cctaaaaggg ccaaagccat
9781 gaaaacaaaa gagccggtta aaaaaatttt tgttggtggc ctttctccag atacacctga
9841 agagaaaata agggagtact ttggtggttt tggtgaggta tgttataaat gttttgaccc
9901 agtttatgtc aaaattagtg tgaatgtgat tgtcccatta tggactcaga gtcacttggc
9961 ttttcaaagc tgttagggta gattatgtga tctgttttgg aataaggata ttgtaaatac
10021 ttcattagca ggtctttgaa ggttggataa tgtgtttttc tcattgagca cctactctat
10081 gcagagtatt gctggggtag agtataccaa agatgaaata gacaaacatt cttaaataca
10141 gacaattaag aggaagaaat ctagattaga aggagacttt tgttgaaaat aggaaaggaa
10201 ttaagaatag ggtgtagtgc cttatcagtt gaaatgcatg tgtaagtgca aatttaatga
10261 gactaatcat tatagactca ttagtgaggc tggacgcgat ggctcatgct gtagtaatcc
10321 cagcacttgg gaggccgaag tgggtggatt actggagacc aggagatgaa gaccatcctg
10381 gacaacatag tggacccgtc tcaattaaaa gtaaaacaaa cttagactca ttagtgaaat
10441 aggtagataa tagaggtttc ttttatgaat taatgaatta atgaaagttg agaaatttgt
10501 ggccttgggc tccagatacg tcccacagac atttattgct tgattgaaac aataagtgct
10561 tttttagtgt tgaggatttg agatattgcc cacaaaactc agtatctagc tttttaaaaa
10621 atatttgcaa gagcacgcaa catgggaact gacgctgccc tcctgtacgg cagcatctct
10681 caaactgagt agtagcttcc atcttggttt gggcatatgc tctccagttt ttagtagtcc
10741 tcaccaccct acttcctgtt ttctctcaaa tacatttttt ttctgttttt cttagatttg
10801 actgttttct tcttgttctt tgtgggcatt tgaatttgtg acccttgagt taggtagtaa
10861 atgtcagtgc gtggtaaagc ttatttttgt aaatagttgt gaagacctta gatggaatgg
10921 gtgttctaat ttgaagaatt ccttaaaagg attagaataa atagggagaa acaggagact
10981 agaacttcag tgccaaatac atgttttctt tgtgtgtttt cccccctcta aacttgtgtt
11041 tcttttaagg tcaataaaat gcatgttagc atattaaaat ttgtttttta ataccaggtg
11101 gaatccatag agctccccat ggacaacaag accaataaga ggcgtgggtt ctgctttatt
11161 acctttaagg aagaagaacc agtgaagaag ataatggaaa agaaatacca caatgttggt
11221 cttagtaaag taagttaagc atccatttac ttgtagagaa aactagctgt tgtaaagagc
11281 ttaaccattt atctttctct gtaaaggctt aagttctttg catgctttaa aaacttctca
11341 ttggttactt accattgacc aactttttgt ggggagcagg atggacacat ttgttagtgt
11401 ttttgtctag gctttcacaa aaatagtttt tagaacttga caagtaaaat gaagtaacat
11461 cactagcaag tactcataag tgattactct taagtactaa atattggttt aattaataaa
11521 ctgactgagg aaagtttcaa attagcctac tctatttaaa catgttggct attctggtta
11581 ttagaaactt atttagcaac ttttattttc ttgagtcagt ttaataatgt aatttttctc
11641 ttttagtgtg aaataaaagt agccatgtcg aaggaacaat atcagcaaca gcaacagtgg
 
11701 ggatctagag gaggatttgc aggaagagct cgtggaagag gtggtggtaa gctagagcct
11761 aagtttactc tatcttaagc ttttctgctt tttaattatc ctgaagtaaa gatctttgct
11821 gatcttctga ctttagtgaa cctattaatg tgctgcaggc cccagtcaaa actggaacca
11881 gggatatagt aactattgga atcaaggcta tggcaactat ggatataaca gccaaggtta
11941 cggtggttat ggaggatatg actacactgg ttacaacaac tactatggat atggtgatta
12001 tagcagtaag tactatactt tttatattaa ctgctatttg acatttattt tgtacaaatt
12061 tggataggca gaaaggttag tgtagccttg ccaagtgcaa atgtcttcag gtttcaaatt
12121 cctggaaact tgaaactgca gccattttat tgcttggttc ctcccagcct atatcacaca
12181 cacattataa gggtagggtg tatgtgtggt tctatatatg tttgctgggc atttgtttta
12241 cttggattta aaaattttaa gctcagttca gatcttttaa gctcaaggta ttctaaatgt
12301 cacttcttgg accaaccaat aatcttggca tgatgtgtct taatcctata aaactgaata
12361 ataccacatg ttgccagtta aaactaatag tatcctcgct ttaggattat taatgtagaa
12421 actcttaaaa cagatgttga gcttgataga accaaaaaac ttgactttta gacatggaaa
12481 gccctgactt cattgtgcaa ctaaggtagt tgctctccac ctgatttgta gcaactgttg
12541 agtcgtcagg taaagggttc tactagaagc aatcttacat tttttggag gagagtggtt
12601 gcattggttg cattgtttta agtggttttt cttttccttc cttggttaga ccagttcttg
12661 gagttatatc ctttcttagg tgactaggcc tgctgcacaa taataggtta attaaagtca
12721 gaagaaggtc agcaaagatg gattgggtga gattggggcc cttttcttag aagggcagag
12781 atactaagca ctgattgtgg ttgacaattt gttctaaatt ttaagatatt ttttgctggt
12841 ggttgtgaaa gggtcagctg tccatccttt gaaacttaaa acttttaaac tgtaaggtg
12901 aggggattgt ctcccatttt atacaataag tcaagtaatc agctcattct gaatgcctgc
12961 cattgtatgc attcactaca tatttggtaa attatttgat aaatgattgc tcagggtgaa
13021 tttttcacac ttgggaatta agctaccctt aatttttga gattgtttaa aattaggtac
13081 tgttctgatt attagtatgt aaccactacc gttctggttc taacacttgt tttattttag
13141 accagcagag tggttatggg aaggtatcca ggcgaggtgg tcatcaaaat agctacaaac
13201 catactaaat tattccattt gcaacttatc cccaacaggt atgttctaaa aatagttttt
13261 ttttgtcatt tacaatagta gtttttataa tctatattgt tcataaaaca atgcttaatt
13321 taagagtttc acagcaccca gaagtgctta ccatattata acatagtgac tttcaaaaga
13381 tatgtaacac aggtgctctt aagcttttgc ctttttgtcc tattattaac aagtcagtaa
13441 agttaacagg taaagtactg ctaatgggta caaattaagg aattgcagca aaaaagtatt
13501 gcctactaac tctgacatta taccttgttt gtaccgccag cgggaacttc attgcaggcc
13561 ctgtgtcgcg ctgacttcag attctcacag gcccgctcaa tgcggacagg gtaacgagat
13621 gctccacgct ctcgaatgct gccgtttggt atggtctctt ccaacatcct gtatcagcat
13681 tataaaataa aatggatact tcaagctttg ccttcactta tttctttgct tttaaaaac
13741 tatttgtaat gtaattttaa tgcatttttt acaggcccag taatggttaa atacgtcagc
13801 ttactgaata attttaacta tttattcttc taaggataca gcttgtctct ggattttcca
13861 gtcttaattt tatattttat taatctattt taatgcttgc ttttcccatt tatagacgtt
13921 gtagcagtaa ttgcaagaag ttcttgagct gaattcctgt tgtgacaact tcctataatt
13981 acagtagata actttttctt ttagtcgtat ataacttttc tataacttgt gatggacaag
14041 agatatgctt atccaataaa ataagcttaa atattagatg ctcttgggtc aaaatgtcct
14101 tttaccaaat tgaccttttt atgagttctt tgggtaaata ctttaaagct tttatattt
14161 taaagaatac ttgtaaaagc atatcacatc ttaaaccagt ggtgcacatg tggatttaca
14221 gctcatggac tctactgttc agctttaatt tataaaacat atcacacatt taatgttata
14281 cagtatttac atatagtgga acatagggat aactcagttt tatgtaaatt tttgttaagt
14341 gttgtagcct gcccagagtg acttctattt tttcttcttt gtctccaggt ggtgaagcag
14401 tattttccaa tttgaagatt catttgaagg tggctcctgc cacctgctaa tagcagttca
14461 aactaaattt tttgtatcaa gtccctgaat ggaagtatga cgttgggtcc ctctgaagtt
14521 taattctgag ttctcattaa aagaaatttg ctttcattgt tttatttctt aattgctatg
14581 cttcagaatc aatttgtgtt ttatgccctt tcccccagta ttgtagagca agtcttgtgt
14641 taaaagccca gtgtgacagt gtcatgatgt agtagtgtct tactggtttt ttaataaatc
14701 cttttgtata aaaatgtatt ggctctttta tcatcagaat aggaaaaaat tgtcatggat
14761 tcaagttatt aaaagcataa gtttggaaga caggcttgcc gaaattgagg acatgattaa
14821 aattgcagtg aagtttgaaa tgtttttagc aaaatctaat ttttgccata atgtgtcctc
14881 cctgtccaaa ttgggaatga cttaatgtca atttgtttgt tggttgtttt aataatactt
14941 ccttatgtag ccattaagat ttatatgaat attttcccca atg
SEQ ID NO. 42
   NP_852000
   GSK-3 Binding Protein - FRAT1 
mpcrreeeee ageeaegeee eedsflllqq svalgssgev drlvaqiget lqldaaqhsp
aspcgppgap lrapgplaaa vpadkarspa vplllppala etvgpappgv lrcalgdrgr
vrgraapycv aelatgpsal splppqadld gppgagkqgi pqplsgpcrr gwlrgaaasr
rlqqrrgsqp etrtgdddph rllqqlvlsg nlikeavrrl hsrrlqlrak lpqrpllgpl
sapvheppsp rspraacsdp gasgraqlrt gdgvlvpgs
SEQ ID NO. 43
   NM_005479
   Homo sapiens frequently rearranged in advanced T-cell lymphomas FRAT1), transcript variant 1, mRNA 
1 ggattccggc tcccgcggct gcaggcgcgc ggctagagtg cctggcgggc tccggcttcc
61 gcgtccgccc cggccccggt ccagacttag tcttcagctc cgcgcccgct ccgccgcggc
121 ccaccgcgcc cgccggcagc cgagccccca gcgacgcccg cacagctccg ggtgcccaga
181 cagggggcca tgccgtgccg gagggaggag gaagaggaag ccggcgagga ggcggagggg
241 gaggaagagg aggaggacag cttcctccta ctgcagcagt cagtggcgct gggcagctcg
301 ggcgaggtgg accggctggt ggcccagatc ggcgagacgc tgcagctgga cgcggcgcag
361 cacagcccgg cctcgccgtg cgggcccccg ggggcgccgc tgcgggcccc gggccccctg
421 gctgcggcgg tgccggcgga caaggccagg tccccggcgg tgccgctgct gctgccgccc
481 gcgttggcgg agactgtggg cccggcgccc cctggggtcc tgcgctgcgc cctgggggac
541 cgcggccgcg tgcggggccg cgctgcgccc tactgcgtgg ccgagctcgc cacaggcccc
601 agcgcgctgt ccccactgcc ccctcaggcc gaccttgatg ggcctccggg agctggcaag
661 cagggcatcc cgcagccgct gtcgggtccg tgccggcgag gatggctccg ggcgccgcc
721 gcctcccgcc gcctgcagca gcgacgcggg tcccaaccag aaacccgcac aggcgacgac
781 gacccgcacc ggcttctgca gcagctagtg ctctctggaa acctcatcaa ggaggccgtg
841 cgaaggcttc attcgcgacg gctgcagtta cgtgcaaagc ttccccaacg cccgctcctg
901 ggacctctgt cggccccggt gcatgaaccc ccttcgcctc gcagccctcg cgcggcctgc
961 agtgaccctg gcgcctccgg gagggcgcag ctcagaactg gcgacggcgt tcttgtgcct
1021 ggcagctaac acgcccgggg tggccacagc gccagcctca gactggaggg caaggggttc
1081 ccttgagggc tgcagttcta ctcaggctgg tggagaactc tggcttttgg aagcgagagt
1141 aaaaagctaa tgacgaggaa ccgaaaaatc gcgagtgttt cgcgggtaac tggggttgag
1201 ggccaaaata tttggaatga aggacttggc cctatttaag gcagatttta cagagcgcac
1261 ctcaaacgta caagtcagta ggactcctta tttggcgtga cccgacctgg ccgcggagcc
1321 tgcatttcct cgcagcctct cagtgccctc cagccccgcg accatgtggc cacaatccac
1381 gcttctccgg atcgcggtgc gccggaacca cggaggatga tgccagttac ttgctttacc
1441 ttttcagggc tggctcctga tccactttgg gggaggagaa catgagtaga taatttcagg
1501 gtgcagccca atctgccaga cttaaaaaaa ccatcttgtg tctttggagg tgctgcttaa
1561 taccaaacat gcggtgccat gaagggaccc tttgggggtt gaataggagt taacccctgc
1621 gctctctttg caactgtctc tcttctcaga gtggtggggg aaggctgtac gacacgggtg
1681 gggaaaggag gtgggggcgg ggagtattga atggtggtgg aagggtagag aggcgcggag
1741 tgaaccccac gccctgtcta aagtgtattt tcagagccgg cccgcctctc ctcggttcaa
1801 ggtcactgtt tcctgggcac gcactgggtt gcgggacaga gtagccaggt tctgccggtg
1861 ctcggagaag agcgcagtgt tttgcaagtg ctggagtctc ctgaggacac gcgcgtcgcc
1921 gccaccgcgg gtgtgggaaa gcgcggacgt gctgggcggc tgtgcttcgg taggcgacca
1981 ccgcccctgg ccgcgctccg ggctttcacg gaaactcccg agaccggccc ctgggttcct
2041 cctctcctac tcggctctgc agtcctactc aagcgggtgg ctctgggatc ctgggggcct
 
2101 gggttggggg ctagggagac gccatgtgat ggacactcca gggacacaca gcctagcaca
2161 gcagcttata atgggctctc cggggccatt tgcaataaca gctgcaattc cctggataga
2221 cgagttgatt tcctccctct gcccctcccc cagccatgcc agctggcctt tgtaagtgca
2281 ggaaaccgag tagaaaatgt gaccctccaa atggagaagc tgcaggcttt gccattgtga
2341 accatggtga agtgcttgga acatactgtt cactcactct aaaggcgctg agactgtgct
2401 gttgttctcg tttttatagt caatggcttg ttcatcatcc agatgtggct actgacatat
2461 ctacacttcg caccggagtg tctggaattg tggctatcct gattatagga ttttaactta
2521 actgaaatgc ctgctttgaa taaatgtgtt gggttttttg tttggtttta ttttatactt
2581 gccatcagtg aaaaagatgt acagaacaca tttctctgat ctccataaac atgaaaacac
2641 ttgaaatctc aaa
SEQ ID NO. 44
   NM_181355
   Homo sapiens frequently rearranged in advanced T-cell lymphomas FRAT1), transcript variant 2, mRNA 
1 ggattccggc tcccgcggct gcaggcgcgc ggctagagtg cctggcgggc tccggcttcc
61 gcgtccgccc cggccccggt ccagacttag tcttcagctc cgcgcccgct ccgccgcggc
121 ccaccgcgcc cgccggcagc cgagccccca gcgacgcccg cacagctccg ggtgcccaga
181 cagggggcca tgccgtgccg gagggaggag gaagaggaag ccggcgagga ggcggagggg
241 gaggaagagg aggaggacag cttcctccta ctgcagcagt cagtggcgct gggcagctcg
301 ggcgaggtgg accggctggt ggcccagatc ggcgagacgc tgcagctgga cgcggcgcag
361 cacagcccgg cctcgccgtg cgggcccccg ggggcgccgc tgcgggcccc ggggcccctg
421 gctgcggcgg tgccggcgga caaggccagg tccccggcgg tgccgctgct gctgccgccc
481 gcgttggcgg agactgtggg cccggcgccc cctggggtcc tgcgctgcgc cctgggggac
541 cgcggccgcg tgcggggccg cgctgcgccc tactgcgtgg ccgagctcgc cacaggcccc
601 agcgcgctgt ccccactgcc ccctcaggcc gaccttgatg ggcctccggg agctggcaag
661 cagggcatcc cgcagccgct gtcgggtccg tgccggcgag gatggctccg ggcgccgcc
721 gcctcccgcc gcctgcagca gcgacgcggg tcccaaccag aaacccgcac aggcgacgac
781 gacccgcacc ggcttctgca gcagctagtg ctctctggaa acctcatcaa ggaggccgtg
841 cgaaggcttc attcgcgacg gctgcagtta cgtgcaaagc ttccccaacg cccgctcctg
901 ggacctctgt cggccccggt gcatgaaccc ccttcgcctc gcagccctcg cgcggcctgc
961 agtgaccctg gcgcctccgg gagggcgcag ctcagaactg gcgacggcgt tcttgtgcct
1021 ggcagctaac acgcccgggg tggccacagc gccagcctca gactggaggg caaggggttc
1081 ccttgagggc tgcagttcta ctcaggctgg tggagaactc tggcttttgg aagcgagagt
1141 aaaaagctaa tgacgaggaa ccgaaaaatc gcgagtgttt cgcgggtaac tggggttgag
1201 ggccaaaata tttggaatga aggacttggc cctatttaag gcagatttta cagagcgcac
1261 ctcaaacgta caagtcagta ggactcctta tttggcgtga cccgacctgg ccgcggagcc
1321 tgcatttcct cgcagcctct cagtgccctc cagccccgcg accatgtggc cacaatccac
1381 gcttctccgg atcgcggtgc gccggaacca cggaggatga tgccagttac ttgctttacc
1441 ttttcagggc tggctcctga tccactttgg gggaggagaa catgagtaga taatttcagg
1501 gtgcagccca atctgccaga cttaaaaaaa ccatcttgtg tctttggagg tgctgcttaa
1561 taccaaacat gcggtgccat gaagggaccc tttgggggtt gaataggagt taacccctgc
1621 gctctctttg caactgtctc tcttctcaga gtggtggggg aaggctgtac gacacgggtg
1681 gggaaaggag gtgggggcgg ggagtattga atggtggtgg aagggtagag aggcgcggag
1741 tgaaccccac gccctgtcta aagtgtattt tcagagccgg cccgcctctc ctcggttcaa
1801 ggtcactgtt tcctgggcac gcactgggtt gcgggacaga gtagccaggt tctgccggtg
1861 ctcggagaag agcgcagtgt tttgcaagtg ctggagtctc ctgaggacac gcgcgtcgcc
1921 gccaccgcgg gtgtgggaaa gcgcggacgt gctgggcggc tgtgcttcgt caatggcttg
1981 ttcatcatcc agatgtggct actgacatat ctacacttcg caccggagtg tctggaattg
2041 tggctatcct gattatagga ttttaactta actgaaatgc ctgctttgaa taaatgtgtt
2101 gggttttttg tttggtttta ttttatactt gccatcagtg aaaaagatgt acagaacaca
2161 tttctctgat ctccataaac atgaaaacac ttgaaatctc aaa
SEQ ID NO. 45
   NP_444254
   myosin light chain isform kinase 2 
mgdvklvass hisktslsvd psrvdsmplt eapafilppr nlcikegata kfegrvrgyp
epqvtwhrng qpitsggrfl ldcgirgtfs lvihavheed rgkytceatn gsgarqvtve
ltvegsfakq lgqpvvsktl gdrfsasave trpsiwgecp pkfatklgrv vvkegqmgrf
sckitgrpqp qvtwlkgnvp lqpsarvsvs ekngmqvlei hgvnqddvgv ytclvvngsg
kasmsaelsi qgldsanrsf vretkatnsd vrkevtnvis kesklds1ea aaksknc ssp
qrggsppwaa nsqpqppres klesckdspr tapqtpvlqk tsssitlqaa rvqpeprapg
lgvlspsgee rkrpapprpa tfptrqpglg sqdvvskaan rripmegqrd safpkfeskp
qsqevkenqt vkfrceglav mevapsfssv lkdcaviegq dfvlqcsvrg tpvpritwll
ngqpiqyars tceagvaelh iqdalpedhg tytclaenal gqvscsawvt vhekkssrks
eyllpvapsk ptapiflqgl sdlkvmdgsq vtmtvqvsgn pppeviwlhn gneiqesedf
hfeqrgtqhs lciqevfped tgtytceawn sagevrtqav ltvqephdgt qpwfiskprs
vtaslgqsvl iscaiagdpf ptvhwlrdgk alckdtghfe vlqnedvftl vlkkvqpwha
gqyeillknr vgecscqvsl mlqnssaral prgrepasce dlcgggvgad gggsdrygsl
rpgwpargqg wleeedgedv rgvlkrrvet rqhteeairq qeveqldfrd llgkkvstkt
lseddlkeip aeqmdfranl qrqvkpktvs eeerkvhspq qvdfrsvlak kgtsktpvpe
kvpppkpatp dfrsvlggkk klpaengsss aetlnakave sskplsnaqp sgplkpvgna
kpaetlkpmg nakpaetlkp mgnakpdenl ksaskeelkk dvkndvnckr ghagttdnek
rsesqgtapa fkqklqdvhv aegkklllqc qvssdppati iwtlngktlk ttkfiilsqe
gslcsvsiek alpedrglyk cvakndagqa ecscqvtvdd apasentkap emksrrpkss
lppvlgtesd atvkkkpapk tppkaamppq iiqfpedqkv ragesvelfg kvtgtqpitc
twmkfrkqiq esehmkvens engskltila arqehcgcyt llvenklgsr qaqvnltvvd
kpdppagtpc asdirssslt lswygssydg gsavqsysie iwdsanktwk elatcrstsf
nvqdllpdhe ykfrvrainv ygtsepsqes elttvgekpe epkdevevsd ddekepevdy
rtvtinteqk vsdfydieer lgsgkfgqvf rlvekktrkv wagkffkays akekenirqe
isimnclhhp klvqcvdafe ekanivmvle ivsggelfer iidedfelte recikymrqi
segveyihkq givhldlkpe nimcvnktgt riklidfgla rrlenagslk vlfgtpefva
pevinyepig yatdmwsigv icyilvsgls pfmgdndnet lanvtsatwd fddeafdeis
ddakdfisnl lkkdmknrld ctqclqhpwl mkdtknmeak klskdrmkky marrkwqktg
navraigrls smamisglsg rksstgspts plnaeklese edvsqaflea vaeekphvkp
yfsktirdle vvegsaarfd ckiegypdpe vvwfkddqsi resrhfqidy dedgncslii
sdvcgdddak ytckavnslg eatctaeliv etmeegegeg eeeee
SEQ ID NO. 46
   AF069601
   Homo sapiens myosin light chain kinase isoform 2 (MLCK) mRNA, complete cds 
ccggctgcct ctgctgcagt tcagagcaac ttcaggagct tcccagccga gagcttcagg
acgcctttcc tgtcccactg gcccagttgc cacaacaaac aacagagaag acggtgacca
tgggggatgt gaagctggtt gcctcgtcac acatttccaa aacctccctc agtgtggatc
cctcaagagt tgactccatg cccctgacag aggcccctgc tttcattttg ccccctcgga
acctctgcat caaagaagga gccaccgcca agttcgaagg gcgggtccgg ggttacccag
agccccaggt gacatggcac agaaacgggc aacccatcac cagcgggggc cgcttcctgc
tggattgcgg catccggggg actttcagcc ttgtgattca tgctgtccat gaggaggaca
ggggaaagta tacctgtgaa gccaccaatg gcagtggtgc tcgccaggtg acagtggagt
tgacagtaga aggaagtttt gcgaagcagc ttggtcagcc tgttgtttcc aaaaccttag
gggatagatt ttcagcttca gcagtggaga cccgtcctag catctggggg gagtgcccac
 
caaagtttgc taccaagctg ggccgagttg tggtcaaaga aggacagatg ggacgattct
cctgcaagat cactggccgg ccccaaccgc aggtcacctg gctcaaggga aatgttccac
tgcagccgag tgcccgtgtg tctgtgtctg agaagaacgg catgcaggtt ctggaaatcc
atggagtcaa ccaagatgac gtgggagtgt acacgtgcct ggtggtgaac gggtcgggga
aggcctcgat gtcagctgaa ctttccatcc aaggtttgga cagtgccaat aggtcatttg
tgagagaaac aaaagccacc aattcagatg tcaggaaaga ggtgaccaat gtaatctcaa
aggagtcgaa gctggacagt ctggaggctg cagccaaaag caagaactgc tccagccccc
agagaggtgg ctccccaccc tgggctgcaa acagccagcc tcagccccca agggagtcca
agctggagtc atgcaaggac tcgcccagaa cggccccgca gaccccggtc cttcagaaga
cttccagctc catcaccctg caggccgcaa gagttcagcc ggaaccaaga gcaccaggcc
tgggggtcct atcaccttct ggagaagaga ggaagaggcc agctcctccc cgtccagcca
ccttccccac caggcagcct ggcctgggga gccaagatgt tgtgagcaag gctgctaaca
ggagaatccc catggagggc cagagggatt cagcattccc caaatttgag agcaagcccc
aaagccagga ggtcaaggaa aatcaaactg tcaagttcag atgtgaaggg cttgccgtga
tggaggtggc cccctccttc tccagtgtcc tgaaggactg cgctgttatt gagggccagg
attttgtgct gcagtgctcc gtacggggga ccccagtgcc ccggatcact tggctgctga
atgggcagcc catccagtac gctcgctcca cctgcgaggc cggcgtggct gagctccaca
tccaggatgc cctgccggag gaccatggca cctacacctg cctagctgag aatgccttgg
ggcaggtgtc ctgcagcgcc tgggtcaccg tccatgaaaa gaagagtagc aggaagagtg
agtaccttct gcctgtggct cccagcaagc ccactgcacc catcttcctg cagggcctct
ctgatctcaa agtcatggat ggaagccagg tcactatgac tgtccaagtg tcagggaatc
caccccctga agtcatctgg ctgcacaatg ggaatgagat ccaagagtca gaggacttcc
actttgaaca gagaggaact cagcacagcc tttggatcca ggaagtgttc ccggaggaca
cgggcacgta cacctgcgag gcctggaaca gcgctggaga ggtccgcacc caggccgtgc
tcacggtaca agagcctcac gatggcaccc agccctggtt catcagtaag cctcgctcag
tgacagcctc cctgggccag agtgtcctca tctcctgcgc catagctggt gacccctttc
ctaccgtgca ctggctcaga gatggcaaag ccctctgcaa agacactggc cacttcgagg
tgcttcagaa tgaggacgtg ttcaccctgg ttctaaagaa ggtgcagccc tggcatgccg
gccagtatga gatcctgctc aagaaccggg ttggcgaatg cagttgccag gtgtcactga
tgctacagaa cagctctgcc agagcccttc cacgggggag ggagcctgcc agctgcgagg
acctctgtgg tggaggagtt ggtgctgatg gtggtggtag tgaccgctat gggtccctga
ggcctggctg gccagcaaga gggcagggtt ggctagagga ggaagacggc gaggacgtgc
gaggggtgct gaagaggcgc gtggagacga ggcagcacac tgaggaggcg atccgccagc
aggaggtgga gcagctggac ttccgagacc tcctggggaa gaaggtgagt acaaagaccc
tatcggaaga cgacctgaag gagatcccgg ccgagcagat ggatttccgt gccaacctgc
agcggcaagt gaagccaaag actgtgtctg aggaagagag gaaggtgcac agcccccagc
aggtcgattt tcgctctgtc ctggccaaga aggggacttc caagaccccc gtgcctgaga
aggtgccacc gccaaaacct gccaccccgg attttcgctc agtgctgggt ggcaagaaga
aattaccagc agagaatggc agcagcagtg ccgagaccct gaatgccaag gcagtggaga
gttccaagcc cctgagcaat gcacagcctt cagggccctt gaaacccgtg ggcaacgcca
agcctgctga gaccctgaag ccaatggcca acgccaagcc tgccgagacc ctgaagccca
tgggcaatgc caagcctgat gagaacctga aatccgctag caaagaagaa ctcaagaaag
acgttaagaa tgatgtgaac tgcaagagag gccatgcagg gaccacagat aatgaaaaga
gatcagagag ccaggggaca gccccagcct tcaagcagaa gctgcaagat gttcatgtgg
cagagggcaa gaagctgctg ctccagtgcc aggtgtcttc tgacccccca gccaccatca
tctggacgct gaatggaaag accctcaaga ccaccaagtt catcatcctc tcccaggaag
gctcactctg ctccgtctcc atcgagaagg cactgcctga ggacagaggc ttatacaagt
gtgtagccaa gaatgacgct ggccaggcgg agtgctcctg ccaagtcacc gtggatgatg
ctccagccag tgagaacacc aaggccccag agatgaaatc ccggaggccc aagagctctc
ttcctcccgt gctaggaact gagagtgatg cgactgtgaa aaagaaacct gcccccaaga
cacctccgaa ggcagcaatg ccccctcaga tcatccagtt ccctgaggac cagaaggtac
gcgcaggaga gtcagtggag ctgtttggca aagtgacagg cactcagccc atcacctgta
cctggatgaa gttccgaaag cagatccagg aaagcgagca catgaaggtg gagaacagcg
agaatggcag caagctcacc atcctggccg cgcgccagga gcactgcggc tgctacacac
tgctggtgga gaacaagctg ggcagcaggc aggcccaggt caacctcact gtcgtggata
 
agccagaccc cccagctggc acaccttgtg cctctgacat tcggagctcc tcactgaccc
tgtcctggta tggctcctca tatgatgggg gcagtgctgt acagtcctac agcatcgaga
tctgggactc agccaacaag acgtggaagg aactagccac atgccgcagc acctctttca
acgtccagga cctgctgcct gaccacgaat ataagttccg tgtacgtgca atcaacgtgt
atggaaccag tgagccaagc caggagtctg aactcacaac ggtaggagag aaacctgaag
agccgaagga tgaagtggag gtgtcagatg atgatgagaa ggagcccgag gttgattacc
ggacagtgac aatcaatact gaacaaaaag tatctgactt ctacgacatt gaggagagat
taggatctgg gaaatttgga caggtctttc gacttgtaga aaagaaaact cgaaaagtct
gggcagggaa gttcttcaag gcatattcag caaaagagaa agagaatatc cggcaggaga
ttagcatcat gaactgcctc caccacccta agctggtcca gtgtgtggat gcctttgaag
aaaaggccaa catcgtcatg gtcctggaga tcgtgtcagg aggggagctg tttgagcgca
tcattgacga ggactttgag ctgacggagc gtgagtgcat caagtacatg cggcagatct
cggagggagt ggagtacatc cacaagcagg gcatcgtgca cctggacctc aagccggaga
acatcatgtg tgtcaacaag acgggcacca ggatcaagct catcgacttt ggtctggcca
ggaggctgga gaatgcgggg tctctgaagg tcctctttgg caccccagaa tttgtggctc
ctgaagtgat caactatgag cccatcggct acgccacaga catgtggagc atcggggtca
tctgctacat cctagtcagt ggcctttccc ccttcatggg agacaacgat aacgaaacct
tggccaacgt tacctcagcc acctgggact tcgacgacga ggcattcgat gagatctccg
acgatgccaa ggatttcatc agcaatctgc tgaagaaaga tatgaaaaac cgcctggact
gcacgcagtg ccttcagcat ccatggctaa tgaaagatac caagaacatg gaggccaaga
aactctccaa ggaccggatg aagaagtaca tggcaagaag gaaatggcag aaaacgggca
atgctgtgag agccattgga agactgtcct ctatggcaat gatctcaggg ctcagtggca
ggaaatcctc aacagggtca ccaaccagcc cgctcaatgc agaaaaacta gaatctgaag
aagatgtgtc ccaagctttc cttgaggctg ttgctgagga aaagcctcat gtaaaaccct
atttctctaa gaccattcgc gatttagaag ttgtggaggg aagtgctgct agatttgact
gcaagattga aggataccca gaccccgagg ttgtctggtt caaagatgac cagtcaatca
gggagtcccg ccacttccag atagactacg atgaggacgg gaactgctct ttaattatta
gtgatgtttg cggggatgac gatgccaagt acacctgcaa ggctgtcaac agtcttggag
aagccacctg cacagcagag ctcattgtgg aaacgatgga ggaaggtgaa ggggaagggg
aagaggaaga agagtgaaac aaagccagag aaaagcagtt tctaagtcat attaaaagga
ctatttctct caaaatcca
SEQ ID NO. 47
   AAH07433 and P09493
   tropomyosin 1 alpha chain. 
1 mdaikkkmqm lkldkenald raeqaeadkk aaedrskqle delvslqkkl kgtedeldky
61 sealkdaqek lelaekkatd aeadvaslnr riqlveeeld raqerlatal qkleeaekaa
121 desergmkvi esraqkdeek meiqeiqlke akhiaedadr kyeevarklv iiesdlerae
181 eraelsegqv rqleeqlrim dqtlkalmaa edkysqkedr yeeeikvlsd klkeaetrae
241 faersvtkle ksiddledel yaqklkykai seeldhalnd mtsm
SEQ ID NO. 48
   NM_000366 and BC007433
   Homo sapiens tropomyosin 1 (alpha), mRNA (cDNA clone), complete cds 
1 gaggaatgcg gtcgccccct tgggaaagta catatctggg agaagcaggc ggctccgcgc
61 tcgcactccc gctcctccgc ccgaccgcgc gctcgccccg ccgctcctgc tgcagcccca
121 gggcccctcg ccgccgccac catggacgcc atcaagaaga agatgcagat gctgaagctc
181 gacaaggaga acgccttgga tcgagctgag caggcggagg ccgacaagaa ggcggcggaa
241 gacaggagca agcagctgga agatgagctg gtgtcactgc aaaagaaact caaggcacc
301 gaagatgaac tggacaaata ttctgaggct ctcaaagatg cccaggagaa gctggagctg
 
361 gcagagaaaa aggccaccga tgctgaagcc gacgtagctt ctctgaacag acgcatccag
421 ctggttgagg aagagttgga tcgtgcccag gagcgtctgg caacagcttt gcagaagctg
481 gaggaagctg agaaggcagc agatgagagt gagagaggca tgaaagtcat tgagagtcga
541 gcccaaaaag atgaagaaaa aatggaaatt caggagatcc aactgaaaga ggcaaagcac
601 attgctgaag atgccgaccg caaatatgaa gaggtggccc gtaagctggt catcattgag
661 agcgacctgg aacgtgcaga ggagcgggct gagctctcag aaggccaagt ccgacagctg
721 gaagaacaat taagaataat ggatcagacc ttgaaagcat taatggctgc agaggataag
781 tactcgcaga aggaagacag atatgaggaa gagatcaagg tcctttccga caagctgaag
841 gaggctgaga ctcgggctga gtttgcggag aggtcagtaa ctaaattgga gaaaagcatt
901 gatgacttag aagacgagct gtacgctcag aaactgaagt acaaagccat cagcgaggag
961 ctggaccacg ctctcaacga tatgacttcc atgtaaacgt tcatccactc tgcctgctta
1021 caccctgccc tcatgctaat ataagtttct ttgcttcact tctcccaaga ctccctcgtc
1081 gagctggatg tcccacctct ctgagctctg catttgtcta ttctccagct gaccctggtt
1141 ctctctctta gcatcctgcc ttagagccag gcacacactg tgctttctat tgtacagaag
1201 ctcttcgttt cagtgtcaaa taaacactgt gtaagctaaa aaaaaaaaaa aaaaaa
SEQ ID NO. 49
   EITALAPSTMK
SEQ ID NO. 50
   MLTELEK
SEQ ID NO. 51
   ALNSIIDVYHK
SEQ ID NO. 52 .
   GADVWFK

## Claims

1. A method for screening a subject for endometrial cancer comprising (a) detecting in a sample from the subject an amount of an endometrial cancer marker or polynucleotide encoding the endometrial cancer marker; and (b) comparing the amount of endometrial cancer marker or polynucleotide detected to a standard, where detection of a level of endometrial cancer marker or polynucleotide different than that of a standard is indicative of endometrial cancer, and wherein the endometrial cancer marker is chaperonin 10 of SEQ ID NO. 1, a sequence with 80% sequence identity to SEQ ID NO. 1 having the immunogenic activity of SEQ ID NO. 1 or a fragment of SEQ ID NO. 1 having the ability to form antibodies specific for SEQ ID NO. 1.

2. A method of claim 1 wherein the level of endometrial cancer marker is significantly higher compared to the standard and is indicative of endometrial cancer.

3. A method of claim 1 wherein the level of endometrial cancer marker is significantly lower compared to the standard and is indicative of endometrial cancer.

4. A method as claimed in claim 1, 2 or 3 wherein the sample is obtained from tissues, extracts, cell cultures, cell lysates, lavage fluid, or physiological fluids.

5. A method as claimed in claim 4 wherein the sample is obtained from a tumor tissue.

6. A method as claimed in claim 1 wherein the polynucleotide detected is mRNA.

7. A method as claimed in claim 6 wherein the mRNA is detected using an amplification reaction.

8. A method as claimed in claim 7 wherein the amplification reaction is a polymerase chain reaction employing oligonucleotide primers that hybridize to the polynucleotide, or complements of such polynucleotide.

9. A method as claimed in claim 6 wherein the mRNA is detected using a hybridization technique employing oligonucleotide probes that hybridize to the polynucleotide or complements of such polynucleotide.

10. A method as claimed in claim 9 wherein the mRNA is detected by (a) isolating mRNA from the sample and combining the mRNA with reagents to convert it to cDNA; (b) treating the converted cDNA with amplification reaction reagents and primers that hybridize to the polynucleotide, to produce amplification products; (c) analyzing the amplification products to detect an amount of mRNA encoding the endometrial cancer marker; and (d) comparing the amount of mRNA to an amount detected for normal tissue derived using similar primers.

11. A method as claimed in any one of claims 1 to 5 wherein the endometrial cancer marker is detected by contacting the sample with an antibody that specifically binds to the endometrial cancer marker.

12. A method as claimed in claim 11 wherein the antibody is used in a radioimmunoassay, enzyme immunoassay, microarray, competitive immunoassay, non-competitive immunoassay, or immunofluorescence, immunoprecipiation, latex agglutination, hemagglutination, histochemical or bioluminescence assay.

13. A method as claimed in any preceding claim wherein the standard comprises levels of the endometrial cancer marker or polynucleotide for control samples from healthy subjects.

14. A method as claimed in any preceding claim wherein the standard comprises levels of the endometrial cancer marker or polynucleotide for another sample or an earlier sample of the subject.

## Patentansprüche

1. Verfahren zum Durchmustern eines Subjekts auf endometrialen Krebs, welches folgendes umfasst: (a) Nachweisen einer Menge eines endometrialen Krebsmarkers oder für den endometrialen Krebsmarker codierenden Polynukleotids in einer Probe von dem Subjekt und (b) Vergleichen der nachgewiesenen Menge von endometrialem Krebsmarker oder Polynukleotid mit einem Standard, wobei der Nachweis eines Gehalts an endometrialem Krebsmarker oder Polynukleotid, der von einem Standard abweicht, auf endometrialen Krebs hinweist, und wobei der endometriale Krebsmarker Chaperonin 10 aus SEQ ID NO. 1, eine Sequenz mit 80% Sequenzidentität zu SEQ ID NO. 1 mit einer immunogenen Aktivität von SEQ ID NO. 1 oder ein Fragment von SEQ ID NO. 1 mit der Fähigkeit, für SEQ ID NO. 1 spezifische Antikörper zu bilden, ist.

2. Verfahren gemäß Anspruch 1, wobei der Gehalt an endometrialem Krebsmarker signifikant höher ist im Vergleich zu dem Standard und auf endometrialen Krebs hinweist.

3. Verfahren gemäß Anspruch 1, wobei der Gehalt an endometrialem Krebsmarker signifikant niedriger ist im Vergleich zu dem Standard und auf endometrialen Krebs hinweist.

4. Verfahren gemäß Anspruch 1, 2 oder 3, wobei die Probe aus Geweben, Extrakten, Zellkulturen, Zelllysaten, Spülflüssigkeiten oder physiologischen Flüssigkeiten erhalten wird.

5. Verfahren gemäß Anspruch 4, wobei die Probe aus einem Tumorgewebe erhalten wird.

6. Verfahren gemäß Anspruch 1, wobei das nachgewiesene Nukleotid mRNA ist.

7. Verfahren gemäß Anspruch 6, wobei die mRNA unter Verwendung einer Amplifizierungsreaktion nachgewiesen wird.

8. Verfahren gemäß Anspruch 7, wobei die Amplifizierungsreaktion eine Polymerasekettenreaktion ist, unter Verwendung von Oligonukleotidprimern, die an das Polynukleotid hybridisieren, oder von Komplementen solcher Polynukleotide.

9. Verfahren gemäß Anspruch 6, wobei die mRNA unter Verwendung einer Hybridisierungstechnik nachgewiesen wird, unter Verwendung von Oligonukleotidsonden, die an das Polynukleotid hybridisieren, oder von Komplementen solcher Polynukleotide.

10. Verfahren gemäß Anspruch 9, wobei die mRNA nachgewiesen wird durch (a) Isolieren von mRNA aus der Probe und Zusammenbringen der mRNA mit Reagenzien, um sie in cDNA umzuwandeln, (b) Behandeln der umgewandelten cDNA mit Amplifizierungsreaktionsreagenzien und Primern, die an das Polynukleotid hybridisieren, um Amplifizierungsprodukte zu erhalten, (c) Analysieren der Amplifizierungsprodukte, um eine Menge an mRNA nachzuweisen, die den endometrialen Krebsmarker codiert, und (d) Vergleichen der Menge an mRNA mit einer Menge, die bei normalem Gewebe nachgewiesen wird und die unter Verwendung gleicher Primer erhalten wird.

11. Verfahren gemäß einem der Ansprüche 1 bis 5, wobei der endometriale Krebsmarker durch Inkontaktbringen der Probe mit einem Antikörper, der spezifisch an den endometrialen Krebsmarker bindet, nachgewiesen wird.

12. Verfahren gemäß Anspruch 11, wobei der Antikörper in einem Radioimmuntest, Enzymimmuntest, Mikroarray, kompetitiven Immuntest, nicht-kompetitiven Immuntest oder Immunfluoreszenz, Immunpräzipitation, Latexagglutination, Hämagglutination, histochemischen oder Biolumineszenztest verwendet wird.

13. Verfahren gemäß einem der vorangehenden Ansprüche, wobei der Standard Gehalte des endometrialen Krebsmarkers oder Polynukleotid zu Kontrollproben aus gesunden Subjekten enthält.

14. Verfahren gemäß einem der vorangehenden Ansprüche, wobei der Standard Gehalte des endometrialen Krebsmarkers oder Polynukleotid zu einer anderen Probe oder einer vorangegangenen Probe des Subjekts enthält.

## Revendications

1. Procédé de dépistage d'un cancer endométrial chez un sujet comprenant (a) la détection dans un échantillon provenant du sujet d'une quantité d'un marqueur de cancer endométrial ou d'un polynucléotide codant pour le marqueur de cancer endométrial ; et (b) la comparaison de la quantité de marqueur de cancer endométrial ou de polynucléotide détectée à un étalon, où la détection d'un taux de marqueur de cancer endométrial ou de polynucléotide différent de celui d'un étalon indique un cancer endométrial, et où le marqueur de cancer endométrial est la chaperonine 10 de SEQ ID NO : 1, une séquence présentant 80 % d'identité de séquence avec SEQ ID NO : 1 ayant l'activité immunogène de SEQ ID NO : 1 ou un fragment de SEQ ID NO : 1 ayant la capacité de former des anticorps spécifiques de SEQ ID NO : 1.

2. Procédé selon la revendication 1, dans lequel le taux de marqueur de cancer endométrial est significativement supérieur comparativement à l'étalon et indique un cancer endométrial.

3. Procédé selon la revendication 1, dans lequel le taux de marqueur de cancer endométrial est significativement inférieur comparativement à l'étalon et indique un cancer endométrial.

4. Procédé selon la revendication 1, 2 ou 3, dans lequel l'échantillon est obtenu à partir de tissus, d'extraits, de cultures cellulaires, de lysats cellulaires, de liquide de lavage ou de liquides physiologiques.

5. Procédé selon la revendication 4, dans lequel l'échantillon est obtenu à partir d'un tissu tumoral.

6. Procédé selon la revendication 1, dans lequel le polynucléotide détecté est de l'ARNm.

7. Procédé selon la revendication 6, dans lequel l'ARNm est détecté en utilisant une réaction d'amplification.

8. Procédé selon la revendication 7, dans lequel la réaction d'amplification est une réaction en chaîne de la polymérase employant des amorces oligonucléotidiques qui s'hybrident au polynucléotide, ou à des complémentaires d'un tel polynucléotide.

9. Procédé selon la revendication 6, dans lequel l'ARNm est détecté en utilisant une technique d'hybridation employant des sondes oligonucléotidiques qui s'hybrident au polynucléotide, ou à des complémentaires d'un tel polynucléotide.

10. Procédé selon la revendication 9, dans lequel l'ARNm est détecté par (a) isolement de l'ARNm à partir de l'échantillon et combinaison de l'ARNm avec des réactifs pour le convertir en ADNc ; (b) traitement de l'ADNc converti avec des réactifs de réaction d'amplification et des amorces qui s'hybrident au polynucléotide, pour produire des produits d'amplification ; (c) analyse des produits d'amplification pour détecter une quantité d'ARNm codant pour le marqueur de cancer endométrial ; et (d) comparaison de la quantité d'ARNm à une quantité détectée pour du tissu normal dérivé en utilisant des amorces similaires.

11. Procédé selon l'une quelconque des revendications 1 à 5, dans lequel le marqueur de cancer endométrial est détecté par la mise en contact de l'échantillon avec un anticorps qui se lie spécifiquement au marqueur de cancer endométrial.

12. Procédé selon la revendication 11, dans lequel l'anticorps est utilisé dans un test radio-immunologique, un test immuno-enzymatique, une micropuce, un test immunologique compétitif, un test immunologique non compétitif ou un test d'immunofluorescence, d'immunoprécipitation, d'agglutination au latex, d'hémagglutination, histochimique ou de bioluminescence.

13. Procédé selon l'une quelconque des revendications précédentes, où l'étalon comprend des taux de marqueur de cancer endométrial ou de polynucléotide pour les échantillons contrôles provenant de sujets en bonne santé.

14. Procédé selon l'une quelconque des revendications précédentes, où l'étalon comprend des taux de marqueur de cancer endométrial ou de polynucléotide pour un autre échantillon ou un échantillon antérieur du sujet.
